# EUROPEAN PATENT APPLICATION

(11) **EP 4 835 088 A1**
(43) Date of publication of application: **04.11.2026**
(21) Application number: 24913118.6
(22) Date of filing: 27.12.2024
(51) Int. Cl.: C12N 15/87, A61K 9/127, A61K 31/7088, A61K 31/7105, A61K 47/14, A61K 47/18, A61K 47/24, A61K 47/28, A61K 48/00, A61P 37/04, A61P 43/00

(54) **AGENT FOR DELIVERING NUCLEIC ACID TO IMMUNE CELLS AND METHOD FOR DELIVERING NUCLEIC ACID TO IMMUNE CELLS**

(30) Priority: 28.12.2023 JP 2023222204; 10.07.2024 JP 2024110674
(71) Applicant: FUJIFILM Corporation, Tokyo 106-8620 (JP)
(72) Inventor: NITABARU, Tatsuya, Ashigarakami-gun, Kanagawa 258-8577 (JP); NAKAMURA, Naoto, Ashigarakami-gun, Kanagawa 258-8577 (JP); UMETANI, Sayako, Ashigarakami-gun, Kanagawa 258-8577 (JP); FUKUNAGA, Hirofumi, Ashigarakami-gun, Kanagawa 258-8577 (JP)
(74) Representative: Klunker IP Patentanwälte PartG mbB
(86) International application number: PCT/JP2024/046426
(87) International publication number: WO 2025/143232

(57) **Abstract**

An object of the present invention is to provide a nucleic acid delivery agent for an immune cell, the agent being capable of delivering nucleic acid to both an immune cell subjected to activation treatment and an immune cell not subjected to activation treatment, and a method for delivering nucleic acid to an immune cell using the nucleic acid delivery agent for an immune cell. According to the present invention, there is provided a nucleic acid delivery agent for an immune cell, that contains a lipid composition containing an ionizable lipid that is a compound represented by Formula (1) or a salt thereof, a non-ionizable lipid, a lipid having a nonionic polymer, and a nucleic acid.

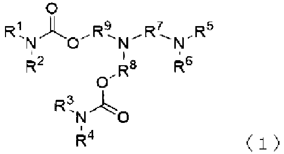

In the formula, the meanings of R¹ to R⁹ are as described in the present specification.

## Description

### Technical Field

The present invention relates to a nucleic acid delivery agent to an immune cell, containing a lipid. The present invention further relates to a method for delivering nucleic acid to an immune cell, using the nucleic acid delivery agent.

### Background Art

In immune cell therapy including chimeric antigen receptor (CAR) T cell therapy, immune cells that are genetically modified to express a therapeutic exogenous gene such as CAR or to modulate the expression of an endogenous gene are used. In the genetic modification of immune cells, a virus vector method is generally used, but there are problems such as safety concerns due to viruses and high cost. In addition, as a non-viral method, an electroporation method has been used in the related art, but there is a problem that cytotoxicity and DNA damage due to electroporation occur, which leads to proliferation delay and chromosomal abnormalities.

Regarding nucleic acid delivery to immune cells using lipid nanoparticles (LNP), Patent Document 1 describes that Cas9 mRNA and gRNA are encapsulated in separate LNP to perform genome editing at a plurality of sites, and used for genome editing of a T cell. In addition, Patent Document 2 describes that nucleic acid (mRNA) is delivered to a T cell using an LNP encapsulating the nucleic acid.

### Prior Art Documents

### Patent Documents

Patent Document 1: WO2021/222287A
Patent Document 2: WO2020/210901A

### Summary of Invention

### Object to be solved by the invention

In the lipid nanoparticles described in Patent Documents 1 and 2, a treatment of sufficiently activating an immune cell is required, and there is a limitation on the culturing method that can be used. Since it is known that the activation treatment of the immune cell reduces the efficacy of the treatment of the immune cell, it is desirable not to perform the activation treatment.

In view of such circumstances, an object of the present invention is to provide a nucleic acid delivery agent for an immune cell, the agent being capable of delivering nucleic acid to both an immune cell subjected to activation treatment and an immune cell not subjected to activation treatment. Another object of the present invention is to provide a method for delivering nucleic acid to an immune cell using the nucleic acid delivery agent for an immune cell.

### Means for solving the object

As a result of intensive studies to achieve the above-described object, the present inventors have found that a lipid composition containing an ionizable lipid that is a compound represented by Formula (1) or a salt thereof, a non-ionizable lipid, a lipid having a nonionic polymer, and nucleic acid is capable of delivering the nucleic acid to both an immune cell subjected to activation treatment and an immune cell not subjected to activation treatment, thereby completing the present invention. According to an aspect of the present invention, the following inventions are provided.
<1> A nucleic acid delivery agent for an immune cell, comprising:
   a lipid composition containing an ionizable lipid that is a compound represented by Formula (1) or a salt thereof, a non-ionizable lipid, a lipid having a nonionic polymer, and a nucleic acid, 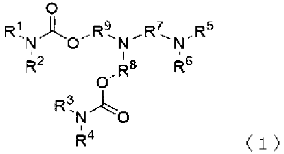
   in the formula, R¹, R², R³, and R⁴ each independently represent a hydrogen atom or a hydrocarbon group having 1 to 24 carbon atoms which may be substituted,
   substituents on the hydrocarbon groups having 1 to 24 carbon atoms which may be substituted, the hydrocarbon groups being represented by R¹, R², R³, and R⁴, each independently represent -C(O)O-R¹¹, -OC(O)-R¹², -O-R¹³, -CO-R¹⁴, -OC(O)O-R¹⁵, or -S-S-R¹⁶,
   R¹¹, R¹², R¹³, R¹⁴, R¹⁵, and R¹⁶ each independently represent a hydrocarbon group having 1 to 24 carbon atoms which may be substituted with -S-R¹⁷, where R¹⁷ represents a hydrocarbon group having 1 to 12 carbon atoms,
   R⁵ and R⁶ each independently represent a hydrocarbon group having 1 to 18 carbon atoms which may be substituted,
   substituents on the hydrocarbon groups having 1 to 18 carbon atoms which may be substituted, the hydrocarbon groups being represented by R⁵ and R⁶, each independently represent -OH, -COOH, -NR²¹R²², -OC(O)O-R²³, -C(O)O-R²⁴, -OC(O)-R²⁵, -O-R²⁶, -C(O)NR²⁷R²⁸, -NR²⁹C(O)R³⁰, -N(R³¹)S(O)₂R³², -N(R³³)C(O)N(R³⁴)R³⁵, -N(R³⁶)C(S)N(R³⁷)R³⁸, -OC(O)N(R³⁹)R⁴⁰, or -N(R⁴¹)C(O)OR⁴²,
   R²¹ and R²² each independently represent a hydrogen atom or a hydrocarbon group having 1 to 8 carbon atoms,
   R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, and R⁴² each independently represent a hydrogen atom or a hydrocarbon group having 1 to 24 carbon atoms which may be substituted, and substituents on the hydrocarbon groups having 1 to 24 carbon atoms which may be substituted, the hydrocarbon group being represented by R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, and R⁴², represent an aryl group having 6 to 20 carbon atoms, a heterocyclic group, -OH, -COOH, or -NR⁵¹R⁵², where R⁵¹ and R⁵² each independently represent a hydrogen atom or a hydrocarbon group having 1 to 8 carbon atoms,
   R⁷, R⁸, and R⁹ each independently represent a hydrocarbon group having 2 to 8 carbon atoms, and
   R⁵ and R⁶, or R⁵ and R⁷ may be taken together to form a 4- to 7-membered ring.
<2> The nucleic acid delivery agent for an immune cell according to <1>, in which a molar ratio of the ionizable lipid to total lipids in the lipid composition is 20 to 60 mol%.
<3> The nucleic acid delivery agent for an immune cell according to <1> or <2>, in which the non-ionizable lipid contains a sterol or a derivative thereof, and a phospholipid.
<4> The nucleic acid delivery agent for an immune cell according to <3>, in which the phospholipid is selected from the group consisting of distearoylphosphatidylcholine, dioleoylphosphatidylcholine, and dioleoylphosphatidylethanolamine.
<5> The nucleic acid delivery agent for an immune cell according to <3> or <4>, in which a molar ratio of the sterol or the derivative thereof to total lipids in the lipid composition is 30 to 70 mol%.
<6> The nucleic acid delivery agent for an immune cell according to any one of <3> to <5>, in which a molar ratio of the phospholipid to total lipids in the lipid composition is 1 to 30 mol%.
<7> The nucleic acid delivery agent for an immune cell according to any one of <1> to <6>, in which the lipid having the nonionic polymer is a lipid having a polyethylene glycol chain.
<8> The nucleic acid delivery agent for an immune cell according to <7>, in which the lipid having the polyethylene glycol chain is selected from dimyristoyl-rac-glycerol polyethylene glycol, distearoyl-rac-glycerol polyethylene glycol, and distearoylphosphatidylethanolamine polyethylene glycol.
<9> The nucleic acid delivery agent for an immune cell according to any one of <1> to <8>, in which a molar ratio of the lipid having the nonionic polymer to total lipids in the lipid composition is 0.1 to 3 mol%.
<10> The nucleic acid delivery agent for an immune cell according to any one of <1> to <9>, in which a mass ratio of total lipids in the lipid composition to the nucleic acid is 7:1 to 1000:1.
<11> The nucleic acid delivery agent for an immune cell according to any one of <1> to <10>, in which the immune cell is an activated cell or a non-activated cell.
<12> The nucleic acid delivery agent for an immune cell according to any one of <1> to <11>, in which the ionizable lipid is one or more of the following compounds. 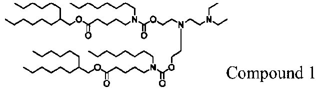 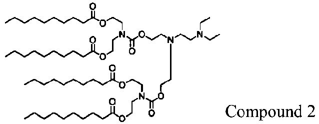 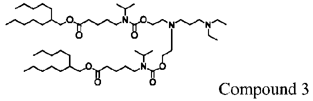 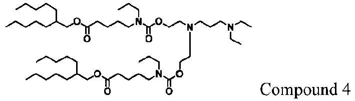 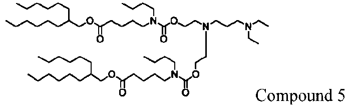 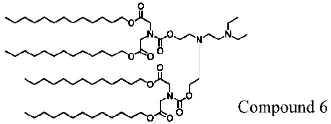 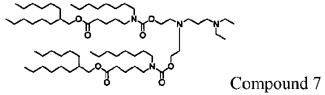 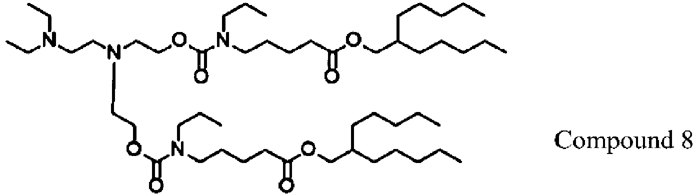 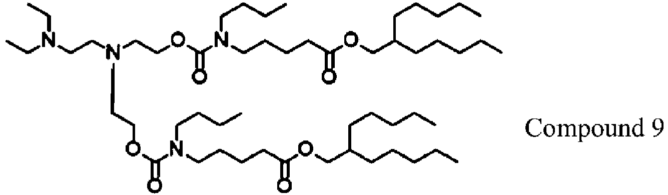 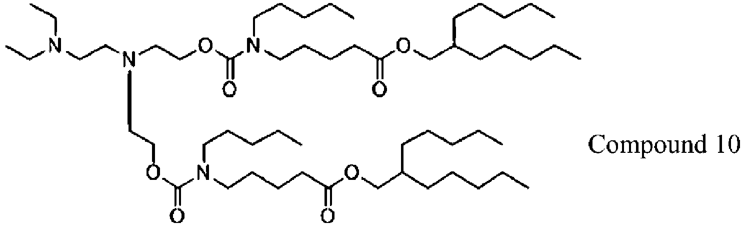 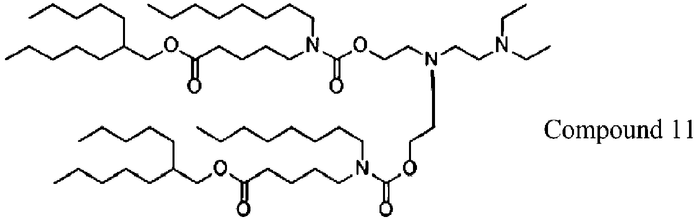 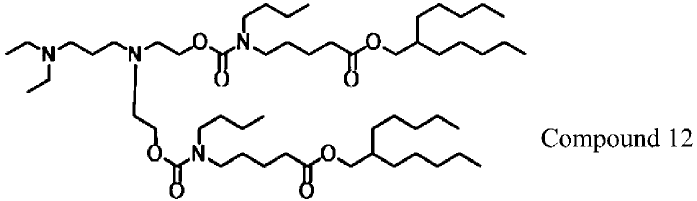 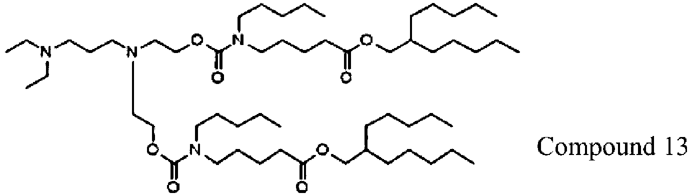 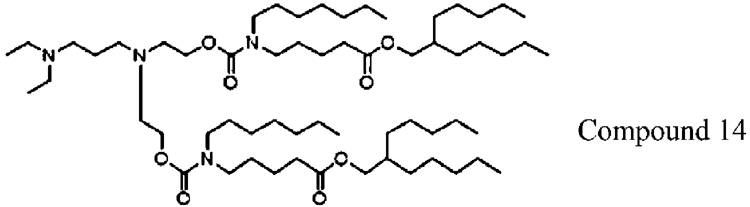 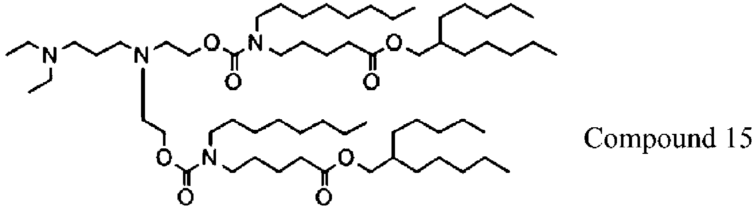 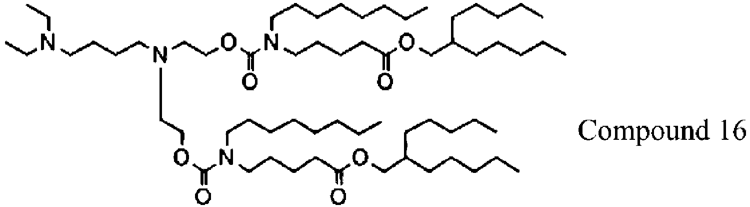 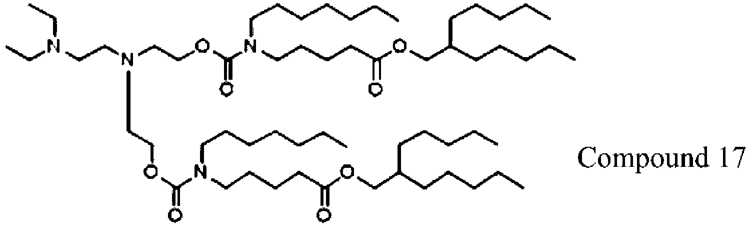 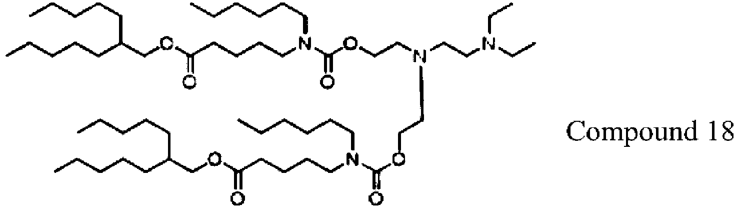 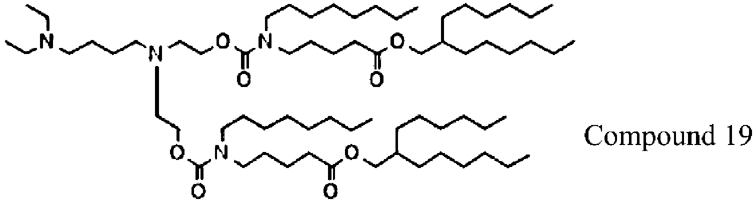 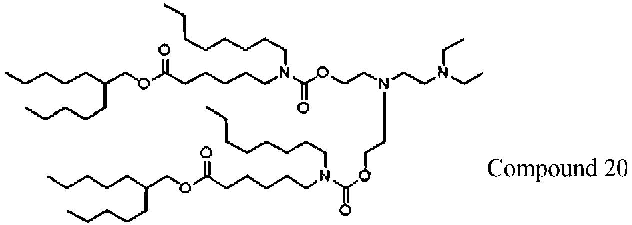 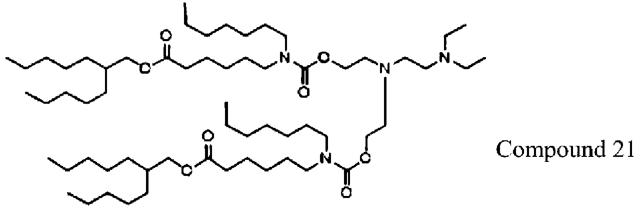 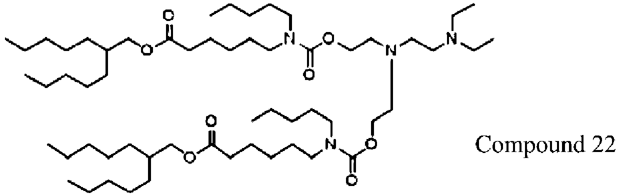 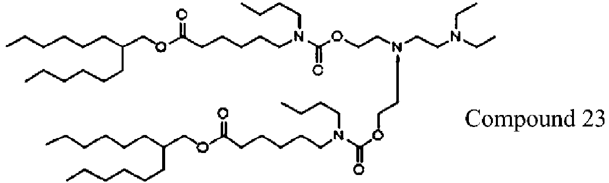 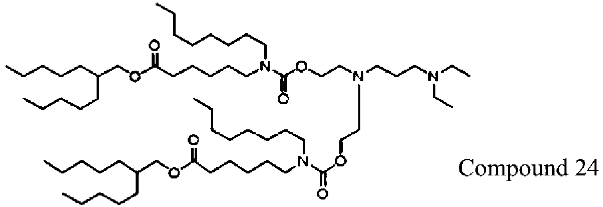 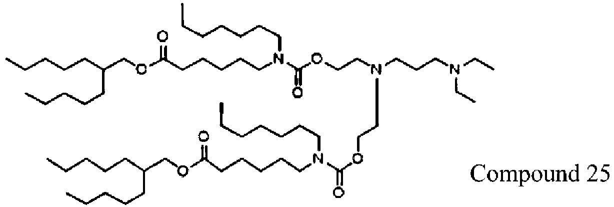 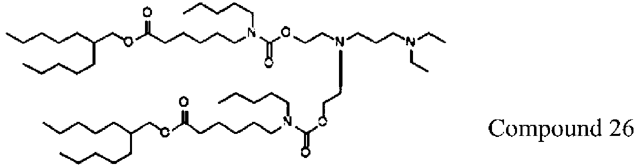 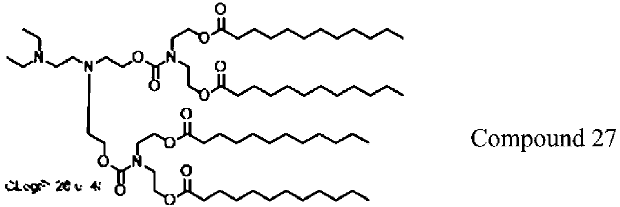 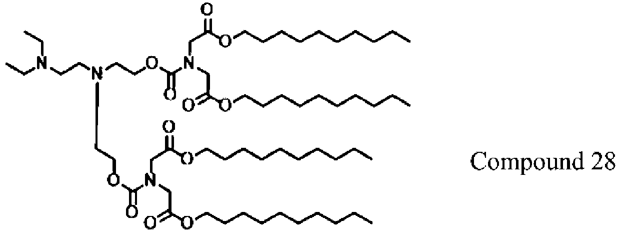 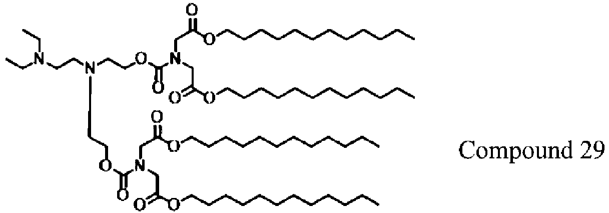 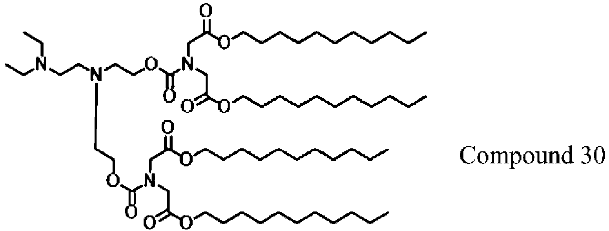 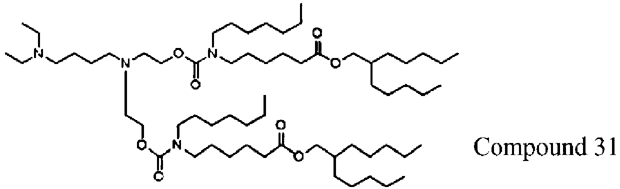 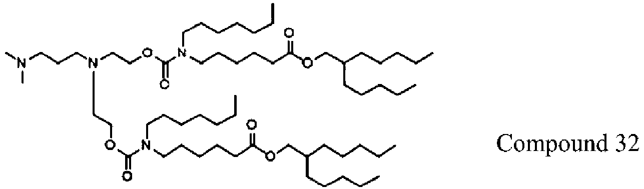 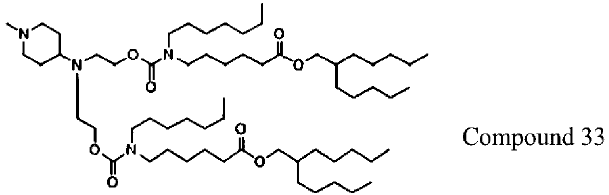 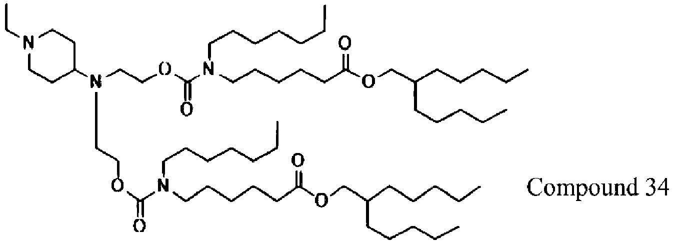 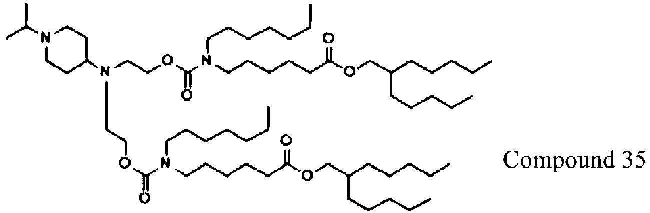 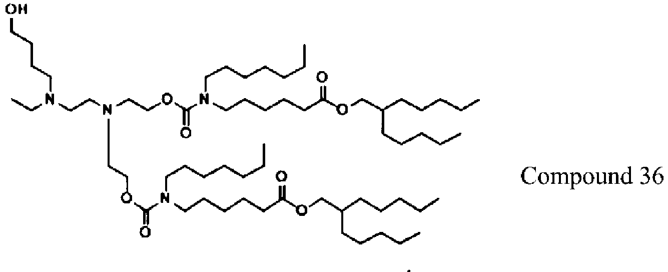 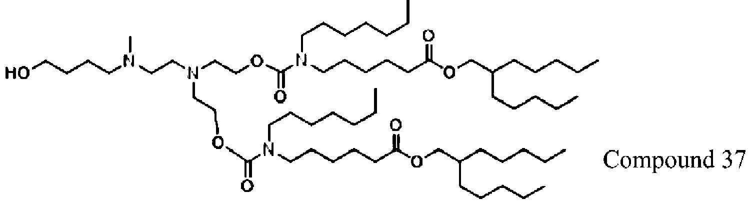 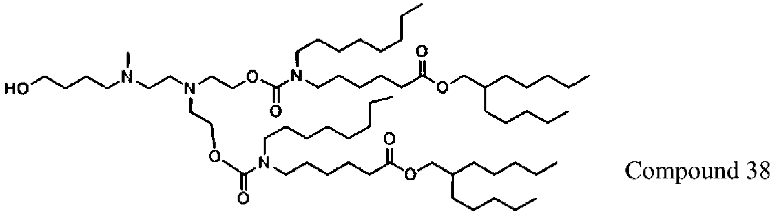 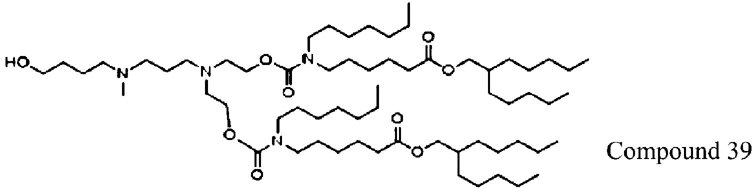 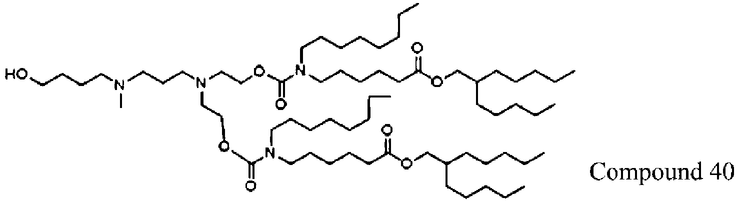 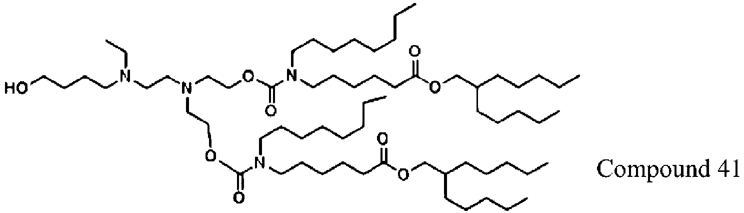 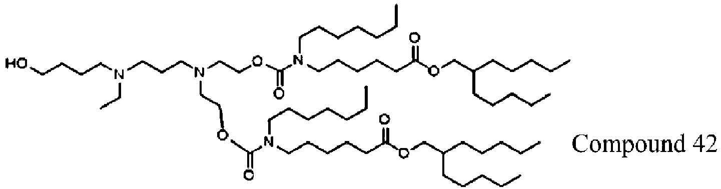 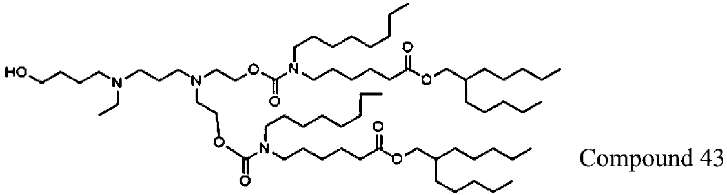 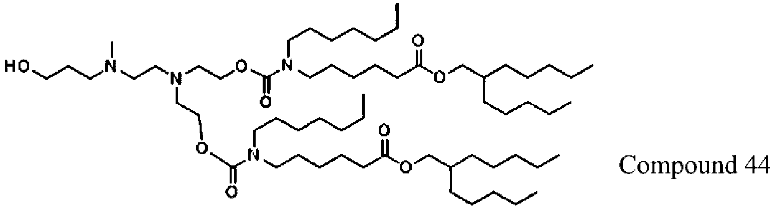 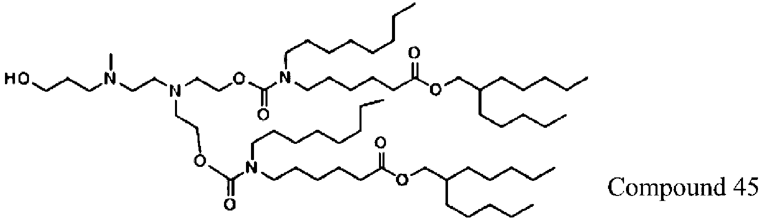 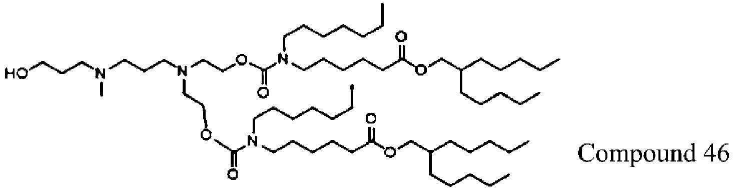 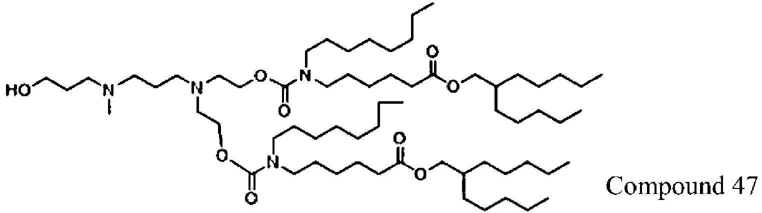 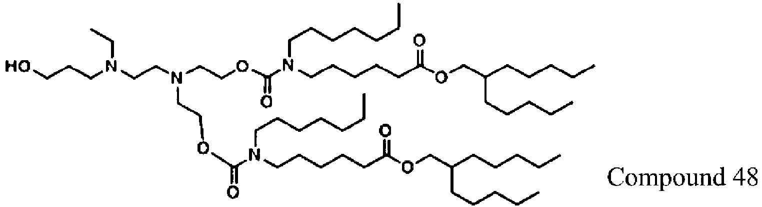 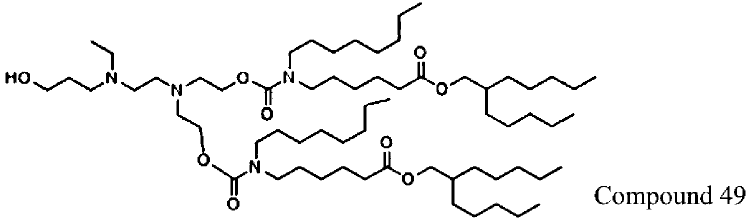 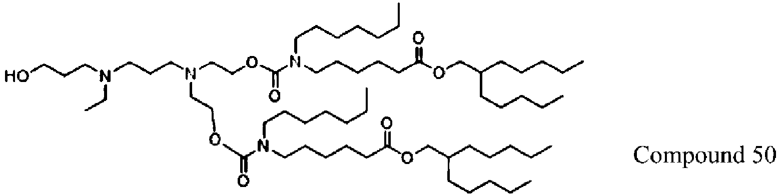 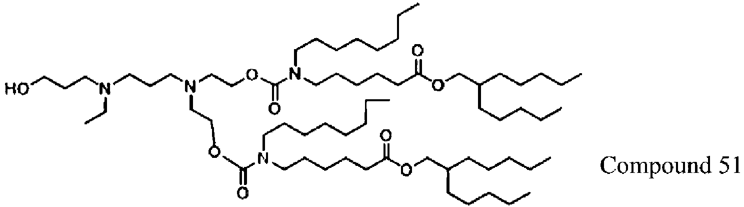 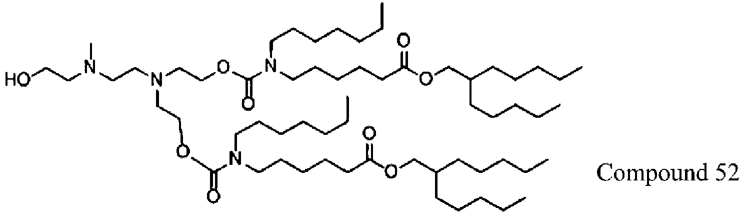 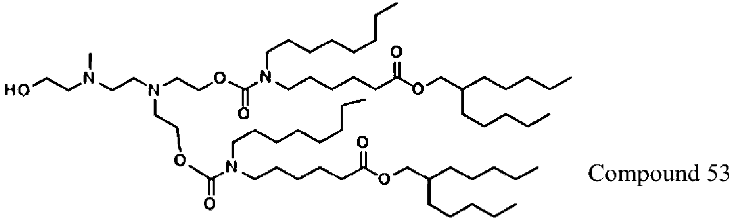 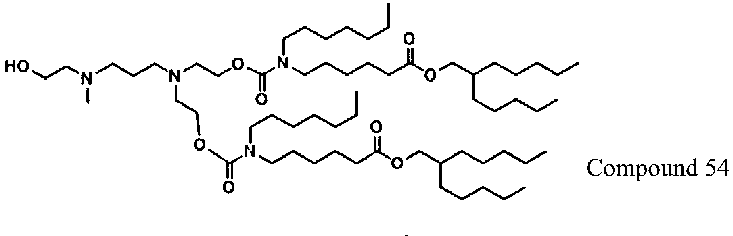 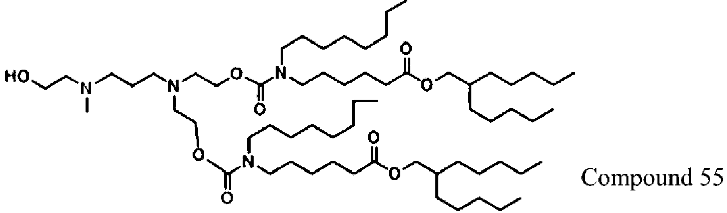 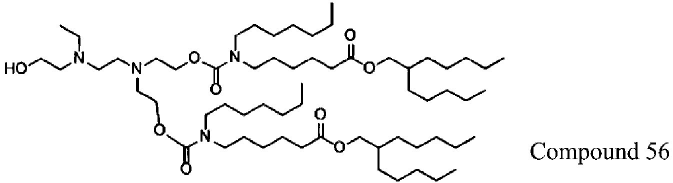 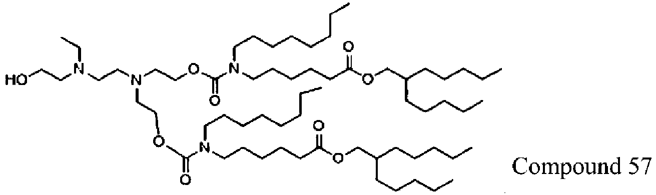 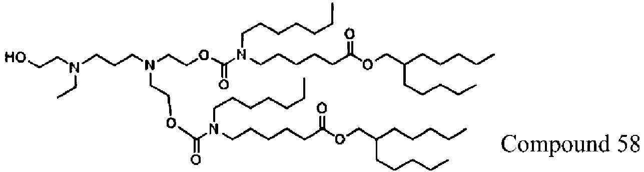 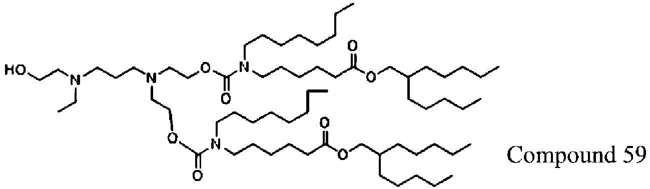 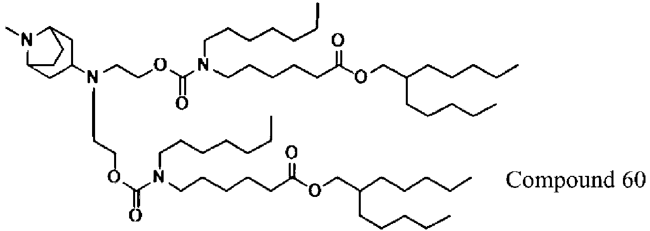 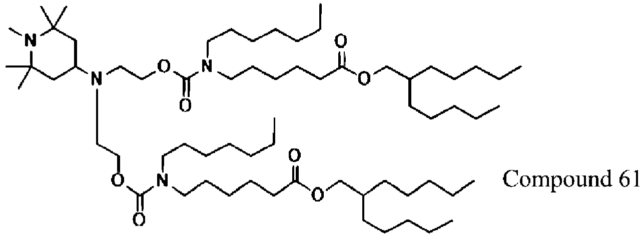 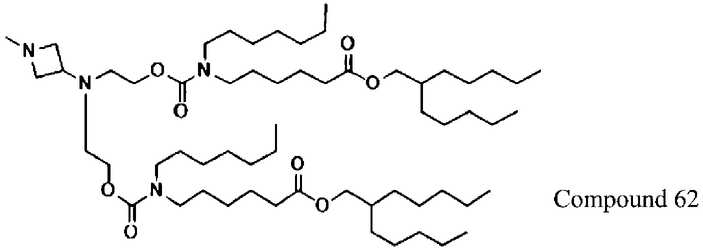 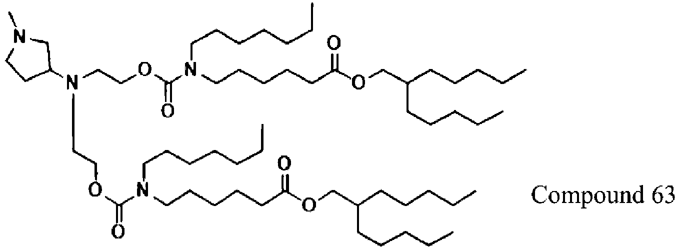 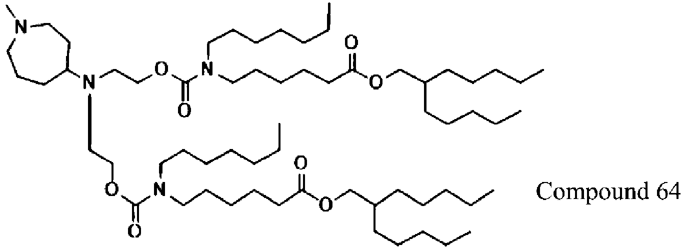 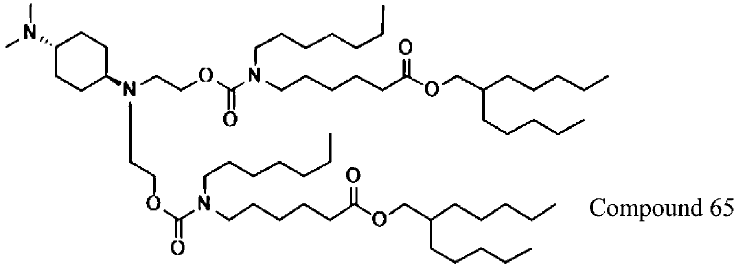 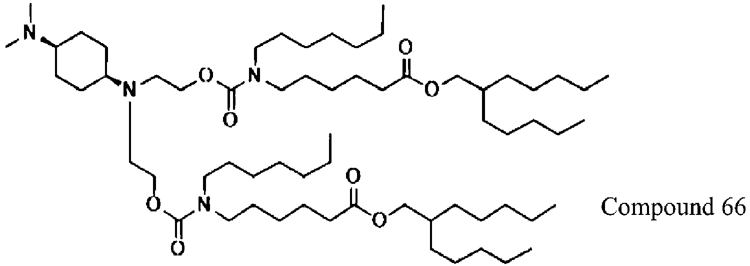 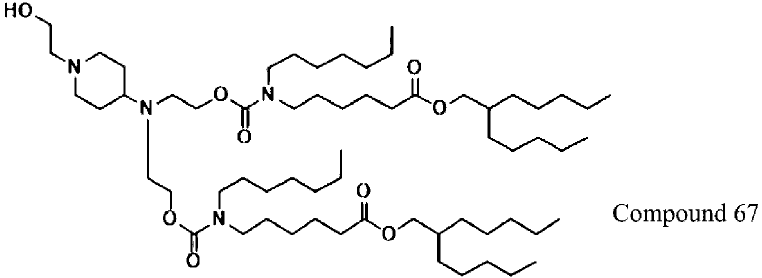 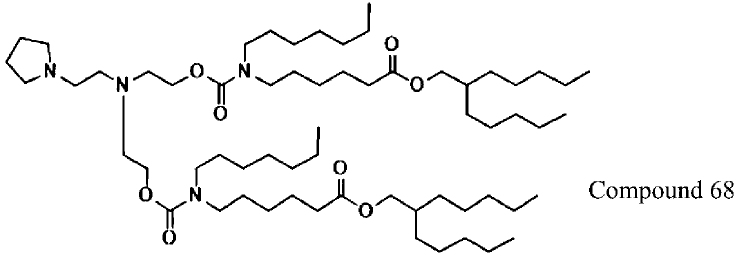 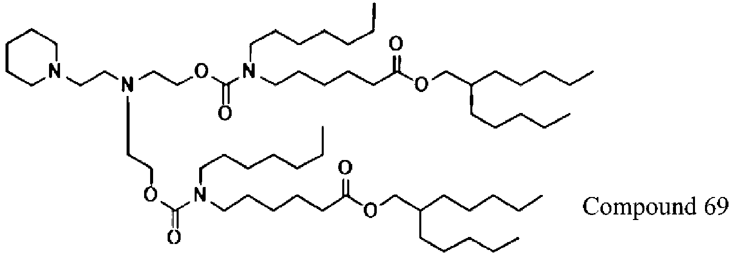 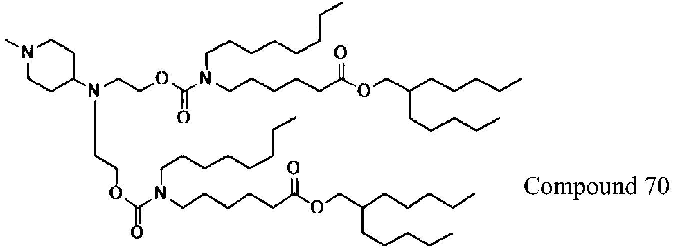 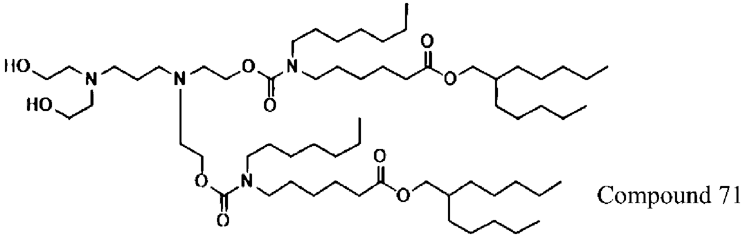 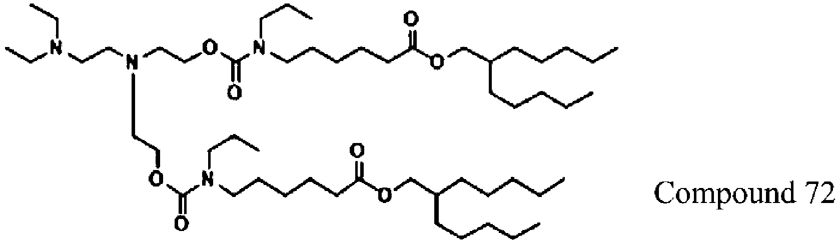 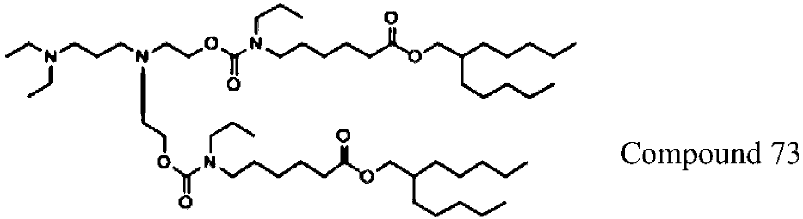 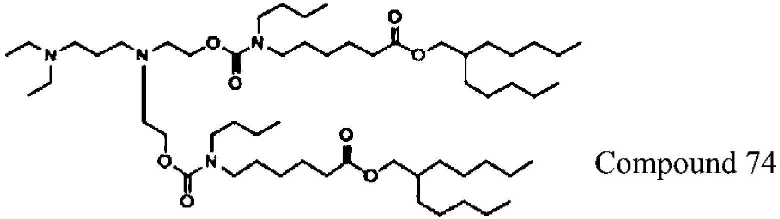
<13> A method for delivering nucleic acid to an immune cell (excluding an in vivo delivery method), the method comprising:
   bringing the nucleic acid delivery agent for an immune cell according to any one of <1> to <12> into contact with the immune cell.
<14> The method according to <13>, in which the immune cell is an activated cell or a non-activated cell.
<15> The method according to <13>, comprising:
   a step of adding, before bringing the nucleic acid delivery agent into contact with the immune cell, (i) an apolipoprotein and/or (ii) a protein including a cell-binding domain and a heparin-binding domain to the nucleic acid delivery agent or the immune cell.
<16> A method for producing the nucleic acid delivery agent according to any one of <1> to <12>, the method comprising:
   a step of preparing lipid particles not containing nucleic acid using an ionizable lipid that is a compound represented by Formula (1), a non-ionizable lipid, and a lipid having a nonionic polymer; and
   a step of mixing the lipid particles not containing nucleic acid with the nucleic acid, 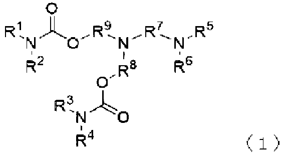
   in the formula, R¹, R², R³, and R⁴ each independently represent a hydrogen atom or a hydrocarbon group having 1 to 24 carbon atoms which may be substituted,
   substituents on the hydrocarbon groups having 1 to 24 carbon atoms which may be substituted, the hydrocarbon groups being represented by R¹, R², R³, and R⁴, each independently represent -C(O)O-R¹¹, -OC(O)-R¹², -O-R¹³, -CO-R¹⁴, -OC(O)O-R¹⁵, or -S-S-R¹⁶,
   R¹¹, R¹², R¹³, R¹⁴, R¹⁵, and R¹⁶ each independently represent a hydrocarbon group having 1 to 24 carbon atoms which may be substituted with -S-R¹⁷, where R¹⁷ represents a hydrocarbon group having 1 to 12 carbon atoms,
   R⁵ and R⁶ each independently represent a hydrocarbon group having 1 to 18 carbon atoms which may be substituted,
   substituents on the hydrocarbon groups having 1 to 18 carbon atoms which may be substituted, the hydrocarbon groups being represented by R⁵ and R⁶, each independently represent -OH, -COOH, -NR²¹R²², -OC(O)O-R²³, -C(O)O-R²⁴, -OC(O)-R²⁵, -O-R²⁶, -C(O)NR²⁷R²⁸, -NR²⁹C(O)R³⁰, -N(R³¹)S(O)₂R³², -N(R³³)C(O)N(R³⁴)R³⁵, -N(R³⁶)C(S)N(R³⁷)R³⁸, -OC(O)N(R³⁹)R⁴⁰, or -N(R⁴¹)C(O)OR⁴²,
   R²¹ and R²² each independently represent a hydrogen atom or a hydrocarbon group having 1 to 8 carbon atoms,
   R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, and R⁴² each independently represent a hydrogen atom or a hydrocarbon group having 1 to 24 carbon atoms which may be substituted, and substituents on the hydrocarbon groups having 1 to 24 carbon atoms which may be substituted, the hydrocarbon group being represented by R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, and R⁴², represent an aryl group having 6 to 20 carbon atoms, a heterocyclic group, -OH, -COOH, or -NR⁵¹R⁵², where R⁵¹ and R⁵² each independently represent a hydrogen atom or a hydrocarbon group having 1 to 8 carbon atoms,
   R⁷, R⁸, and R⁹ each independently represent a hydrocarbon group having 2 to 8 carbon atoms, and
   R⁵ and R⁶, or R⁵ and R⁷ may be taken together to form a 4- to 7-membered ring.
<17> At least one compound selected from the group consisting of the following compounds, or a salt thereof, 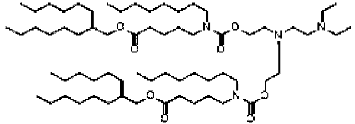
   bis(2-hexyloctyl) 11-(2-(diethylamino)ethyl)-6,16-dioctyl-7,15-dioxo-8,14-dioxa-6,11,16-triazahenicosanedioat e, 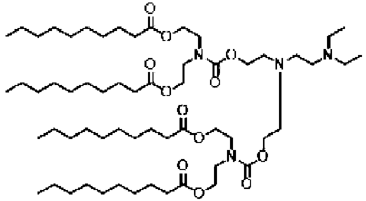
   2-(2-(2-(bis(2-decanoyloxyethyl)carbamoyloxy)ethyl-(2-(diethylamino)ethyl)amino)e thoxycarbonyl-(2-decanoyloxyethyl)amino)ethyl decanoate, 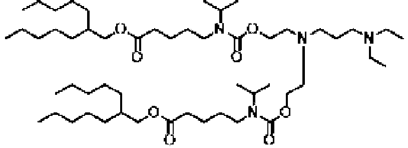
   bis(2-pentylheptyl) 11-(3-(diethylamino)propyl)-6,16-diisopropyl-7,15-dioxo-8,14-dioxa-6,11,16-triazahenicosane dioate, 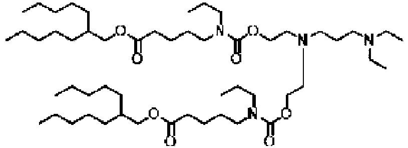
   bis(2-pentylheptyl) 11-(3-(diethylamino)propyl)-7,15-dioxo-6,16-dipropyl-8,14-dioxa-6,11,16-triazahenicosanedi oate, 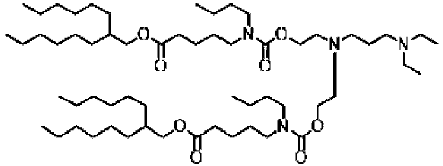
   bis(2-hexyloctyl) 6,16-dibutyl-11-(3-(diethylamino)propyl)-7,15-dioxo-8,14-dioxa-6,11,16-triazahenicosanedioa te, 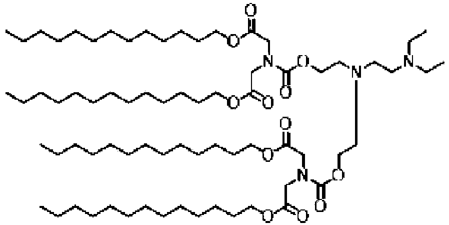
   ditridecyl 8-(2-(diethylamino)ethyl)-4,12-dioxo-3,13-bis(2-oxo-2-(tridecyloxy)ethyl)-5,11-dioxa-3,8,13-t riazapentadecanedioate, 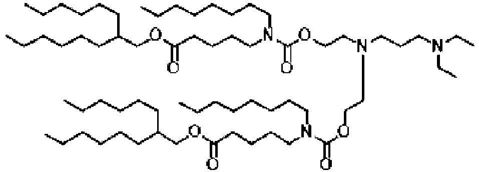
   bis(2-hexyloctyl) 11-(3-(diethylamino)propyl)-6,16-dioctyl-7,15-dioxo-8,14-dioxa-6,11,16-triazahenicosanedioa te, 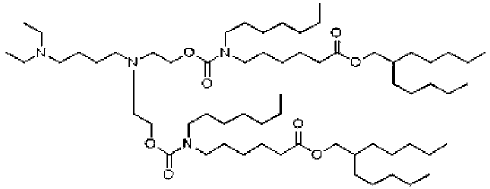
   bis(2-pentylheptyl) 12-(4-(diethylamino)butyl)-7,17-diheptyl-8,16-dioxo-9,15-dioxa-7,12,17-triazatricosanedioate, 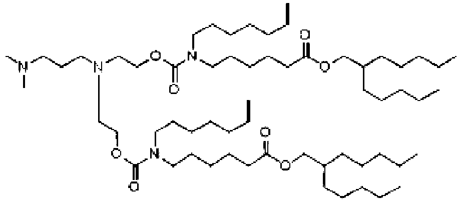
   bis(2-pentylheptyl) 12-(3-(dimethylamino)propyl)-7,17-diheptyl-8,16-dioxo-9,15-dioxa-7,12,17-triazatricosanedio ate, 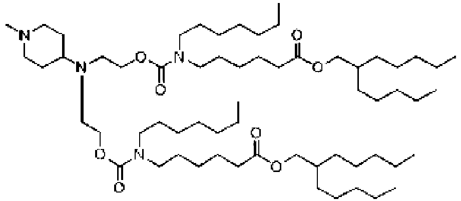
   bis(2-pentylheptyl) 7,17-diheptyl-12-(1-methylpiperidin-4-yl)-8,16-dioxo-9,15-dioxa-7,12,17-triazatricosanedioat e, 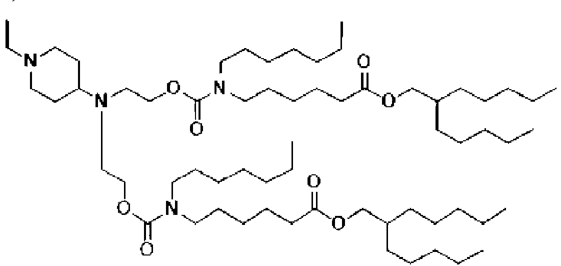
   bis(2-pentylheptyl) 12-(1-ethylpiperidin-4-yl)-7,17-diheptyl-8,16-dioxo-9,15-dioxa-7,12,17-triazatricosanedioate, 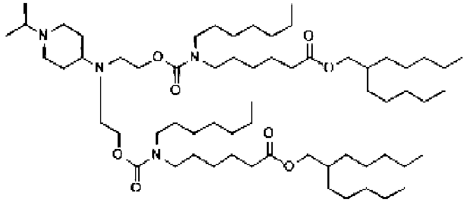
   bis(2-pentylheptyl) 7,17-diheptyl-12-(1-isopropylpiperidin-4-yl)-8,16-dioxo-9,15-dioxa-7,12,17-triazatricosanedio ate, 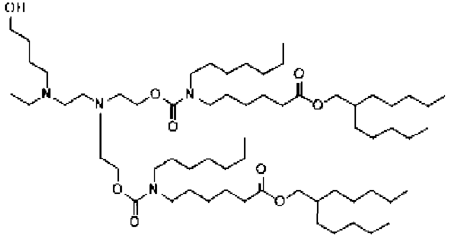
   bis(2-pentylheptyl) 12-(2-(ethyl(4-hydroxybutyl)amino)ethyl)-7,17-diheptyl-8,16-dioxo-9,15-dioxa-7,12,17-triaza tricosanedioate, 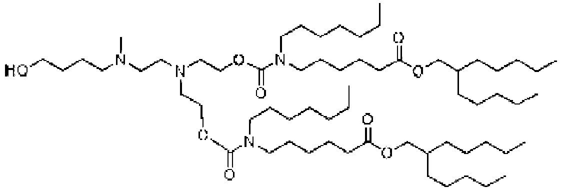
   bis(2-pentylheptyl) 7,17-diheptyl-12-(2-((4-hydroxybutyl)(methyl)amino)ethyl)-8,16-dioxo-9,15-dioxa-7,12,17-tri azatricosanedioate, 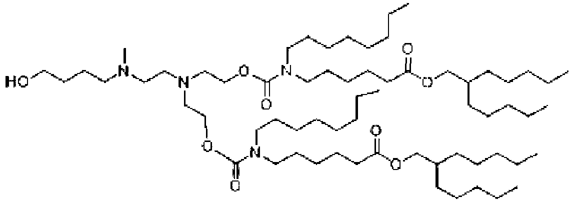
   bis(2-pentylheptyl) 12-(2-((4-hydroxybutyl)(methyl)amino)ethyl)-7,17-dioctyl-8,16-dioxo-9,15-dioxa-7,12,17-tria zatricosanedioate, 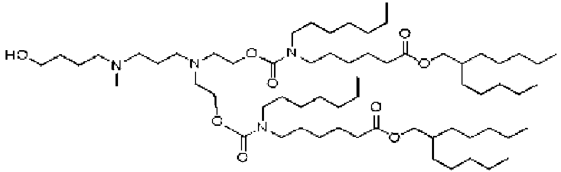
   bis(2-pentylheptyl) 7,17-diheptyl-12-(3-((4-hydroxybutyl)(methyl)amino)propyl)-8,16-dioxo-9,15-dioxa-7,12,17-t riazatricosanedioate, 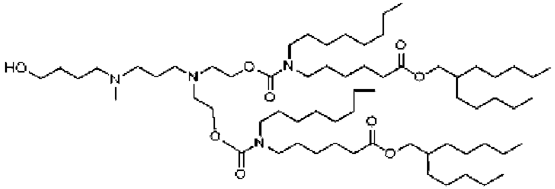
   bis(2-pentylheptyl) 12-(3-((4-hydroxybutyl)(methyl)amino)propyl)-7,17-dioctyl-8,16-dioxo-9,15-dioxa-7,12,17-tri azatricosanedioate, 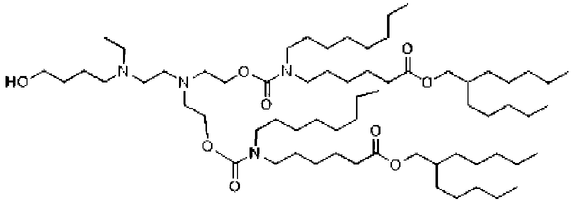
   bis(2-pentylheptyl) 12-(2-(ethyl(4-hydroxybutyl)amino)ethyl)-7,17-dioctyl-8,16-dioxo-9,15-dioxa-7,12,17-triazatr icosanedioate, 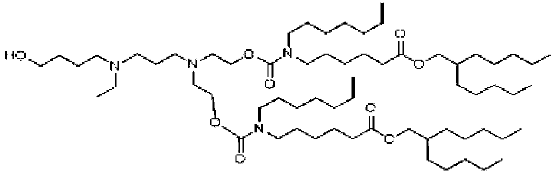
   bis(2-pentylheptyl) 12-(3-(ethyl(4-hydroxybutyl)amino)propyl)-7,17-diheptyl-8,16-dioxo-9,15-dioxa-7,12,17-triaz atricosanedioate, 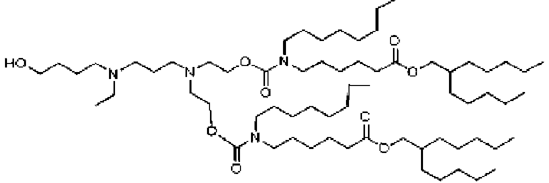
   bis(2-pentylheptyl) 12-(3-(ethyl(4-hydroxybutyl)amino)propyl)-7,17-dioctyl-8,16-dioxo-9,15-dioxa-7,12,17-triaza tricosanedioate, 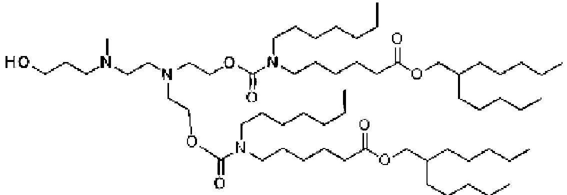
   bis(2-pentylheptyl) 7,17-diheptyl-12-(2-((3-hydroxypropyl)(methyl)amino)ethyl)-8,16-dioxo-9,15-dioxa-7,12,17-t riazatricosanedioate, 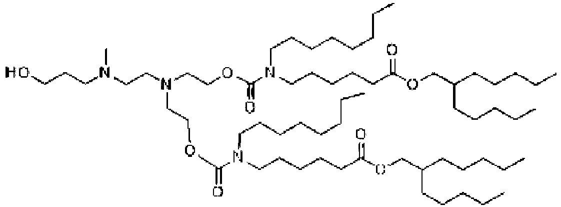
   bis(2-pentylheptyl) 12-(2-((3-hydroxypropyl)(methyl)amino)ethyl)-7,17-dioctyl-8,16-dioxo-9,15-dioxa-7,12,17-tri azatricosanedioate, 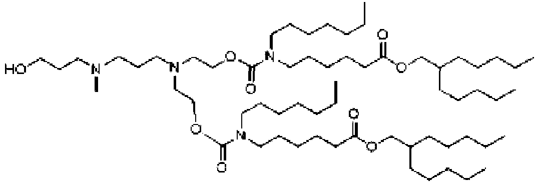
   bis(2-pentylheptyl) 7,17-diheptyl-12-(3-((3-hydroxypropyl)(methyl)amino)propyl)-8,16-dioxo-9,15-dioxa-7,12,17 -triazatricosanedioate, 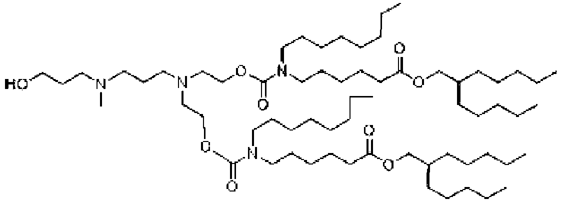
   bis(2-pentylheptyl) 12-(3-((3-hydroxypropyl)(methyl)amino)propyl)-7,17-dioctyl-8,16-dioxo-9,15-dioxa-7,12,17-t riazatricosanedioate, 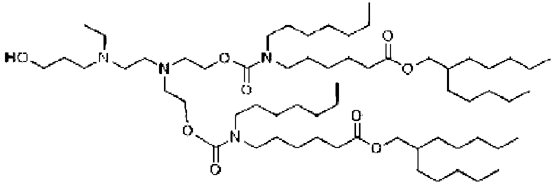
   bis(2-pentylheptyl) 12-(2-(ethyl(3-hydroxypropyl)amino)ethyl)-7,17-diheptyl-8,16-dioxo-9,15-dioxa-7,12,17-triaz atricosanedioate, 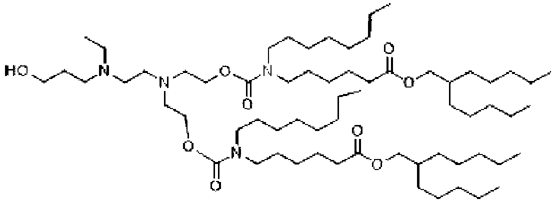
   bis(2-pentylheptyl) 12-(2-(ethyl(3-hydroxypropyl)amino)ethyl)-7,17-dioctyl-8,16-dioxo-9,15-dioxa-7,12,17-triaza tricosanedioate, 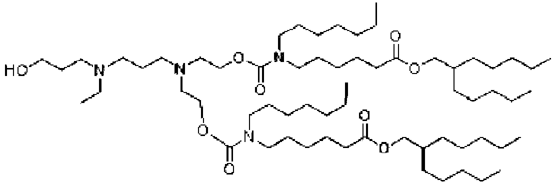
   bis(2-pentylheptyl) 12-(3-(ethyl(3-hydroxypropyl)amino)propyl)-7,17-diheptyl-8,16-dioxo-9,15-dioxa-7,12,17-tri azatricosanedioate, 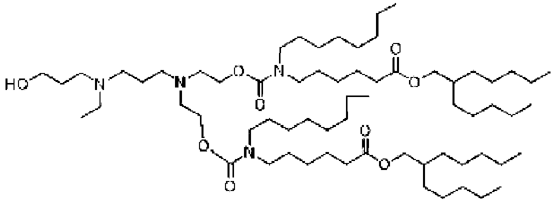
   bis(2-pentylheptyl) 12-(3-(ethyl(3-hydroxypropyl)amino)propyl)-7,17-dioctyl-8,16-dioxo-9,15-dioxa-7,12,17-triaz atricosanedioate, 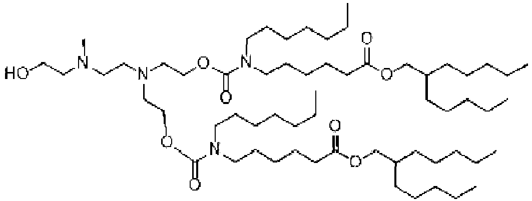
   bis(2-pentylheptyl) 7,17-diheptyl-12-(2-((2-hydroxyethyl)(methyl)amino)ethyl)-8,16-dioxo-9,15-dioxa-7,12,17-tri azatricosanedioate, 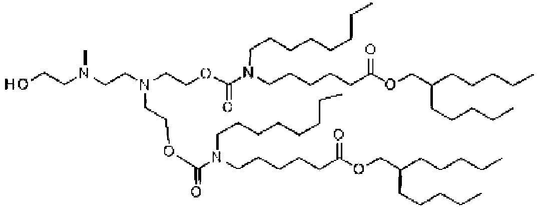
   bis(2-pentylheptyl) 12-(2-((2-hydroxyethyl)(methyl)amino)ethyl)-7,17-dioctyl-8,16-dioxo-9,15-dioxa-7,12,17-tria zatricosanedioate, 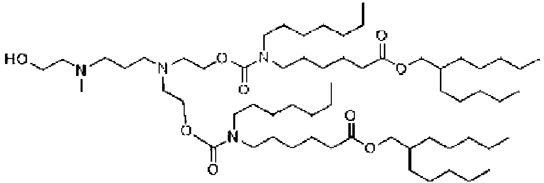
   bis(2-pentylheptyl) 7,17-diheptyl-12-(3-((2-hydroxyethyl)(methyl)amino)propyl)-8,16-dioxo-9,15-dioxa-7,12,17-t riazatricosanedioate, 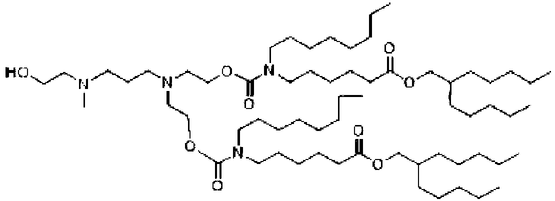
   bis(2-pentylheptyl) 12-(3-((2-hydroxyethyl)(methyl)amino)propyl)-7,17-dioctyl-8,16-dioxo-9,15-dioxa-7,12,17-tri azatricosanedioate, 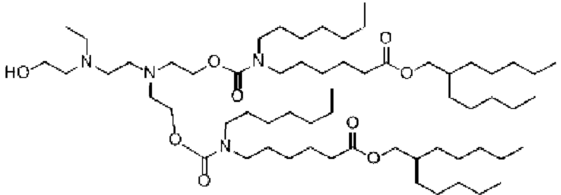
   bis(2-pentylheptyl) 12-(2-(ethyl(2-hydroxyethyl)amino)ethyl)-7,17-diheptyl-8,16-dioxo-9,15-dioxa-7,12,17-triazat ricosanedioate, 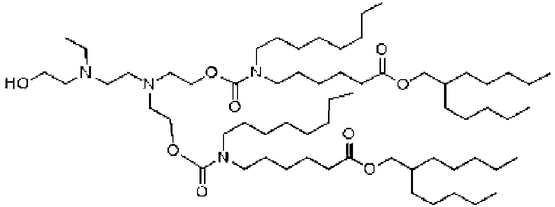
   bis(2-pentylheptyl) 12-(2-(ethyl(2-hydroxyethyl)amino)ethyl)-7,17-dioctyl-8,16-dioxo-9,15-dioxa-7,12,17-triazatr icosanedioate, 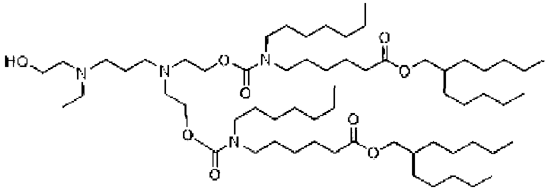
   bis(2-pentylheptyl) 12-(3-(ethyl(2-hydroxyethyl)amino)propyl)-7,17-diheptyl-8,16-dioxo-9,15-dioxa-7,12,17-triaz atricosanedioate, 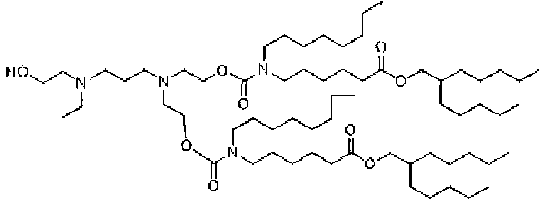
   bis(2-pentylheptyl) 12-(3-(ethyl(2-hydroxyethyl)amino)propyl)-7,17-dioctyl-8,16-dioxo-9,15-dioxa-7,12,17-triaza tricosanedioate, 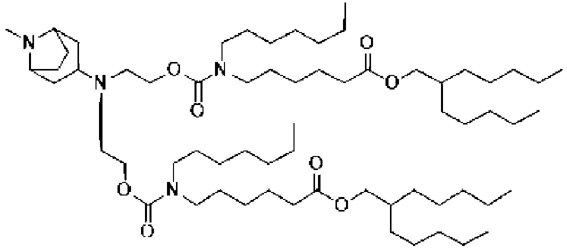
   bis(2-pentylheptyl) 7,17-diheptyl-12-(8-methyl-8-azabicyclo[3.2.1]octan-3-yl)-8,16-dioxo-9,15-dioxa-7,12,17-tria zatricosanedioate, 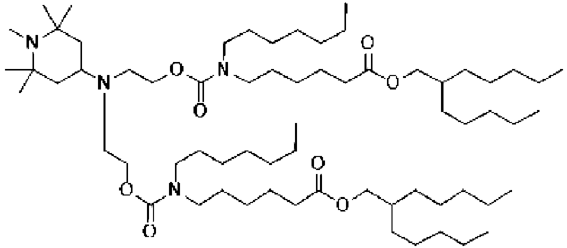
   bis(2-pentylheptyl) 7,17-diheptyl-8,16-dioxo-12-(1,2,2,6,6-pentamethylpiperidin-4-yl)-9,15-dioxa-7,12,17-triazatr icosanedioate, 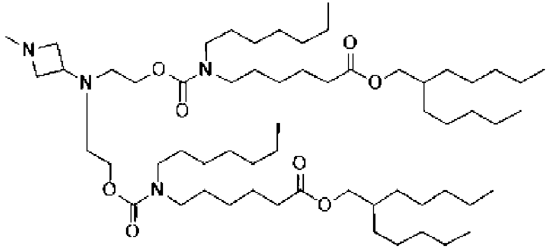
   bis(2-pentylheptyl) 7,17-diheptyl-12-(1-methylazetidin-3-yl)-8,16-dioxo-9,15-dioxa-7,12,17-triazatricosanedioate, 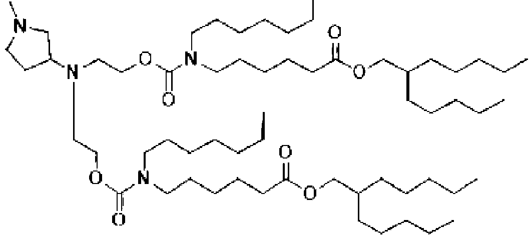
   bis(2-pentylheptyl) 7,17-diheptyl-12-(1-methylpyrrolidin-3-yl)-8,16-dioxo-9,15-dioxa-7,12,17-triazatricosanedioa te, 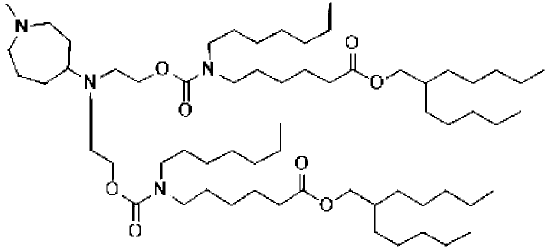
   bis(2-pentylheptyl) 7,17-diheptyl-12-(1-methylazepan-4-yl)-8,16-dioxo-9,15-dioxa-7,12,17-triazatricosanedioate, 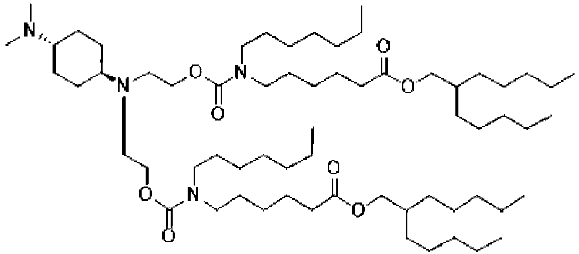
   bis(2-pentylheptyl) 12-((1r,4r)-4-(dimethylamino)cyclohexyl)-7,17-diheptyl-8,16-dioxo-9,15-dioxa-7,12,17-triaza tricosanedioate, 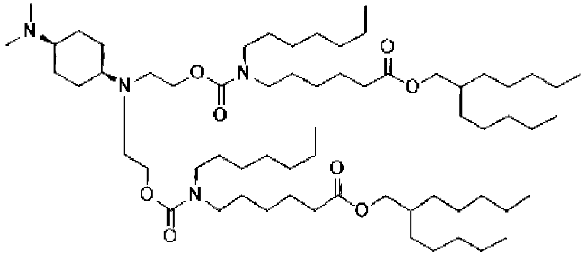
   bis(2-pentylheptyl) 12-((1s,4s)-4-(dimethylamino)cyclohexyl)-7,17-diheptyl-8,16-dioxo-9,15-dioxa-7,12,17-triaza tricosanedioate, 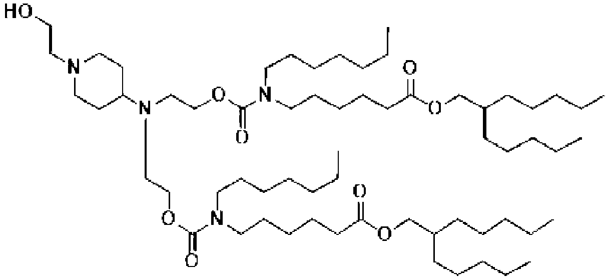
   bis(2-pentylheptyl) 7,17-diheptyl-12-(1-(2-hydroxyethyl)piperidin-4-yl)-8,16-dioxo-9,15-dioxa-7,12,17-triazatrico sanedioate, 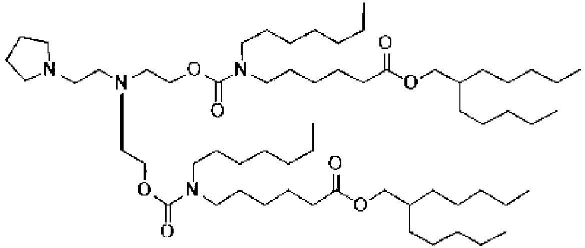
   bis(2-pentylheptyl) 7,17-diheptyl-8,16-dioxo-12-(2-(pyrrolidin-1-yl)ethyl)-9,15-dioxa-7,12,17-triazatricosanedioat e, 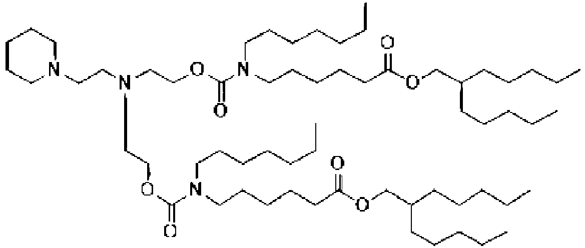
   bis(2-pentylheptyl) 7,17-diheptyl-8,16-dioxo-12-(2-(piperidin-1-yl)ethyl)-9,15-dioxa-7,12,17-triazatricosanedioate , 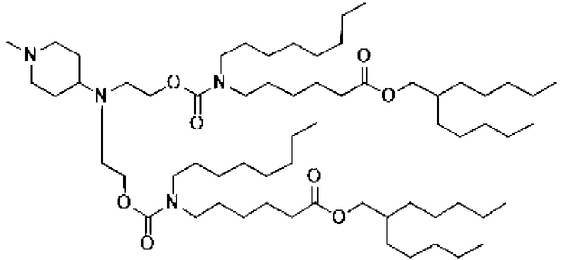
   bis(2-pentylheptyl) 12-(1-methylpiperidin-4-yl)-7,17-dioctyl-8,16-dioxo-9,15-dioxa-7,12,17-triazatricosanedioate, 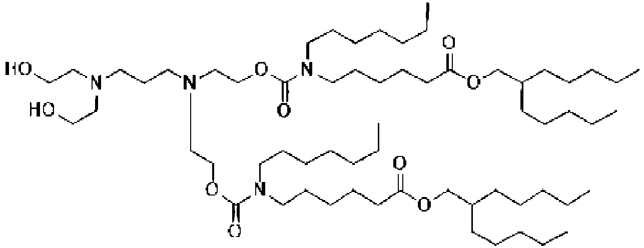
   bis(2-pentylheptyl) 12-(3-(bis(2-hydroxyethyl)amino)propyl)-7,17-diheptyl-8,16-dioxo-9,15-dioxa-7,12,17-triazat ricosanedioate, 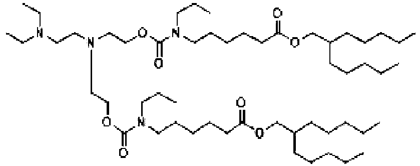
   bis(2-pentylheptyl) 12-(2-(diethylamino)ethyl)-8,16-dioxo-7,17-dipropyl-9,15-dioxa-7,12,17-triazatricosanedioate, 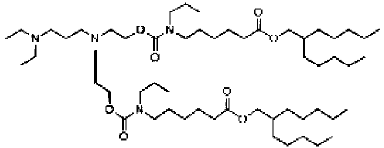
   bis(2-pentylheptyl) 12-(3-(diethylamino)propyl)-8,16-dioxo-7,17-dipropyl-9,15-dioxa-7,12,17-triazatricosanedioat e, and 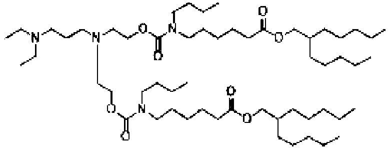
   bis(2-pentylheptyl) 7,17-dibutyl-12-(3-(diethylamino)propyl)-8,16-dioxo-9,15-dioxa-7,12,17-triazatricosanedioate .

### Effect of the invention

According to the present invention, it is possible to deliver nucleic acid to both an immune cell subjected to activation treatment and an immune cell not subjected to activation treatment.

### BRIEF DESCRIPTION OF THE DRAWINGS

[FIG. 1] FIG. 1 shows results of measuring nucleic acid delivery to activated T cells.
[FIG. 2] FIG. 2 shows results of measuring nucleic acid delivery to non-activated T cells.
[FIG. 3] FIG. 3 shows results of measuring nucleic acid delivery to activated T cells in an activation culture medium to which no ApoE3 is added.
[FIG. 4] FIG. 4 shows results of measuring nucleic acid delivery to non-activated T cells in an activation culture medium to which no ApoE3 is added.
[FIG. 5] FIG. 5 shows results of measuring TCR KO efficiency in activated T cells.
[FIG. 6] FIG. 6 shows results of measuring nucleic acid delivery to activated T cells under culture conditions in which various proteins are added.
[FIG. 7] FIG. 7 shows results of measuring nucleic acid delivery to long-term cultured T cells.

### Embodiments for carrying out the invention

Hereinafter, the present invention will be described in detail.

In the present specification, "to" shows a range including numerical values described before and after "to" as a minimum value and a maximum value, respectively.

### [Nucleic acid delivery agent to immune cell]

A nucleic acid delivery agent for an immune cell according to the embodiment of the present invention contains a lipid composition containing an ionizable lipid that is a compound represented by Formula (1) or a salt thereof, a non-ionizable lipid, a lipid having a nonionic polymer, and a nucleic acid, 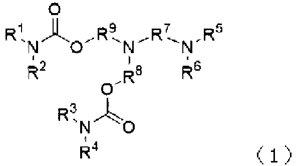
in the formula, R¹, R², R³, and R⁴ each independently represent a hydrogen atom or a hydrocarbon group having 1 to 24 carbon atoms which may be substituted,
substituents on the hydrocarbon groups having 1 to 24 carbon atoms which may be substituted, the hydrocarbon groups being represented by R¹, R², R³, and R⁴, each independently represent -C(O)O-R¹¹, -OC(O)-R¹², -O-R¹³, -CO-R¹⁴, -OC(O)O-R¹⁵, or -S-S-R¹⁶,
R¹¹, R¹², R¹³, R¹⁴, R¹⁵, and R¹⁶ each independently represent a hydrocarbon group having 1 to 24 carbon atoms which may be substituted with -S-R¹⁷, where R¹⁷ represents a hydrocarbon group having 1 to 12 carbon atoms,
R⁵ and R⁶ each independently represent a hydrocarbon group having 1 to 18 carbon atoms which may be substituted,
substituents on the hydrocarbon groups having 1 to 18 carbon atoms which may be substituted, the hydrocarbon groups being represented by R⁵ and R⁶, each independently represent -OH, -COOH, -NR²¹R²², -OC(O)O-R²³, -C(O)O-R²⁴, -OC(O)-R²⁵, -O-R²⁶, -C(O)NR²⁷R²⁸, -NR²⁹C(O)R³⁰, -N(R³¹)S(O)₂R³², -N(R³³)C(O)N(R³⁴)R³⁵, -N(R³⁶)C(S)N(R³⁷)R³⁸, -OC(O)N(R³⁹)R⁴⁰, or -N(R⁴¹)C(O)OR⁴²,
R²¹ and R²² each independently represent a hydrogen atom or a hydrocarbon group having 1 to 8 carbon atoms,
R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, and R⁴² each independently represent a hydrogen atom or a hydrocarbon group having 1 to 24 carbon atoms which may be substituted, and substituents on the hydrocarbon groups having 1 to 24 carbon atoms which may be substituted, the hydrocarbon group being represented by R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, and R⁴², represent an aryl group having 6 to 20 carbon atoms, a heterocyclic group, -OH, -COOH, or -NR⁵¹R⁵², where R⁵¹ and R⁵² each independently represent a hydrogen atom or a hydrocarbon group having 1 to 8 carbon atoms,
R⁷, R⁸, and R⁹ each independently represent a hydrocarbon group having 2 to 8 carbon atoms, and
R⁵ and R⁶, or R⁵ and R⁷ may be taken together to form a 4- to 7-membered ring.

The nucleic acid delivery agent to an immune cell according to the embodiment of the present invention can be used for producing an immune cell in which gene expression is modified, and can deliver a target nucleic acid to an immune cell in an non-activated state, and thus it is possible to prevent exhaustion of cells and to improve cell therapy performance.

### <Compound represented by Formula (1) or salt thereof>

A hydrocarbon group having 1 to 24 carbon atoms, a hydrocarbon group having 1 to 18 carbon atoms, a hydrocarbon group having 1 to 12 carbon atoms, and a hydrocarbon group having 1 to 8 carbon atoms are each preferably an alkyl group, an alkenyl group, or an alkynyl group.

The alkyl group may be linear or branched, or may be chainlike or cyclic. Specifically, examples of the alkyl group include a methyl group, an ethyl group, a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, a 2-butylhexyl group, a 2-butyloctyl group, a 1-pentylhexyl group, a 2-pentylheptyl group, a 3-pentyloctyl group, a 1-hexylheptyl group, a 1-hexylnonyl group, a 2-hexyloctyl group, a 2-hexyldecyl group, a 3-hexylnonyl group, a 1-heptyloctyl group, a 2-heptylnonyl group, a 2-heptylundecyl group, a 3-heptyldecyl group, a 1-octylnonyl group, a 2-octyldecyl group, a 2-octyldodecyl group, a 3-octylundecyl group, a 2-nonylundecyl group, a 3-nonyldodecyl group, a 2-decyldodecyl group, a 2-decyltetradecyl group, a 3-decyltridecyl group, a 2-(4,4-dimethylpentan-2-yl)-5,7,7-trimethyloctyl group, and the like.

The alkenyl group may be linear or branched, or may be chainlike or cyclic. Specifically, examples of the alkenyl group include an allyl group, a prenyl group, a pentenyl group, a hexenyl group, a heptenyl group, an octenyl group, a nonenyl group (preferably a (Z)-2-nonenyl group or an (E)-2-nonenyl group), a decenyl group, an undecenyl group, a dodecenyl group, a dodecadienyl group, a tridecenyl group (preferably a (Z)-tridec-8-enyl group), a tetradecenyl group (preferably a tetradec-9-enyl group), a pentadecenyl group (preferably a (Z)-pentadec-8-enyl group), a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11-dienyl group), an octadecenyl group (preferably a (Z)-octadec-9-enyl group), an octadecadienyl group (preferably a (9Z,12Z)-octadeca-9,12-dienyl group), and the like.

The alkynyl group may be linear or branched, or may be chainlike or cyclic. Specifically, examples thereof include a propargyl group, a butynyl group, a pentynyl group, a hexynyl group, a heptynyl group, an octynyl group, a nonynyl group, a decynyl group, an undecynyl group, a dodecynyl group, a tetradecynyl group, a pentadecynyl group, a hexadecynyl group, a heptadecynyl group, and an octadecynyl group.

All of the above alkenyl groups preferably have one double bond or two double bonds. All of the above alkynyl groups preferably have one triple bond or two triple bonds.

The hydrocarbon groups having 2 to 8 carbon atoms represented by R⁷, R⁸, and R⁹ is each preferably an alkylene group, an alkenylene group, or an alkynylene group.

The alkylene group, the alkenylene group, or the alkynylene group having 2 to 8 carbon atoms may be linear or branched, or may be chainlike or cyclic.

Specific examples thereof include an ethylene group, a trimethylene group, a tetramethylene group, a pentamethylene group, a hexamethylene group, a heptamethylene group, and an octamethylene group.

The aryl group having 6 to 20 carbon atoms is preferably an aryl group having 6 to 18 carbon atoms, and more preferably an aryl group having 6 to 10 carbon atoms. Specifically, examples of the aryl group include a phenyl group, a naphthyl group, an anthracenyl group, and a phenanthrenyl group.

The heterocyclic group means a heteroaryl group or a heteroaliphatic ring group.

The heteroaryl group means an aromatic heterocyclic group, may be an aromatic heterocyclic group in which an aromatic heterocyclic ring, an aromatic hydrocarbon ring, a heteroaliphatic ring, or an aliphatic hydrocarbon ring is fused, and is preferably a monocyclic nitrogen-containing heteroaryl group, a monocyclic oxygen-containing heteroaryl group, a monocyclic sulfur-containing heteroaryl group, a monocyclic nitrogen- and oxygen-containing heteroaryl group, a monocyclic nitrogen- and sulfur-containing heteroaryl group, a bicyclic nitrogen-containing heteroaryl group, a bicyclic oxygen-containing heteroaryl group, a bicyclic sulfur-containing heteroaryl group, a bicyclic nitrogen- and oxygen-containing heteroaryl group, or a bicyclic nitrogen- and sulfur-containing heteroaryl group. The 5-membered ring heteroaryl group is a monocyclic heteroaryl group, in which the number of atoms constituting the ring is five.

In addition, the aromatic heterocyclic ring means an aromatic ring having a heteroatom as a ring member, may be fused with an aromatic heterocyclic ring, an aromatic hydrocarbon ring, a heteroaliphatic ring, or an aliphatic hydrocarbon ring, and is preferably a monocyclic nitrogen-containing aromatic heterocyclic ring, a monocyclic oxygen-containing aromatic heterocyclic ring, a monocyclic sulfur-containing aromatic heterocyclic ring, a monocyclic nitrogen- and oxygen-containing aromatic heterocyclic ring, a monocyclic nitrogen- and sulfur-containing aromatic heterocyclic ring, a bicyclic nitrogen-containing aromatic heterocyclic ring, a bicyclic oxygen-containing aromatic heterocyclic ring, a bicyclic sulfur-containing aromatic heterocyclic ring, a bicyclic nitrogen- and oxygen-containing aromatic heterocyclic ring, or a bicyclic nitrogen- and sulfur-containing aromatic heterocyclic ring.

The monocyclic nitrogen-containing heteroaryl group means a heteroaryl group (this heteroaryl group may be partially saturated) in which a ring containing at least one nitrogen atom, such as a pyrrolinyl group, a pyrrolyl group, a tetrahydropyridyl group, a pyridyl group, an imidazolinyl group, an imidazolyl group, a pyrazolinyl group, a pyrazolyl group, a pyrazinyl group, a pyridazinyl group, a pyrimidinyl group, a triazolyl group, or a tetrazolyl group, has aromaticity. This heteroaryl group may be further fused with another aromatic ring or aliphatic ring.

The monocyclic oxygen-containing heteroaryl group means a heteroaryl group (this heteroaryl group may be partially saturated) in which a ring containing at least one oxygen atom, such as a furanyl group or a pyranyl group, has aromaticity. This heteroaryl group may be further fused with another aromatic ring or aliphatic ring.

The monocyclic nitrogen- and oxygen-containing heteroaryl group means an oxazolyl group, an isoxazolyl group, an oxadiazolyl group, or the like. This heteroaryl group may be further fused with another aromatic ring or aliphatic ring.

The monocyclic nitrogen- and sulfur-containing heteroaryl group means a thiazolyl group, an isothiazolyl group, a thiadiazolyl group, or the like. This heteroaryl group may be further fused with another aromatic ring or aliphatic ring.

The bicyclic nitrogen-containing heteroaryl group means a bicyclic heteroaryl group (this heteroaryl group may be partially saturated) in which a ring containing at least one nitrogen atom, such as an indolyl group, an isoindolyl group, a benzimidazolyl group, an indazolyl group, a benzotriazolyl group, a quinolyl group, an isoquinolyl group, a tetrahydroquinolyl group, a tetrahydroisoquinolyl group, a quinolizidyl group, a cinnolinyl group, a phthalazinyl group, a quinazolinyl group, a quinoxalinyl group, a naphthyridinyl group, a pyrrolopyridyl group, an imidazopyridyl group, a pyrazolopyridyl group, a pyridopyrazyl group, a purinyl group, a pteridinyl group, a 5,6,7,8-tetrahydrophthalazinyl group, a 5,6,7,8-tetrahydrocinnolinyl group, a 1,2,3,4-tetrahydropyrido[2,3-d]pyrazidinyl group, a 5,6,7,8-tetrahydro-[1,2,4]triazolo[4,3-a]pyrazidinyl group, a 5,6,7,8-tetrahydropyrido[3,4-d]pyrazidinyl group, a 5,6,7,8-tetrahydropyrido[3,2-c]pyrazidinyl group, a 5,6,7,8-tetrahydropyrido[4,3-c]pyrazidinyl group, a 6,7-dihydro-5H-cyclopenta[d]pyrazidinyl group, a 6,7-dihydro-5H-cyclopenta[c]pyrazidinyl group, a 2,3-dihydro-1H-pyrrolo[2,3-d]pyrazidinyl group, a 6,7-dihydro-5H-pyrrolo[3,4-d]pyrazidinyl group, a 6,7-dihydro-5H-pyrrolo[3,2-c]pyrazidinyl group, a 6,7-dihydro-5H-pyrrolo[3,4-c]pyrazidinyl group, or a 6,7-dihydro-5H-pyrrolo[2,3-c]pyrazidinyl group, has aromaticity.

The bicyclic oxygen-containing heteroaryl group means a bicyclic heteroaryl group (this heteroaryl group may be partially saturated) in which a ring containing at least one oxygen atom, such as a benzofuranyl group, an isobenzofuranyl group, and a chromenyl group, has aromaticity.

The bicyclic nitrogen- and oxygen-containing heteroaryl group means a bicyclic heteroaryl group (this heteroaryl group may be partially saturated) in which a ring containing at least one nitrogen atom and at least one oxygen atom, such as a benzoxazolyl group, a benzoisoxazolyl group, a benzoxadiazolyl group, a dihydropyranopyridyl group, a dihydrodioxinopyridyl group, a dihydropyridooxadienyl group, a 3,4-dihydro-2H-pyrano[2,3-d]pyridazinyl group, a 7,8-dihydro-5H-pyrano[3,4-d]pyridazinyl group, a 7,8-dihydro-6H-pyrano[3,2-c]pyridazinyl group, a 7,8-dihydro-5H-pyrano[4,3-c]pyridazinyl group, a 2,3-dihydrofuro[2,3-d]pyridazinyl group, a 5,7-dihydrofuro[3,4-d]pyridazinyl group, a 6,7-dihydrofuro[3,2-c]pyridazinyl group, a 5,7-dihydrofuro[3,4-c]pyridazinyl group, and a 5,6-dihydrofuro[2,3-c]pyridazinyl group, has aromaticity.

The heteroaliphatic ring group means a nitrogen-containing heteroaliphatic ring group, an oxygen-containing heteroaliphatic ring group, a sulfur-containing heteroaliphatic ring group, a nitrogen- and oxygen-containing heteroaliphatic ring group, a nitrogen- and sulfur-containing heteroaliphatic ring group, a heterobridged ring group, or a heterospiro ring group.

In addition, the heteroaliphatic ring means an aliphatic ring having a heteroatom as a ring member, and preferred examples thereof include a nitrogen-containing heteroaliphatic ring, an oxygen-containing heteroaliphatic ring, a sulfur-containing heteroaliphatic ring, a nitrogen- and oxygen-containing heteroaliphatic ring, a nitrogen- and sulfur-containing heteroaliphatic ring, a heterobridged ring, and a heterospiro ring.

The nitrogen-containing heteroaliphatic ring group means a heterocyclic aliphatic ring group in which a ring containing at least one nitrogen atom, such as an azetidinyl, a pyrrolidinyl, a piperidinyl, a homopiperidinyl, an octahydroazocinyl, an imidazolidinyl, a pyrazolidinyl, a piperazinyl, or a homopiperazinyl group, does not have aromaticity. This nitrogen-containing heteroaliphatic ring group may be further fused with another aromatic ring or aliphatic ring.

The oxygen-containing heteroaliphatic ring group means a tetrahydrofuranyl group, a tetrahydropyranyl group, an oxetanyl group, a 1,3-dioxanyl group, or the like. This oxygen-containing heteroaliphatic ring group may be further fused with another aromatic ring or aliphatic ring.

The nitrogen- and oxygen-containing heteroaliphatic ring group means a morpholinyl group, a 1,4-oxazepanyl group, or the like. This nitrogen- and oxygen-containing heteroaliphatic ring group may be further fused with another aromatic ring or aliphatic ring.

The heteroaliphatic ring C₁₋₈ alkyl group refers to a linear, branched, or cyclic C₁₋₈ alkyl group to which a heteroaliphatic ring group such as a pyrrolidinylmethyl group, a pyrrolidinylethyl group, a pyrrolidinylpropyl group, a pyrrolidinyloctyl group, a piperidinylmethyl group, or a tetrahydrofuranylmethyl group is bonded.

In Formula (1), preferably,
R¹ represents -R^{1a}-L¹-R^{1b}, where R^{1a} represents a hydrocarbon group having 1 to 18 carbon atoms,
L¹ represents -C(O)O-, -OC(O)-, -OC(O)O-, or -S-S-, and
R^{1b} represents a hydrocarbon group having 1 to 18 carbon atoms,
R³ represents -R^{3a}-L³-R^{3b}, where R^{3a} represents a hydrocarbon group having 3 to 18 carbon atoms,
L³ represents -C(O)O-, -OC(O)-, -OC(O)O-, or -S-S-, and
R^{3b} represents a hydrocarbon group having 1 to 18 carbon atoms,
R² and R⁴ each independently represent a hydrocarbon group having 1 to 18 carbon atoms, which may be substituted, and substituents on the hydrocarbon groups having 1 to 18 carbon atoms represented by R² and R⁴, which may be substituted, each independently represent -C(O)O-R¹¹, -OC(O)-R¹², -O-R¹³, -CO-R¹⁴, -OC(O)O-R¹⁵, or -S-S-R¹⁶,
R¹¹, R¹², R¹³, R¹⁴, R¹⁵, and R¹⁶ each independently represent a hydrocarbon group having 1 to 18 carbon atoms,
R⁵ and R⁶ each independently represent a hydrocarbon group having 1 to 12 carbon atoms, which may be substituted,
substituents on the hydrocarbon groups having 1 to 12 carbon atoms represented by R⁵ and R⁶, which may be substituted, each independently represent -OH, -O-R²⁶, -C(O)NR²⁷R²⁸, or -NR²⁹C(O)R³⁰,
R²⁶, R²⁷, R²⁸, R²⁹, and R³⁰ each independently represent a hydrogen atom or a hydrocarbon group having 1 to 12 carbon atoms, which may be substituted, and substituents on the hydrocarbon groups having 1 to 12 carbon atoms represented by R²⁶, R²⁷, R²⁸, R²⁹, and R³⁰, which may be substituted, represent an aryl group having 6 to 10 carbon atoms or a heterocyclic group, and
R⁷, R⁸, and R⁹ each independently represent -(CH₂)ₙ-, and n represents an integer of 2 to 8, and
R⁵ and R⁶, or R⁵ and R⁷ may be taken together to form a 4- to 7-membered ring.

In Formula (1), more preferably,
R¹ represents -R^{1a}-L¹-R^{1b}, where R^{1a} represents a hydrocarbon group having 1 to 18 carbon atoms,
L¹ represents -C(O)O- or -OC(O)-, and R^{1b} represents a hydrocarbon group having 1 to 18 carbon atoms,
R³ represents -R^{3a}-L³-R^{3b}, where R^{3a} represents a hydrocarbon group having 3 to 18 carbon atoms,
L³ represents -C(O)O- or -OC(O)-, and R^{3b} represents a hydrocarbon group having 3 to 18 carbon atoms,
R² and R⁴ each independently represent a hydrocarbon group having 1 to 10 carbon atoms,
R⁵ and R⁶ each independently represent a hydrocarbon group having 1 to 6 carbon atoms, which may be substituted,
substituents on the hydrocarbon groups having 1 to 6 carbon atoms represented by R⁵ and R⁶, which may be substituted, each independently represent -OH, -O-R²⁶, -C(O)NR²⁷R²⁸, or -NR²⁹C(O)R³⁰,
R²⁶, R²⁷, R²⁸, R²⁹, and R³⁰ each independently represent a hydrogen atom or a hydrocarbon group having 1 to 12 carbon atoms, which may be substituted, and substituents on the hydrocarbon groups having 1 to 12 carbon atoms represented by R²⁶, R²⁷, R²⁸, R²⁹, and R³⁰, which may be substituted, represent an aryl group having 6 to 10 carbon atoms, and
R⁷, R⁸, and R⁹ each independently represent -(CH₂)ₙ-, and n represents an integer of 2 to 8.

In Formula (1), most preferably,
R¹ represents -R^{1a}-L¹-R^{1b}, where R^{1a} represents a hydrocarbon group having 1 to 5 carbon atoms, L¹ represents -C(O)O- or -OC(O)-, and R^{1b} represents a hydrocarbon group having 1 to 18 carbon atoms,
R³ represents -R^{3a}-L³-R^{3b}, where R^{3a} represents a hydrocarbon group having 1 to 5 carbon atoms, L³ represents -C(O)O- or -OC(O)-, and R^{3b} represents a hydrocarbon group having 7 to 14 carbon atoms,
R² and R⁴ each independently represent a hydrocarbon group having 3 to 8 carbon atoms,
R⁵ and R⁶ each independently represent a hydrocarbon group having 1 to 4 carbon atoms, which may be substituted, and substituents on the hydrocarbon groups having 1 to 18 carbon atoms represented by R⁵ and R⁶, which may be substituted, each independently preferably represent -OH, and
R⁷, R⁸, and R⁹ each independently represent -(CH₂)ₙ-, and n represents an integer of 2 to 4.
R⁵ and R⁶, or R⁵ and R⁷ may be taken together to form a 5- to 7-membered ring.

The compound represented by Formula (1) may form a salt.

Examples of the salt in a basic group include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylenesulfonic acid, and naphthalenesulfonic acid.

Examples of the salt in an acidic group include salts with alkali metals such as sodium and potassium; salts with alkaline earth metals such as calcium and magnesium; ammonium salts; salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine; and the like.

Among the above salts, preferred examples of the salt include pharmacologically acceptable salts.

The ionizable lipid is preferably one or more of the following compounds, but the present invention is not limited thereto in interpretation.

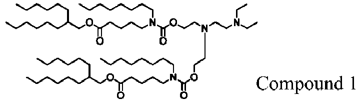

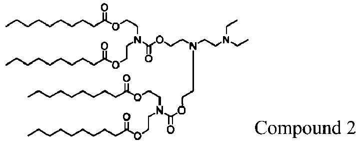

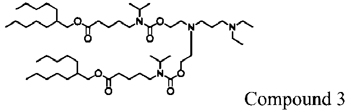

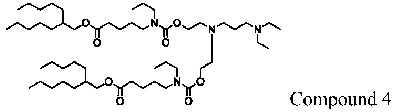

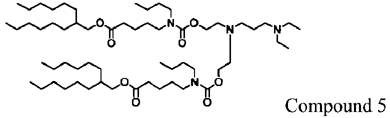

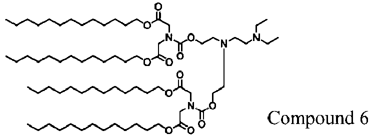

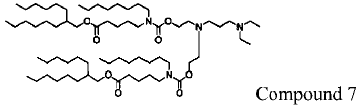

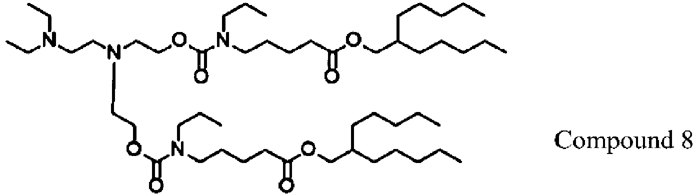

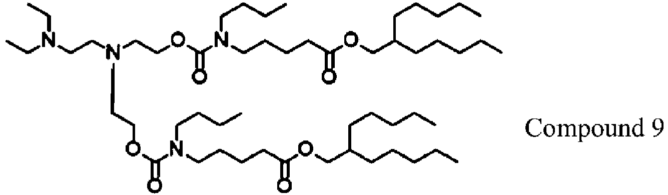

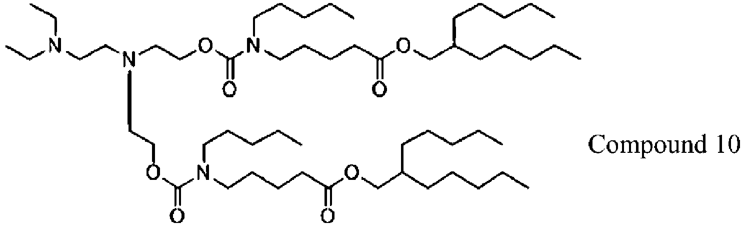

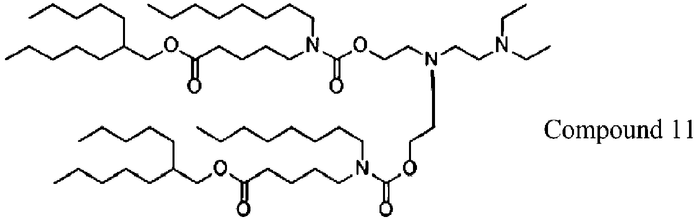

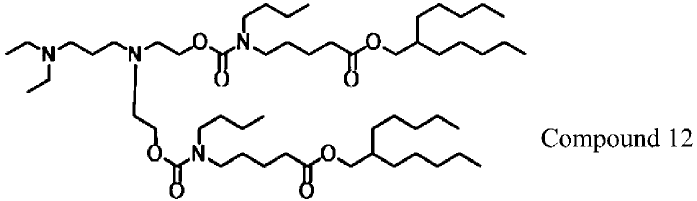

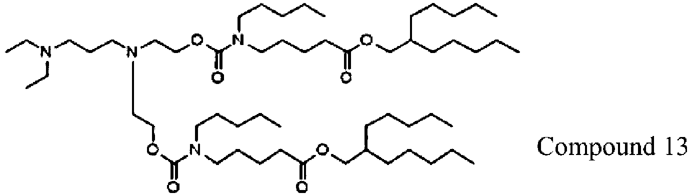

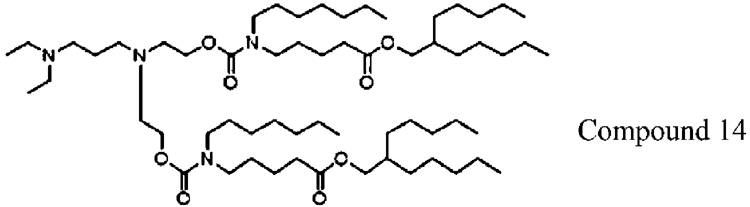

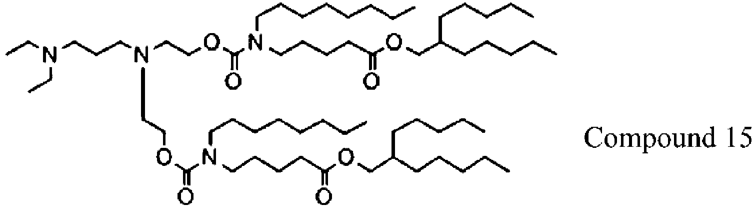

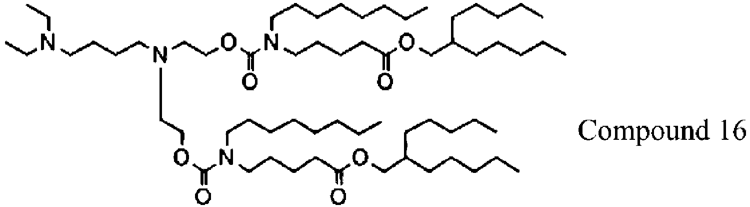

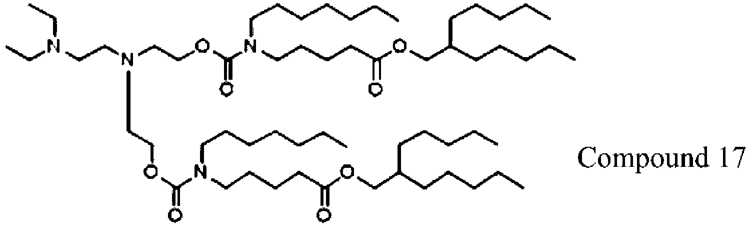

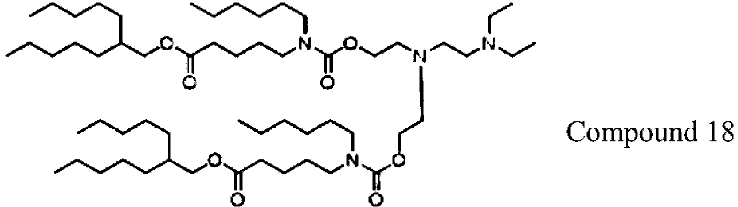

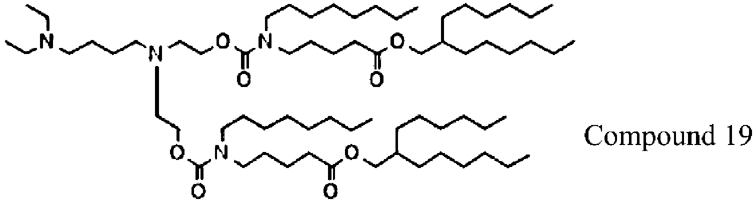

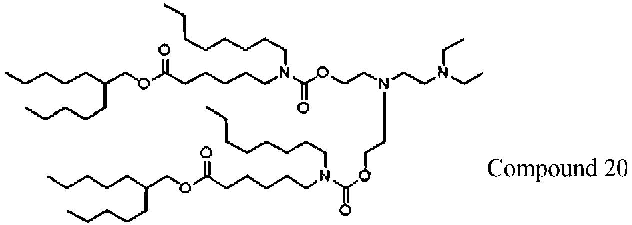

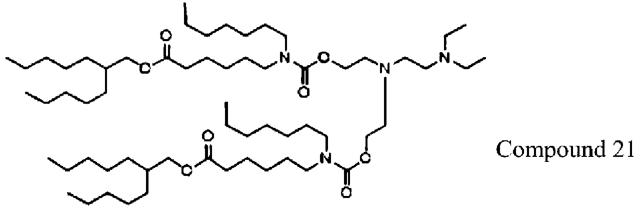

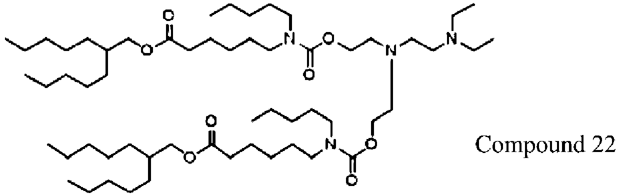

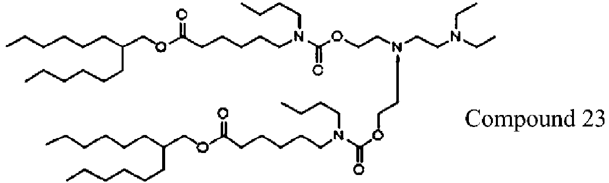

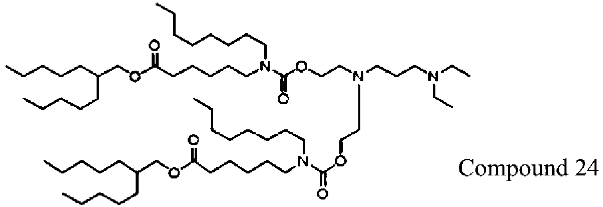

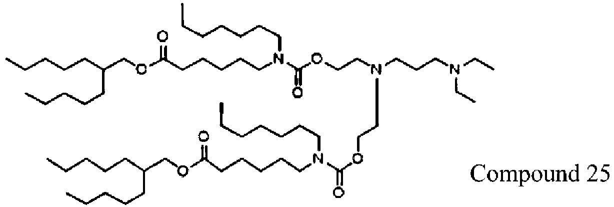

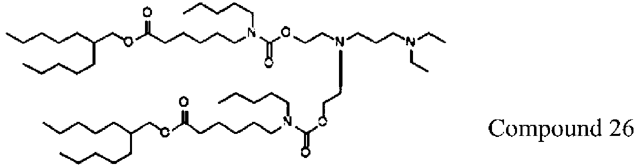

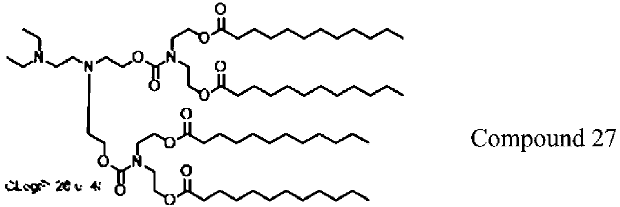

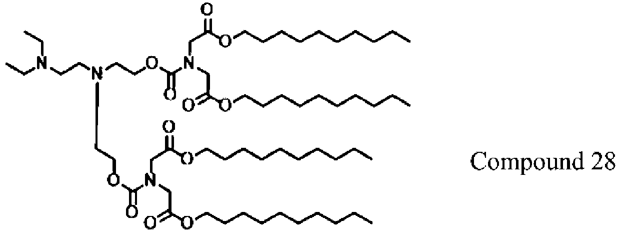

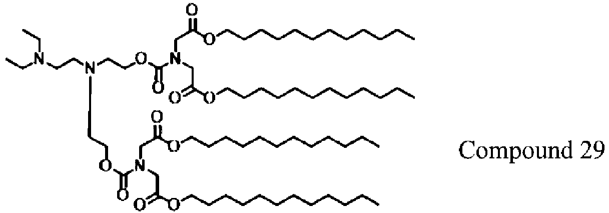

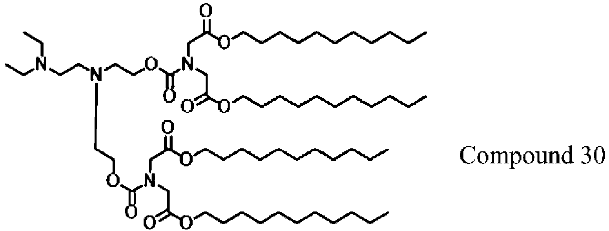

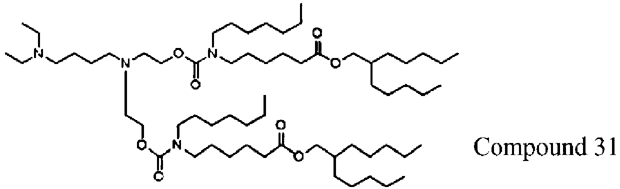

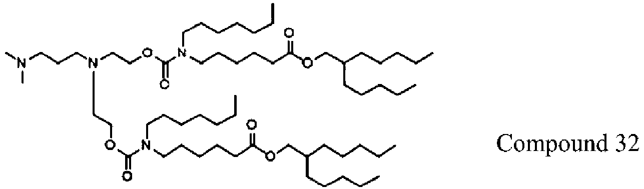

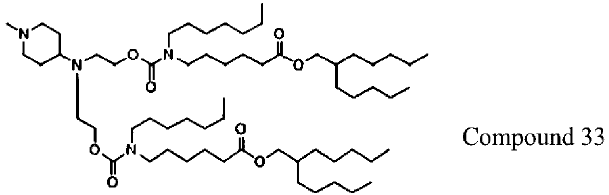

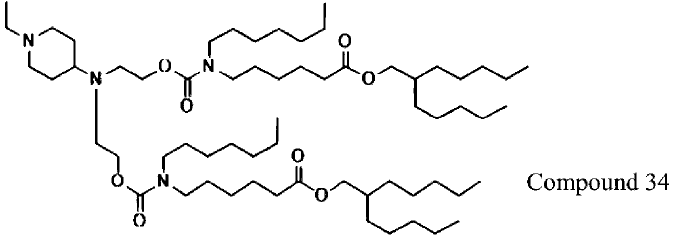

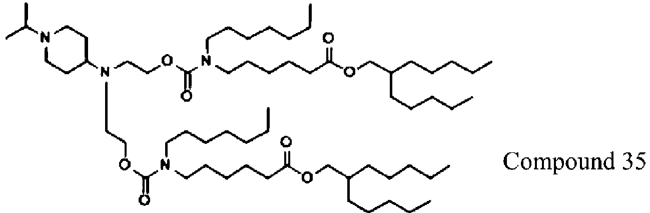

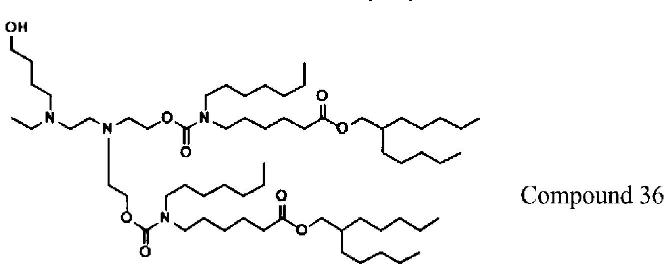

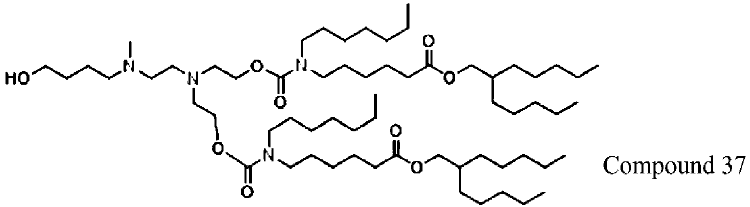

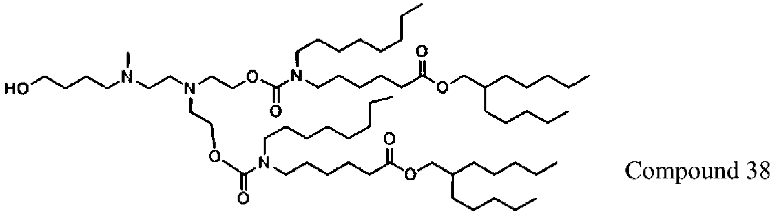

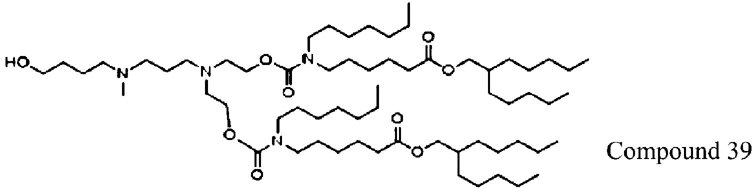

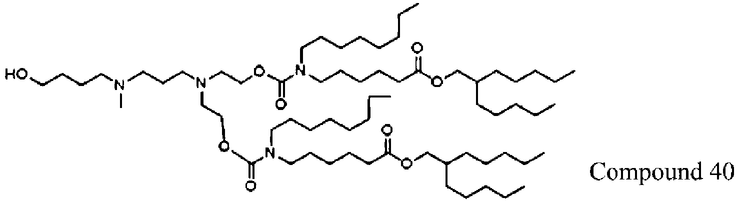

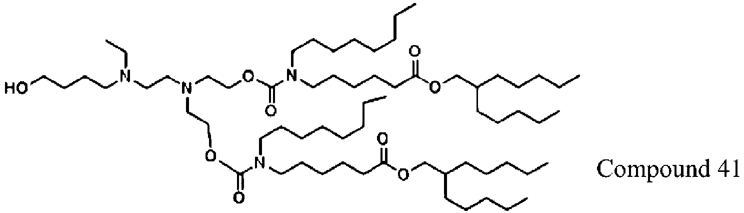

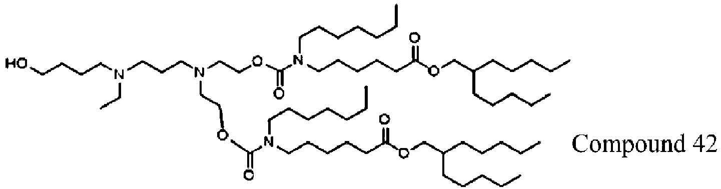

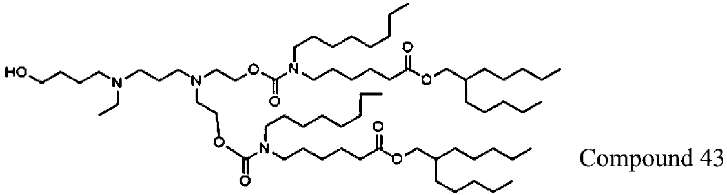

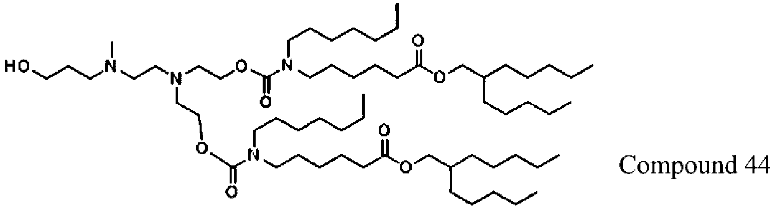

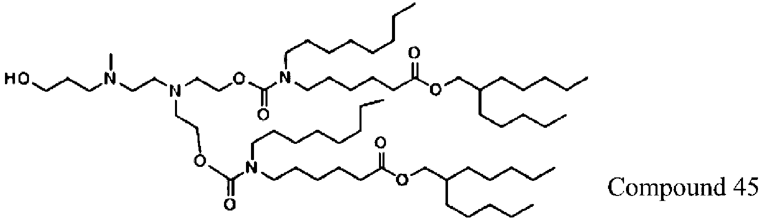

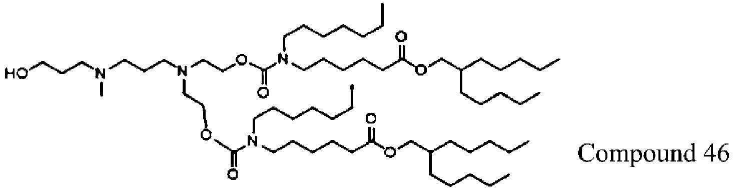

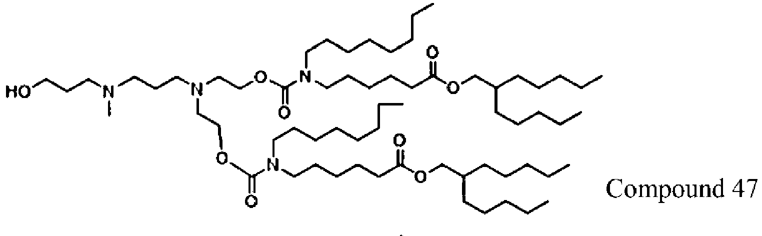

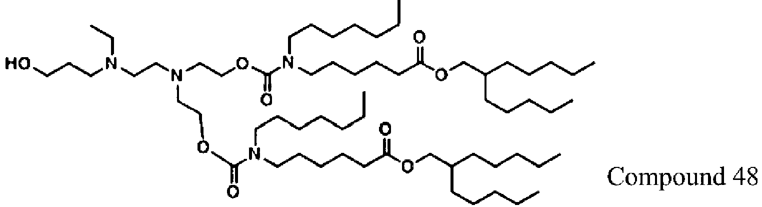

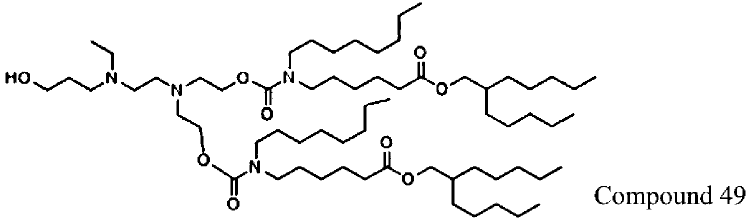

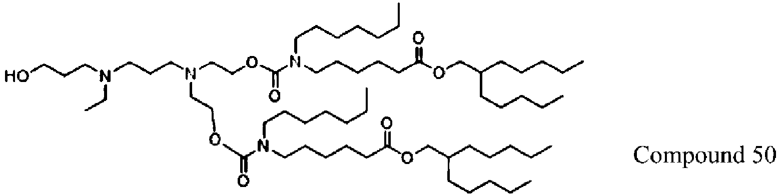

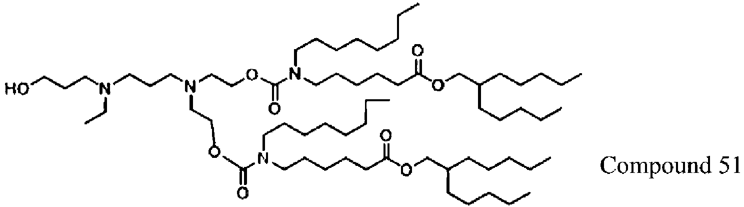

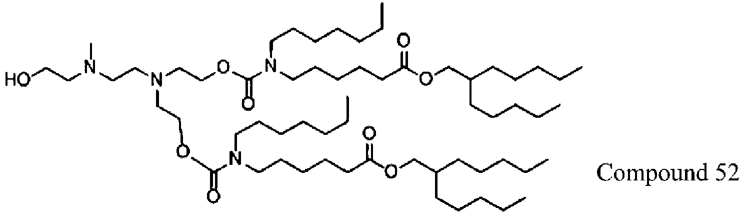

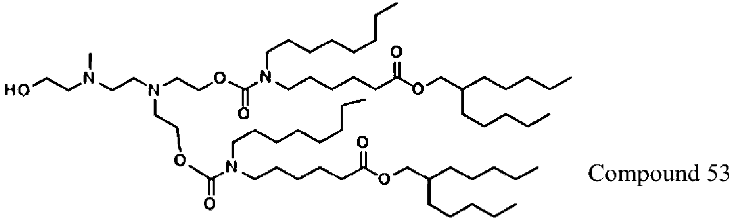

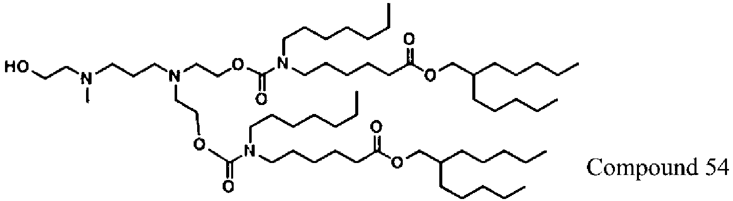

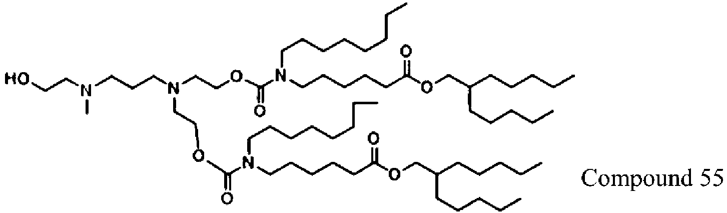

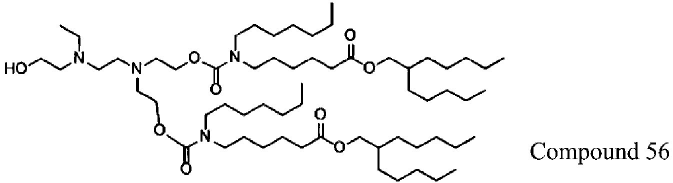

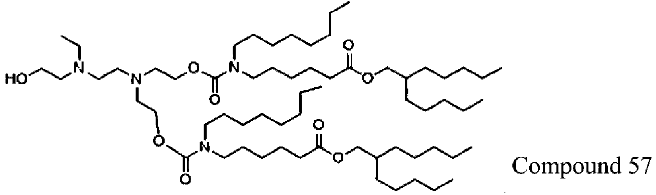

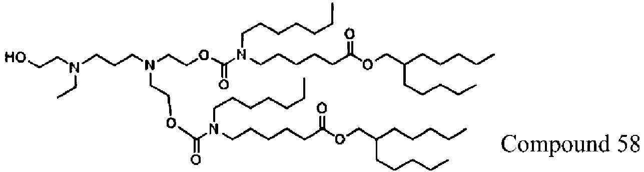

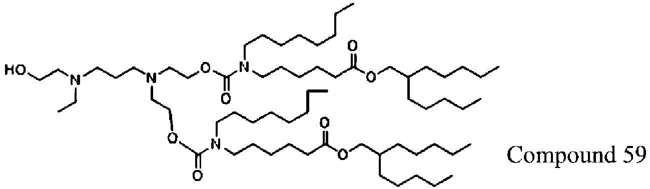

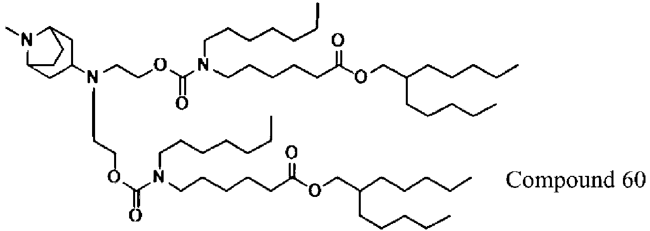

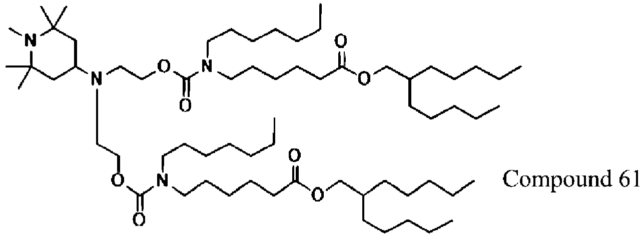

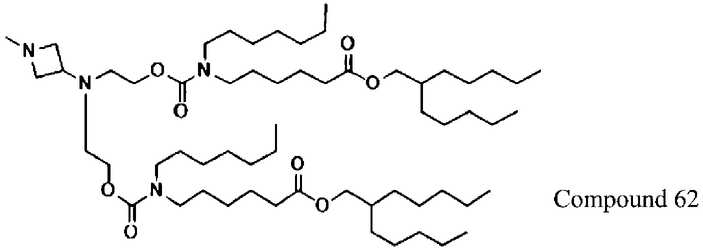

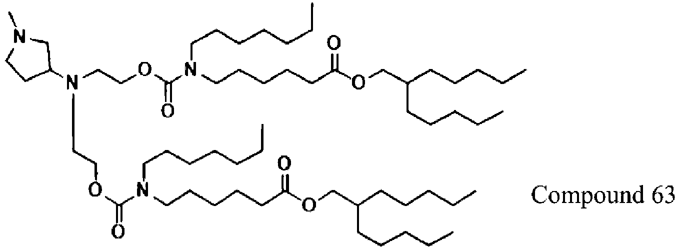

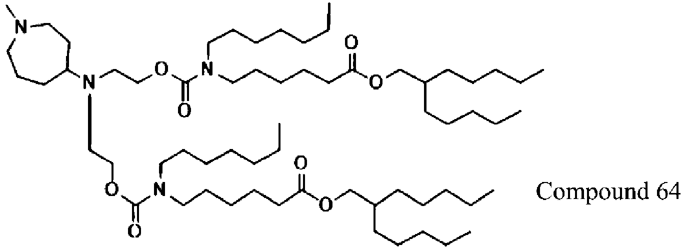

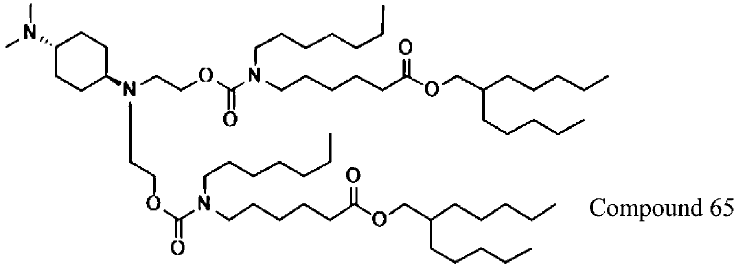

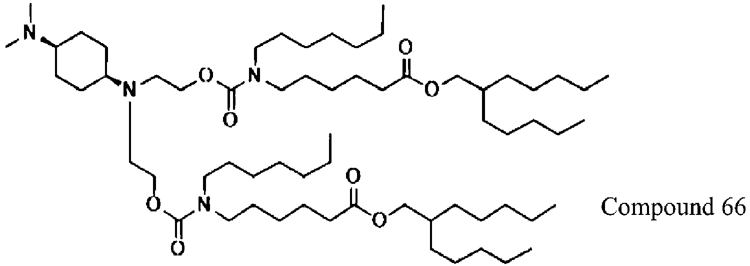

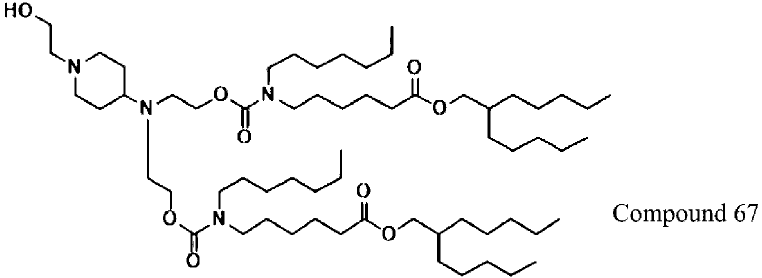

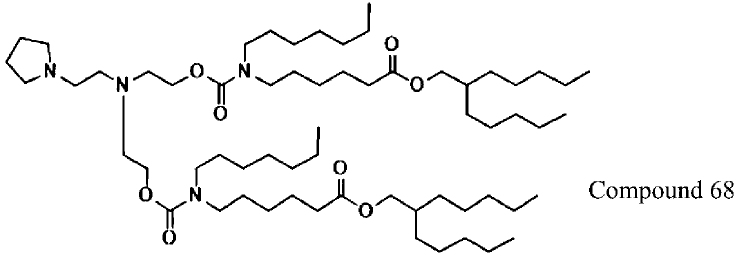

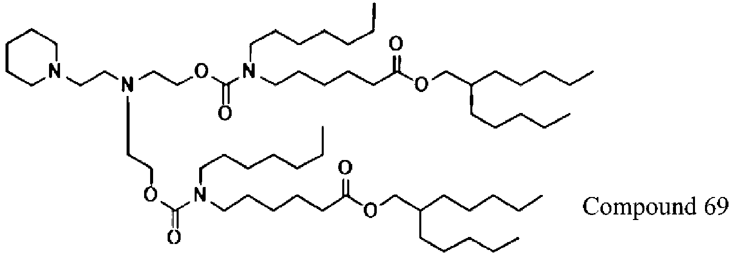

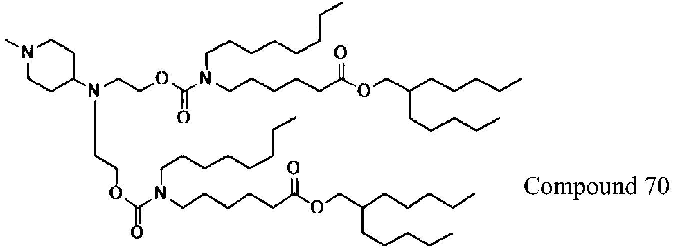

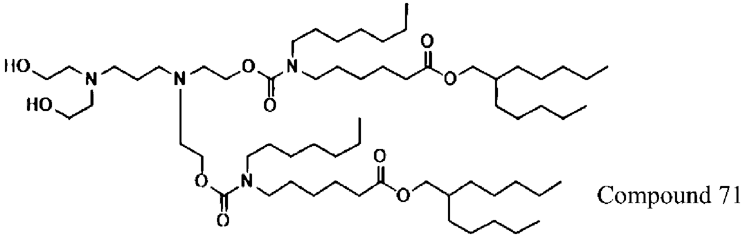

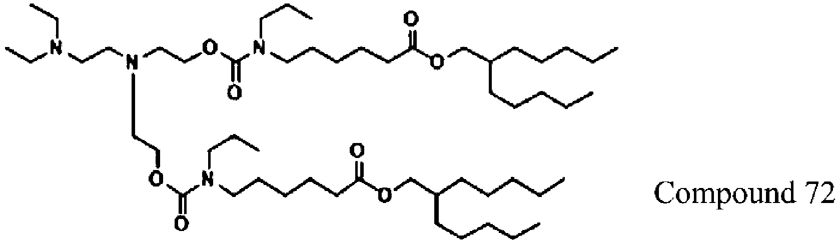

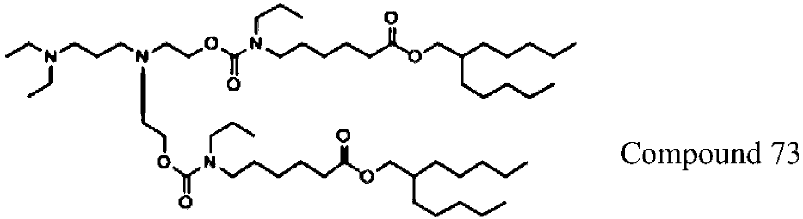

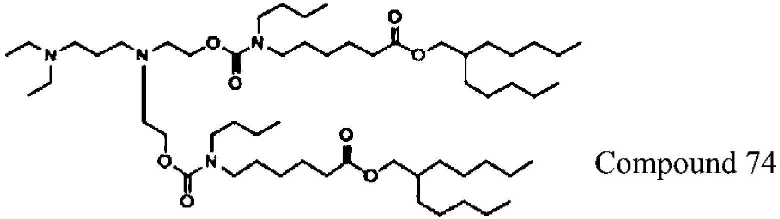

The compounds 1 to 7 and 31 to 74 are novel compounds. According to the present invention, the compounds 1 to 7 and 31 to 74 are provided.

In the lipid composition, the blending amount of the compound represented by Formula (1) or a salt thereof is preferably 20 mol% to 60 mol%, more preferably 30 mol% to 60 mol%, and still more preferably 40 mol% to 60 mol% in terms of molar ratio with respect to the total lipids in the lipid composition.

### <Method for producing compound represented by Formula (1)>

A method for producing the compound represented by Formula (1) will be described.

The compound represented by Formula (1) can be produced by combining known methods, and can be produced, for example, according to the following production method.

### [Production method 1]

A method for producing the compound represented by Formula [1] from the compound represented by Formula [2].

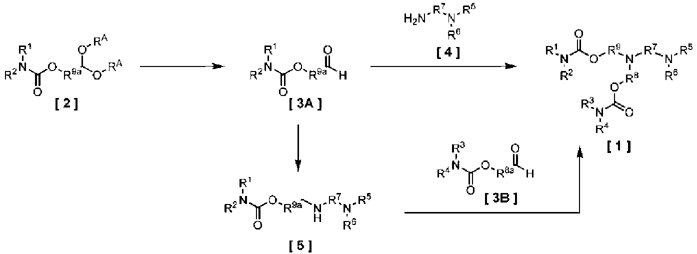

In the formulae, R¹, R², R³, R⁴, R⁵, R⁶, R⁷, R⁸, and R⁹ have the same meanings as described above, and R^{8a}, R^{9a}, and R^{A} each represent a hydrocarbon group having 1 to 7 carbon atoms.

(1-1)
The compound represented by Formula [3A] can be produced by reacting the compound represented by Formula [2] in the presence of water and acid, in the presence of a solvent, or in the absence of a solvent.

Examples of the acid used in this reaction include an inorganic acid and an organic acid. An organic acid is preferable, specific examples thereof include formic acid, acetic acid, trifluoroacetic acid, 4-toluenesulfonic acid, methanesulfonic acid, and the like, and formic acid is more preferable.

The used amount of the acid is only required to be 1 to 100 times (v/w) and preferably 1 to 10 times (v/w) the amount of the compound represented by Formula [2].

The used amount of the water is only required to be 0.1 to 100 times (v/w) and preferably 0.1 to 10 times (v/w) the amount of the compound represented by Formula [2].

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

The used amount of the solvent is not particularly limited, but is only required to be 0.1 to 50 times (v/w) the amount of the compound represented by Formula [2].

This reaction may be carried out at -30°C to 150°C preferably at 0°C to 100°C for 5 minutes to 48 hours.

(1-2)
The compound represented by Formula [1] can be produced by reacting the compound represented by Formula [3A] with the compound represented by Formula [4] in the presence of a reducing agent.

As the compound represented by Formula [4], for example, N,N-diethylethylenediamine, N,N-diethyl-1,3-diaminopropane, and the like are known.

The solvent used in this reaction is not particularly limited as long as the solvent does not affect the reaction. Examples of the solvent include halogenated hydrocarbons, alcohols, ethers, esters, amides, nitriles, sulfoxides, and aromatic hydrocarbons. These solvents may be used by being mixed together.

Examples of the preferred solvent include esters, and ethyl acetate is more preferable.

The used amount of the solvent is not particularly limited, but is only required to be 1 to 500 times (v/w) the amount of the compound represented by Formula [3A].

Examples of the reducing agent used in this reaction include sodium borohydride, sodium cyanoborohydride, pyridine borane, 2-picoline borane, and sodium triacetoxyborohydride, and sodium triacetoxyborohydride is more preferable.

The used amount of the reducing agent is only required to be 1 to 100 times and preferably 1 to 10 times the molar amount of the compound represented by Formula [3A].

The used amount of the compound represented by Formula [4] is only required to be 0.1 to 1 times the molar amount of the compound represented by Formula [3A].

This reaction may be carried out at -30°C to 150°C preferably at 0°C to 100°C for 5 minutes to 48 hours.

(1-3)
The compound represented by Formula [5] can be produced by reacting the compound represented by Formula [3A] with the compound represented by Formula [4] in the presence of a reducing agent.

This reaction may be performed according to the production method (1-2), and the compound represented by Formula [4] is only required to be used in an amount of 1 to 10 times the molar amount of the compound represented by Formula [3A].

(1-4)
The compound represented by Formula [1] can be produced by reacting the compound represented by Formula [3B] with the compound represented by Formula [5] in the presence of a reducing agent.

This reaction may be performed according to the production method (1-2), and the compound represented by Formula [3B] is only required to be used in an amount of 1 to 10 times the molar amount of the compound represented by Formula [5].

### [Production method 2]

A method for producing the compound represented by Formula [2] from the compound represented by Formula [6] and the compound represented by Formula [7].

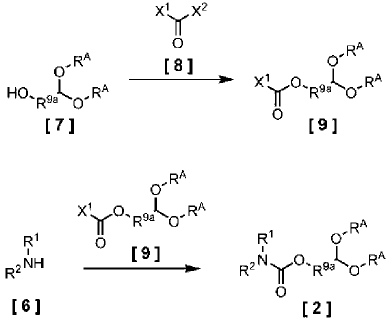

In the formulae, R¹, R², R^{9a}, and R^{A} have the same meanings as described above, and X¹ and X² each represent a leaving group.

Examples of the leaving group include a chloro group, a fluoro group, a bromo group, a trichloromethoxy group, a 4-nitro-phenoxy group, a 2,4-dinitrophenoxy group, a 2,4,6-trichlorophenoxy group, a pentafluorophenoxy group, a 2,3,5,6-tetrafluorophenoxy group, an imidazolyl group, a triazolyl group, a 3,5-dioxo-4-methyl-1,2,4-oxadiazolidinyl group, an N-hydroxysuccinimidyl group, and the like.

(2-1)
The compound represented by Formula [9] can be produced by reacting the compound represented by Formula [7] with the compound represented by Formula [8] in the presence or absence of a base.

As the compound represented by Formula [8], for example, 1,1'-carbonyldi(1,2,4-triazole), 1,1'-carbonyldiimidazole, 4-nitrophenyl chloroformate, triphosgene, phosgene, and the like are known.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

Preferred examples of the solvent include ethers, and tetrahydrofuran is more preferable.

The used amount of the solvent is not particularly limited, but is only required to be 1 to 500 times (v/w) the amount of the compound represented by Formula [7].

Examples of the base used in this reaction include an inorganic base and an organic base. As the base, an organic base is preferable. Specifically, examples thereof include triethylamine, N,N-diisopropylethylamine, 4-methylmorpholine, pyridine, 1,8-diazabicyclo[5.4.0]-7-undecene, N,N-dimethylaminopyridine, and the like.

The used amount of the base is only required to be 1 to 50 times and preferably 1 to 10 times the molar amount of the compound represented by Formula [7].

The used amount of the compound represented by Formula [8] is not particularly limited, but is only required to be 1 to 10 times the molar amount of the compound represented by Formula [7].

This reaction may be carried out at -30°C to 150°C preferably at 0°C to 100°C for 5 minutes to 48 hours.

(2-2)
The compound represented by Formula [2] can be produced by reacting the compound represented by Formula [6] with the compound represented by Formula [9] in the presence of a base.

As the compound represented by Formula [6], for example, dioctylamine and the like are known.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

Preferred examples of the solvent include nitriles, and acetonitrile is more preferable.

The used amount of the solvent is not particularly limited, but is only required to be 1 to 500 times (v/w) the amount of the compound represented by Formula [6].

Examples of the base used in this reaction include an inorganic base and an organic base. Specifically, examples thereof include potassium carbonate, sodium carbonate, lithium carbonate, potassium phosphate, sodium phosphate, lithium phosphate, triethylamine, N,N-diisopropylethylamine, 4-methylmorpholine, pyridine, 1,8-diazabicyclo[5.4.0]-7-undecene, N,N-dimethylaminopyridine, and the like.

The used amount of the base is only required to be 1 to 50 times and preferably 1 to 10 times the molar amount of the compound represented by Formula [6].

The used amount of the compound represented by Formula [9] is not particularly limited, but is only required to be 0.1 to 10 times the molar amount of the compound represented by Formula [6].

This reaction may be carried out at -30°C to 150°C preferably at 0°C to 100°C for 5 minutes to 48 hours.

### [Production method 3]

A method for producing the compound represented by Formula [6A].

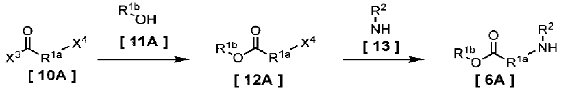

In the formulae, R², R^{1a}, and R^{1b} have the same meanings as described above, X³ represents a hydroxyl group or a leaving group, X⁴ represents a leaving group, and the leaving group has the same meaning as described above.

(3-1)
The compound represented by Formula [12A] can be produced by reacting the compound represented by Formula [10A] with the compound represented by Formula [11A] in the presence or absence of an acid, in the presence or absence of a condensing agent or an acid halide, and in the presence or absence of a base.

As the compound represented by Formula [10A], for example, 5-bromovaleric acid, chloroacetyl chloride, and the like are known.

As the compound represented by Formula [11A], for example, 2-butyl-1-octanol, 2-pentyl-1-heptanol, 1-decanol, and the like are known.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

Preferred examples of the solvent include aromatic hydrocarbons and ethers, and toluene and tetrahydrofuran are more preferable.

The used amount of the solvent is not particularly limited, but is only required to be 1 to 500 times (v/w) the amount of the compound represented by Formula [10A].

Examples of the acid used in this reaction include an inorganic acid and an organic acid. As the acid, sulfonic acids are preferable. Specifically, examples thereof include sulfuric acid, 4-toluenesulfonic acid, methanesulfonic acid, and the like.

Examples of the condensing agent used in this reaction include carbodiimides such as N,N'-dicyclohexylcarbodiimide and 1-ethyl-3-(3-dimethylaminopropyl)carbodiimide; carbonyls such as carbonyldiimidazole; acid azides such as diphenylphosphoryl azide; acid cyanides such as diethylphosphoryl cyanide; uroniums such as 2-ethoxy-1-ethoxycarbonyl-1,2-dihydroquinoline; O-benzotriazol-1-yl-1,1,3,3-tetramethyluronium=hexafluorophosphate; O-(7-azabenzotriazol-1-yl)-1,1,3,3-tetramethyluronium=hexafluorophosphate; and the like.

Examples of the acid halide used in this reaction include carboxylic acid halides such as acetyl chloride and trifluoroacetyl chloride; sulfonic acid halides such as methanesulfonyl chloride and tosyl chloride; chloroformic acid esters such as ethyl chloroformate and isobutyl chloroformate; and the like.

Examples of the base used in this reaction include an inorganic base and an organic base. An organic base is preferable. Specifically, examples thereof include triethylamine, N,N-diisopropylethylamine, 4-methylmorpholine, pyridine, 1,8-diazabicyclo[5.4.0]-7-undecene, N,N-dimethylaminopyridine, and the like.

The used amount of the base is only required to be 1 to 50 times and preferably 1 to 10 times the molar amount of the compound represented by Formula [10A].

The used amount of the compound represented by Formula [11A] is not particularly limited, but is required to be 0.8 to 10 times (v/w) the amount of the compound represented by Formula [10A].

This reaction may be carried out at -30°C to 150°C preferably at 0°C to 100°C for 5 minutes to 48 hours.

(3-2)
The compound represented by Formula [6A] can be produced by reacting the compound represented by Formula [12A] with the compound represented by Formula [13] in the presence or absence of a base and in the presence or absence of an additive.

As the compound represented by Formula [13], for example, 1-butylamine, 1-hexylamine, and the like are known.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

Preferred examples of the solvent include nitriles and ethers, and acetonitrile and tetrahydrofuran are more preferable.

The used amount of the solvent is not particularly limited, but is only required to be 1 to 500 times (v/w) the amount of the compound represented by Formula [12A].

Examples of the base used in this reaction include an inorganic base and an organic base. Specifically, examples thereof include potassium hydroxide, sodium hydroxide, lithium hydroxide, potassium carbonate, sodium carbonate, lithium carbonate, potassium phosphate, sodium phosphate, lithium phosphate, triethylamine, N,N-diisopropylethylamine, 4-methylmorpholine, pyridine, 1,8-diazabicyclo[5.4.0]-7-undecene, N,N-dimethylaminopyridine, and the like, and the potassium carbonate is more preferable.

The used amount of the base is only required to be 1 to 50 times and preferably 1 to 10 times the molar amount of the compound represented by Formula [12A].

The used amount of the compound represented by Formula [13] is not particularly limited, but is only required to be 1 to 10 times the molar amount of the compound represented by Formula [12A].

Specific examples of the additive used in this reaction include lithium iodide, sodium iodide, potassium iodide, benzyltriethylammonium iodide, benzyltriethylammonium bromide, and the like.

The used amount of the additive is only required to be 0.1 to 10 times the molar amount of the compound represented by Formula [12A].

This reaction may be carried out at -30°C to 150°C preferably at 0°C to 100°C for 5 minutes to 48 hours.

### [Production method 4]

A method for producing the compound represented by Formula [6B].

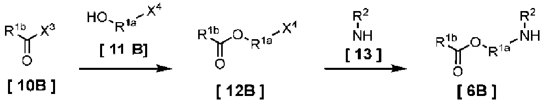

In the formulae, R², R^{1a}, R^{1b}, X³, and X⁴ have the same meanings as described above.

(4-1)
The compound represented by Formula [12B] can be produced by the same method as in the production method (3-1), except that the compound represented by Formula [10B] is used instead of the compound represented by Formula [10A] and the compound represented by Formula [11B] is used instead of the compound represented by Formula [11A].

(4-2)
The compound represented by Formula [6B] can be produced by the same method as in the production method (3-2) except that the compound represented by Formula [12B] is used instead of the compound represented by Formula [12A].

### [Production method 5]

A method for producing the compound represented by Formula [6C].

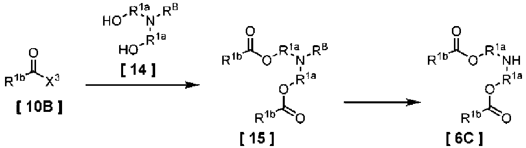

In the formulae, R^{1a}, R^{1b}, and X³ have the same meanings as described above, and R^{B} represents an amino protecting group.

(5-1)
The compound represented by Formula [15] can be produced by reacting the compound represented by Formula [10B] with the compound represented by Formula [14] in the presence or absence of an acid, in the presence or absence of a condensing agent or an acid halide, and in the presence or absence of a base.

As the compound represented by Formula [10B], for example, decanoic acid, decanoyl chloride, and the like are known.

As the compound represented by Formula [14], for example, tert-butylbis(2-hydroxyethyl)carbamate is known.

This reaction may be performed according to the production method (3-1).

(5-2)
The compound represented by Formula [6C] can be produced by deprotecting the compound represented by Formula [15].

This reaction may be performed, for example, according to the method described in T. W. Greene et al., Protective Groups in Organic Synthesis, 4th Edition, pp. 696 to 926, 2007, John Wiley & Sons, INC.

### [Production method 6]

A method for producing the compound represented by Formula [6D].

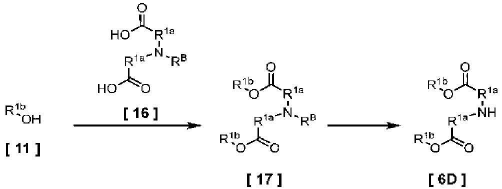

In the formulae, R^{1a}, R^{1b}, and R^{B} have the same meanings as described above.

(6-1)
The compound represented by Formula [6D] can be produced by reacting the compound represented by Formula [11] with the compound represented by Formula [16] in the presence or absence of an acid, in the presence or absence of a condensing agent or an acid halide, and in the presence or absence of a base.

As the compound represented by Formula [11], for example, 1-decanol and the like are known.

As the compound represented by Formula [16], for example, N-(tert-butoxycarbonyl)iminodiacetic acid and the like are known.

This reaction may be performed according to the production method (3-1).

(6-2)
The compound represented by Formula [6D] can be produced by deprotecting the compound represented by Formula [17].

This reaction may be performed according to the production method (3-2).

In a case where the compounds used in the above-described production methods have isomers (for example, an optical isomer, a geometric isomer, a tautomer, and the like), these isomers can also be used.

In addition, in a case where solvates, hydrates, and various forms of crystals are present, these solvates, hydrates, and various forms of crystals can also be used.

In a case where the compounds used in the above-described production methods have, for example, an amino group, a hydroxyl group, and a carboxyl group, these groups can be protected with ordinary protecting groups in advance, and these protecting groups can be eliminated by known methods after the reaction.

The compounds obtained by the above-described production methods can be transformed into other compounds by being subjected to known reaction such as condensation, addition, oxidation, reduction, transition, substitution, halogenation, dehydration, or hydrolysis, or by being subjected to these reactions that are appropriately combined.

### <Sterol>

The lipid composition in the present invention preferably contains a sterol or a derivative thereof as a non-ionizable lipid. In a case where the lipid composition according to the embodiment of the present invention contain a sterol, the fluidity of the membrane can be reduced, and hence the lipid composition can be effectively stabilized.

The sterol is not particularly limited, and examples thereof can include cholesterol, phytosterol (sitosterol, stigmasterol, fucosterol, spinasterol, brassicasterol, and the like), ergosterol, cholestanone, cholestenone, coprostanol, cholesteryl-2'-hydroxyethyl ether, and cholesteryl-4'-hydroxybutyl ether. Among these, cholesterol is preferable.

In the lipid composition, the blending amount of the sterol or the derivative thereof is preferably 30% to 70% by mole, more preferably 30% by mole to 65% by mole, and still more preferably 30% by mole to 60% by mole in terms of a molar ratio with respect to the total lipids in the lipid composition.

### <Phospholipid>

The lipid composition in the present invention preferably contains a phospholipid as a non-ionizable lipid. The phospholipid is not particularly limited, and examples thereof include phosphatidylcholine, phosphatidylethanolamine, sphingomyelin, ceramide, and the like. Among these, phosphatidylcholine is preferable. In addition, the phospholipid may be used alone or in combination with a plurality of different neutral lipids.

The phosphatidylcholine is not particularly limited, and examples thereof include soybean lecithin (SPC), hydrogenated soybean lecithin (HSPC), egg yolk lecithin (EPC), hydrogenated egg yolk lecithin (HEPC), dimyristoylphosphatidylcholine (DMPC), dipalmitoylphosphatidylcholine (DPPC), distearoylphosphatidylcholine (DSPC), 1-palmitoyl-2-oleoylphosphatidylcholine (POPC), dioleoylphosphatidylcholine (DOPC), and the like.

The phosphatidylethanolamine is not particularly limited, and examples thereof include dimyristoylphosphatidylethanolamine (DMPE), dipalmitoylphosphatidylethanolamine (DPPE), distearoylphosphatidylethanolamine (DSPE), dioleoylphosphatidylethanolamine (DOPE), dilinoleoylphosphatidylethanolamine (DLoPE), diphytanoylphosphatidylethanolamine (D(Phy)PE), 1-palmitoyl-2-oleoylphosphatidylethanolamine (POPE), ditetradecylphosphatidylethanolamine, dihexadecylphosphatidylethanolamine, dioctadecylphosphatidylethanolamine and diphytanylphosphatidylethanolamine.

The sphingomyelin is not particularly limited, and examples thereof include egg yolk-derived sphingomyelin, milk-derived sphingomyelin, and the like.

The ceramide is not particularly limited, and examples thereof include egg yolk-derived ceramide, milk-derived ceramide, and the like.

The phospholipid is preferably selected from the group consisting of distearoylphosphatidylcholine, dioleoylphosphatidylcholine, and dioleoylphosphatidylethanolamine.

In the lipid composition, the blending amount of the phospholipid is preferably 1% to 30% by mole and more preferably 1% to 20% by mole in terms of the molar ratio to the total amount of the lipids in the lipid composition.

### <Lipid having nonionic hydrophilic polymer chain>

The lipid composition in the present invention may contain a lipid having a nonionic hydrophilic polymer. In the present invention, by containing the lipid having a nonionic hydrophilic polymer, it is possible to obtain the dispersion stabilizing effect of the lipid composition.

The nonionic hydrophilic polymer is not particularly limited, and examples thereof include a nonionic vinyl-based polymer, a nonionic polyamino acid, a nonionic polyester, a nonionic polyether, a nonionic natural polymer, a nonionic modified natural polymer, and a block polymer or a graft copolymer having two or more types of these polymers as constitutional units.

Among these nonionic hydrophilic polymers, a nonionic polyether, a nonionic polyester, a nonionic polyamino acid, or a nonionic synthetic polypeptide is preferable, a nonionic polyether or a nonionic polyester is more preferable, a nonionic polyether or a nonionic monoalkoxy polyether is even more preferable, and polyethylene glycol (hereinafter, polyethylene glycol will be also called PEG) is particularly preferable. That is, the lipid having a nonionic polymer is preferably a lipid having a polyethylene glycol chain.

The lipid having a nonionic hydrophilic polymer is not particularly limited, and examples thereof include PEG-modified phosphoethanolamine, a diacylglycerol PEG derivative, a monoacylglycerol PEG derivative, a dialkylglycerol PEG derivative, a cholesterol PEG derivative, a ceramide PEG derivative, and the like. Among these, monoacylglycerol PEG derivative or diacylglycerol PEG derivative is preferable.

The lipid having a polyethylene glycol chain is particularly preferably selected from dimyristoyl-rac-glycerol polyethylene glycol, distearoyl-rac-glycerol polyethylene glycol, and distearoylphosphatidylethanolamine polyethylene glycol.

The weight-average molecular weight of the PEG chain of the nonionic hydrophilic polymer derivative is preferably 500 to 5,000 and more preferably 750 to 3,000.

The nonionic hydrophilic polymer chain may be branched or may have a substituent such as a hydroxymethyl group.

In the lipid composition, the blending amount of the lipid having a nonionic hydrophilic polymer chain is preferably 0.1 to 3 mol%, more preferably 0.3 to 3 mol%, and still more preferably 0.5 to 3 mol% with respect to the total lipids in the lipid composition in terms of molar ratio.

### <Nucleic acid>

The lipid composition contains nucleic acid.

Examples of the nucleic acid include a circular double-stranded DNA (plasmid DNA, a small circular double-stranded DNA without a drug resistance gene, or the like), a single-stranded DNA, a double-stranded DNA, a small interfering RNA (siRNA), a micro RNA (miRNA), an mRNA, an antisense oligonucleotide (also referred to as an ASO), a ribozyme, an aptamer, a saRNA, and a sgRNA, and any of these may be contained. Two or more types of nucleic acids may be used. In addition, the lipid composition may contain a modified nucleic acid. The nucleic acid is particularly preferably a circular double-stranded DNA or an RNA, and most preferably a plasmid DNA or an mRNA. The number of bases is preferably 5 to 20,000 bases.

The nucleic acid may be a sequence for gene editing, and may be an mRNA encoding a DNA nuclease.

The nucleic acid may be a sequence for gene editing, and may be a guide RNA.

The nucleic acid may be a nucleic acid mixture which is a sequence for gene editing and contains an mRNA encoding a Cas nuclease and a guide RNA. That is, the nucleic acid may be a nucleic acid for gene editing that contains an mRNA encoding a Cas nuclease and a guide RNA. The above-described mixture may further contain any donor DNA.

The nucleic acid may be a nucleic acid mixture which is a sequence for single-base editing and contains an mRNA encoding a deaminase and a mutant Cas nuclease, and a guide RNA.

The nucleic acid may be a nucleic acid mixture which is a sequence for substituting a target DNA nucleotide and contains an mRNA encoding a fusion protein of an artificial reverse transcriptase and a Cas9 endonuclease, and a prime editing guide RNA.

The nucleic acid may be a nucleic acid mixture which is a sequence for guide RNA-dependent gene transcription activation (CRISPR activator system or the like) and contains an mRNA encoding a fusion protein of an activator protein (VP64, p65, or Rta) and a mutant Cas nuclease, and a guide RNA. Alternatively, the nucleic acid may be a nucleic acid mixture which contains an mRNA encoding an activator protein (MS2, p65, or HSF1), an mRNA encoding a fusion protein of VP64 and a mutant Cas nuclease, and a guide RNA.

The nucleic acid may be a nucleic acid mixture containing a sequence for guide RNA-dependent gene transcription suppression (CRISPR interference system or the like), an mRNA encoding a fusion protein of a transcriptional suppression factor (KRAB or the like) and a mutant Cas nuclease, and a guide RNA.

The nucleic acid may be an mRNA or DNA encoding a DNA recombinase, and may be a nucleic acid mixture which further contains any donor DNA.

The nucleic acid may be an mRNA or DNA encoding a DNA recombinase, and may be a nucleic acid mixture which further contains any donor DNA. The DNA recombinase is not particularly limited, and examples thereof include a transposase (for example, Sleeping beauty transposase, piggyBac transposase, Tol2, or the like), a Cre recombinase, and a serine integrase.

The nucleic acid may be an mRNA encoding a reverse transcriptase, which is a sequence for inserting an exogenous gene into a host cell genome, and may be a nucleic acid mixture which further contains any RNA as a donor.

The nucleic acid may be an mRNA encoding a sequence for expressing an exogenous gene, or a DNA containing the above-described exogenous gene, a promoter sequence, and a terminal sequence.

The nucleic acid may be a sequence for gene editing or gene transcription suppression of a gene (target gene) present in an immune cell. The target gene is not particularly limited, and examples thereof include a T cell receptor gene (TRAC or TRBC), MHC-I (or HLA-I), MHC-II, B2M, a gene related to a self-antigen, a gene related to an inhibitory receptor or a ligand thereof (PDCD1, CD274 (or PD-L1), PDCD1LG2, LAG3, or CTLA4), a gene related to cytotoxicity (TGFBR2, PGE2, EP2, EP4, FAS, or FASLG), a gene related to cell exhaustion or differentiation (SOCS1, ZC3H12A, NR4A1, NR4A2, NR4A3, PRDM1, BLIMP1, or TIM3), a gene related to cell death (CASP3, CASP6, or CASP7), a gene related to an inflammation response (CGAS, STING, TBK1, or the like), a methylated gene (TET1, TET2, or DNMT3A), a gene related to immune escape (CD47 or NKG2A), and others (DGK, EZH2, CSF2, PAX5, LDLR, MAP4K1, CISH, CD5, CD52, ADORA2A, CD39, CD73, CD5, MCM3AP, EIF3D, CAD, HGS, RPL19, MAK16, PDGFRA, NRF1, EP400, CBLB, RPS7, CPSF4, IL2RG, RPL38, IL2RB, JAK3, MCM2, SNRPC, PSMD4, MAP4K1, BRD9, RNF20, RNF40, NFKB2, NMT1, MYB, TSC1, EIF3K, RPL19, TBX21, PRDM1, SUV39H1, or ARID1A).

The nucleic acid may be a sequence for expressing an exogenous gene that enhances the function of an immune cell, or a sequence for transcriptionally activating a gene (target gene) present in an immune cell. The exogenous gene or the target gene for transcriptional activation is not particularly limited, and examples thereof include a cytokine (IFNG, IL2, IL7, IL12, IL15, IL18, IL19, or IL21), a cytokine receptor (IL2RA, IL2RB, IL2RG, IL12B1, or IL12B2), a costimulatory factor (CD28 or TNFRSF9), a gene involved in immune evasion (CD47 or HLA-E), a metabolism-related gene (GLUT1 or PPARGC1A), a gene related to exhaustion suppression (FOXO1, TCF7, or LEF1), a gene related to cell survival (BCL2), others (a dominant negative form TGFBR2, AKT1, LTBR, AHCY, DUPD1, AKR1C4, ATF6B, ITM2A, AHNAK, BATF, GPD1, CDK2, CDK1, GPN3, MRPL51, DBI, CALML2, IL12B, IFNL2, CLIC1, HOMER1, ADA, CYP27A1, MRPL18, RAN, SLC10A7, CRLF2, VAV1, TRIM21, LHX6, FOXO4, IRX4, FOXQ1, OTUD7B, LCP2, FOSB, RAC2, FOSL1, APOBEC3D, RIPK3, EMP1, ANXA2R, CDKN2C, OTUD7A, CD2, LAT, LCP2, TBX21, or EOMES), and a sequence encoding a secreted protein that induces inflammation.

The nucleic acid may be a sequence for expressing a chimeric antigen receptor gene, a T cell receptor gene, an antibody, a bispecific antibody, a multispecific antibody, a single chain Fv (scFv), a nanobody, or a bispecific T cell engager (BiTE).

The nucleic acid may be a sequence for expressing an exogenous gene involved in cell initialization (or reprogramming). The exogenous gene is not particularly limited, and examples thereof include Oct3/4, Sox2, Klf4, C-Myc, Nanog, and Lin28.

In the lipid composition, a mass ratio of the total lipids of the lipid composition to the nucleic acid is preferably 5:1 to 1,000:1, more preferably 5:1 to 500:1, still more preferably 7:1 to 200:1, and particularly preferably 7:1 to 100:1.

### <Method for producing lipid composition>

A method for producing the lipid composition will be described.

The method for producing the lipid composition is not limited, and for example, the lipid composition can be produced by a method in which all of the constituent components of the lipid composition or some of oil-soluble components of the lipid composition are dissolved in an organic solvent or the like to form an oil phase, water-soluble components of the lipid composition are dissolved in water to form a water phase, and then the oil phase and the water phase are mixed together. A micromixer may be used for mixing, or an emulsification using an emulsifying machine such as a homogenizer, an ultrasonic emulsifying machine, a high-pressure injection emulsifying machine, or the like may be performed.

Alternatively, the lipid particles can also be produced by a method in which a lipid-containing solution is subjected to evaporation to dryness using an evaporator under reduced pressure or subjected to spray drying using a spray drier such that a dried mixture containing a lipid is prepared, and the mixture is added to an aqueous solvent and further emulsified using the aforementioned emulsifying machine or the like.

One example of the method for producing the lipid composition containing nucleic acid is a method including
a step (a) of dissolving the constituent components of the lipid composition containing the compound represented by Formula (1) in an organic solvent to obtain an oil phase, and dissolving the nucleic acid in an aqueous solvent to obtain a water phase;
a step (b) of mixing the oil phase and the water phase obtained in the step (a) to obtain a dispersion liquid of lipid particles;
a step (c) of diluting the dispersion liquid of the lipid particles obtained in the step (b);
a step (d) of removing the organic solvent from the dispersion liquid of the lipid particles; and
a step (e) of adjusting the concentration of the dispersion liquid of lipid particles.

In the step (a), the constituent components of the lipid composition containing the compound represented by Formula (1) are dissolved in an organic solvent (an alcohol such as ethanol, an ester, or the like). The total lipid concentration is not particularly limited, but is generally 1 mmol/L to 100 mmol/L, preferably 5 mmol/L to 80 mmol/L, and more preferably 10 mmol/L to 70 mmol/L.

The water phase can be obtained by dissolving a nucleic acid (for example, mRNA or the like) in water or a buffer solution. The concentration of the nucleic acid is not particularly limited, but is preferably 1 to 1,000 µg/mL and more preferably 10 to 500 µg/mL. A component such as a buffer component or an antioxidant for pH adjustment can be added as necessary. The pH of the water phase is preferably 2.0 to 7.0 and more preferably 3.0 to 6.0. Acetic acid, citric acid, malic acid, phosphoric acid, MES, HEPES, or the like is preferably used as a buffer component for adjusting the pH, and as necessary, salts such as sodium chloride and potassium chloride may be added for the purpose of adjusting a ion strength, or sugars or sugar alcohols, such as sucrose, trehalose, and mannitol may be added for the purpose of adjusting an osmotic pressure.

In the step (b), the oil phase and the water phase may be mixed by any method, and a batch type or an in-line method using a channel device may be used. As the in-line method, a microchannel device is preferably used, and as the microchannel device to be used, a Y-shaped mixer, a T-shaped mixer, a herringbone mixer, a ring micromixer, an impingement jet mixer, or the like can be used. The mixing ratio (volume ratio) of water phase:oil phase is preferably 5:1 to 1:1 and more preferably 4:1 to 2:1.

In the step (c), by mixing the dispersion liquid of lipid particles with the dilution solution, the content of the organic solvent can be reduced and the lipid particles can be stabilized. The dilution solution may be water, but the pH or ion strength may be adjusted with the dilution solution. The component contained in the dilution solution can be optionally selected depending on the purpose. For example, for the purpose of adjusting the pH, a buffer solution (for example, a citrate buffer solution, a citrate buffered physiological saline, an acetate buffer solution, an acetate buffered physiological saline, a phosphate buffered physiological saline, a Tris buffer solution, an MES buffer solution, a HEPES buffer solution, or the like) may be used. In addition, sodium chloride, potassium chloride, sucrose, trehalose, fructose, mannitol, or the like may be contained for the purpose of adjusting the ion strength or the osmotic pressure, and a buffer solution to which these additives are further added can also be used.

The dispersion liquid of lipid particles and the dilution solution may be mixed by any method, and a batch type or an in-line method using a channel device may be used. As the channel device used at the time of mixing, a Y-shaped mixer, a T-shaped mixer, or the like can be used. In addition, the time from mixing the oil phase with the water phase to mixing the dilution solution therewith is not particularly limited, but the dilution is preferably performed within 30 seconds and more preferably performed within 10 seconds after mixing the oil phase with the water phase.

The mixing ratio (liquid amount ratio) of the dispersion liquid of lipid particles to the dilution solution is preferably 1:0.5 to 1:10 and more preferably 1:1 to 1:5.

In some embodiments, in the step (c), the dispersion liquid of lipid particles may be mixed with the dilution solution a plurality of times depending on the purpose. In addition, the dilution solutions to be used may be the same as or different from each other.

In the dispersion liquid of lipid particles, the particle diameter of the lipid particles may change depending on the pH, and thus the adjustment of the pH of the dispersion liquid is important. Therefore, for example, to adjust the pH of the dispersion liquid of lipid particles after mixing with the dilution solution, a buffer solution having a concentration and a pH suitable for the adjustment or a buffer solution further containing other components may be used.

Furthermore, the plurality of dilution steps may be continuously performed, and the interval between the dilution step and the next dilution step can be optionally set, and for example, the interval may be 10 seconds, 30 seconds, 1 minute, 5 minutes, 10 minutes, 30 minutes, 1 hour, 2 hours, 3 hours, 4 hours, 6 hours, 12 hours, or 24 hours.

In addition, the pH of the dispersion liquid of lipid particles after performing the step (c) is preferably 3.0 to 10.0, more preferably 3.5 to 9.0, and particularly preferably 4.0 to 8.5.

The lipid composition can be subjected to sizing if necessary. The method of sizing is not particularly limited, and the particle size can be reduced by using an extruder or the like.

In addition, the dispersion liquid containing the lipid composition can be subjected to freezing or freeze-drying by a general method.

In the step (d), a method of removing the organic solvent from the dispersion liquid of lipid particles is not particularly limited, and a general method can be used. For example, a pH buffer solution such as phosphate buffered physiological saline or a Tris buffer solution can be used as the dialysate, and additives such as any salt or sugar can be added as necessary for the purpose of adjusting the osmotic pressure or protecting the dialysate from freezing.

In the step (e), the concentration of the dispersion liquid of lipid particles obtained in the step (d) can be adjusted. In a case of diluting the dispersion liquid, the dispersion liquid can be diluted to an appropriate concentration by using a solution such as phosphate buffered physiological saline, physiological saline, a Tris buffer solution, or a sucrose-containing Tris buffer solution as a dilution solution. In a case of concentrating the dispersion liquid, the dispersion liquid obtained in the step (d) can be concentrated by ultrafiltration using an ultrafiltration membrane, or the like. It is preferable to use the concentrated dispersion as it is. Alternatively, it is preferable to adjust the concentrated dispersion liquid to a desired concentration by using the aforementioned diluent after the concentration.

In addition, in some embodiments, the organic solvent removal step (step (d)) and the concentration adjustment step (step (e)) can be continuously performed using tangential flow filtration (TFF). In the present step, the organic solvent removal step and the concentration adjustment step may be performed in any order. The organic solvent removal step and the concentration adjustment step may be each performed a plurality of times as necessary.

As the solution that can be used in the dialysis in the step (d) or the dilution in the step (e), an excipient, a freeze protective agent, a buffering agent, or an antioxidant may be added. The excipient or the freeze protective agent is not particularly limited, and examples thereof include sugars and sugar alcohols. Examples of the sugars include sucrose, trehalose, maltose, glucose, lactose, fructose, and the like, and examples of the sugar alcohols include mannitol, sorbitol, inositol, xylitol, and the like. The buffering agent is not particularly limited, and examples thereof include ACES, BES, bicine, CAPS, CHES, DIPSO, EPPS, HEPES, HEPPSO, MES, MOPS, MOPSO, TAPS, TAPSO, TES, tricine, tris, phosphoric acid, acetic acid, citric acid, and the like. Examples of the antioxidant include EDTA, ascorbic acid, tocopherol, and the like.

The dispersion liquid of the lipid particles may be subjected to sterile filtration. As a filtration method, a hollow fiber membrane, a reverse osmosis membrane, a membrane filter, or the like can be used to remove insoluble substances from the dispersion liquid of lipid particles. In the present invention, the filtration method is not particularly limited, but it is preferable to filter the dispersion liquid through a filter having a pore diameter capable of sterilization (preferably a filtration sterilization filter with a pore diameter of 0.2 µm).

In addition, it is preferable to perform the sterile filtration after the step (d) or the step (e).

Furthermore, as necessary, the dispersion liquid of the lipid particles can be subjected to freezing or freeze-drying. The dispersion liquid of the lipid particles can be subjected to freezing or freeze-drying by a general method, and the method is not particularly limited.

In addition, the method for producing a nucleic acid delivery agent is not particularly limited, and examples thereof include a method including a step of preparing lipid particles not containing nucleic acid using an ionizable lipid that is a compound represented by Formula (1), a non-ionizable lipid, and a lipid having a nonionic polymer, and a step of mixing the lipid particles not containing nucleic acid with the nucleic acid.

The lipid particles not containing a nucleic acid can be manufactured by dissolving all or some of the components of the lipid particles not containing a nucleic acid, which are oil-soluble components, in an organic solvent or the like to form an oil phase, and mixing the oil phase with a water phase. A micromixer may be used for mixing, or an emulsification using an emulsifying machine such as a homogenizer, an ultrasonic emulsifying machine, a high-pressure injection emulsifying machine, or the like may be performed.

Alternatively, the lipid particles can also be produced by a method in which a lipid-containing solution is subjected to evaporation to dryness using an evaporator under reduced pressure or subjected to spray drying using a spray drier such that a dried mixture containing a lipid is prepared, and the mixture is added to an aqueous solvent and further emulsified using the aforementioned emulsifying machine or the like.

One of the examples of the method for producing the lipid particles containing a nucleic acid is a method including
a step (A) of dissolving the constituent components of the lipid particles containing the compound in an organic solvent to obtain an oil phase,
a step (B) of mixing the oil phase obtained in the step (A) with a water phase to obtain a dispersion liquid of lipid particles,
a step (C) of diluting the dispersion liquid obtained in the step (B),
a step (D) of removing the organic solvent from the dispersion liquid of the lipid particles, and
a step (E) of adjusting the concentration of the dispersion liquid of lipid particles.

In the step (A), the components of the lipid particles not containing a nucleic acid are dissolved in an organic solvent (an alcohol such as ethanol or an ester). The total lipid concentration is not particularly limited, but is generally 1 mmol/L to 100 mmol/L, preferably 5 mmol/L to 50 mmol/L, and more preferably 10 mmol/L to 30 mmol/L.

In the step (B), the oil phase and the water phase may be mixed by any method, and a batch type or an in-line method using a channel device may be used. As the in-line method, a microchannel device is preferably used, and as the microchannel device to be used, a Y-shaped mixer, a T-shaped mixer, a herringbone mixer, a ring micromixer, an impingement jet mixer, or the like can be used. The mixing ratio (volume ratio) of water phase:oil phase is preferably 5:1 to 1:1 and more preferably 4:1 to 2:1.

A component such as a buffer component or an antioxidant for pH adjustment can be added as necessary. The pH of the water phase is preferably 2.0 to 7.0 and more preferably 3.0 to 6.0. Acetic acid, citric acid, malic acid, phosphoric acid, MES, HEPES, or the like is preferably used as a buffer component for adjusting the pH, and as necessary, salts such as sodium chloride and potassium chloride may be added for the purpose of adjusting an ion strength, or sugars or sugar alcohols, such as sucrose, trehalose, and mannitol may be added for the purpose of adjusting an osmotic pressure.

In the step (C), by mixing the dispersion liquid of lipid particles with the dilution solution, the content of the organic solvent can be reduced and the lipid particles can be stabilized. The dilution solution may be water, but the pH or ion strength may be adjusted with the dilution solution. The component contained in the dilution solution can be optionally selected depending on the purpose. For example, for the purpose of adjusting the pH, a buffer solution (for example, a citrate buffer solution, a citrate buffered physiological saline, an acetate buffer solution, an acetate buffered physiological saline, a phosphate buffered physiological saline, a Tris buffer solution, an MES buffer solution, a HEPES buffer solution, or the like) may be used. In addition, sodium chloride, potassium chloride, sucrose, trehalose, fructose, mannitol, or the like may be contained for the purpose of adjusting the ion strength or the osmotic pressure, and a buffer solution to which these additives are further added can also be used.

The dispersion liquid of lipid particles and the dilution solution may be mixed by any method, and a batch type or an in-line method using a channel device may be used. As the channel device used at the time of mixing, a Y-shaped mixer, a T-shaped mixer, or the like can be used. In addition, the time from mixing the oil phase with the water phase to mixing the dilution solution therewith is not particularly limited, but the dilution is preferably performed within 30 seconds and more preferably performed within 10 seconds after mixing the oil phase with the water phase.

The mixing ratio (liquid amount ratio) of the dispersion liquid of lipid particles to the dilution solution is preferably 1:0.5 to 1:10 and more preferably 1:1 to 1:5.

In some embodiments, in the step (C), the dispersion liquid of lipid particles may be mixed with the dilution solution a plurality of times depending on the purpose. In addition, the dilution solutions to be used may be the same as or different from each other. In the dispersion liquid of lipid particles, the particle diameter of the lipid particles may change depending on the pH, and thus the adjustment of the pH of the dispersion liquid is important. Therefore, for example, to adjust the pH of the dispersion liquid of lipid particles after mixing with the dilution solution, a buffer solution having a concentration and a pH suitable for the adjustment or a buffer solution further containing other components may be used.

Furthermore, the plurality of dilution steps may be continuously performed, and the interval between the dilution step and the next dilution step can be optionally set, and for example, the interval may be 10 seconds, 30 seconds, 1 minute, 5 minutes, 10 minutes, 30 minutes, 1 hour, 2 hours, 3 hours, 4 hours, 6 hours, 12 hours, or 24 hours.

In addition, the pH of the dispersion liquid of lipid particles after performing the step (C) is preferably 3.0 to 10.0, more preferably 3.5 to 9.0, and particularly preferably 4.0 to 8.5.

The lipid particles can be sized as necessary. The method of sizing is not particularly limited, and the particle size can be reduced by using an extruder or the like.

In addition, the dispersion liquid containing the lipid composition can be subjected to freezing or freeze-drying by a general method.

In the step (D), a method of removing the organic solvent from the dispersion liquid of lipid particles is not particularly limited, and a general method can be used. For example, a pH buffer solution such as phosphate buffered physiological saline or a Tris buffer solution can be used as the dialysate, and additives such as any salt or sugar can be added as necessary for the purpose of adjusting the osmotic pressure or protecting the dialysate from freezing.

In the step (E), the concentration of the dispersion liquid of lipid particles obtained in the step (D) can be adjusted. In a case of diluting the dispersion liquid, the dispersion liquid can be diluted to an appropriate concentration by using a solution such as phosphate buffered physiological saline, physiological saline, a Tris buffer solution, or a sucrose-containing Tris buffer solution as a dilution solution. In a case of concentrating the dispersion liquid, the dispersion liquid obtained in the step (D) can be concentrated by ultrafiltration using an ultrafiltration membrane, or the like. It is preferable to use the concentrated dispersion as it is. Alternatively, it is preferable to adjust the concentrated dispersion liquid to a desired concentration by using the aforementioned diluent after the concentration.

In addition, in some embodiments, the organic solvent removal step (step (D)) and the concentration adjustment step (step (E)) can be continuously performed using tangential flow filtration (TFF). In the present step, the organic solvent removal step and the concentration adjustment step may be performed in any order. The organic solvent removal step and the concentration adjustment step may be each performed a plurality of times as necessary.

As the solution that can be used in the dialysis in the step (D) or the dilution in the step (E), an excipient, a freeze protective agent, a buffering agent, or an antioxidant may be added. The excipient or the freeze protective agent is not particularly limited, and examples thereof include sugars and sugar alcohols. Examples of the sugars include sucrose, trehalose, maltose, glucose, lactose, fructose, and the like, and examples of the sugar alcohols include mannitol, sorbitol, inositol, xylitol, and the like. The buffering agent is not particularly limited, and examples thereof include ACES, BES, bicine, CAPS, CHES, DIPSO, EPPS, HEPES, HEPPSO, MES, MOPS, MOPSO, TAPS, TAPSO, TES, tricine, tris, phosphoric acid, acetic acid, citric acid, and the like. Examples of the antioxidant include EDTA, ascorbic acid, tocopherol, and the like.

The dispersion liquid of the lipid particles may be subjected to sterile filtration. As a filtration method, a hollow fiber membrane, a reverse osmosis membrane, a membrane filter, or the like can be used to remove insoluble substances from the dispersion liquid of lipid particles. In the present invention, the filtration method is not particularly limited, but it is preferable to filter the dispersion liquid through a filter having a pore diameter capable of sterilization (preferably a filtration sterilization filter with a pore diameter of 0.2 µm). In addition, it is preferable to perform the sterile filtration after the step (D) or the step (E).

Furthermore, as necessary, the dispersion liquid of the lipid particles not containing a nucleic acid can be subjected to freezing or freeze-drying. The dispersion liquid of the lipid particles of the present invention can be subjected to freezing or freeze-drying by a general method, and the method is not particularly limited.

Preferably, in the present invention, the lipid particles not containing a nucleic acid can be cryopreserved, and the lipid particles not containing a nucleic acid cryopreserved can be thawed before mixing with the nucleic acid.

In the present invention, a step of mixing the lipid particles not containing a nucleic acid prepared as described above with a nucleic acid is performed. The step of mixing the lipid particles not containing nucleic acid with nucleic acid is not particularly limited, and the step can be performed by any of a method of mixing liquids using a flow channel, mixing in which a liquid is made to travel in a reciprocating direction in a container, pipette mixing, stirrer mixing in a batch container, mixing in which a container is rotated to stir a content liquid, or flask agitation.

The step of mixing the lipid particles not containing nucleic acid with nucleic acid may preferably include a step of incubating the lipid particles not containing a nucleic acid and the aqueous solution containing a nucleic acid at 0°C to 30°C for 0.1 to 120 minutes, and a step of adjusting the pH of the mixture obtained above to 6.5 to 8.5.

The aqueous solution containing a nucleic acid can be obtained by dissolving a nucleic acid in water or a buffer solution. The concentration of the nucleic acid is not particularly limited, but is preferably 1 to 2,000 µg/mL and more preferably 10 to 1,000 µg/mL. A component such as a buffer component or an antioxidant for pH adjustment can be added as necessary.

In the step of mixing the lipid particles not containing nucleic acid with nucleic acid, a mass ratio of the lipid concentration to the nucleic acid concentration in the solution after the mixing is preferably 5:1 to 1,000:1, more preferably 5:1 to 500:1, still more preferably 7:1 to 200:1, and particularly preferably 7:1 to 100:1.

### <Lipid composition>

The lipid composition may be a lipid particle. The lipid particles mean particles composed of lipids, and include a composition having any structure selected from a lipid aggregate in which lipids are aggregated, a micelle, a liposome, a lipid nanoparticle (LNP), or a lipoplex. The liposome has a lipid bilayer structure and has a water phase in the inside, and includes a liposome which has a single bilayer membrane, and a multilayer liposome which has multiple layers stacked together. The present invention may include any of these liposomes. As the lipid particle, a lipid nanoparticle (LNP) is preferable.

The form of the lipid particles can be checked by electron microscopy, structural analysis using X-rays, and the like. For example, by a method using Cryo transmission electron microscopy (CryoTEM method), it is possible to check, for example, whether or not a lipid particle is, such as a liposome, a bimolecular lipid membrane structure (lamella structure) and a structure having an inner water layer, and whether or not a lipid particle has a structure having an inner core with a high electron density and packed with constituent components including a lipid. The X-ray small angle scattering (SAXS) analysis also makes it possible to check whether or not a lipid particle has a bimolecular lipid membrane structure (lamella structure).

The particle size of the lipid particles is not particularly limited, and is preferably 10 to 1,000 nm, more preferably 30 to 500 nm, and even more preferably 50 to 250 nm. The particle size of the lipid particles can be measured by a general method (for example, a dynamic light scattering method, a laser diffraction method, or the like).

### <Method for delivering nucleic acid to immune cell>

According to the present invention, there is provided a method for delivering nucleic acid to an immune cell, including bringing the nucleic acid delivery agent for an immune cell according to the embodiment of the present invention into contact with the immune cell. However, a delivery method in vivo may be excluded. That is, the nucleic acid or the like can be introduced into the immune cell by mixing the lipid composition with the nucleic acid and transfecting the immune cell in ex vivo, in vitro, or in vivo.

### <Parmaceutical use>

According to the present invention, the nucleic acid delivery agent for an immune cell according to the embodiment of the present invention can be used for a pharmaceutical use. In a case of being used for a pharmaceutical use, the nucleic acid delivery agent according to the embodiment of the present invention can be administered to a living body alone or in a mixture with a pharmaceutically acceptable carrier. In addition, in a case of being used for a pharmaceutical use, a route of administration in a case of administering the nucleic acid delivery agent is not particularly limited, and the nucleic acid delivery agent can be administered by any method.

To further deliver the nucleic acid delivery agent according to the embodiment of the present invention to the immune cell, a molecule (hereinafter, also referred to as a target molecule) targeting the immune cell may be bonded to the surface of the lipid composition. The target molecule is not particularly limited, and a low-molecular-weight molecule, a peptide, a nucleic acid, or an antibody can be used.

In addition, in a case where the target molecule is bonded to the surface of the lipid composition, the lipid composition may contain a lipid having a modification group for chemical or electrical bonding between the lipid composition and the target molecule. Examples of the lipid having the modification group include a lipid having a maleimide group and a polyethylene glycol chain.

The immune cell may be any of an activated cell or a non-activated cell, and can be optionally selected depending on a method for producing the cell.

The activation treatment refers to, for example, in a case of a T cell, performing a main stimulation signal through a TCR/CD3 complex using an anti-CD3 antibody, an anti-CD28 antibody, or the like, a co-stimulation signal through CD28, or stimulation through a lectin pathway. This activation treatment is usually performed in the presence of a cytokine signal such as IL-2, IL-7, or IL-15, and these activation treatments induce the expression of a cytokine receptor such as IL-2R or active cell proliferation. For example, the activated T cells mean T cells that have been subjected to such an activation treatment.

In addition, the non-activated cell refers to, for example, in a case of a T cell, culturing in the presence of a cytokine signal such as IL-2, IL-7, or IL-15 without performing the above-described activation treatment. However, the method is not limited to the method described here.

The immune cell is preferably a mammal-derived cell and more preferably a human-derived cell.

The immune cell is not particularly limited, and can be selected from, for example, a lymphocyte (for example, a T cell, a B cell, a natural killer cell (NK cell), an NKT cell, or an iNKT cell), a monocyte, a macrophage, a mast cell, a dendritic cell, a granulocyte (for example, a neutrophil, an eosinophil, or a basophil), a hematopoietic stem/progenitor cell, a primary immune cell, a CD3⁺ cell, a CD4⁺ cell, a CD8⁺ T cell, a regulatory T cell (Treg), a B cell, an NK cell, a natural lymphocyte, or a dendritic cell (DC).

The immune cells may be preferably selected from peripheral blood mononuclear cells (PBMC), lymphocytes, T cells, CD4⁺ cells, CD8⁺ cells, memory T cells, naive T cells, or stem cell memory T cells.

The immune cells may be primary cells or cells derived from pluripotent stem cells.

A step of adding, before bringing the nucleic acid delivery agent into contact with the immune cell, (i) an apolipoprotein and/or (ii) a protein including a cell-binding domain and a heparin-binding domain to the nucleic acid delivery agent or the immune cell may be included.

The apolipoprotein is a group of proteins that binds to a lipoprotein and acts as an activator of an enzyme group involved in the recognition of the lipoprotein or lipid metabolism, or as a coenzyme. The apolipoproteins are roughly classified into five types from apolipoprotein A to E depending on the structure and function, and some of them are classified into subclasses such as apolipoprotein A-I and C-II. In the present invention, for example, apolipoprotein E, particularly apolipoprotein E3 may be used. The origin of the apolipoprotein is not particularly limited, and an apolipoprotein of a mammal such as a human can be used.

The recombinant protein containing a cell-binding domain and a heparin-binding domain is a cell adhesion protein (such as fibronectin or vitronectin) or a recombinant protein containing only a cell-binding domain and a heparin-binding domain derived from a cell adhesion protein. The recombinant protein is preferably a recombinant protein containing only a cell-binding domain and a heparin-binding domain, and more preferably RetroNectin.

Next, the present invention will be described based on examples, but the present invention is not limited thereto.

### Examples

Unless otherwise specified, in purification by column chromatography, the automatic purification device ISOLERA (Biotage AB, Inc.), the medium pressure preparative purification device Purif-espoir-2 (Shoko Science Co., Ltd.), or the medium pressure liquid chromatograph YFLC W-prep 2XY (Yamazen Corporation) was used.

Unless otherwise specified, as a carrier in silica gel column chromatography, Chromatorex Q-Pack SI 50 (FUJI SILYSIA CHEMICAL LTD.), or high flash column WO01, WO02, WO03, WO04, or WO05 (Yamazen Corporation) was used.

For NH silica gel, Chromatorex Q-Pack NH 60 (FUJI SILYSIA CHEMICAL LTD.) was used.

NMR spectra were measured using a Bruker AVNEO400 (manufactured by Bruker Corporation) and using tetramethylsilane as an internal standard, and all δ values were shown in ppm.

MS spectra were measured using an ACQUITY SQD LC/MS System (manufactured by Waters Corporation).

### [Synthesis Example 1]

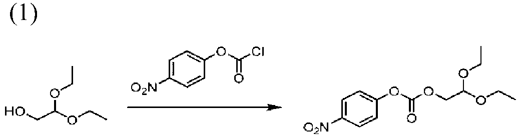

4-Nitrophenyl chloroformate (11.3 g) was added in two divided portions to a mixture of 2,2-diethoxyethanol (5.0 g), tetrahydrofuran (25 mL), and triethylamine (15.6 mL) under ice cooling, and the mixture was stirred under ice cooling for 1 hour. Water (25 mL) and hexane (25 mL) were added to the reaction mixture under ice cooling, and the organic layer was separated. The obtained organic layer was washed with water (25 mL) and saturated saline, and anhydrous sodium sulfate was then added thereto to dry the organic layer, and the solvent was distilled off under reduced pressure. The obtained residue was purified by silica gel column chromatography (ethyl acetate-hexane) to obtain 2,2-diethoxyethyl(4-nitrophenyl) carbonate (12.2 g) as a light yellow oily substance.

¹H-NMR (CDCl₃) δ: 8.30-8.26 (2H, m), 7.41-7.37 (2H, m), 4.79 (1H, t, J = 5.3 Hz), 4.28 (2H, d, J = 5.3 Hz), 3.80-3.72 (2H, m), 3.66-3.58 (2H, m), 1.26 (6H, t, J = 7.0 Hz).

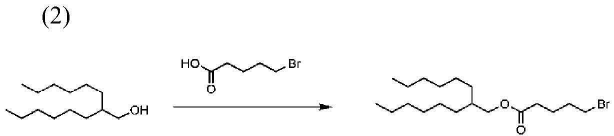

Sulfuric acid (0.5 mL) was added to a toluene (25 mL) mixture of 2-hexyl-1-octanol (5.0 g) and 5-bromovaleric acid (4.6 g), and the mixture was stirred at 110°C for 5 hours. The reaction mixture was cooled to room temperature and then purified by silica gel column chromatography (ethyl acetate-hexane) to obtain 2-hexyloctyl 5-bromopentanoate (8.2 g) as a colorless oily substance.

¹H-NMR (CDCl₃) δ: 3.98 (2H, d, J = 5.7 Hz), 3.42 (2H, t, J = 6.5 Hz), 2.34 (2H, t, J = 7.2 Hz), 1.94-1.87 (2H, m), 1.82-1.74 (2H, m), 1.65-1.57 (1H, m), 1.32-1.23 (20H, m), 0.90-0.86 (6H, m).

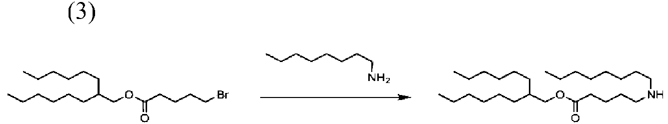

Potassium carbonate (1.3 g) was added to a mixture of 2-hexyloctyl 5-bromopentanoate (1.2 g), n-octylamine (1.2 g), and 1-methyl-2-pyrrolidone (6 mL), and the mixture was stirred at 60°C for 5 hours. The reaction mixture was cooled to room temperature, then ethyl acetate (12 mL) and water (6 mL) were added thereto, and the organic layer was separated. The organic layer was washed with saturated saline, and anhydrous sodium sulfate was then added thereto to dry the organic layer, and the solvent was distilled off under reduced pressure. The obtained residue was purified by silica gel column chromatography (hexane-ethyl acetate-methanol) to obtain 2-hexyloctyl 5-(octylamino)pentanoate (1.25 g) as a light yellow oily substance.

¹H-NMR (CDCl₃) δ: 3.96 (2H, d, J = 5.8 Hz), 2.63-2.52 (4H, m), 2.32 (2H, t, J = 7.4 Hz), 2.06-1.98 (1H, m), 1.70-1.40 (7H, m), 1.34-1.20 (30H, m), 0.90-0.87 (9H, m).

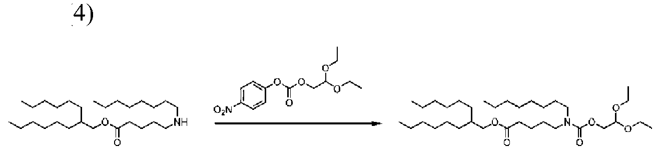

A mixture of 2-hexyloctyl 5-(octylamino)pentanoate (1.25 g), acetonitrile (4 mL), 2,2-diethoxyethyl(4-nitrophenyl) carbonate (0.49 g), and triethylamine (0.46 mL) was stirred at 60°C for 4 hours. Ethyl acetate (4 mL) and water (4 mL) were added to the reaction mixture cooled to room temperature, and the organic layer was separated. The obtained organic layer was washed with water and saturated saline, anhydrous sodium sulfate was then added thereto to dry the organic layer, and the solvent was distilled off under reduced pressure. The obtained residue was purified by silica gel column chromatography (ethyl acetate-hexane) to obtain 2-hexyloctyl 5-(((2,2-diethoxyethoxy)carbonyl)(octyl)amino)pentanoate (0.83 g) as a light yellow oily substance.

¹H-NMR (CDCl₃) δ: 4.69 (1H, t, J = 5.4 Hz), 4.08 (2H, d, J = 5.5 Hz), 3.97 (2H, d, J = 5.7 Hz), 3.74-3.62 (2H, m), 3.60-3.52 (2H, m), 3.26-3.14 (4H, m), 2.36-2.30 (2H, m), 1.65-1.46 (7H, m), 1.33-1.19 (36H, m), 0.90-0.85 (9H, m).

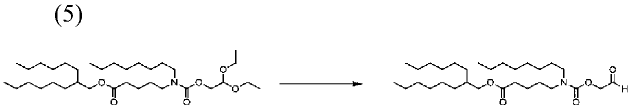

A mixture of 2-hexyloctyl 5-(((2,2-diethoxyethoxy)carbonyl)(octyl)amino)pentanoate (0.83 g), formic acid (6 mL), and water (1.5 mL) was stirred at 50°C for 3 hours, toluene was then added thereto, and the solvent was distilled off under reduced pressure. The operation of adding toluene again and distilling off the solvent under reduced pressure was repeated twice to obtain 2-hexyloctyl 5-(octyl((2-oxoethoxy)carbonyl)amino)pentanoate (0.94 g) as a crude product of a light yellow oily substance.

¹H-NMR (CDCl₃) δ: 4.10 (4H, t, J = 6.3 Hz), 3.97 (4H, d, J = 5.7 Hz), 3.25-3.11 (8H, m), 2.79 (4H, t, J = 6.4 Hz), 2.69-2.63 (2H, m), 2.56-2.47 (6H, m), 2.36-2.29 (4H, m), 1.64-1.49 (14H, m), 1.32-1.21 (60H, m), 1.01 (6H, t, J = 7.0 Hz), 0.90-0.85 (18H, m).

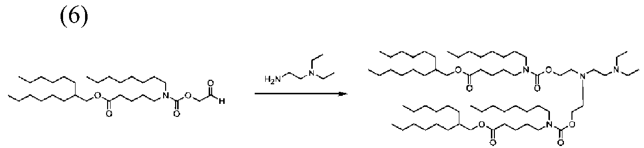

N,N-diethylethylenediamine (0.083 g), acetic acid (43 mg), and sodium triacetoxyborohydride (0.91 g) were added to an ethyl acetate (8 mL) solution of 2-hexyloctyl 5-(octyl((2-oxoethoxy)carbonyl)amino)pentanoate (0.73 g) at room temperature, and the mixture was stirred at room temperature for 5 hours. A 20% potassium carbonate aqueous solution (10 mL) was added to the reaction mixture, the organic layer was then separated and washed with water and saturated saline, anhydrous sodium sulfate was then added thereto to dry the organic layer, and the solvent was distilled off under reduced pressure. The obtained residue was purified by silica gel column chromatography (methanol-ethyl acetate-hexane), thereby obtaining bis(2-hexyloctyl) 11-(2-(diethylamino)ethyl)-6,16-dioctyl-7,15-dioxo-8,14-dioxa-6,11,16-triazahenicosanedioat e (referred to as a compound 1) (0.39 g) as a light yellow oily substance.

¹H-NMR (CDCl₃) δ: 4.10 (4H, t, J = 6.3 Hz), 3.97 (4H, d, J = 5.7 Hz), 3.25-3.11 (8H, m), 2.79 (4H, t, J = 6.4 Hz), 2.69-2.63 (2H, m), 2.56-2.47 (6H, m), 2.36-2.29 (4H, m), 1.64-1.49 (14H, m), 1.32-1.21 (60H, m), 1.01 (6H, t, J = 7.0 Hz), 0.90-0.85 (18H, m).

MS m/z (M + H): 1108.

### [Synthesis Example 2]

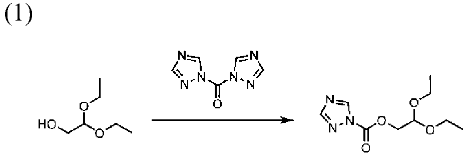

1,1'-Carbonyldi(1,2,4-triazole) (18.3 g) was added to a tetrahydrofuran (100 mL) solution of 2,2-diethoxyethanol (10.0 g), and the mixture was heated at 30°C and stirred for 1 hour. The reaction mixture was cooled to room temperature, then hexane (100 mL) and saturated aqueous sodium bicarbonate (100 mL) were added thereto, and the organic layer was separated. The obtained organic layer was washed with water (50 mL) and saturated saline, anhydrous sodium sulfate was then added thereto to dry the organic layer, and the solvent was distilled off under reduced pressure. The obtained residue was purified by silica gel column chromatography (ethyl acetate-hexane) to obtain 2,2-diethoxyethyl 1H-1,2,4-triazole-1-carboxylate (10.4 g) as a colorless oily substance.

¹H-NMR (CDCl₃) δ: 8.83 (1H, s), 8.09 (1H, s), 4.87 (1H, t, J = 5.3 Hz), 4.49 (2H, d, J = 5.3 Hz), 3.80-3.73 (2H, m), 3.66-3.56 (2H, m), 1.23 (6H, t, J = 7.0 Hz).

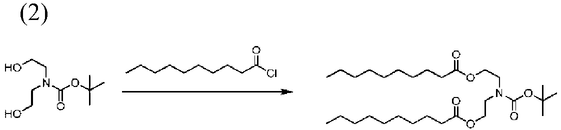

Decanoyl chloride (11 mL) was added dropwise to a tetrahydrofuran (50 mL) solution of tert-butyl N,N-bis(2-hydroxyethyl)carbamate (5.00 g) and triethylamine (8.15 mL) under ice cooling, and the mixture was stirred at room temperature for 4 hours. Hexane (50 mL) and water (50 mL) were added to the reaction mixture, and the organic layer was separated. The obtained organic layer was washed with water (50 mL) and saturated saline, anhydrous sodium sulfate was then added thereto to dry the organic layer, and the solvent was distilled off under reduced pressure to obtain yellow oily ((tert-butoxycarbonyl)azanediyl)bis(ethan-2,1-diyl)bis(decanoate) (13.1 g) as a crude product.

¹H-NMR (CDCl₃) δ: 4.21-4.13 (4H, m), 3.52-3.44 (4H, m), 2.30 (4H, t, J = 7.5 Hz), 1.67-1.55 (4H, m), 1.46 (9H, s), 1.33-1.23 (24H, m), 0.88 (6H, t, J = 6.8 Hz).

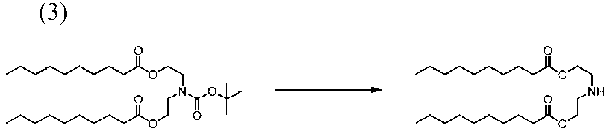

Trifluoroacetic acid (20 mL) was added to a mixture of ((tert-butoxycarbonyl)azanediyl)bis(ethan-2,1-diyl)bis(decanoate) (13.0 g) and water (1 mL) at room temperature, and the mixture was stirred overnight. The mixture was distilled off under reduced pressure, hexane (60 mL), ethyl acetate (30 mL), and a 20% potassium carbonate aqueous solution (40 mL) were added to the residue, and the organic layer was separated. Anhydrous sodium sulfate was added to the obtained organic layer, the organic layer was dried, the solvent was distilled off under reduced pressure, and the residue was purified by silica gel column chromatography (ethyl acetate-hexane) to obtain azanediylbis(ethane-2,1-diyl)bis(decanoate) (7.39 g) as a light yellow oily substance.

MS m/z (M + H): 415.

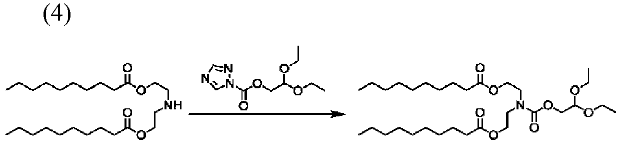

A mixture of azanediylbis(ethane-2,1-diyl)bis(decanoate) (1.00 g), 2,2-diethoxyethyl 1H-1,2,4-triazole-1-carboxylate (0.55 g), acetonitrile (4 mL), and triethylamine (1.0 mL) was stirred at 50°C for 2 hours. Ethyl acetate (4 mL) and water (4 mL) were added to the reaction mixture cooled to room temperature, and the organic layer was separated. Anhydrous sodium sulfate was added to the obtained organic layer to dry the mixture, and the solvent was distilled off under reduced pressure. The obtained residue was purified by silica gel column chromatography (ethyl acetate-hexane) to obtain (((2,2-diethoxyethoxy)carbonyl)azanediyl)bis(ethane-2,1-diyl) bis(decanoate) (0.27 g) as a colorless oily substance.

¹H-NMR (CDCl₃) δ: 4.70 (1H, t, J = 5.5 Hz), 4.24-4.16 (4H, m), 4.10 (2H, d, J = 5.5 Hz), 3.77-3.47 (8H, m), 2.30 (4H, t, J = 7.6 Hz), 1.66-1.54 (4H, m), 1.35-1.18 (30H, m), 0.92-0.83 (6H, m).

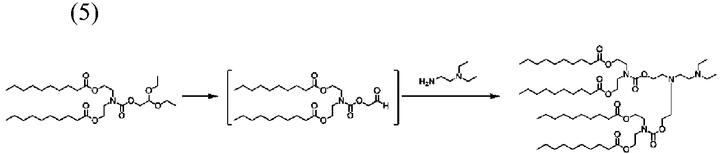

A mixture of (((2,2-diethoxyethoxy)carbonyl)azanediyl)bis(ethane-2,1-diyl) bis(decanoate) (0.27 g), formic acid (1.2 mL), and water (0.3 mL) was stirred at 30°C for 2 hours, and then the volatile components were distilled off under reduced pressure. Ethyl acetate (2.4 mL), N,N-diethylethylenediamine (27.6 mg), and sodium triacetoxyborohydride (303 mg) were added to the obtained crude (((2-oxoethoxy)carbonyl)azanediyl)bis(ethane-2,1-diyl) bis(decanoate) at room temperature, and the mixture was stirred at room temperature for 2 hours. A 20% potassium carbonate aqueous solution was added to the reaction mixture, the organic layer was then separated and washed with water and saturated saline, anhydrous sodium sulfate was then added thereto to dry the organic layer, and the solvent was distilled off under reduced pressure. The obtained residue was purified by NH silica gel column chromatography (ethyl acetate-hexane), thereby obtaining bis(2-hexyloctyl) 11-(2-(diethylamino)ethyl)-6,16-dioctyl-7,15-dioxo-8,14-dioxa-6,11,16-triazahenicosanedioat e (0.14 g) (referred to as a compound 2) as a light yellow oily substance.

¹H-NMR (CDCl₃) δ: 4.22-4.11 (12H, m), 3.56-3.49 (8H, m), 2.79 (4H, t, J = 6.4 Hz), 2.68-2.63 (2H, m), 2.54-2.47 (6H, m), 2.30 (8H, t, J = 7.5 Hz), 1.64-1.54 (8H, m), 1.33-1.22 (48H, m), 1.01 (6H, t, J = 7.1 Hz), 0.88 (12H, t, J = 6.8 Hz).

MS m/z (M + H): 1084.

### [Synthesis Example 3]

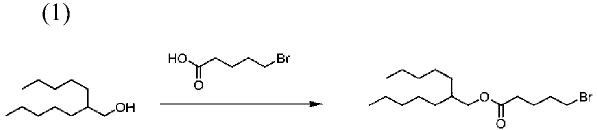

4-Toluenesulfonic acid monohydrate (168 mg) was added to a toluene (30 mL) mixture of 2-pentyl-1-heptanol (6.0 g) and 5-bromovaleric acid (6.4 g) at room temperature, and then the mixture was stirred for 2 hours with heating under reflux while removing water with a Dean-Stark apparatus. The reaction mixture was cooled to room temperature and then purified by silica gel column chromatography (ethyl acetate-hexane) to obtain 2-pentylheptyl 5-bromopentanoate (10.8 g) as a colorless oily substance.

¹H-NMR (CDCl₃) δ: 3.98 (2H, d, J = 5.8 Hz), 3.42 (2H, t, J = 6.6 Hz), 2.35 (2H, t, J = 7.2 Hz), 1.94-1.87 (2H, m), 1.82-1.74 (2H, m), 1.65-1.59 (1H, m), 1.34-1.23 (16H, m), 0.89 (6H, t, J = 6.9 Hz).

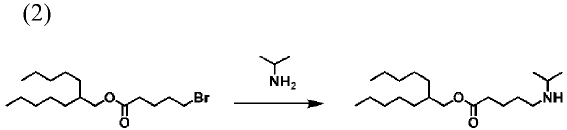

Potassium carbonate (1.45 g) was added to a mixture of 2-pentylheptyl 5-bromopentanoate (1.2 g), isopropylamine (0.65 g), and acetonitrile (6 mL), and the mixture was stirred at 60°C for 1 hour. The reaction mixture was cooled to room temperature, then ethyl acetate (24 mL) and water (12 mL) were added thereto, and the organic layer was separated. The organic layer was washed with water and saturated saline, anhydrous sodium sulfate was then added thereto to dry the organic layer, and the solvent was distilled off under reduced pressure. The obtained residue was purified by silica gel column chromatography (hexane-ethyl acetate-methanol) to obtain 2-pentylheptyl 5-(isopropylamino)pentanoate (0.69 g) as a light yellow oily substance.

¹H-NMR (CDCl₃) δ: 3.97 (2H, d, J = 5.8 Hz), 2.78 (1H, sept, J = 6.2 Hz), 2.60 (2H, t, J = 7.3 Hz), 2.33 (2H, t, J = 7.5 Hz), 1.72-1.40 (6H, m), 1.36-1.20 (16H, m), 1.05 (6H, t, J = 6.2 Hz), 0.88 (6H, t, J = 6.9 Hz).

MS m/z (M + H): 328.

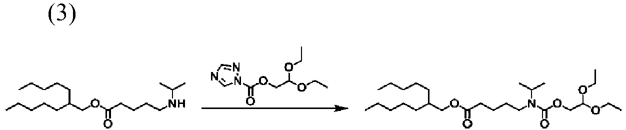

A mixture of 2-pentylheptyl 5-(isopropylamino)pentanoate (0.38 g), 2,2-diethoxyethyl 1H-1,2,4-triazole-1-carboxylate (0.27 g), acetonitrile (2 mL), triethylamine (0.33 mL), and N,N-dimethylaminopyridine (10 mg) was stirred at 60°C for 3 hours. Ethyl acetate (20 mL) and water (20 mL) were added to the reaction mixture cooled to room temperature, and the organic layer was separated. The obtained organic layer was washed with saturated aqueous ammonium chloride solution and saturated saline, anhydrous sodium sulfate was then added thereto to dry the organic layer, and the solvent was distilled off under reduced pressure. The obtained residue was purified by silica gel column chromatography (ethyl acetate-hexane) to obtain 2-pentylheptyl 5-(((2,2-diethoxyethoxy)carbonyl)(isopropyl)amino)pentanoate (0.55 g) as a colorless oily substance.

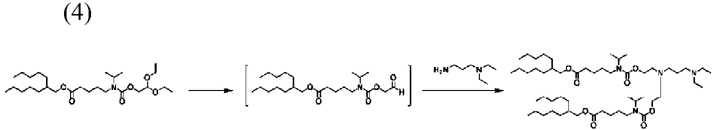

A mixture of 2-pentylheptyl 5-(((2,2-diethoxyethoxy)carbonyl)(isopropyl)amino)pentanoate (0.55 g), formic acid (2.2 mL), and water (0.55 mL) was stirred at 40°C for 2 hours. Ethyl acetate and water were added to the reaction mixture cooled to room temperature, and the organic layer was washed twice with saturated aqueous sodium bicarbonate. Anhydrous sodium sulfate was added to the obtained organic layer to dry the mixture, and the solvent was distilled off under reduced pressure. Ethyl acetate (1.5 mL), N,N-diethylpropane-1,3-diamine (23 mg), and sodium triacetoxyborohydride (0.23 g) were added to the obtained crude 2-pentylheptyl 5-(isopropyl((2-oxoethoxy)carbonyl)amino)pentanoate (0.15 g) at room temperature, and the mixture was stirred at room temperature for 1 hour. A 10% potassium carbonate aqueous solution was added to the reaction mixture, the organic layer was then separated and washed with water and saturated saline, anhydrous sodium sulfate was then added thereto to dry the organic layer, and the solvent was distilled off under reduced pressure. The obtained residue was purified by silica gel column chromatography (hexane-ethyl acetate-methanol) and NH silica gel column chromatography (ethyl acetate-hexane) to obtain bis(2-pentylheptyl) 11-(3-(diethylamino)propyl)-6,16-diisopropyl-7,15-dioxo-8,14-dioxa-6,11,16-triazahenicosane dioate (0.108 g) (referred to as a compound 3) as a colorless oily substance.

¹H-NMR (CDCl₃) δ: 4.30-4.05 (2H, m), 4.11 (4H, t, J = 6.4 Hz), 3.97 (4H, d, J = 5.8 Hz), 3.16-3.00 (4H, m), 2.77 (4H, t, J = 6.4 Hz), 2.57-2.47 (6H, m), 2.43-2.39 (2H, m), 2.32 (4H, t, J = 7.0 Hz), 1.66-1.53 (12H, m), 1.35-1.21 (32H, m), 1.13 (12H, d, J = 6.8 Hz), 1.00 (6H, t, J = 7.1 Hz), 0.88 (12H, t, J = 6.9 Hz).

MS m/z (M + H): 926.

### [Synthesis Example 4]

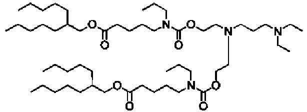

Bis(2-pentylheptyl) 11-(3-(diethylamino)propyl)-7,15-dioxo-6,16-dipropyl-8,14-dioxa-6,11,16-triazahenicosanedi oate (referred to as a compound 4) as a colorless oily substance was obtained in the same manner as in Synthesis Example 3, except that in Synthesis Example 3(2), n-propylamine was used instead of isopropylamine.

¹H-NMR (CDCl₃) δ: 4.10 (4H, t, J = 6.4 Hz), 3.97 (4H, d, J = 5.8 Hz), 3.26-3.09 (8H, m), 2.76 (4H, t, J = 6.4 Hz), 2.57-2.48 (6H, m), 2.43-2.39 (2H, m), 2.34-2.31 (4H, m), 1.66-1.49 (16H, m), 1.36-1.21 (32H, m), 1.01 (6H, t, J = 7.1 Hz), 0.90-0.84 (18H, m).

MS m/z (M + H): 926.

### [Synthesis Example 5]

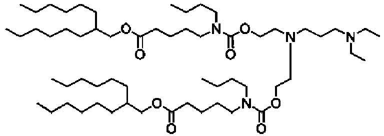

Bis(2-hexyloctyl) 6,16-dibutyl-11-(3-(diethylamino)propyl)-7,15-dioxo-8,14-dioxa-6,11,16-triazahenicosanedioa te (referred to as a compound 5) as a colorless oily substance was obtained in the same manner as in Synthesis Example 3, except that in Synthesis Example 3(2), 2-hexyloctyl 5-bromopentanoate was used instead of 2-pentylheptyl 5-bromopentanoate, and n-butylamine was used instead of isopropylamine.

¹H-NMR (CDCl₃) δ: 4.09 (4H, t, J = 6.4 Hz), 3.96 (4H, d, J = 5.8 Hz), 3.26-3.12 (8H, m), 2.76 (4H, t, J = 6.4 Hz), 2.66-2.36 (8H, m), 2.32 (4H, t, J = 6.2 Hz), 1.66-1.43 (16H, m), 1.36-1.18 (44H, m), 1.08-0.95 (6H, m), 0.95-0.83 (18H, m).

MS m/z (M + H): 1010.

### [Synthesis Example 6]

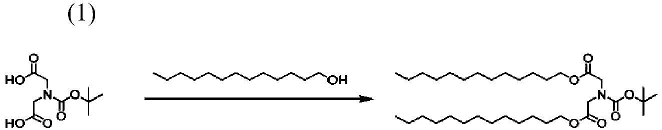

1-Tridecanol (3.44 g), triethylamine (5.98 mL), and N,N-dimethylaminopyridine (1.05 g) were added to a dichloromethane (20 mL) mixture of N-(tert-butoxycarbonyl)iminodiacetic acid (2.00 g) and 1-ethyl-3-(3-dimethylaminopropyl)carbodiimide hydrochloride (1.97 g) at room temperature, and the mixture was stirred for 10 minutes. 1-Ethyl-3-(3-dimethylaminopropyl)carbodiimide hydrochloride (1.97 g) was added to the reaction mixture, and the mixture was stirred at 40°C for 4 hours. Water (20 mL) was added to the reaction mixture, and the organic layer was separated. Ethyl acetate (20 mL) was added to the aqueous layer, the organic layer was washed with saturated saline, combined with the organic layer obtained above, anhydrous sodium sulfate was added thereto to dry the organic layer, and the solvent was distilled off under reduced pressure. The residue was purified by silica gel column chromatography (ethyl acetate-hexane) to obtain ditridecyl 2,2'-((tert-butoxycarbonyl)azanediyl)diacetate (4.26 g) as a colorless oily substance.

¹H-NMR (CDCl₃) δ: 4.17 to 3.94 (8H, m), 1.68 to 1.57 (4H, m), 1.44 (9H, s), 1.38 to 1.17 (40H, m), 0.92 to 0.84 (6H, m).

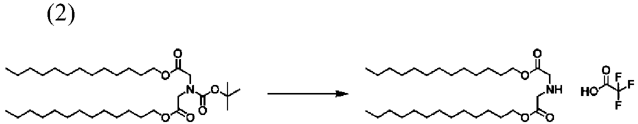

Trifluoroacetic acid (6.0 mL) was added under ice cooling to a mixture of ditridecyl 2,2'-((tert-butoxycarbonyl)azanediyl)diacetate (4.26 g), toluene (2 mL), and water (0.3 mL), and the mixture was stirred at room temperature for 1 hour and then distilled off under reduced pressure. Toluene (20 mL) was added to the residue, and the operation of distilling off under reduced pressure was repeated 3 times. Hexane (40 mL) was added to the resulting residue, and the mixture was stirred under ice cooling. The precipitated solid was collected by filtration to give the trifluoroacetic acid salt of ditridecyl 2,2'-azanediyldiacetate (4.69 g) as a white solid.

¹H-NMR (CDCl₃) δ: 5.57 (2H, brs), 4.22 (4H, t, J = 6.8 Hz), 4.00 (4H, s), 1.71 to 1.59 (4H, m), 1.39 to 1.16 (40H, m), 0.93 to 0.83 (6H, m).

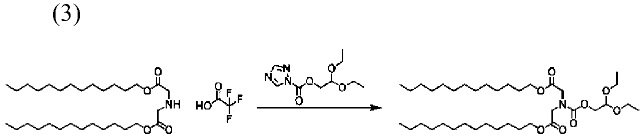

A mixture of trifluoroacetic acid salt of ditridecyl 2,2'-azanediyldiacetate (1.50 g), 2,2-diethoxyethyl 1H-1,2,4-triazole-1-carboxylate (0.56 g), acetonitrile (7.5 mL), triethylamine (1.03 mL), and N,N-dimethylaminopyridine (0.30 g) was stirred at 70°C for 3 hours and at 80°C for 1 hour. Ethyl acetate (10 mL) and water (5 mL) were added to the reaction mixture cooled to room temperature, and the organic layer was separated. The obtained organic layer was washed with saturated saline, anhydrous sodium sulfate was added thereto to dry the organic layer, and the solvent was distilled off under reduced pressure. The obtained residue was purified by silica gel column chromatography (ethyl acetate-hexane) to obtain ditridecyl 2,2-(((2,2-diethoxyethoxy)carbonyl)azanediyl)diacetate (1.06 g) as a colorless oily substance.

¹H-NMR (CDCl₃) δ: 4.66 (1H, t, J = 5.5 Hz), 4.18 to 4.06 (10H, m), 3.75 to 3.49 (4H, m), 1.68 to 1.57 (4H, m), 1.37 to 1.20 (40H, m), 1.21 (6H, t, J = 7.1 Hz), 0.92 to 0.84 (6H, m).

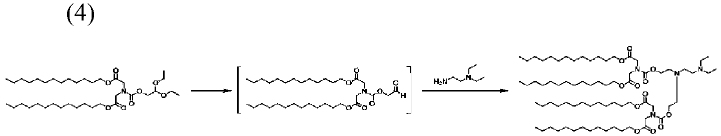

Ditridecyl 8-(2-(diethylamino)ethyl)-4,12-dioxo-3,13-bis(2-oxo-2-(tridecyloxy)ethyl)-5,11-dioxa-3,8,13-t riazapentadecanedioate (referred to as Compound 6) was obtained as a colorless oil in the same manner as in Synthesis Example 2(5), except that in Synthesis Example 2(5), ditridecyl 2,2'-(((2,2-diethoxyethoxy)carbonyl)azanediyl)diacetate was used instead of (((2,2-diethoxyethoxy)carbonyl)azanediyl)bis(ethane-2,1-diyl) bis(decanoate).

¹H-NMR (CDCl₃) δ: 4.19-4.06 (20H, m), 2.84-2.71 (4H, m), 2.68-2.56 (2H, m), 2.55-2.41 (6H, m), 1.69-1.49 (16H, m), 1.38-1.18 (72H, m), 1.07-0.95 (6H, m), 0.88 (12H, t, J = 6.8 Hz).

### [Synthesis Example 7]

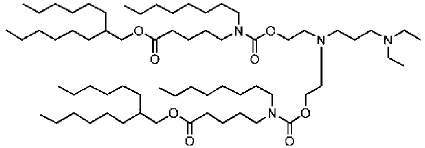

Bis(2-hexyloctyl) 11-(3-(diethylamino)propyl)-6,16-dioctyl-7,15-dioxo-8,14-dioxa-6,11,16-triazahenicosanedioa te (referred to as a compound 7) was obtained by the same method as that in Synthesis Example 3, except that in Synthesis Example 3(3), 2-hexyloctyl 5-(octylamino)pentanoate was used instead of 2-pentylheptyl 5-(isopropylamino)pentanoate.

¹H-NMR (CDCl₃) δ: 4.09 (4H, t, J = 6.4 Hz), 3.96 (4H, d, J = 5.8 Hz), 3.27-3.07 (8H, m), 2.76 (4H, t, J = 6.4 Hz), 2.67-2.36 (8H, m), 2.32 (4H, t, J = 6.4 Hz), 1.67-1.44 (16H, m), 1.36-1.16 (60H, m), 1.06-0.96 (6H, m), 0.92-0.82 (18H, m).

MS m/z (M + H): 1123.

### [Synthesis Example 8]

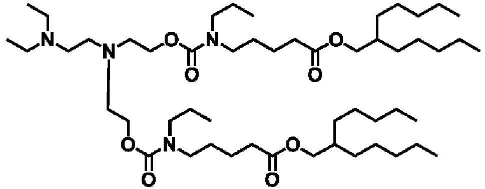

Bis(2-pentylheptyl) 11-(2-(diethylamino)ethyl)-7,15-dioxo-6,16-dipropyl-8,14-dioxa-6,11,16-triazahenicosanedioa te (compound 8) was synthesized according to Examples described in WO2024/158042A.

### [Synthesis Example 9]

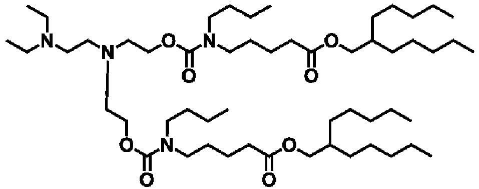

Bis(2-pentylheptyl) 6,16-dibutyl-11-(2-(diethylamino)ethyl)-7,15-dioxo-8,14-dioxa-6,11,16-triazahenicosanedioat e (compound 9) was synthesized according to Examples described in WO2024/158042A.

### [Synthesis Example 10]

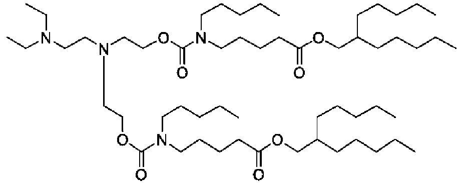

Bis(2-pentylheptyl) 11-(2-(diethylamino)ethyl)-7,15-dioxo-6,16-dipentyl-8,14-dioxa-6,11,16-triazahenicosanedioa te (compound 10) was synthesized according to Examples described in WO2024/158042A.

### [Synthesis Example 11]

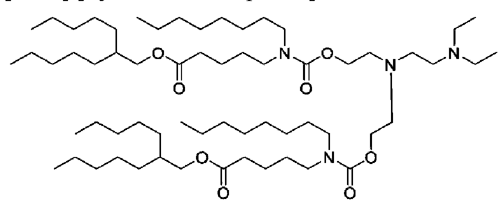

Bis(2-pentylheptyl) 11-(2-(diethylamino)ethyl)-6,16-dioctyl-7,15-dioxo-8,14-dioxa-6,11,16-triazahenicosanedioat e (compound 11) was synthesized according to Examples described in WO2024/158042A.

### [Synthesis Example 12]

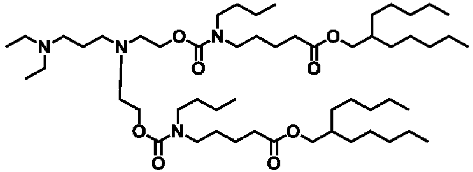

Bis(2-pentylheptyl) 6,16-dibutyl-11-(3-(diethylamino)propyl)-7,15-dioxo-8,14-dioxa-6,11,16-triazahenicosanedioa te (compound 12) was synthesized according to Examples described in WO2024/158042A.

### [Synthesis Example 13]

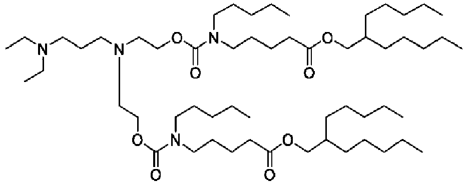

Bis(2-pentylheptyl) 11-(3-(diethylamino)propyl)-7,15-dioxo-6,16-dipentyl-8,14-dioxa-6,11,16-triazahenicosanedio ate (compound 13) was synthesized according to Examples described in WO2024/158042A.

### [Synthesis Example 14]

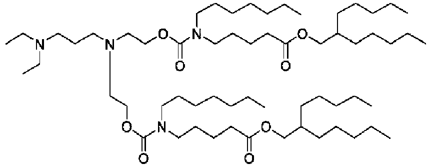

Bis(2-pentylheptyl) 11-(3-(diethylamino)propyl)-6,16-diheptyl-7,15-dioxo-8,14-dioxa-6,11,16-triazahenicosanedio ate (compound 14) was synthesized according to Examples described in WO2024/158042A.

### [Synthesis Example 15]

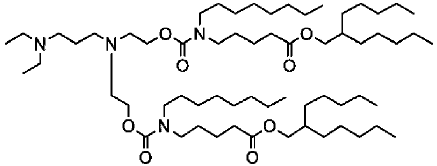

Bis(2-pentylheptyl) 11-(3-(diethylamino)propyl)-6,16-dioctyl-7,15-dioxo-8,14-dioxa-6,11,16-triazahenicosanedioa te (compound 15) was synthesized according to Examples described in WO2024/158042A.

### [Synthesis Example 16]

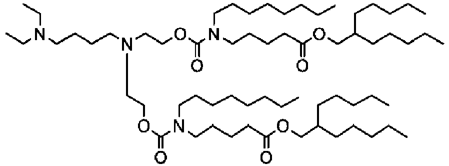

Bis(2-pentylheptyl) 11-(4-(diethylamino)butyl)-6,16-dioctyl-7,15-dioxo-8,14-dioxa-6,11,16-triazahenicosanedioat e (compound 16) was synthesized according to Examples described in WO2024/158042A.

### [Synthesis Example 17]

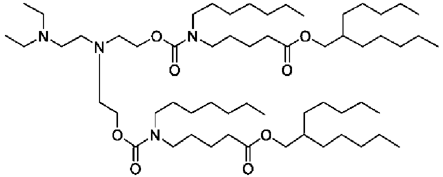

Bis(2-pentylheptyl) 11-(2-(diethylamino)ethyl)-6,16-diheptyl-7,15-dioxo-8,14-dioxa-6,11,16-triazahenicosanedioa te (compound 17) was synthesized according to Examples described in WO2024/158042A.

### [Synthesis Example 18]

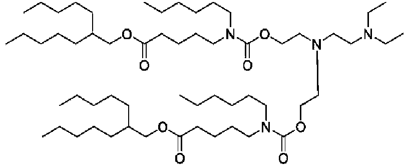

Bis(2-pentylheptyl) 11-(2-(diethylamino)ethyl)-6,16-dihexyl-7,15-dioxo-8,14-dioxa-6,11,16-triazahenicosanedioat e (compound 18) was synthesized according to Examples described in WO2024/158042A.

### [Synthesis Example 19]

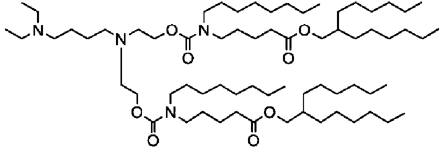

Bis(2-hexyloctyl) 11-(4-(diethylamino)butyl)-6,16-dioctyl-7,15-dioxo-8,14-dioxa-6,11,16-triazahenicosanedioat e (compound 19) was synthesized according to Examples described in WO2024/158042A.

### [Synthesis Example 20]

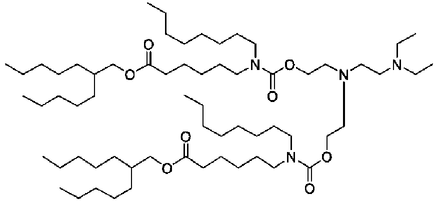

Bis(2-pentylheptyl) 12-(2-(diethylamino)ethyl)-7,17-dioctyl-8,16-dioxo-9,15-dioxa-7,12,17-triazatricosanedioate (compound 20) was synthesized according to Examples described in WO2024/158042A.

### [Synthesis Example 21]

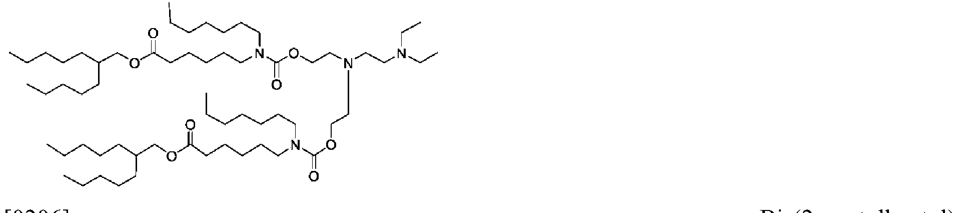

Bis(2-pentylheptyl) 12-(2-(diethylamino)ethyl)-7,17-diheptyl-8,16-dioxo-9,15-dioxa-7,12,17-triazatricosanedioate (compound 21) was synthesized according to Examples described in WO2024/158042A.

### [Synthesis Example 22]

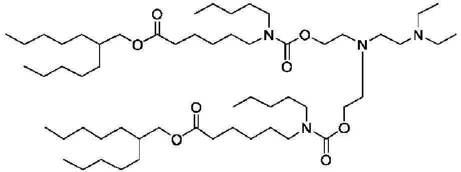

Bis(2-pentylheptyl) 12-(2-(diethylamino)ethyl)-8,16-dioxo-7,17-dipentyl-9,15-dioxa-7,12,17-triazatricosanedioate (compound 22) was synthesized according to Examples described in WO2024/158042A.

### [Synthesis Example 23]

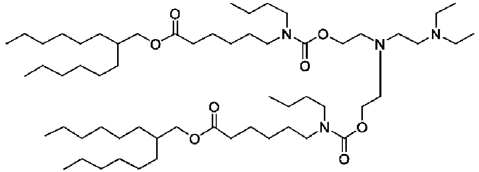

Bis(2-hexyloctyl) 7,17-dibutyl-12-(2-(diethylamino)ethyl)-8,16-dioxo-9,15-dioxa-7,12,17-triazatricosanedioate (compound 23) was synthesized according to Examples described in WO2024/158042A.

### [Synthesis Example 24]

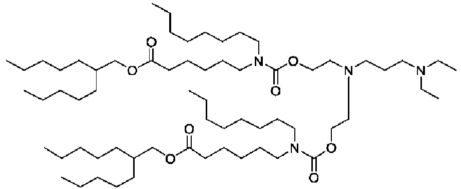

Bis(2-pentylheptyl) 12-(3-(diethylamino)propyl)-7,17-dioctyl-8,16-dioxo-9,15-dioxa-7,12,17-triazatricosanedioate (compound 24) was synthesized according to Examples described in WO2024/158042A.

### [Synthesis Example 25]

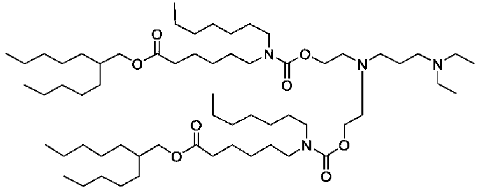

Bis(2-pentylheptyl) 12-(3-(diethylamino)propyl)-7,17-diheptyl-8,16-dioxo-9,15-dioxa-7,12,17-triazatricosanedioat e (compound 25) was synthesized according to Examples described in WO2024/158042A.

### [Synthesis Example 26]

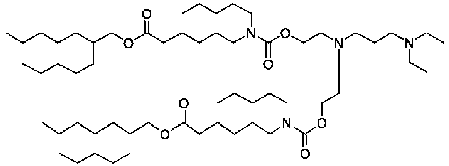

Bis(2-pentylheptyl) 12-(3-(diethylamino)propyl)-8,16-dioxo-7,17-dipentyl-9,15-dioxa-7,12,17-triazatricosanedioat e (compound 26) was synthesized according to Examples described in WO2024/158042A.

### [Synthesis Example 27]

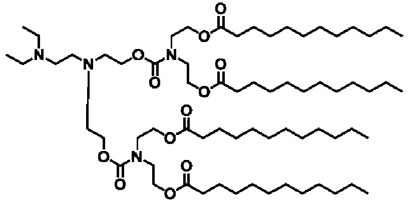

2-(2-(2-(bis(2-dodecanoyloxyethyl)carbamoyloxy)ethyl-(2-(diethylamino)ethyl)amino)ethoxy carbonyl-(2-dodecanoyloxyethyl)amino)ethyl dodecanoate (compound 27) was synthesized according to Examples described in WO2024/158042A.

### [Synthesis Example 28]

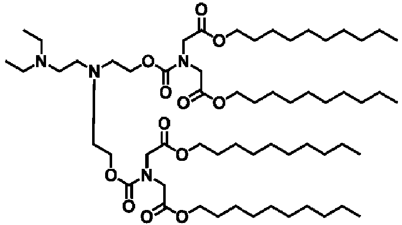

Decyl 2-(2-(2-(bis(2-deoxy-2-oxo-ethyl)carbamoyloxy)ethyl-(2-(diethylamino)ethyl)amino)ethoxyca rbonyl-(2-deoxy-2-oxo-ethyl)amino)acetate (compound 28) was synthesized according to Examples described in WO2024/158042A.

### [Synthesis Example 29]

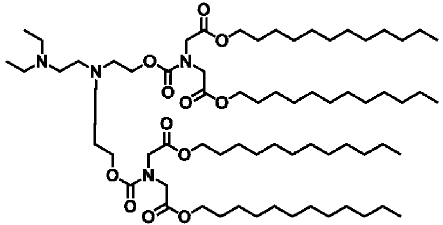

Didodecyl 8-(2-(diethylamino)ethyl)-3,13-bis(2-(dodecyloxy)-2-oxoethyl)-4,12-dioxo-5,11-dioxa-3,8,13-triazapentadecanedioate (compound 29) was synthesized according to Examples described in WO2024/158042A.

### [Synthesis Example 30]

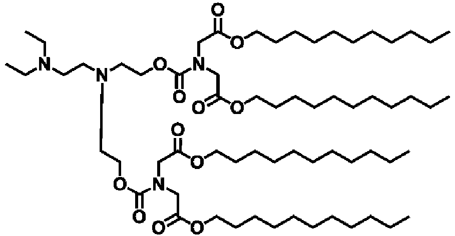

Diundecyl 8-(2-(diethylamino)ethyl)-4,12-dioxo-3,13-bis(2-oxo-2-(undecyloxy)ethyl)-5,11-dioxa-3,8,13-triazapentadecanedioate (compound 30) was synthesized according to Examples described in WO2024/158042A.

### [Synthesis Example 31]

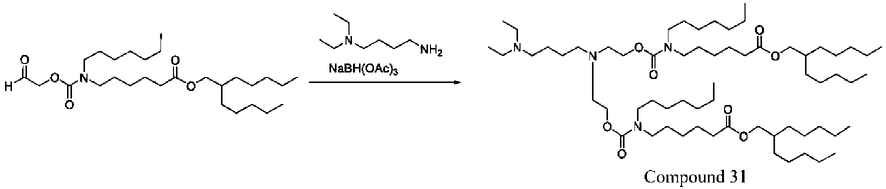

A solution of ethyl acetate (10 mL) of 6-(heptyl((2-oxoethoxy)carbonyl)amino)hexanoic acid 2-pentylheptyl ester (1.0 g) synthesized according to Examples described in WO2024/158042A was cooled to 0°C, and 4-(diethylamino)butylamine (0.14 g) and sodium triacetoxyborohydride (1.3 g) were sequentially added thereto. The coolant was removed, the mixture was stirred at room temperature for 1 hour, the completion of the reaction was confirmed, and then a 10% aqueous solution of sodium hydrogen carbonate was added thereto to stop the reaction. The organic layer was washed with a 10% aqueous solution of sodium hydrogen carbonate, and then with saturated saline, and dried over anhydrous sodium sulfate, and the solvent was distilled off. The obtained residue was purified by silica gel column chromatography (ethyl acetate/methanol), and then by NH silica gel column chromatography (hexane/ethyl acetate), thereby obtaining bis(2-pentylheptyl) 12-(4-(diethylamino)butyl)-7,17-diheptyl-8,16-dioxo-9,15-dioxa-7,12,17-triazatricosanedioate (compound 31) (594 mg) as a colorless oily substance.
LC/MS
rt (min): 1.73
MS (ESI, m/z): 1080.3 [M+H]⁺

### [Synthesis Example 32]

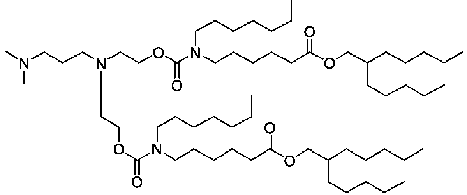

Using 6-(heptyl((2-oxoethoxy)carbonyl)amino)hexanoic acid 2-pentylheptyl and 3-(dimethylamino)propylamine, bis(2-pentylheptyl) 12-(3-(dimethylamino)propyl)-7,17-diheptyl-8,16-dioxo-9,15-dioxa-7,12,17-triazatricosanedio ate (compound 32) was synthesized by the same method as in Synthesis Example 31.
LC/MS
rt (min): 1.52
MS (ESI, m/z): 1038.3 [M+H]⁺

### [Synthesis Example 33]

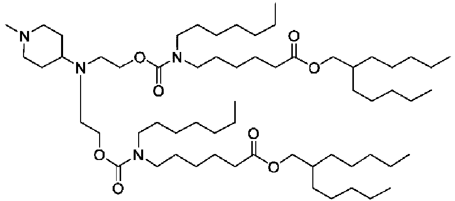

Using 6-(heptyl((2-oxoethoxy)carbonyl)amino)hexanoic acid 2-pentylheptyl and 1-methylpiperidin-4-amine, bis(2-pentylheptyl) 7,17-diheptyl-12-(1-methylpiperidin-4-yl)-8,16-dioxo-9,15-dioxa-7,12,17-triazatricosanedioat e (compound 33) was synthesized by the same method as in Synthesis Example 31.
LC/MS
rt (min): 1.77
MS (ESI, m/z): 1050.3 [M+H]⁺

### [Synthesis Example 34]

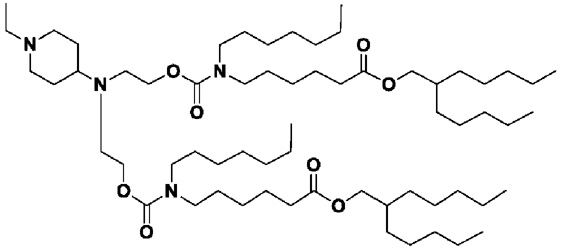

Using 6-(heptyl((2-oxoethoxy)carbonyl)amino)hexanoic acid 2-pentylheptyl and 1-ethylpiperidin-4-amine, bis(2-pentylheptyl) 12-(1-ethylpiperidin-4-yl)-7,17-diheptyl-8,16-dioxo-9,15-dioxa-7,12,17-triazatricosanedioate (compound 34) was synthesized by the same method as in Synthesis Example 31.
LC/MS
rt (min): 1.48
MS (ESI, m/z): 1065.2 [M+H]⁺

### [Synthesis Example 35]

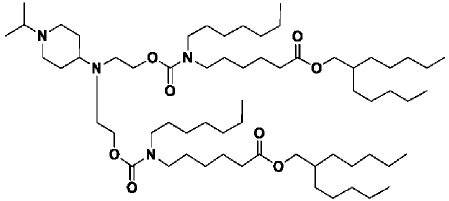

Using 6-(heptyl((2-oxoethoxy)carbonyl)amino)hexanoic acid 2-pentylheptyl and 1-isopropylpiperidin-4-amine, bis(2-pentylheptyl) 7,17-diheptyl-12-(1-isopropylpiperidin-4-yl)-8,16-dioxo-9,15-dioxa-7,12,17-triazatricosanedio ate (compound 35) was synthesized by the same method as in Synthesis Example 31.
LC/MS
rt (min): 1.52
MS (ESI, m/z): 1079.3 [M+H]⁺

### [Synthesis Example 36]

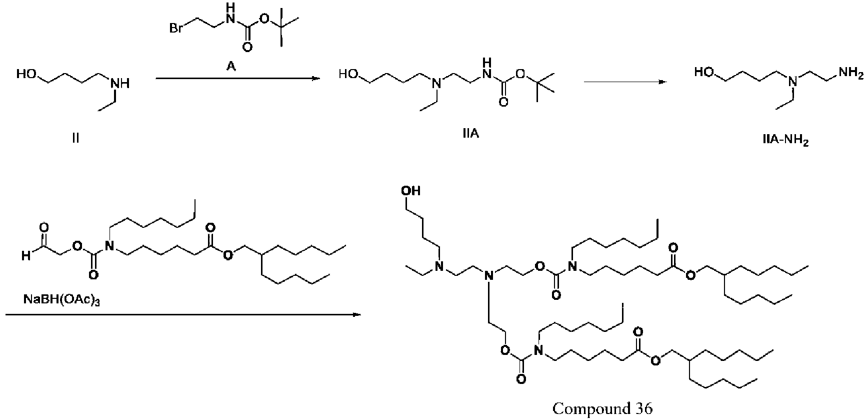

(1)
   4-(Ethylamino)butan-1-ol (II) (1.3 mL) was added to a solution of tert-butyl (2-bromoethyl)carbamate (A) (1.12 g) and potassium carbonate (2.0 g) in dimethylformamide (10 mL), and the mixture was stirred at 80°C for 30 minutes. The reaction solution was separated with ethyl acetate/1% hydrochloric acid, and the obtained aqueous layer was made basic with potassium carbonate (5 g) and then extracted with ethyl acetate. The mixture was dried over anhydrous sodium sulfate, and the solvent was distilled off to obtain tert-butyl (2-(ethyl(4-hydroxybutyl)amino)ethyl)carbamate (IIA) (1.08 g) as a colorless oily substance.
   LC/MS
   rt (min): 0.63
   MS (ESI, m/z): 261.3 [M+H]⁺
(2) Trifluoroacetic acid (5 mL) was added to tert-butyl (2-(ethyl(4-hydroxybutyl)amino)ethyl)carbamate (IIA) (650 mg), and the mixture was stirred at room temperature for 30 minutes. Trifluoroacetic acid was distilled off under reduced pressure, the obtained residue was desalted using an ion exchange resin (DIAION SA10A (Mitsubishi Chemical Corporation), regenerated to OH type), and azeotropic dehydration was carried out with ethanol to obtain 4-((2-aminoethyl)(ethyl)amino)butan-1-ol (IIA-NH₂).
   LC/MS
   rt (min): 0.19
   MS (ESI, m/z): 161.2 [M+H]⁺
(3) Using 6-(heptyl((2-oxoethoxy)carbonyl)amino)hexanoic acid 2-pentylheptyl (0.50 g) and 4-((2-aminoethyl)(ethyl)amino)butan-1-ol (IIA-NH₂) (93 mg), bis(2-pentylheptyl) 12-(2-(ethyl(4-hydroxybutyl)amino)ethyl)-7,17-diheptyl-8,16-dioxo-9,15-dioxa-7,12,17-triaza tricosanedioate (Compound 36) (375 mg) was obtained by the same method as in Synthesis Example 31.
   LC/MS
   rt (min): 1.43
   MS (ESI, m/z): 1097.2 [M+H]⁺

### [Synthesis Examples 37 to 59]

According to the same method as in Synthesis Example 36(1) to 36(3),
using 4-(methylamino)butane-1-ol (I),
4-(ethylamino)butane-1-ol (II),
3-(methylamino)propane-1-ol (III),
3-(ethylamino)propane-1-ol (IV),
2-(methylamino)ethane-1-ol (V),
2-(ethylamino)ethane-1-ol (VI), and
tert-butyl (2-bromoethyl)carbamate (A) and
tert-butyl (3-bromopropyl)carbamate (B),
tert-butyl (2-((4-hydroxybutyl)(methyl)amino)ethyl)carbamate (IA),
tert-butyl (3-((4-hydroxybutyl)(methyl)amino)propyl)carbamate (IB),
tert-butyl (2-(ethyl(4-hydroxybutyl)amino)ethyl)carbamate (IIA),
tert-butyl (3-(ethyl(4-hydroxybutyl)amino)propyl)carbamate (IIB),
tert-butyl (2-((3-hydroxypropyl)(methyl)amino)ethyl)carbamate (IIIA),
tert-butyl (3-((3-hydroxypropyl)(methyl)amino)propyl)carbamate (IIIB),
tert-butyl (2-(ethyl(3-hydroxypropyl)amino)ethyl)carbamate (IVA),
tert-butyl (3-(ethyl(3-hydroxypropyl)amino)propyl)carbamate (IVB),
tert-butyl (2-((2-hydroxyethyl)(methyl)amino)ethyl)carbamate (VA),
tert-butyl (3-((2-hydroxyethyl)(methyl)amino)propyl)carbamate (VB),
tert-butyl (2-(ethyl(2-hydroxyethyl)amino)ethyl)carbamate (VIA), and
tert-butyl (3-(ethyl(2-hydroxyethyl)amino)propyl)carbamate (VIB) were synthesized, and
subsequently, by deprotecting the BOC group to synthesize
4-((2-aminoethyl)(methyl)amino)butane-1-ol (IA-NH₂),
4-((3-aminopropyl)(methyl)amino)butane-1-ol (IB-NH₂),
4-((2-aminoethyl)(ethyl)amino)butane-1-ol (IIA-NH₂),
4-((3-aminopropyl)(ethyl)amino)butane-1-ol (IIB-NH₂),
3-((2-aminoethyl)(methyl)amino)propane-1-ol (IIIA-NH₂),
3-((3-aminopropyl)(methyl)amino)propane-1-ol (IIIB-NH₂),
3-((2-aminoethyl)(ethyl)amino)propane-1-ol (IVA-NH₂),
3-((3-aminopropyl)(ethyl)amino)propane-1-ol (IVB-NH₂),
2-((2-aminoethyl)(methyl)amino)ethane-1-ol (VA-NH₂),
2-((3-aminopropyl)(methyl)amino)ethan-1-ol (VB-NH₂),
2-((2-aminoethyl)(ethyl)amino)ethane-1-ol (VIA-NH₂), and
2-((3-aminopropyl)(ethyl)amino)ethan-1-ol (VIB-NH₂) were synthesized, and
using the thus-obtained amines and 2-pentylheptyl 6-(heptyl((2-oxoethoxy)carbonyl)amino)hexanoate or 2-pentyl 6-(octyl((2-oxoethoxy)carbonyl)amino)hexanoate,
the compounds (Compound 37 to Compound 59) were synthesized in the same manner as in Synthesis Example 31 (Table 2).

**[Table 1]**

| No. | Structural formula | LCMS (rt, m/z) | No. | Structural formula | LCMS (rt, m/z) |
|---|---|---|---|---|---|
| IA | 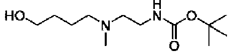 | 0.59 | IA-NH₂ | 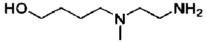 | 0.19 |
| | | 247.3 | | | 147.1 |
| IB | 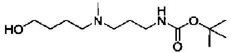 | 0.63 | IB-NH₂ | 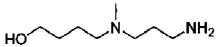 | 0.19 |
| | | 261.3 | | | 161.2 |
| IIA | 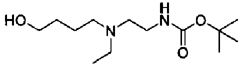 | 0.64 | IIA-NH₂ | 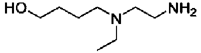 | 0.19 |
| | | 261.3 | | | 161.2 |
| IIB | 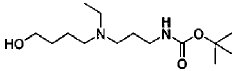 | 0.66 | IIB-NH₂ | 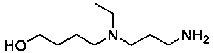 | 0.19 |
| | | 275.3 | | | 175.2 |
| IIIA | 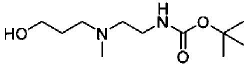 | 0.56 | IIIA-NH₂ | 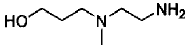 | 0.19 |
| | | 233.2 | | | 133.1 |
| IIIB | 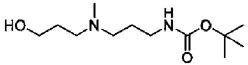 | 0.57 | IIIB-NH₂ | 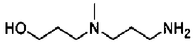 | 0.19 |
| | | 247.3 | | | 147.1 |
| IVA | 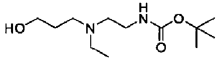 | 0.57 | IVA-NH₂ | 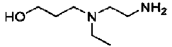 | 0.19 |
| | | 247.3 | | | 147.1 |
| IVB | 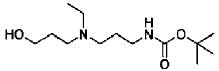 | 0.63 | IVB-NH₂ | 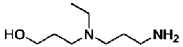 | 0.19 |
| | | 261.3 | | | 161.2 |
| VA | 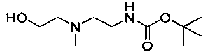 | 0.53 | VA-NH₂ | 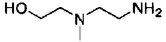 | 0.20 |
| | | 219.2 | | | 119.1 |
| VB | 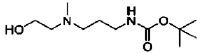 | 0.57 | VB-NH₂ | 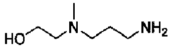 | 0.19 |
| | | 233.2 | | | 133.1 |
| VIA | 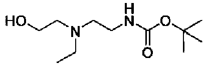 | 0.59 | VIA-NH₂ | 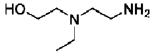 | 0.20 |
| | | 233.3 | | | 133.1 |
| VIB | 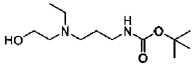 | 0.60 | VIB-NH₂ | 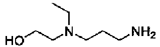 | 0.19 |
| | | 247.3 | | | 147.1 |

**[Table 2]**

| Compound No. | Structural formula | LCMS (rt, m/z) |
|---|---|---|
| Compound 37 | 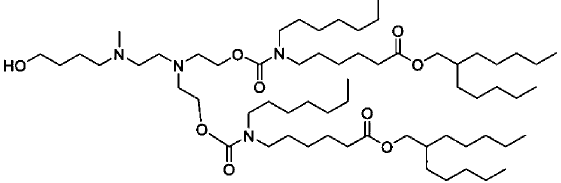 Bis(2-pentylheptyl)7,17-diheptyl-12-(2-((4-hydroxybutyl)(methyl)amino)ethyl)-8,16-dioxo-9,15-dioxa-7,12,17-triazatricosanedioate | 1.43, 1083.1 |
| Compound 38 | 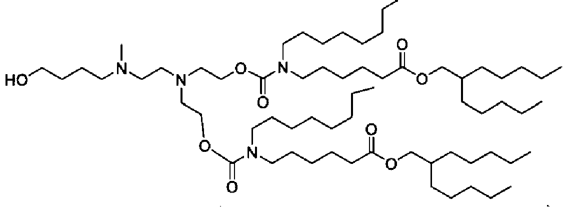 Bis(2-pentylheptyl)12-(2-((4-hydroxybutyl)(methyl) amino)ethyl)-7,17-dioctyl-8,16-dioxo-9,15-dioxa-7,12,17-triazatricosanedioate | 1.61, 1110.4 |
| Compound 39 | 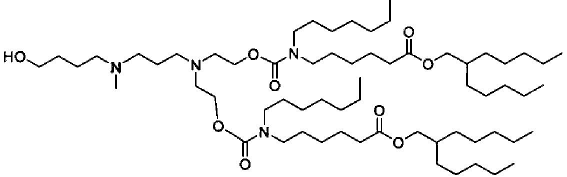 Bis(2-pentylheptyl)7,17-diheptyl-12-(3-((4-hydroxybutyl) (methyl)amino)propyl)-8,16-dioxo-9,15-dioxa-7,12,17-triazatricosanedioate | 1.43, 1097.3 |
| Compound 40 | 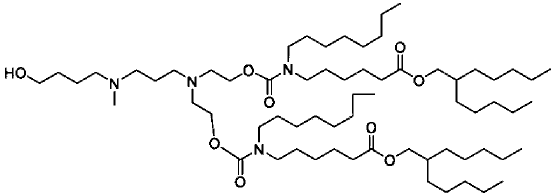 Bis(2-pentylheptyl)12-(3-((4-hydroxybutyl)(methyl) amino)propyl)-7,17-dioctyl-8,16-dioxo-9,15-dioxa-7,12,17-triazatricosanedioate | 1.57, 1124.4 |
| Compound 36 | 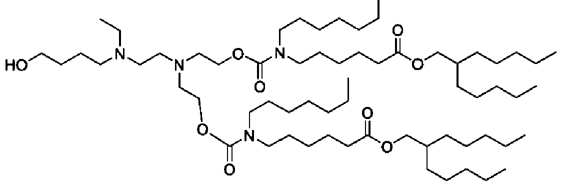 Bis(2-pentylheptyl)12-(2-(ethyl(4-hydroxybutyl)amino) ethyl)-7,17-diheptyl-8,16-dioxo-9,15-dioxa-7,12,17-triazatricosanedioate | 1.43, 1097.2 |
| Compound 41 | 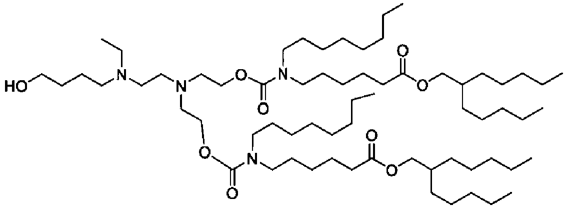 Bis(2-pentylheptyl)12-(2-(ethyl(4-hydroxybutyl)amino) ethyl)-7,17-dioctyl-8,16-dioxo-9,15-dioxa-7,12,17-triazatricosanedioate | 1.61, 1125.3 |
| Compound 42 | 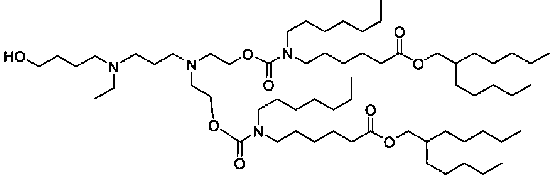 Bis(2-pentylheptyl)12-(3-(ethyl(4-hydroxybutyl)amino) propyl)-7,17-diheptyl-8,16-dioxo-9,15-dioxa-7,12,17-triazatricosanedioate | 1.41, 1111.3 |
| Compound 43 | 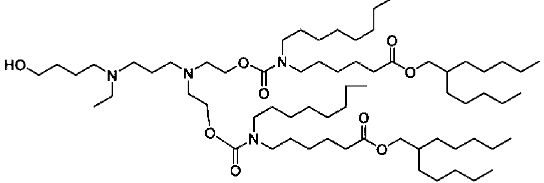 Bis(2-pentylheptyl)12-(3-(ethyl(4-hydroxybutyl)amino) propyl)-7,17-dioctyl-8,16-dioxo-9,15-dioxa-7,12,17-triazatricosanedioate | 1.63, 1139.3 |
| Compound 44 | 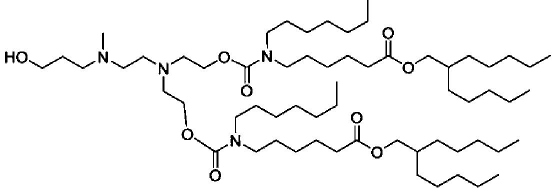 Bis(2-pentylheptyl)7,17-diheptyl-12-(2-((3-hydroxypropyl)(methyl)amino)ethyl)-8,16-dioxo-9,15-dioxa-7,12,17-triazatricosanedioate | 1.38, 1069.2 |
| Compound 45 | 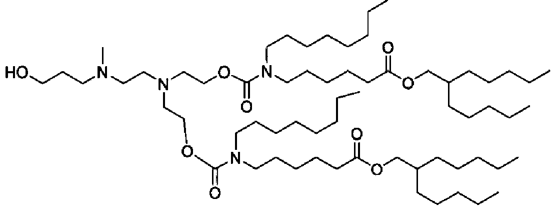 Bis(2-pentylheptyl)12-(2-((3-hydroxypropyl)(methyl) amino)ethyl)-7,17-dioctyl-8,16-dioxo-9,15-dioxa-7,12,17-triazatricosanedioate | 1.59, 1097.1 |
| Compound 46 | 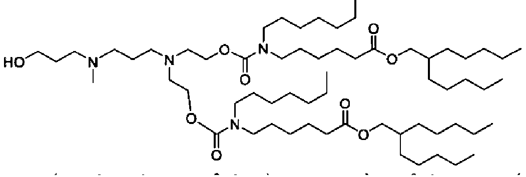 Bis(2-pentylheptyl)7,17-diheptyl-12-(3-((3-hydroxypropyl)(methyl)amino)propyl)-8,16-dioxo-9,15-dioxa-7,12,17-triazatricosanedioate | 1.40, 1083.3 |
| Compound 47 | 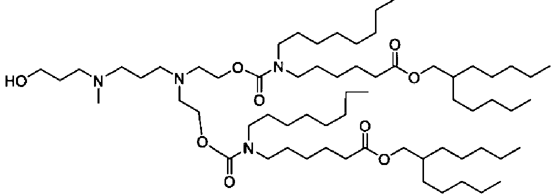 Bis(2-pentylheptyl)12-(3-((3-hydroxypropyl)(methyl) amino)propyl)-7,17-dioctyl-8,16-dioxo-9,15-dioxa-7,12,17-triazatricosanedioate | 1.67, 1111.3 |
| Compound 48 | 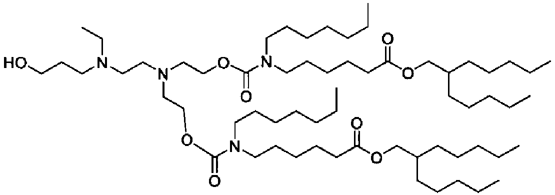 Bis(2-pentylheptyl)12-(2-(ethyl(3-hydroxypropyl)amino) ethyl)-7,17-diheptyl-8,16-dioxo-9,15-dioxa-7,12,17-triazatricosanedioate | 1.46, 1083.2 |
| Compound 49 | 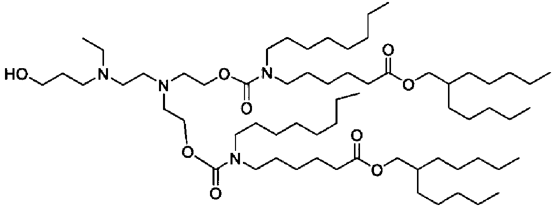 Bis(2-pentylheptyl)12-(2-(ethyl(3-hydroxypropyl)amino) ethyl)-7,17-dioctyl-8,16-dioxo-9,15-dioxa-7,12,17-triazatricosanedioate | 1.62, 1111.2 |
| Compound 50 | 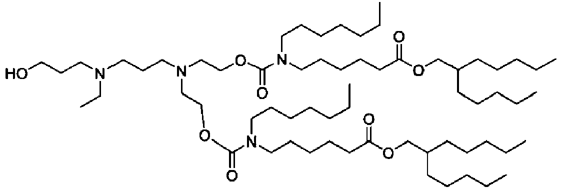 Bis(2-pentylheptyl)12-(3-(ethyl(3-hydroxypropyl)amino) propyl)-7,17-diheptyl-8,16-dioxo-9,15-dioxa-7,12,17-triazatricosanedioate | 1.39, 1097.3 |
| Compound 51 | 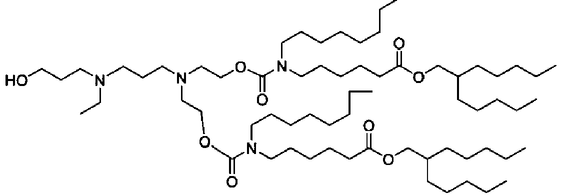 Bis(2-pentylheptyl)12-(3-(ethyl(3-hydroxypropyl)amino) propyl)-7,17-dioetyl-8,16-dioxo-9,15-dioxa-7,12,17-triazatricosanedioate | 1.69, 1124.4 |
| Compound 52 | 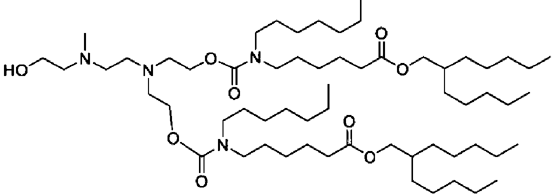 Bis(2-pentylheptyl)7,17-diheptyl-12-(2-((2-hydroxyethyl) (methyl)amino)ethyl)-8,16-dioxo-9,15-dioxa-7,12,17-triazatricosanedioate | 1.45, 1055.1 |
| Compound 53 | 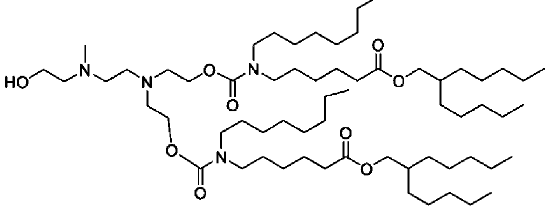 Bis(2-pentylheptyl)12-(2-((2-hydroxyethyl)(methyl) amino)ethyl)-7,17-dioctyl-8,16-dioxo-9,15-dioxa-7,12,17-triazatricosanedioate | 1.48, 1082.3 |
| Compound 54 | 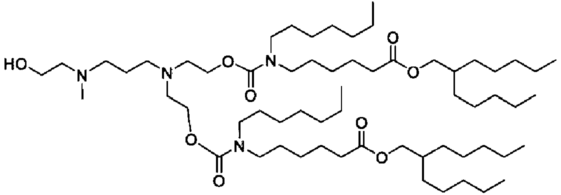 Bis(2-pentylheptyl)7,17-diheptyl-12-(3-((2-hydroxyethyl) (methyl)amino)propyl)-8,16-dioxo-9,15-dioxa-7,12,17-triazatricosanedioate | 1.44, 1069.2 |
| Compound 55 | 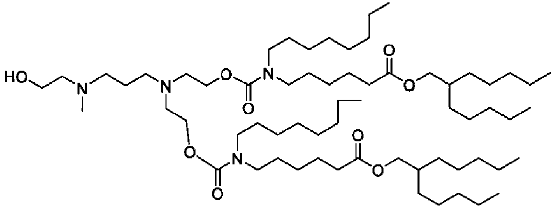 Bis(2-pentylheptyl)12-(3-((2-hydroxyethyl)(methyl) amino)propyl)-7,17-dioctyl-8,16-dioxo-9,15-dioxa-7,12,17-triazatricosanedioate | 1.61, 1096.3 |
| Compound 56 | 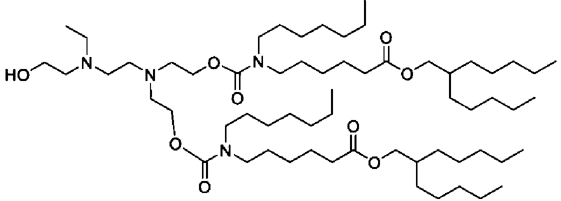 Bis(2-pentylheptyl)12-(2-(ethyl(2-hydroxyethyl)amino) ethyl)-7,17-diheptyl-8,16-dioxo-9,15-dioxa-7,12,17-triazatricosanedioate | 1.46, 1069.1 |
| Compound 57 | 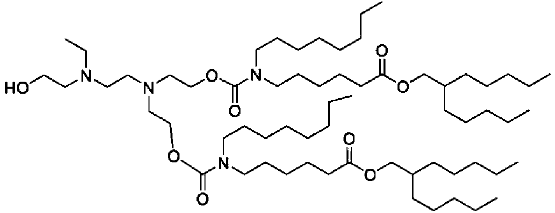 Bis(2-pentylheptyl)12-(2-(ethyl(2-hydroxyethyl)amino) ethyl)-7,17-dioctyl-8,16-dioxo-9,15-dioxa-7,12,17-triazatricosanedioate | 1.66, 1097.3 |
| Compound 58 | 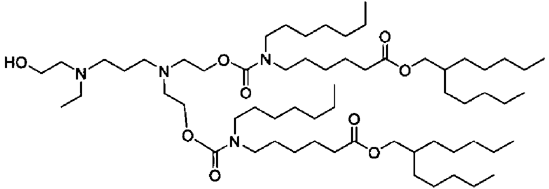 Bis(2-pentylheptyl)12-(3-(ethyl(2-hydroxyethyl)amino) propyl)-7,17-diheptyl-8,16-dioxo-9,15-dioxa-7,12,17-triazatricosanedioate | 1.41, 1083.3 |
| Compound 59 | 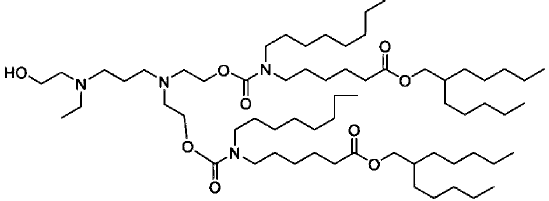 Bis(2-pentylheptyl)12-(3-(ethyl(2-hydroxyethyl)amino) propyl)-7,17-dioctyl-8,16-dioxo-9,15-dioxa-7,12,17-triazatricosanedioate | 1.59, 1111.3 |

### [Synthesis Example 60]

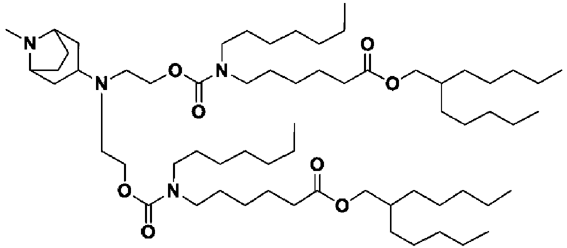

Using 2-pentylheptyl 6-(heptyl((2-oxoethoxy)carbonyl)amino)hexanoate and 8-methyl-8-azabicyclo[3.2.1]octan-3-amine, bis(2-pentylheptyl) 7,17-diheptyl-12-(8-methyl-8-azabicyclo[3.2.1]octan-3-yl)-8,16-dioxo-9,15-dioxa-7,12,17-tria zatricosanedioate (Compound 60) was synthesized in the same manner as in Synthesis Example 31.
LC/MS
rt (min): 1.52
MS (ESI, m/z): 1177.3 [M+H]⁺

### [Synthesis Example 61]

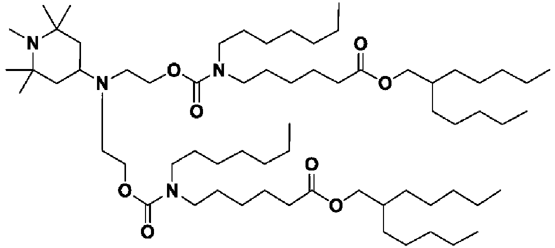

Using 2-pentylheptyl 6-(heptyl((2-oxoethoxy)carbonyl)amino)hexanoate and 1,2,2,6,6-pentamethylpiperidin-4-amine, bis(2-pentylheptyl) 7,17-diheptyl-8,16-dioxo-12-(1,2,2,6,6-pentamethylpiperidin-4-yl)-9,15-dioxa-7,12,17-triazatr icosanedioate (Compound 61) was synthesized in the same manner as in Synthesis Example 31.
LC/MS
rt (min): 1.55
MS (ESI, m/z): 1107.3 [M+H]⁺

### [Synthesis Example 62]

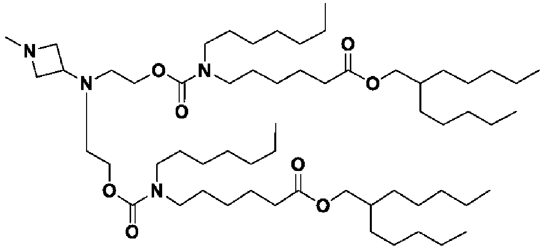

Using 2-pentylheptyl 6-(heptyl((2-oxoethoxy)carbonyl)amino)hexanoate and 1-methylazetidine-3-amine, bis(2-pentylheptyl) 7,17-diheptyl-12-(1-methylazetidin-3-yl)-8,16-dioxo-9,15-dioxa-7,12,17-triazatricosanedioate (Compound 62) was synthesized by the same method as in Synthesis Example 31.
LC/MS
rt (min): 1.46
MS (ESI, m/z): 1023.1 [M+H]⁺

### [Synthesis Example 63]

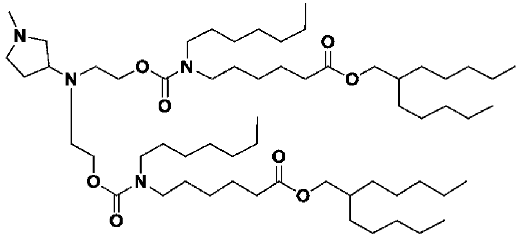

Using 2-pentylheptyl 6-(heptyl((2-oxoethoxy)carbonyl)amino)hexanoate and 1-methylpiperidin-4-amine, bis(2-pentylheptyl) 7,17-diheptyl-12-(1-methylpiperidin-4-yl)-8,16-dioxo-9,15-dioxa-7,12,17-triazatricosanedioat e (compound 63) was synthesized by the same method as in Synthesis Example 31.
LC/MS
rt (min): 1.51
MS (ESI, m/z): 1037.1 [M+H]⁺

### [Synthesis Example 64]

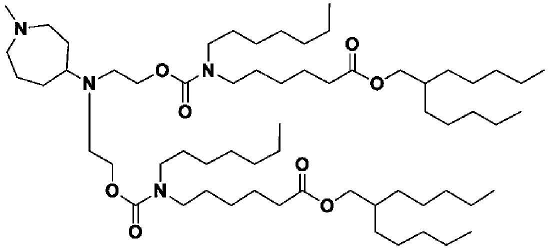

Using 2-pentylheptyl 6-(heptyl((2-oxoethoxy)carbonyl)amino)hexanoate and 1-methylazepan-4-amine, bis(2-pentylheptyl) 7,17-diheptyl-12-(1-methylazepan-4-yl)-8,16-dioxo-9,15-dioxa-7,12,17-triazatricosanedioate (Compound 64) was synthesized by the same method as in Synthesis Example 31.
LC/MS
rt (min): 1.45
MS (ESI, m/z): 1065.4 [M+H]⁺

### [Synthesis Examples 65 and 66]

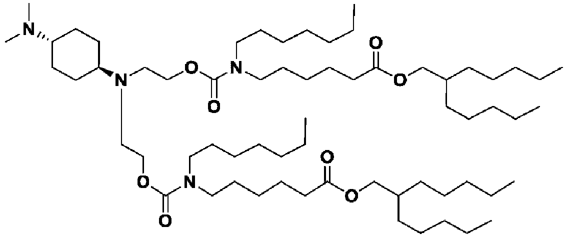

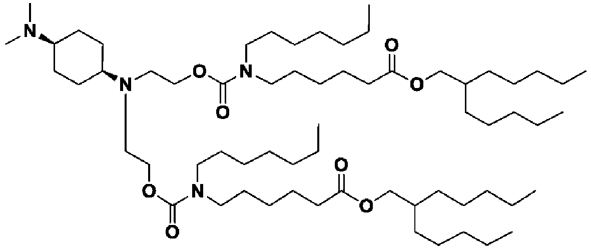

Using 2-pentylheptyl 6-(heptyl((2-oxoethoxy)carbonyl)amino)hexanoate and rac-N1,N1-dimethylcyclohexane-1,4-diamine, condensates were synthesized by the same method as in Synthesis Example 31, and two compounds having different polarities (low-polarity compound: Compound 65, high-polarity compound: Compound 66) were obtained by silica gel column chromatography.

Bis(2-pentylheptyl) 12-((1r,4r)-4-(dimethylamino)cyclohexyl)-7,17-diheptyl-8,16-dioxo-9,15-dioxa-7,12,17-triaza tricosanedioate (Compound 65).
LC/MS
rt (min): 1.46
MS (ESI, m/z): 1079.4 [M+H]⁺

Bis(2-pentylheptyl) 12-((1r,4r)-4-(dimethylamino)cyclohexyl)-7,17-diheptyl-8,16-dioxo-9,15-dioxa-7,12,17-triaza tricosanedioate (Compound 66).
LC/MS
rt (min): 1.12
MS (ESI, m/z): 1079.4 [M+H]⁺

### [Synthesis Example 67]

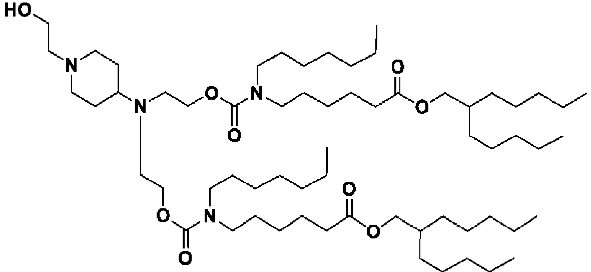

Using 2-pentylheptyl 6-(heptyl((2-oxoethoxy)carbonyl)amino)hexanoate and 2-(4-aminopiperidin-1-yl)ethan-1-ol, bis(2-pentylheptyl) 7,17-diheptyl-12-(1-(2-hydroxyethyl)piperidin-4-yl)-8,16-dioxo-9,15-dioxa-7,12,17-triazatrico sanedioate (Compound 67) was synthesized by the same method as in Synthesis Example 31.
LC/MS
rt (min): 1.49
MS (ESI, m/z): 1081.4 [M+H]⁺

### [Synthesis Example 68]

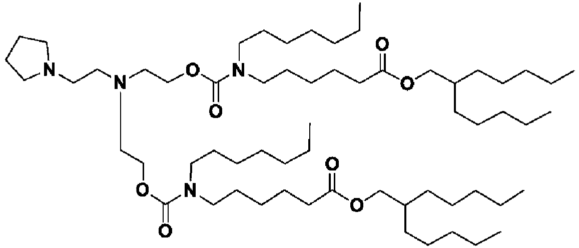

Using 2-pentylheptyl 6-(heptyl((2-oxoethoxy)carbonyl)amino)hexanoate and 2-(pyrrolidin-1-yl)ethan-1-amine, bis(2-pentylheptyl) 7,17-diheptyl-8,16-dioxo-12-(2-(pyrrolidin-1-yl)ethyl)-9,15-dioxa-7,12,17-triazatricosanedioat e (Compound 68) was synthesized by the same method as in Synthesis Example 31.
LC/MS
rt (min): 1.50
MS (ESI, m/z): 1051.1 [M+H]⁺

### [Synthesis Example 69]

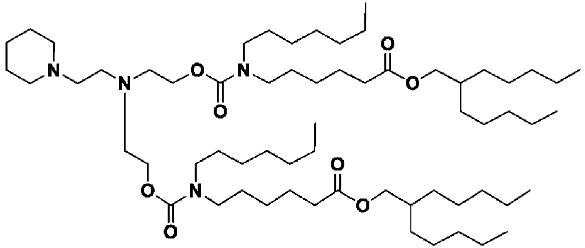

Using 2-pentylheptyl 6-(heptyl((2-oxoethoxy)carbonyl)amino)hexanoate and 2-(pyrrolidin-1-yl)ethan-1-amine, bis(2-pentylheptyl) 7,17-diheptyl-8,16-dioxo-12-(2-(pyrrolidin-1-yl)ethyl)-9,15-dioxa-7,12,17-triazatricosanedioat e (Compound 69) was synthesized by the same method as in Synthesis Example 31.
LC/MS
rt (min): 1.52
MS (ESI, m/z): 1065.1 [M+H]⁺

### [Synthesis Example 70]

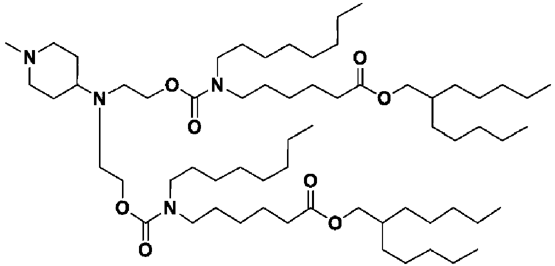

Using 2-pentylheptyl 6-(octyl((2-oxoethoxy)carbonyl)amino)hexanoate and 1-methylpiperidin-4-amine, bis(2-pentylheptyl) 12-(1-methylpiperidin-4-yl)-7,17-dioctyl-8,16-dioxo-9,15-dioxa-7,12,17-triazatricosanedioate (Compound 70) was synthesized by the same method as in Synthesis Example 31.
LC/MS
rt (min): 1.69
MS (ESI, m/z): 1079.2 [M+H]⁺

### [Synthesis Example 71]

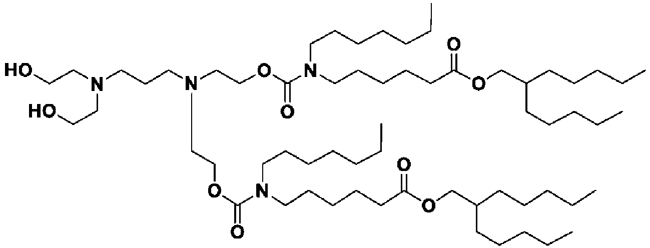

Using 2-pentylheptyl 6-(heptyl((2-oxoethoxy)carbonyl)amino)hexanoate and 2,2'-((3-aminopropyl)azanediyl)bis(ethane-1-ol), bis(2-pentylheptyl) 12-(3-(bis(2-hydroxyethyl)amino)propyl)-7,17-diheptyl-8,16-dioxo-9,15-dioxa-7,12,17-triazat ricosanedioate (Compound 71) was synthesized by the same method as in Synthesis Example 31.
LC/MS
rt (min): 1.38
MS (ESI, m/z): 1099.4 [M+H]⁺

### [Synthesis Example 72]

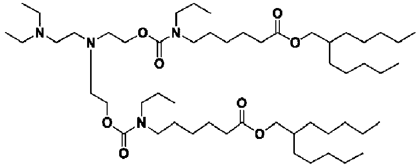

Using 2-pentylheptyl 6-(((2-oxoethoxy)carbonyl)(propyl)amino)hexanoate and 2-(diethylamino)ethylamine, bis(2-pentylheptyl) 11-(2-(diethylamino)ethyl)-7,15-dioxo-6,16-dipropyl-8,14-dioxa-6,11,16-triazahenicosanedioa te was synthesized in the same manner as in Synthesis Example 31 (Compound 72).
LC/MS
rt (min): 0.91
MS (ESI, m/z): 940.2 [M+H]⁺

### [Synthesis Example 73]

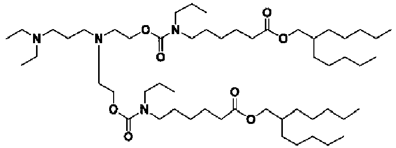

Using 2-pentylheptyl 6-(((2-oxoethoxy)carbonyl)(propyl)amino)hexanoate and 3-diethylaminopropylamine, bis(2-pentylheptyl) 12-(3-(diethylamino)propyl)-8,16-dioxo-7,17-dipropyl-9,15-dioxa-7,12,17-triazatricosanedioat e (Compound 73) was synthesized by the same method as in Synthesis Example 31.
LC/MS
rt (min): 0.91
MS (ESI, m/z): 954.2 [M+H]⁺

### [Synthesis Example 74]

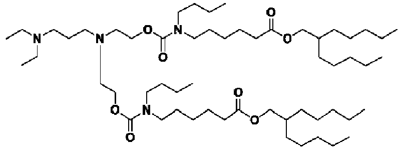

Using 2-pentylheptyl 6-(butyl((2-oxoethoxy)carbonyl)amino)hexanoate and 3-diethylaminopropylamine, bis(2-pentylheptyl) 7,17-dibutyl-12-(3-(diethylamino)propyl)-8,16-dioxo-9,15-dioxa-7,12,17-triazatricosanedioate was synthesized in the same manner as in Synthesis Example 31 (Compound 74).
LC/MS
rt (min): 0.99
MS (ESI, m/z): 982.3 [M+H]⁺

### [Test Example 1]

### <Preparation of GFP mRNA lipid particles>

The ionizable lipid, DSPC (1,2-distearoyl-sn-glycero-3-phosphocholine, product name: COATSOME (R) MC-8080, manufactured by NOF Corporation), cholesterol, and DMG-PEG2000 (1,2-dimyristoyl-rac-glycero-3-methoxypolyethylene glycol-2000, product name: SUNBRIGHT (R) GM-020, manufactured by NOF Corporation) shown in Table 3 were dissolved in ethanol at a molar ratio shown in Table 3 so that the total lipid concentration was 12.5 mmol/L, thereby obtaining an oil phase.

GFP mRNA (product name: CleanCap GFP mRNA (5 moU); manufactured by TriLink BioTechnologies, Inc.) was diluted with a 50 mmol/L citrate buffer at a pH of 4 so that the weight ratio of the total lipid concentration after mixing the oil phase and the water phase to the mRNA concentration was as shown in Table 3, thereby obtaining a water phase. Subsequently, the water phase and the oil phase were mixed using NanoAssemblr (Precision NanoSystems) so that the volume ratio of the water phase to the oil phase was water phase:oil phase = 3:1, and the mixed solution was diluted 2-fold with water to obtain a dispersion liquid of mRNA lipid particles. The dispersion liquid was dialyzed against a 20 mmol/L Tris buffer solution at pH 7.4 containing 8% sucrose using a dialysis cassette (Slide-A-Lyzer G2, MWCO: 10 kD, Thermo Fisher Scientific, Inc.) to remove ethanol, thereby obtaining mRNA-encapsulating lipid particles. The prepared sample was stored at -70°C until use.

**[Table 3]**

| <Table 3> GFP mRNA-encapsulating lipid particles | | | | | |
|---|---|---|---|---|---|
| | Compound No. | Helper lipid | Formulation of ionizable lipid/helper lipid/cholesterol/DMG-PEG2000 (mol%) | Lipid/RNA (wt/wt) | Oil-phase lipid concentration (mM) |
| Example 1 | Compound 1 | DSPC | 50/10/38.5/1.5 | 20 | 12.5 |
| Example 2 | Compound 2 | DSPC | 50/10/38.5/1.5 | 20 | 12.5 |
| Example 3 | Compound 3 | DSPC | 50/10/38.5/1.5 | 20 | 12.5 |
| Example 4 | Compound 4 | DSPC | 50/10/38.5/1.5 | 20 | 12.5 |
| Example 5 | Compound 5 | DSPC | 50/10/38.5/1.5 | 20 | 12.5 |
| Example 6 | Compound 6 | DSPC | 50/10/38.5/1.5 | 20 | 12.5 |
| Example 7 | Compound 7 | DSPC | 50/10/38.5/1.5 | 20 | 12.5 |

### <Preparation of GFP pDNA lipid particles>

The ionizable lipid, DOPE (L-α-dioleoyl phosphatidylethanolamine, product name: COATSOME (R) ME-8181, manufactured by NOF Corporation), DSPC (1,2-distearoyl-sn-glycero-3-phosphocholine, product name: COATSOME (R) MC-8080, manufactured by NOF Corporation), one phospholipid (helper lipid) selected from the phospholipids, cholesterol, and DMG-PEG2000 (1,2-dimyristoyl-rac-glycero-3-methoxypolyethylene glycol-2000, product name: SUNBRIGHT (R) GM-020, manufactured by NOF Corporation) shown in Table 4 were dissolved in ethanol at a molar ratio shown in Table 1 so that the total lipid concentration was 12.5 mmol/L, thereby obtaining an oil phase.

GFP pDNA (GenScript, custom synthesized plasmid DNA) was diluted with a 50 mmol/L citrate buffer at a pH of 4 so that the weight ratio of the total lipid concentration after mixing the oil phase and the water phase to the pDNA concentration was as shown in Table 4, thereby obtaining a water phase. Subsequently, the water phase and the oil phase were mixed using NanoAssemblr (Precision NanoSystems) so that the volume ratio of the water phase to the oil phase was water phase:oil phase = 3:1, and the mixed solution was diluted 2-fold with water to obtain a dispersion liquid of pDNA lipid particles. The dispersion liquid was dialyzed against a 20 mmol/L Tris buffer solution at pH 7.4 containing 8% sucrose using a dialysis cassette (Slide-A-Lyzer G2, MWCO: 10 kD, Thermo Fisher Scientific, Inc.) to remove ethanol, thereby obtaining GFP pDNA-encapsulating lipid particles. The prepared sample was stored at -70°C until use.

**[Table 4]**

| <Table 4> GFP pDNA-encapsulating lipid particles | | | | | |
|---|---|---|---|---|---|
| | Compound No. | Helper lipid | Formulation of ionizable lipid/helper lipid/cholesterol/DMG-PEG2000 (mol%) | Lipid/DNA (wt/wt) | Oil-phase lipid concentration (mM) |
| Example 130 | Compound 33 | DSPC | 40/5/53.5/1.5 | 20 | 12.5 |
| Example 131 | Compound 33 | DSPC | 40/10/48.5/1.5 | 20 | 12.5 |
| Example 132 | Compound 33 | DSPC | 40/20/38.5/1.5 | 20 | 12.5 |
| Example 133 | Compound 33 | DSPC | 40/30/28.5/1.5 | 20 | 12.5 |
| Example 134 | Compound 33 | DSPC | 40/40/18.5/1.5 | 20 | 12.5 |
| Example 135 | Compound 21 | DOPE | 40/5/53.5/1.5 | 20 | 12.5 |
| Example 136 | Compound 21 | DOPE | 40/10/48.5/1.5 | 20 | 12.5 |
| Example 137 | Compound 21 | DOPE | 40/20/38.5/1.5 | 20 | 12.5 |
| Example 138 | Compound 21 | DOPE | 40/30/28.5/1.5 | 20 | 12.5 |
| Example 139 | Compound 21 | DOPE | 40/40/18.5/1.5 | 20 | 12.5 |
| Example 140 | Compound 21 | DOPE | 40/0/58.5/1.5 | 20 | 12.5 |
| Example 141 | Compound 21 | DOPE | 40/2.5/56/1.5 | 20 | 12.5 |
| Example 142 | Compound 21 | DOPE | 40/50/8.5/1.5 | 20 | 12.5 |
| Example 143 | Compound 21 | DOPE | 40/58.5/0/1.5 | 20 | 12.5 |
| Example 144 | Compound 33 | DSPC | 40/0/58.5/1.5 | 20 | 12.5 |
| Example 145 | Compound 33 | DSPC | 40/2.5/56/1.5 | 20 | 12.5 |
| Example 146 | Compound 33 | DSPC | 40/50/8.5/1.5 | 20 | 12.5 |
| Example 147 | Compound 33 | DSPC | 40/58.5/0/1.5 | 20 | 12.5 |

### <Preparation of lipid particles not containing nucleic acid (empty LNP)>

The ionizable lipid shown in Table 5, one phospholipid (helper lipid) selected from DOPE (L-α-dioleoyl phosphatidylethanolamine, product name: COATSOME (R) ME-8181, manufactured by NOF Corporation), DSPC (1,2-distearoyl-sn-glycero-3-phosphocholine, product name: COATSOME (R) MC-8080, manufactured by NOF Corporation), DOPC (1,2-dioleoyl-sn-glycero-3-phosphocholine, product name: COATSOME (R) MC-8181, manufactured by NOF Corporation), DPPC (1,2-dipalmitoyl-sn-glycero-3-phosphocholine, product name: COATSOME (R) MC-6060, manufactured by NOF Corporation), and DMPC (1,2-dimyristoyl-sn-glycero-3-phosphocholine, product name: COATSOME (R) MC-4040, manufactured by NOF Corporation), cholesterol, and DMG-PEG2000 (1,2-dimyristoyl-rac-glycero-3-methoxypolyethylene glycol-2000, product name: SUNBRIGHT (R) GM-020, manufactured by NOF Corporation) were dissolved in ethanol at the molar ratios shown in Table 5 so that the total lipid concentration was 12.5 mmol/L or 62.5 mmol/L, thereby obtaining an oil phase.

A 50 mmol/L citrate buffer at a pH of 4 was mixed with the above-described oil phase using NanoAssemblr (Precision NanoSystems) so that the volume ratio of the citrate buffer to the oil phase was citrate buffer:oil phase = 3:1, and the mixed solution was diluted 2-fold with water to obtain a dispersion liquid of lipid particles. The dispersion liquid was dialyzed against a 20 mmol/L MES buffer solution at pH 6.0 containing 8% sucrose using a dialysis cassette (Slide-A-Lyzer G2, MWCO: 10 kD, Thermo Fisher Scientific, Inc.) to remove ethanol, and a concentration step was performed using an ultrafiltration filter (Amicon ultra 100 kDa, Merck KGaA) as necessary, thereby obtaining lipid particles (empty LNP) not containing a nucleic acid. The empty LNP was stored at -70°C until use.

### <Encapsulation of gRNA and Cas9 mRNA in empty LNP (post-addition method)>

CleanCap (registered trademark) Cas9 mRNA (5 moU) (TriLink, L-7206) and sgRNA (sequence; A*G*A*GUCUCAGCUGGUACA + modified Scaffold, Thermo Fisher A35514, custom synthesis) targeting a human T cell receptor alpha constant (TRAC) gene were mixed at a weight ratio of 4:1 and diluted with water for injection to prepare an RNA solution. The empty LNP stored at -70°C was thawed at 4°C. The RNA solution was added to the present LNP liquid in an equal liquid amount and mixed by pipetting (LNP-RNA mixed solution). After allowing the mixture to stand at room temperature for 5 minutes, a 20 mmol/L Tris buffer containing 8% sucrose was added to the present LNP-RNA mixed solution in an equal liquid amount, and the mixture was mixed by pipetting, thereby preparing Cas9 mRNA/gRNA-encapsulating LNP by a post-addition method.

**[Table 5]**

| <Table 5> Cas9 mRNA/gRNA-encapsulating lipid particles prepared by post-addition method | | | | | |
|---|---|---|---|---|---|
| | Compound No. | Helper lipid | Formulation of ionizable lipid/helper lipid/cholesterol/DMG-PEG2000 (mol%) | Lipid/RNA (wt/wt) | Oil-phase lipid concentration (mM) |
| Example 8 | Compound 8 | DOPE | 35/15/48.5/1.5 | 20 | 12.5 |
| Example 9 | Compound 9 | DOPE | 35/15/48.5/1.5 | 20 | 12.5 |
| Example 10 | Compound 10 | DOPE | 35/15/48.5/1.5 | 20 | 12.5 |
| Example 11 | Compound 11 | DOPE | 35/15/48.5/1.5 | 20 | 12.5 |
| Example 12 | Compound 1 | DOPE | 35/15/48.5/1.5 | 20 | 12.5 |
| Example 13 | Compound 12 | DOPE | 35/15/48.5/1.5 | 20 | 12.5 |
| Example 14 | Compound 13 | DOPE | 35/15/48.5/1.5 | 20 | 12.5 |
| Example 15 | Compound 14 | DOPE | 35/15/48.5/1.5 | 20 | 12.5 |
| Example 16 | Compound 15 | DOPE | 35/15/48.5/1.5 | 20 | 12.5 |
| Example 17 | Compound 16 | DOPE | 35/15/48.5/1.5 | 20 | 12.5 |
| Example 18 | Compound 17 | DOPE | 35/15/48.5/1.5 | 20 | 12.5 |
| Example 19 | Compound 18 | DOPE | 35/15/48.5/1.5 | 20 | 12.5 |
| Example 20 | Compound 5 | DOPE | 35/15/48.5/1.5 | 20 | 12.5 |
| Example 21 | Compound 2 | DOPE | 35/15/48.5/1.5 | 20 | 12.5 |
| Example 22 | Compound 19 | DOPE | 35/15/48.5/1.5 | 20 | 12.5 |
| Example 23 | Compound 20 | DOPE | 35/15/48.5/1.5 | 20 | 12.5 |
| Example 24 | Compound 21 | DOPE | 35/15/48.5/1.5 | 20 | 12.5 |
| Example 25 | Compound 22 | DOPE | 35/15/48.5/1.5 | 20 | 12.5 |
| Example 26 | Compound 23 | DOPE | 35/15/48.5/1.5 | 20 | 12.5 |
| Example 27 | Compound 24 | DOPE | 35/15/48.5/1.5 | 20 | 12.5 |
| Example 28 | Compound 25 | DOPE | 35/15/48.5/1.5 | 20 | 12.5 |
| Example 29 | Compound 26 | DOPE | 35/15/48.5/1.5 | 20 | 12.5 |
| Example 30 | Compound 8 | DSPC | 40/10/48.5/1.5 | 20 | 12.5 |
| Example 31 | Compound 9 | DSPC | 40/10/48.5/1.5 | 20 | 12.5 |
| Example 32 | Compound 10 | DSPC | 40/10/48.5/1.5 | 20 | 12.5 |
| Example 33 | Compound 18 | DSPC | 40/10/48.5/1.5 | 20 | 12.5 |
| Example 34 | Compound 17 | DSPC | 40/10/48.5/1.5 | 20 | 12.5 |
| Example 35 | Compound 11 | DSPC | 40/10/48.5/1.5 | 20 | 12.5 |
| Example 36 | Compound 1 | DSPC | 40/10/48.5/1.5 | 20 | 12.5 |
| Example 37 | Compound 12 | DSPC | 40/10/48.5/1.5 | 20 | 12.5 |
| Example 38 | Compound 13 | DSPC | 40/10/48.5/1.5 | 20 | 12.5 |
| Example 39 | Compound 14 | DSPC | 40/10/48.5/1.5 | 20 | 12.5 |
| Example 40 | Compound 15 | DSPC | 40/10/48.5/1.5 | 20 | 12.5 |
| Example 41 | Compound 16 | DSPC | 40/10/48.5/1.5 | 20 | 12.5 |
| Example 42 | Compound 2 | DSPC | 40/10/48.5/1.5 | 20 | 12.5 |
| Example 43 | Compound 19 | DSPC | 40/10/48.5/1.5 | 20 | 12.5 |
| Example 44 | Compound 21 | DSPC | 40/10/48.5/1.5 | 20 | 12.5 |
| Example 45 | Compound 21 | DOPE | 40/10/48.5/1.5 | 20 | 12.5 |
| Example 46 | Compound 3 | DOPE | 35/15/48.5/1.5 | 20 | 12.5 |
| Example 47 | Compound 27 | DOPE | 35/15/48.5/1.5 | 20 | 12.5 |
| Example 48 | Compound 28 | DOPE | 35/15/48.5/1.5 | 20 | 12.5 |
| Example 49 | Compound 29 | DOPE | 35/15/48.5/1.5 | 20 | 12.5 |
| Example 50 | Compound 30 | DOPE | 35/15/48.5/1.5 | 20 | 12.5 |
| Example 51 | Compound 7 | DOPE | 35/15/48.5/1.5 | 20 | 12.5 |
| Example 52 | Compound 3 | DOPE | 40/10/48.5/1.5 | 20 | 12.5 |
| Example 53 | Compound 27 | DOPE | 40/10/48.5/1.5 | 20 | 12.5 |
| Example 54 | Compound 28 | DOPE | 40/10/48.5/1.5 | 20 | 12.5 |
| Example 55 | Compound 29 | DOPE | 40/10/48.5/1.5 | 20 | 12.5 |
| Example 56 | Compound 30 | DOPE | 40/10/48.5/1.5 | 20 | 12.5 |
| Example 57 | Compound 7 | DOPE | 40/10/48.5/1.5 | 20 | 12.5 |
| Example 58 | Compound 22 | DSPC | 40/10/48.5/1.5 | 20 | 12.5 |
| Example 59 | Compound 23 | DSPC | 40/10/48.5/1.5 | 20 | 12.5 |
| Example 60 | Compound 25 | DSPC | 40/10/48.5/1.5 | 20 | 12.5 |
| Example 61 | Compound 26 | DSPC | 40/10/48.5/1.5 | 20 | 12.5 |
| Example 62 | Compound 20 | DSPC | 40/10/48.5/1.5 | 20 | 12.5 |
| Example 63 | Compound 24 | DSPC | 40/10/48.5/1.5 | 20 | 12.5 |
| Example 64 | Compound 21 | DSPC | 40/10/48.5/1.5 | 20 | 12.5 |
| Example 65 | Compound 11 | DSPC | 40/10/48.5/1.5 | 20 | 12.5 |
| Example 66 | Compound 15 | DSPC | 40/10/48.5/1.5 | 20 | 12.5 |
| Example 67 | Compound 16 | DSPC | 40/10/48.5/1.5 | 20 | 12.5 |
| Example 68 | Compound 2 | DSPC | 40/10/48.5/1.5 | 20 | 12.5 |
| Example 69 | Compound 19 | DSPC | 40/10/48.5/1.5 | 20 | 12.5 |
| Example 70 | Compound 21 | DOPE | 40/5/55/1.5 | 20 | 12.5 |
| Example 71 | Compound 21 | DSPC | 40/5/55/1.5 | 20 | 12.5 |
| Example 72 | Compound 21 | DPPC | 40/5/55/1.5 | 20 | 12.5 |
| Example 73 | Compound 21 | DMPC | 40/5/55/1.5 | 20 | 12.5 |
| Example 74 | Compound 21 | DOPC | 40/5/55/1.5 | 20 | 12.5 |
| Example 75 | Compound 21 | DOPE | 40/10/50/1.5 | 20 | 12.5 |
| Example 76 | Compound 21 | DSPC | 40/10/50/1.5 | 20 | 12.5 |
| Example 77 | Compound 21 | DPPC | 40/10/50/1.5 | 20 | 12.5 |
| Example 78 | Compound 21 | DMPC | 40/10/50/1.5 | 20 | 12.5 |
| Example 79 | Compound 21 | DOPC | 40/10/50/1.5 | 20 | 12.5 |
| Example 80 | Compound 21 | DOPE | 40/30/30/1.5 | 20 | 12.5 |
| Example 81 | Compound 21 | DSPC | 40/30/30/1.5 | 20 | 12.5 |
| Example 82 | Compound 21 | DPPC | 40/30/30/1.5 | 20 | 12.5 |
| Example 83 | Compound 21 | DMPC | 40/30/30/1.5 | 20 | 12.5 |
| Example 84 | Compound 21 | DOPC | 40/30/30/1.5 | 20 | 12.5 |
| Example 85 | Compound 31 | DSPC | 40/10/48.5/1.5 | 20 | 12.5 |
| Example 86 | Compound 32 | DSPC | 40/10/48.5/1.5 | 20 | 12.5 |
| Example 87 | Compound 33 | DSPC | 40/10/48.5/1.5 | 20 | 12.5 |
| Example 88 | Compound 33 | DSPC | 40/10/48.5/1.5 | 20 | 62.5 |
| Example 89 | Compound 24 | DSPC | 40/10/48.5/1.5 | 20 | 62.5 |
| Example 90 | Compound 32 | DSPC | 40/10/48.5/1.5 | 20 | 62.5 |
| Example 91 | Compound 20 | DSPC | 40/10/48.5/1.5 | 20 | 62.5 |
| Example 92 | Compound 2 | DSPC | 40/10/48.5/1.5 | 20 | 12.5 |
| Example 93 | Compound 21 | DOPE | 40/5/55/1.5 | 20 | 12.5 |
| Example 94 | Compound 33 | DSPC | 40/10/48.5/1.5 | 20 | 12.5 |
| Example 95 | Compound 24 | DOPE | 40/5/55/1.5 | 20 | 12.5 |
| Example 96 | Compound 24 | DSPC | 40/5/55/1.5 | 20 | 12.5 |
| Example 97 | Compound 24 | DPPC | 40/5/55/1.5 | 20 | 12.5 |
| Example 98 | Compound 24 | DMPC | 40/5/55/1.5 | 20 | 12.5 |
| Example 99 | Compound 24 | DOPC | 40/5/55/1.5 | 20 | 12.5 |
| Example 100 | Compound 24 | DOPE | 40/10/50/1.5 | 20 | 12.5 |
| Example 101 | Compound 24 | DSPC | 40/10/50/1.5 | 20 | 12.5 |
| Example 102 | Compound 24 | DPPC | 40/10/50/1.5 | 20 | 12.5 |
| Example 103 | Compound 24 | DMPC | 40/10/50/1.5 | 20 | 12.5 |
| Example 104 | Compound 24 | DOPC | 40/10/50/1.5 | 20 | 12.5 |
| Example 105 | Compound 24 | DOPE | 40/30/30/1.5 | 20 | 12.5 |
| Example 106 | Compound 24 | DSPC | 40/30/30/1.5 | 20 | 12.5 |
| Example 107 | Compound 24 | DPPC | 40/30/30/1.5 | 20 | 12.5 |
| Example 108 | Compound 24 | DMPC | 40/30/30/1.5 | 20 | 12.5 |
| Example 109 | Compound 24 | DOPC | 40/30/30/1.5 | 20 | 12.5 |
| Example 110 | Compound 34 | DSPC | 40/10/48.5/1.5 | 20 | 12.5 |
| Example 111 | Compound 35 | DSPC | 40/10/48.5/1.5 | 20 | 12.5 |
| Example 112 | Compound 36 | DSPC | 40/10/48.5/1.5 | 20 | 12.5 |
| Example 113 | Compound 33 | DSPC | 40/10/48.5/1.5 | 20 | 12.5 |
| Example 114 | Compound 21 | DSPC | 40/10/48.5/1.5 | 20 | 12.5 |
| Example 115 | Compound 34 | DOPE | 35/15/48.5/1.5 | 20 | 12.5 |
| Example 116 | Compound 35 | DOPE | 35/15/48.5/1.5 | 20 | 12.5 |
| Example 117 | Compound 36 | DOPE | 35/15/48.5/1.5 | 20 | 12.5 |
| Example 118 | Compound 33 | DOPE | 35/15/48.5/1.5 | 20 | 12.5 |
| Example 119 | Compound 21 | DOPE | 35/15/48.5/1.5 | 20 | 12.5 |
| Example 120 | Compound 34 | DSPC | 40/10/48.5/1.5 | 20 | 12.5 |
| Example 121 | Compound 35 | DSPC | 40/10/48.5/1.5 | 20 | 12.5 |
| Example 122 | Compound 36 | DSPC | 40/10/48.5/1.5 | 20 | 12.5 |
| Example 123 | Compound 33 | DSPC | 40/10/48.5/1.5 | 20 | 12.5 |
| Example 124 | Compound 21 | DSPC | 40/10/48.5/1.5 | 20 | 12.5 |
| Example 125 | Compound 34 | DOPE | 35/15/48.5/1.5 | 20 | 12.5 |
| Example 126 | Compound 35 | DOPE | 35/15/48.5/1.5 | 20 | 12.5 |
| Example 127 | Compound 36 | DOPE | 35/15/48.5/1.5 | 20 | 12.5 |
| Example 128 | Compound 33 | DOPE | 35/15/48.5/1.5 | 20 | 12.5 |
| Example 129 | Compound 21 | DOPE | 35/15/48.5/1.5 | 20 | 12.5 |
| Example 405 | Compound 73 | DOPE | 35/15/48.5/1.5 | 20 | 12.5 |

### <Encapsulation of GFP pDNA in empty LNP (post-addition method)>

GFP pDNA (GenScript, custom synthesized plasmid DNA) was diluted with water for injection to prepare a DNA solution. The empty LNP stored at -70°C was thawed at 4°C. The DNA solution was added to the present LNP solution in an equal liquid amount and mixed by pipetting (LNP-DNA mixed solution). After allowing the mixture to stand at room temperature for 5 minutes, a 20 mmol/L Tris buffer (pH 8.4) containing 8% sucrose was added to the present LNP-DNA mixed solution in an equal liquid amount, and the mixture was mixed by pipetting, thereby preparing GFP pDNA-encapsulating LNP by a post-addition method.

**[Table 6]**

| <Table 6> GFP pDNA-encapsulating LNP prepared by post-addition method | | | | | |
|---|---|---|---|---|---|
| | Compound No. | Helper lipid | Formulation of ionizable lipid/helper lipid/cholesterol/DMG-PEG2000 (mol%) | Lipid/DNA (wt/wt) | Oil-phase lipid concentration (mM) |
| Example 148 | Compound 33 | DSPC | 40/5/53.5/1.5 | 20 | 12.5 |
| Example 149 | Compound 33 | DSPC | 40/10/48.5/1.5 | 20 | 12.5 |
| Example 150 | Compound 33 | DSPC | 40/20/38.5/1.5 | 20 | 12.5 |
| Example 151 | Compound 33 | DSPC | 40/30/28.5/1.5 | 20 | 12.5 |
| Example 152 | Compound 33 | DSPC | 40/40/18.5/1.5 | 20 | 12.5 |
| Example 153 | Compound 21 | DOPE | 40/5/53.5/1.5 | 20 | 12.5 |
| Example 154 | Compound 21 | DOPE | 40/10/48.5/1.5 | 20 | 12.5 |
| Example 155 | Compound 21 | DOPE | 40/20/38.5/1.5 | 20 | 12.5 |
| Example 156 | Compound 21 | DOPE | 40/30/28.5/1.5 | 20 | 12.5 |
| Example 157 | Compound 21 | DOPE | 40/40/18.5/1.5 | 20 | 12.5 |
| Example 158 | Compound 21 | DOPE | 40/0/58.5/1.5 | 20 | 12.5 |
| Example 159 | Compound 21 | DOPE | 40/2.5/56/1.5 | 20 | 12.5 |
| Example 160 | Compound 21 | DOPE | 40/50/8.5/1.5 | 20 | 12.5 |
| Example 161 | Compound 21 | DOPE | 40/58.5/0/1.5 | 20 | 12.5 |
| Example 162 | Compound 33 | DSPC | 40/0/58.5/1.5 | 20 | 12.5 |
| Example 163 | Compound 33 | DSPC | 40/2.5/56/1.5 | 20 | 12.5 |
| Example 164 | Compound 33 | DSPC | 40/50/8.5/1.5 | 20 | 12.5 |
| Example 165 | Compound 33 | DSPC | 40/58.5/0/1.5 | 20 | 12.5 |
| Example 166 | Compound 21 | DOPE | 40/5/53.5/1.5 | 20 | 12.5 |
| Example 167 | Compound 21 | DOPE | 40/10/48.5/1.5 | 20 | 12.5 |
| Example 168 | Compound 21 | DOPE | 40/20/38.5/1.5 | 20 | 12.5 |
| Example 169 | Compound 21 | DOPE | 40/30/28.5/1.5 | 20 | 12.5 |
| Example 170 | Compound 21 | DOPE | 40/40/18.5/1.5 | 20 | 12.5 |
| Example 171 | Compound 33 | DSPC | 40/5/53.5/1.5 | 20 | 12.5 |
| Example 172 | Compound 33 | DSPC | 40/10/48.5/1.5 | 20 | 12.5 |
| Example 173 | Compound 33 | DSPC | 40/20/38.5/1.5 | 20 | 12.5 |
| Example 174 | Compound 33 | DSPC | 40/30/28.5/1.5 | 20 | 12.5 |
| Example 175 | Compound 33 | DSPC | 40/40/18.5/1.5 | 20 | 12.5 |
| Example 176 | Compound 21 | DOPE | 40/5/53.5/1.5 | 20 | 12.5 |
| Example 177 | Compound 21 | DOPE | 40/10/48.5/1.5 | 20 | 12.5 |
| Example 178 | Compound 33 | DSPC | 40/10/48.5/1.5 | 20 | 12.5 |
| Example 179 | Compound 70 | DSPC | 40/10/48.5/1.5 | 20 | 12.5 |
| Example 180 | Compound 33 | DOPE | 40/10/48.5/1.5 | 20 | 12.5 |
| Example 181 | Compound 70 | DOPE | 40/10/48.5/1.5 | 20 | 12.5 |
| Example 182 | Compound 25 | DSPC | 40/10/48.5/1.5 | 20 | 12.5 |
| Example 183 | Compound 71 | DSPC | 40/10/48.5/1.5 | 20 | 12.5 |
| Example 184 | Compound 67 | DOPE | 40/10/48.5/1.5 | 20 | 12.5 |
| Example 185 | Compound 67 | DOPE | 40/10/48.5/1.5 | 20 | 12.5 |
| Example 186 | Compound 21 | DSPC | 40/10/48.5/1.5 | 20 | 12.5 |
| Example 187 | Compound 21 | DOPE | 40/10/48.5/1.5 | 20 | 12.5 |
| Example 188 | Compound 33 | DSPC | 40/10/48.5/1.5 | 20 | 12.5 |
| Example 189 | Compound 70 | DOPE | 40/10/48.5/1.5 | 20 | 12.5 |
| Example 190 | Compound 33 | DSPC | 40/10/48.5/1.5 | 20 | 12.5 |
| Example 191 | Compound 70 | DSPC | 40/10/48.5/1.5 | 20 | 12.5 |
| Example 192 | Compound 33 | DOPE | 40/10/48.5/1.5 | 20 | 12.5 |
| Example 193 | Compound 70 | DOPE | 40/10/48.5/1.5 | 20 | 12.5 |
| Example 194 | Compound 33 | DSPC | 40/10/48.5/1.5 | 20 | 12.5 |
| Example 195 | Compound 70 | DSPC | 40/10/48.5/1.5 | 20 | 12.5 |
| Example 196 | Compound 33 | DOPE | 40/10/48.5/1.5 | 20 | 12.5 |
| Example 197 | Compound 70 | DOPE | 40/10/48.5/1.5 | 20 | 12.5 |
| Example 198 | Compound 8 | DSPC | 40/10/48.5/1.5 | 20 | 12.5 |
| Example 199 | Compound 9 | DSPC | 40/10/48.5/1.5 | 20 | 12.5 |
| Example 200 | Compound 10 | DSPC | 40/10/48.5/1.5 | 20 | 12.5 |
| Example 201 | Compound 18 | DSPC | 40/10/48.5/1.5 | 20 | 12.5 |
| Example 202 | Compound 17 | DSPC | 40/10/48.5/1.5 | 20 | 12.5 |
| Example 203 | Compound 11 | DSPC | 40/10/48.5/1.5 | 20 | 12.5 |
| Example 204 | Compound 1 | DSPC | 40/10/48.5/1.5 | 20 | 12.5 |
| Example 205 | Compound 12 | DSPC | 40/10/48.5/1.5 | 20 | 12.5 |
| Example 206 | Compound 13 | DSPC | 40/10/48.5/1.5 | 20 | 12.5 |
| Example 207 | Compound 14 | DSPC | 40/10/48.5/1.5 | 20 | 12.5 |
| Example 208 | Compound 15 | DSPC | 40/10/48.5/1.5 | 20 | 12.5 |
| Example 209 | Compound 16 | DSPC | 40/10/48.5/1.5 | 20 | 12.5 |
| Example 210 | Compound 2 | DSPC | 40/10/48.5/1.5 | 20 | 12.5 |
| Example 211 | Compound 19 | DSPC | 40/10/48.5/1.5 | 20 | 12.5 |
| Example 212 | Compound 21 | DSPC | 40/10/48.5/1.5 | 20 | 12.5 |
| Example 213 | Compound 21 | DOPE | 40/10/48.5/1.5 | 20 | 12.5 |
| Example 214 | Compound 3 | DOPE | 35/15/48.5/1.5 | 20 | 12.5 |
| Example 215 | Compound 27 | DOPE | 35/15/48.5/1.5 | 20 | 12.5 |
| Example 216 | Compound 28 | DOPE | 35/15/48.5/1.5 | 20 | 12.5 |
| Example 217 | Compound 29 | DOPE | 35/15/48.5/1.5 | 20 | 12.5 |
| Example 218 | Compound 30 | DOPE | 35/15/48.5/1.5 | 20 | 12.5 |
| Example 219 | Compound 7 | DOPE | 35/15/48.5/1.5 | 20 | 12.5 |
| Example 220 | Compound 3 | DOPE | 40/10/48.5/1.5 | 20 | 12.5 |
| Example 221 | Compound 27 | DOPE | 40/10/48.5/1.5 | 20 | 12.5 |
| Example 222 | Compound 18 | DOPE | 40/10/48.5/1.5 | 20 | 12.5 |
| Example 223 | Compound 29 | DOPE | 40/10/48.5/1.5 | 20 | 12.5 |
| Example 224 | Compound 30 | DOPE | 40/10/48.5/1.5 | 20 | 12.5 |
| Example 225 | Compound 7 | DOPE | 40/10/48.5/1.5 | 20 | 12.5 |
| Example 226 | Compound 22 | DSPC | 40/10/48.5/1.5 | 20 | 12.5 |
| Example 227 | Compound 23 | DSPC | 40/10/48.5/1.5 | 20 | 12.5 |
| Example 228 | Compound 25 | DSPC | 40/10/48.5/1.5 | 20 | 12.5 |
| Example 229 | Compound 26 | DSPC | 40/10/48.5/1.5 | 20 | 12.5 |
| Example 230 | Compound 20 | DSPC | 40/10/48.5/1.5 | 20 | 12.5 |
| Example 231 | Compound 24 | DSPC | 40/10/48.5/1.5 | 20 | 12.5 |
| Example 232 | Compound 21 | DSPC | 40/10/48.5/1.5 | 20 | 12.5 |
| Example 233 | Compound 11 | DSPC | 40/10/48.5/1.5 | 20 | 12.5 |
| Example 234 | Compound 15 | DSPC | 40/10/48.5/1.5 | 20 | 12.5 |
| Example 235 | Compound 16 | DSPC | 40/10/48.5/1.5 | 20 | 12.5 |
| Example 236 | Compound 2 | DSPC | 40/10/48.5/1.5 | 20 | 12.5 |
| Example 237 | Compound 19 | DSPC | 40/10/48.5/1.5 | 20 | 12.5 |
| Example 238 | Compound 21 | DOPE | 40/5/55/1.5 | 20 | 12.5 |
| Example 239 | Compound 21 | DSPC | 40/5/55/1.5 | 20 | 12.5 |
| Example 240 | Compound 21 | DPPC | 40/5/55/1.5 | 20 | 12.5 |
| Example 241 | Compound 21 | DMPC | 40/5/55/1.5 | 20 | 12.5 |
| Example 242 | Compound 21 | DOPC | 40/5/55/1.5 | 20 | 12.5 |
| Example 243 | Compound 21 | DOPE | 40/10/50/1.5 | 20 | 12.5 |
| Example 244 | Compound 21 | DSPC | 40/10/50/1.5 | 20 | 12.5 |
| Example 245 | Compound 21 | DPPC | 40/10/50/1.5 | 20 | 12.5 |
| Example 246 | Compound 21 | DMPC | 40/10/50/1.5 | 20 | 12.5 |
| Example 247 | Compound 21 | DOPC | 40/10/50/1.5 | 20 | 12.5 |
| Example 248 | Compound 21 | DOPE | 40/30/30/1.5 | 20 | 12.5 |
| Example 249 | Compound 21 | DSPC | 40/30/30/1.5 | 20 | 12.5 |
| Example 250 | Compound 21 | DPPC | 40/30/30/1.5 | 20 | 12.5 |
| Example 251 | Compound 21 | DMPC | 40/30/30/1.5 | 20 | 12.5 |
| Example 252 | Compound 21 | DOPC | 40/30/30/1.5 | 20 | 12.5 |
| Example 253 | Compound 31 | DSPC | 40/10/48.5/1.5 | 20 | 12.5 |
| Example 254 | Compound 32 | DSPC | 40/10/48.5/1.5 | 20 | 12.5 |
| Example 255 | Compound 33 | DSPC | 40/10/48.5/1.5 | 20 | 12.5 |
| Example 256 | Compound 2 | DSPC | 40/10/48.5/1.5 | 20 | 12.5 |
| Example 257 | Compound 21 | DOPE | 40/5/55/1.5 | 20 | 12.5 |
| Example 258 | Compound 33 | DSPC | 40/10/48.5/1.5 | 20 | 12.5 |
| Example 259 | Compound 24 | DOPE | 40/5/55/1.5 | 20 | 12.5 |
| Example 260 | Compound 24 | DSPC | 40/5/55/1.5 | 20 | 12.5 |
| Example 261 | Compound 24 | DPPC | 40/5/55/1.5 | 20 | 12.5 |
| Example 262 | Compound 24 | DMPC | 40/5/55/1.5 | 20 | 12.5 |
| Example 263 | Compound 24 | DOPC | 40/5/55/1.5 | 20 | 12.5 |
| Example 264 | Compound 24 | DOPE | 40/10/50/1.5 | 20 | 12.5 |
| Example 265 | Compound 24 | DSPC | 40/10/50/1.5 | 20 | 12.5 |
| Example 266 | Compound 24 | DPPC | 40/10/50/1.5 | 20 | 12.5 |
| Example 267 | Compound 24 | DMPC | 40/10/50/1.5 | 20 | 12.5 |
| Example 268 | Compound 24 | DOPC | 40/10/50/1.5 | 20 | 12.5 |
| Example 269 | Compound 24 | DOPE | 40/30/30/1.5 | 20 | 12.5 |
| Example 270 | Compound 24 | DSPC | 40/30/30/1.5 | 20 | 12.5 |
| Example 271 | Compound 24 | DPPC | 40/30/30/1.5 | 20 | 12.5 |
| Example 272 | Compound 24 | DMPC | 40/30/30/1.5 | 20 | 12.5 |
| Example 273 | Compound 24 | DOPC | 40/30/30/1.5 | 20 | 12.5 |
| Example 274 | Compound 34 | DSPC | 40/10/48.5/1.5 | 20 | 12.5 |
| Example 275 | Compound 35 | DSPC | 40/10/48.5/1.5 | 20 | 12.5 |
| Example 276 | Compound 36 | DSPC | 40/10/48.5/1.5 | 20 | 12.5 |
| Example 277 | Compound 33 | DSPC | 40/10/48.5/1.5 | 20 | 12.5 |
| Example 278 | Compound 21 | DSPC | 40/10/48.5/1.5 | 20 | 12.5 |
| Example 279 | Compound 34 | DOPE | 35/15/48.5/1.5 | 20 | 12.5 |
| Example 280 | Compound 35 | DOPE | 35/15/48.5/1.5 | 20 | 12.5 |
| Example 281 | Compound 36 | DOPE | 35/15/48.5/1.5 | 20 | 12.5 |
| Example 282 | Compound 33 | DOPE | 35/15/48.5/1.5 | 20 | 12.5 |
| Example 283 | Compound 21 | DOPE | 35/15/48.5/1.5 | 20 | 12.5 |
| Example 284 | Compound 64 | DSPC | 40/10/48.5/1.5 | 20 | 12.5 |
| Example 285 | Compound 65 | DSPC | 40/10/48.5/1.5 | 20 | 12.5 |
| Example 286 | Compound 66 | DSPC | 40/10/48.5/1.5 | 20 | 12.5 |
| Example 287 | Compound 67 | DSPC | 40/10/48.5/1.5 | 20 | 12.5 |
| Example 288 | Compound 70 | DSPC | 40/10/48.5/1.5 | 20 | 12.5 |
| Example 289 | Compound 71 | DSPC | 40/10/48.5/1.5 | 20 | 12.5 |
| Example 290 | Compound 64 | DOPE | 40/10/48.5/1.5 | 20 | 12.5 |
| Example 291 | Compound 65 | DOPE | 40/10/48.5/1.5 | 20 | 12.5 |
| Example 292 | Compound 66 | DOPE | 40/10/48.5/1.5 | 20 | 12.5 |
| Example 293 | Compound 67 | DOPE | 40/10/48.5/1.5 | 20 | 12.5 |
| Example 294 | Compound 70 | DOPE | 40/10/48.5/1.5 | 20 | 12.5 |
| Example 295 | Compound 71 | DOPE | 40/10/48.5/1.5 | 20 | 12.5 |
| Example 296 | Compound 60 | DSPC | 40/10/48.5/1.5 | 20 | 12.5 |
| Example 297 | Compound 61 | DSPC | 40/10/48.5/1.5 | 20 | 12.5 |
| Example 298 | Compound 62 | DSPC | 40/10/48.5/1.5 | 20 | 12.5 |
| Example 299 | Compound 63 | DSPC | 40/10/48.5/1.5 | 20 | 12.5 |
| Example 300 | Compound 68 | DSPC | 40/10/48.5/1.5 | 20 | 12.5 |
| Example 301 | Compound 69 | DSPC | 40/10/48.5/1.5 | 20 | 12.5 |
| Example 302 | Compound 43 | DSPC | 40/10/48.5/1.5 | 20 | 12.5 |
| Example 303 | Compound 60 | DOPE | 40/10/48.5/1.5 | 20 | 12.5 |
| Example 304 | Compound 61 | DOPE | 40/10/48.5/1.5 | 20 | 12.5 |
| Example 305 | Compound 62 | DOPE | 40/10/48.5/1.5 | 20 | 12.5 |
| Example 306 | Compound 63 | DOPE | 40/10/48.5/1.5 | 20 | 12.5 |
| Example 307 | Compound 68 | DOPE | 40/10/48.5/1.5 | 20 | 12.5 |
| Example 308 | Compound 69 | DOPE | 40/10/48.5/1.5 | 20 | 12.5 |
| Example 309 | Compound 43 | DOPE | 40/10/48.5/1.5 | 20 | 12.5 |
| Example 310 | Compound 56 | DSPC | 40/10/48.5/1.5 | 20 | 12.5 |
| Example 311 | Compound 42 | DSPC | 40/10/48.5/1.5 | 20 | 12.5 |
| Example 312 | Compound 41 | DSPC | 40/10/48.5/1.5 | 20 | 12.5 |
| Example 313 | Compound 57 | DSPC | 40/10/48.5/1.5 | 20 | 12.5 |
| Example 314 | Compound 44 | DSPC | 40/10/48.5/1.5 | 20 | 12.5 |
| Example 315 | Compound 48 | DSPC | 40/10/48.5/1.5 | 20 | 12.5 |
| Example 316 | Compound 46 | DSPC | 40/10/48.5/1.5 | 20 | 12.5 |
| Example 317 | Compound 56 | DOPE | 40/10/48.5/1.5 | 20 | 12.5 |
| Example 318 | Compound 42 | DOPE | 40/10/48.5/1.5 | 20 | 12.5 |
| Example 319 | Compound 41 | DOPE | 40/10/48.5/1.5 | 20 | 12.5 |
| Example 320 | Compound 57 | DOPE | 40/10/48.5/1.5 | 20 | 12.5 |
| Example 321 | Compound 44 | DOPE | 40/10/48.5/1.5 | 20 | 12.5 |
| Example 322 | Compound 48 | DOPE | 40/10/48.5/1.5 | 20 | 12.5 |
| Example 323 | Compound 46 | DOPE | 40/10/48.5/1.5 | 20 | 12.5 |
| Example 324 | Compound 58 | DSPC | 40/10/48.5/1.5 | 20 | 12.5 |
| Example 325 | Compound 45 | DSPC | 40/10/48.5/1.5 | 20 | 12.5 |
| Example 326 | Compound 47 | DSPC | 40/10/48.5/1.5 | 20 | 12.5 |
| Example 327 | Compound 49 | DSPC | 40/10/48.5/1.5 | 20 | 12.5 |
| Example 328 | Compound 59 | DSPC | 40/10/48.5/1.5 | 20 | 12.5 |
| Example 329 | Compound 50 | DSPC | 40/10/48.5/1.5 | 20 | 12.5 |
| Example 330 | Compound 52 | DSPC | 40/10/48.5/1.5 | 20 | 12.5 |
| Example 331 | Compound 58 | DOPE | 40/10/48.5/1.5 | 20 | 12.5 |
| Example 332 | Compound 45 | DOPE | 40/10/48.5/1.5 | 20 | 12.5 |
| Example 333 | Compound 47 | DOPE | 40/10/48.5/1.5 | 20 | 12.5 |
| Example 334 | Compound 49 | DOPE | 40/10/48.5/1.5 | 20 | 12.5 |
| Example 335 | Compound 59 | DOPE | 40/10/48.5/1.5 | 20 | 12.5 |
| Example 336 | Compound 50 | DOPE | 40/10/48.5/1.5 | 20 | 12.5 |
| Example 337 | Compound 52 | DOPE | 40/10/48.5/1.5 | 20 | 12.5 |
| Example 338 | Compound 36 | DSPC | 40/10/48.5/1.5 | 20 | 12.5 |
| Example 339 | Compound 33 | DSPC | 40/10/48.5/1.5 | 20 | 12.5 |
| Example 340 | Compound 21 | DOPE | 40/10/48.5/1.5 | 20 | 12.5 |
| Example 341 | Compound 36 | DSPC | 40/10/48.5/1.5 | 20 | 12.5 |
| Example 342 | Compound 33 | DSPC | 40/10/48.5/1.5 | 20 | 12.5 |
| Example 343 | Compound 21 | DOPE | 40/10/48.5/1.5 | 20 | 12.5 |
| Example 344 | Compound 36 | DOPE | 40/10/48.5/1.5 | 20 | 12.5 |
| Example 345 | Compound 33 | DOPE | 40/10/48.5/1.5 | 20 | 12.5 |
| Example 346 | Compound 21 | DSPC | 40/10/48.5/1.5 | 20 | 12.5 |
| Example 347 | Compound 54 | DSPC | 40/10/48.5/1.5 | 20 | 12.5 |
| Example 348 | Compound 37 | DSPC | 40/10/48.5/1.5 | 20 | 12.5 |
| Example 349 | Compound 39 | DSPC | 40/10/48.5/1.5 | 20 | 12.5 |
| Example 350 | Compound 38 | DSPC | 40/10/48.5/1.5 | 20 | 12.5 |
| Example 351 | Compound 40 | DSPC | 40/10/48.5/1.5 | 20 | 12.5 |
| Example 352 | Compound 51 | DSPC | 40/10/48.5/1.5 | 20 | 12.5 |
| Example 353 | Compound 53 | DSPC | 40/10/48.5/1.5 | 20 | 12.5 |
| Example 354 | Compound 55 | DOPE | 40/10/48.5/1.5 | 20 | 12.5 |
| Example 355 | Compound 54 | DOPE | 40/10/48.5/1.5 | 20 | 12.5 |
| Example 356 | Compound 37 | DOPE | 40/10/48.5/1.5 | 20 | 12.5 |
| Example 357 | Compound 39 | DOPE | 40/10/48.5/1.5 | 20 | 12.5 |
| Example 358 | Compound 38 | DOPE | 40/10/48.5/1.5 | 20 | 12.5 |
| Example 359 | Compound 40 | DOPE | 40/10/48.5/1.5 | 20 | 12.5 |
| Example 360 | Compound 51 | DOPE | 40/10/48.5/1.5 | 20 | 12.5 |
| Example 361 | Compound 53 | DOPE | 40/10/48.5/1.5 | 20 | 12.5 |
| Example 362 | Compound 55 | DOPE | 40/10/48.5/1.5 | 20 | 12.5 |
| Example 363 | Compound 33 | DSPC | 40/10/48.5/1.5 | 20 | 12.5 |
| Example 364 | Compound 36 | DSPC | 40/10/48.5/1.5 | 20 | 12.5 |
| Example 365 | Compound 21 | DSPC | 40/10/48.5/1.5 | 20 | 12.5 |
| Example 366 | Compound 25 | DSPC | 40/10/48.5/1.5 | 20 | 12.5 |
| Example 367 | Compound 20 | DSPC | 40/10/48.5/1.5 | 20 | 12.5 |
| Example 368 | Compound 24 | DSPC | 40/10/48.5/1.5 | 20 | 12.5 |
| Example 369 | Compound 72 | DSPC | 40/10/48.5/1.5 | 20 | 12.5 |
| Example 370 | Compound 73 | DSPC | 40/10/48.5/1.5 | 20 | 12.5 |
| Example 371 | Compound 74 | DSPC | 40/10/48.5/1.5 | 20 | 12.5 |
| Example 372 | Compound 33 | DOPE | 40/10/48.5/1.5 | 20 | 12.5 |
| Example 373 | Compound 36 | DOPE | 40/10/48.5/1.5 | 20 | 12.5 |
| Example 374 | Compound 21 | DOPE | 40/10/48.5/1.5 | 20 | 12.5 |
| Example 375 | Compound 25 | DOPE | 40/10/48.5/1.5 | 20 | 12.5 |
| Example 376 | Compound 20 | DOPE | 40/10/48.5/1.5 | 20 | 12.5 |
| Example 377 | Compound 24 | DOPE | 40/10/48.5/1.5 | 20 | 12.5 |
| Example 378 | Compound 72 | DOPE | 40/10/48.5/1.5 | 20 | 12.5 |
| Example 379 | Compound 73 | DOPE | 40/10/48.5/1.5 | 20 | 12.5 |
| Example 380 | Compound 74 | DOPE | 40/10/48.5/1.5 | 20 | 12.5 |
| Example 381 | Compound 36 | DSPC | 40/10/48.5/1.5 | 20 | 12.5 |
| Example 382 | Compound 33 | DSPC | 40/10/48.5/1.5 | 20 | 12.5 |
| Example 383 | Compound 21 | DOPE | 40/10/48.5/1.5 | 20 | 12.5 |
| Example 384 | Compound 36 | DSPC | 40/10/48.5/1.5 | 20 | 12.5 |
| Example 385 | Compound 33 | DSPC | 40/10/48.5/1.5 | 20 | 12.5 |
| Example 386 | Compound 21 | DOPE | 40/10/48.5/1.5 | 20 | 12.5 |

### <Measurement of particle size>

The particle size of the mRNA-encapsulating lipid particles was measured using a particle diameter measurement system NanoSAQLA (Otsuka Electronics Co., Ltd.) after optionally diluting the lipid particles with phosphate buffered saline (PBS).

The measurement results of the particle size and the polydispersity index (PDI) are shown in Table 7.

### <Evaluation of encapsulation rate of nucleic acid>

### (Quantification of total nucleic acid concentration)

The nucleic acid was diluted with MilliQ water to prepare a diluted sample in 2-fold dilution series from 100 µg/mL to 3.1 µg/mL, and a standard solution for calibration curve was prepared. 50 µL of the calibration curve solution or the lipid particles was mixed with 450 µL of methanol to prepare a measurement solution. The absorbance of each measurement solution at 260 nm and 330 nm was measured using a UV plate reader (Multiskan Go, Thermo Fisher Scientific), the absorbance at 330 nm was subtracted from the absorbance at 260 nm, and the result was defined as the absorbance of each measurement solution. The total nucleic acid concentration was calculated from the calibration curve using the absorbance of each sample measurement solution.

### (Quantification of nucleic acid concentration in outer water phase)

The concentration of the outer water phase nucleic acid was quantified by a standard addition method using a QuanT-iT RiboGreen RNA Assay Kit (Thermo Fisher Scientific). First, a 20× TE buffer included in the above kit was diluted with water, thereby obtaining a 1× TE buffer. TE represents Tris/EDTA (ethylenediaminetetraacetic acid). The nucleic acid was diluted with a TE buffer such that the final concentration was 0 to 400 ng/mL, thereby preparing a nucleic acid dilution series. 10 µL of the lipid particles and 90 µL of the nucleic acid dilution series were mixed in a 96-well plate, 100 µL of a RiboGreen reagent diluted 200 times with a TE buffer was added to each well, and fluorescence (excitation wavelength: 485 nm, fluorescence wavelength: 535 nm) was measured using a fluorescence plate reader (Infinite 200 Pro M nano +, TECAN). From the obtained results, the outer water phase nucleic acid concentration of each measurement solution was calculated according to the standard addition method.

### (Calculation of encapsulation rate)

Using the quantification results of the total RNA concentration and the nucleic acid concentration in the outer water phase obtained in the above-described step, the nucleic acid encapsulation rate of the nucleic acid lipid nanoparticles was calculated according to the following expression. 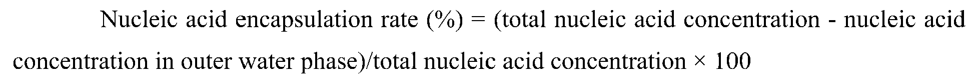 Nucleic acid encapsulation rate (%) = (total nucleic acid concentration - nucleic acid concentration in outer water phase)/total nucleic acid concentration × 100

The results are shown in Table 7.

**[Table 7]**

| | Particle size (nm) | PdI | Encapsulation rate % |
|---|---|---|---|
| Example 30 | 301 | 0.24 | 90% |
| Example 31 | 298 | 0.19 | 89% |
| Example 32 | 250 | 0.18 | 89% |
| Example 33 | 145 | 0.16 | 96% |
| Example 34 | 125 | 0.13 | 98% |
| Example 35 | 81 | 0.13 | 99% |
| Example 36 | 284 | 0.22 | 97% |
| Example 37 | 328 | 0.25 | 94% |
| Example 38 | 249 | 0.20 | 93% |
| Example 39 | 142 | 0.15 | 99% |
| Example 40 | 111 | 0.14 | 100% |
| Example 41 | 133 | 0.19 | 98% |
| Example 42 | 97 | 0.14 | 99% |
| Example 43 | 111 | 0.12 | 99% |
| Example 44 | 86 | 0.07 | 97% |
| Example 45 | 128 | 0.08 | 56% |
| Example 46 | 171 | 0.10 | 58% |
| Example 47 | 690 | 0.23 | 53% |
| Example 52 | 201 | 0.14 | 67% |
| Example 53 | 196 | 0.09 | 68% |
| Example 58 | 197 | 0.10 | 57% |
| Example 60 | 114 | 0.07 | 98% |
| Example 61 | 306 | 0.19 | 57% |
| Example 62 | 84 | 0.12 | 97% |
| Example 63 | 92 | 0.11 | 98% |
| Example 64 | 98 | 0.15 | 97% |
| Example 65 | 93 | 0.15 | 97% |
| Example 66 | 104 | 0.14 | 98% |
| Example 67 | 117 | 0.07 | 98% |
| Example 68 | 98 | 0.13 | 97% |
| Example 69 | 110 | 0.14 | 97% |
| Example 70 | 102 | 0.10 | 94% |
| Example 71 | 97 | 0.19 | 94% |
| Example 72 | 115 | 0.19 | 94% |
| Example 73 | 131 | 0.12 | 85% |
| Example 74 | 111 | 0.06 | 93% |
| Example 76 | 94 | 0.12 | 95% |
| Example 77 | 99 | 0.16 | 95% |
| Example 78 | 97 | 0.10 | 94% |
| Example 81 | 95 | 0.17 | 88% |
| Example 82 | 113 | 0.15 | 80% |
| Example 83 | 132 | 0.15 | 70% |
| Example 85 | 145 | 0.10 | 96% |
| Example 86 | 116 | 0.07 | 97% |
| Example 87 | 162 | 0.51 | 96% |
| Example 88 | 106 | 0.11 | 89% |
| Example 89 | 118 | 0.07 | 93% |
| Example 90 | 138 | 0.12 | 89% |
| Example 91 | 103 | 0.09 | 85% |
| Example 92 | 97 | 0.14 | 99% |
| Example 93 | 102 | 0.10 | 94% |
| Example 94 | 162 | 0.51 | 96% |
| Example 110 | 84 | 0.07 | 98% |
| Example 111 | 90 | 0.09 | 97% |
| Example 112 | 134 | 0.14 | 97% |
| Example 113 | 86 | 0.10 | 98% |
| Example 114 | 94 | 0.07 | 98% |
| Example 120 | 84 | 0.07 | 98% |
| Example 121 | 90 | 0.09 | 97% |
| Example 122 | 134 | 0.14 | 97% |
| Example 123 | 86 | 0.10 | 98% |
| Example 124 | 94 | 0.07 | 98% |
| Example 131 | 99 | 0.04 | 94% |
| Example 132 | 87 | 0.06 | 97% |
| Example 133 | 96 | 0.06 | 90% |
| Example 134 | 97 | 0.03 | 69% |
| Example 135 | 82 | 0.06 | 93% |
| Example 136 | 83 | 0.13 | 94% |
| Example 137 | 88 | 0.10 | 95% |
| Example 138 | 89 | 0.14 | 90% |
| Example 139 | 96 | 0.14 | 93% |
| Example 140 | 85 | 0.08 | 96% |
| Example 141 | 82 | 0.07 | 96% |
| Example 142 | 105 | 0.17 | 95% |
| Example 143 | 92 | 0.16 | 94% |
| Example 144 | 97 | 0.05 | 95% |
| Example 145 | 81 | 0.03 | 96% |
| Example 146 | 109 | 0.14 | 88% |
| Example 147 | 129 | 0.19 | 69% |
| Example 149 | 122 | 0.03 | 86% |
| Example 150 | 84 | 0.06 | 77% |
| Example 151 | 89 | 0.04 | 82% |
| Example 152 | 91 | 0.07 | 51% |
| Example 153 | 140 | 0.04 | 86% |
| Example 154 | 194 | 0.07 | 63% |
| Example 159 | 140 | 0.07 | 80% |
| Example 160 | 817 | 0.28 | 69% |
| Example 161 | 366 | 0.28 | 79% |
| Example 162 | 132 | 0.00 | 95% |
| Example 163 | 114 | 0.01 | 97% |
| Example 164 | 100 | 0.13 | 59% |
| Example 166 | 150 | 0.04 | 70% |
| Example 167 | 206 | 0.12 | 69% |
| Example 170 | 225 | 0.21 | 60% |
| Example 172 | 141 | 0.08 | 80% |
| Example 173 | 97 | 0.08 | 88% |
| Example 174 | 100 | 0.03 | 89% |
| Example 176 | 133 | 0.06 | 87% |
| Example 177 | 237 | 0.14 | 63% |
| Example 178 | 89 | 0.11 | 96% |
| Example 179 | 93 | 0.07 | 94% |
| Example 181 | 171 | 0.04 | 94% |
| Example 182 | 109 | 0.09 | 89% |
| Example 183 | 120 | 0.11 | 96% |
| Example 184 | 277 | 0.14 | 70% |
| Example 186 | 111 | 0.11 | 95% |
| Example 188 | 92 | 0.08 | 92% |
| Example 190 | 89 | 0.11 | 96% |
| Example 191 | 93 | 0.07 | 94% |
| Example 193 | 171 | 0.04 | 94% |
| Example 194 | 89 | 0.11 | 96% |
| Example 195 | 93 | 0.07 | 94% |
| Example 197 | 171 | 0.04 | 94% |
| Example 198 | 301 | 0.24 | 90% |
| Example 199 | 298 | 0.19 | 89% |
| Example 200 | 250 | 0.18 | 89% |
| Example 201 | 145 | 0.16 | 96% |
| Example 202 | 125 | 0.13 | 98% |
| Example 203 | 81 | 0.13 | 99% |
| Example 204 | 284 | 0.22 | 97% |
| Example 205 | 328 | 0.25 | 94% |
| Example 206 | 249 | 0.20 | 93% |
| Example 207 | 142 | 0.15 | 99% |
| Example 208 | 111 | 0.14 | 100% |
| Example 209 | 133 | 0.19 | 98% |
| Example 210 | 97 | 0.14 | 99% |
| Example 211 | 111 | 0.12 | 99% |
| Example 212 | 86 | 0.07 | 97% |
| Example 213 | 128 | 0.08 | 56% |
| Example 214 | 171 | 0.10 | 58% |
| Example 215 | 690 | 0.23 | 53% |
| Example 220 | 201 | 0.14 | 67% |
| Example 221 | 196 | 0.09 | 68% |
| Example 226 | 197 | 0.10 | 57% |
| Example 228 | 114 | 0.07 | 98% |
| Example 229 | 306 | 0.19 | 57% |
| Example 230 | 84 | 0.12 | 97% |
| Example 231 | 92 | 0.11 | 98% |
| Example 232 | 98 | 0.15 | 97% |
| Example 233 | 93 | 0.15 | 97% |
| Example 234 | 104 | 0.14 | 98% |
| Example 235 | 117 | 0.07 | 98% |
| Example 236 | 98 | 0.13 | 97% |
| Example 237 | 110 | 0.14 | 97% |
| Example 238 | 102 | 0.10 | 94% |
| Example 239 | 97 | 0.19 | 94% |
| Example 240 | 115 | 0.19 | 94% |
| Example 241 | 131 | 0.12 | 85% |
| Example 242 | 111 | 0.06 | 93% |
| Example 244 | 94 | 0.12 | 95% |
| Example 245 | 99 | 0.16 | 95% |
| Example 246 | 97 | 0.10 | 94% |
| Example 249 | 95 | 0.17 | 88% |
| Example 250 | 113 | 0.15 | 80% |
| Example 251 | 132 | 0.15 | 70% |
| Example 253 | 145 | 0.10 | 96% |
| Example 254 | 116 | 0.07 | 97% |
| Example 255 | 162 | 0.51 | 96% |
| Example 256 | 97 | 0.14 | 99% |
| Example 257 | 102 | 0.10 | 94% |
| Example 258 | 162 | 0.51 | 96% |
| Example 274 | 84 | 0.07 | 98% |
| Example 275 | 90 | 0.09 | 97% |
| Example 276 | 134 | 0.14 | 97% |
| Example 277 | 86 | 0.10 | 98% |
| Example 278 | 94 | 0.07 | 98% |
| Example 284 | 122 | 0.07 | 94% |
| Example 285 | 106 | 0.08 | 94% |
| Example 286 | 151 | 0.05 | 94% |
| Example 287 | 111 | 0.09 | 95% |
| Example 288 | 97 | 0.11 | 97% |
| Example 289 | 123 | 0.11 | 97% |
| Example 291 | 166 | 0.07 | 92% |
| Example 294 | 192 | 0.04 | 90% |
| Example 296 | 219 | 0.21 | 80% |
| Example 297 | 114 | 0.05 | 74% |
| Example 299 | 97 | 0.13 | 94% |
| Example 300 | 111 | 0.11 | 95% |
| Example 301 | 96 | 0.12 | 86% |
| Example 302 | 141 | 0.11 | 95% |
| Example 304 | 163 | 0.06 | 80% |
| Example 306 | 150 | 0.03 | 92% |
| Example 310 | 104 | 0.10 | 96% |
| Example 311 | 155 | 0.11 | 94% |
| Example 312 | 116 | 0.07 | 94% |
| Example 313 | 99 | 0.12 | 96% |
| Example 314 | 127 | 0.12 | 95% |
| Example 315 | 126 | 0.14 | 94% |
| Example 316 | 144 | 0.03 | 95% |
| Example 317 | 224 | 0.03 | 84% |
| Example 318 | 485 | 0.25 | 68% |
| Example 320 | 195 | 0.07 | 88% |
| Example 322 | 190 | 0.17 | 54% |
| Example 324 | 130 | 0.13 | 96% |
| Example 325 | 116 | 0.07 | 96% |
| Example 326 | 132 | 0.09 | 96% |
| Example 327 | 110 | 0.12 | 95% |
| Example 328 | 119 | 0.08 | 96% |
| Example 329 | 144 | 0.08 | 94% |
| Example 330 | 108 | 0.09 | 95% |
| Example 338 | 128 | 0.14 | 94% |
| Example 339 | 91 | 0.06 | 96% |
| Example 340 | 177 | 0.05 | 79% |
| Example 341 | 122 | 0.09 | 95% |
| Example 342 | 90 | 0.09 | 95% |
| Example 343 | 156 | 0.06 | 75% |
| Example 346 | 108 | 0.07 | 94% |
| Example 347 | 115 | 0.09 | 98% |
| Example 348 | 123 | 0.09 | 97% |
| Example 349 | 133 | 0.11 | 97% |

### [Test Example 2]

### [Materials and methods]

### <Preparation of activation culture medium>

The culture medium used for culturing T cells under the activation culture conditions consists of TexMACS^{TM} Medium (Miltenyi Biotec, 130-097-196) and 5 ng/ml human interleukin-2 (IL-2, Roche, 11147528001) (hereinafter, referred to as an activation culture medium).

### <Preparation and activation culture of T cells>

Frozen T cells (Human PB Pan-T, Cryo, STEMCELL Technologies, ST-70024) derived from peripheral blood of a healthy human donor were thawed by being placed in a water bath at 37°C for several minutes. The thawed T cells were resuspended in TexMACS^{™} Medium containing 1% BSA (bovine serum albumin) (SIGMA, A9576) and 20 U/ml DNase I (Worthington Biochemical, LS002139), further washed by centrifugation, and resuspended in an activation culture medium. The T cells were adjusted to a cell concentration of 1.0 × 10⁶ cells/ml using the present culture medium, and Dynabeads Human T-Activator CD3/CD28 (Thermo Fisher, DB11131) was added thereto so that the concentration thereof was 1.0 × 10⁶ beads/ml. The cells were seeded in a 24-well plate for cell culture and activated by being cultured in a 37°C and 5% CO₂ incubator for 3 days. On the third day of activation, the Dynabeads were removed from the T cell culture solution. The T cells pretreated as described above were used as activated T cells.

### <Preparation of non-activation culture medium>

The culture medium used in a case of culturing T cells under inactivation culture conditions consists of TexMACS^{™} Medium (Miltenyi Biotec, 130-097-196), 5 ng/ml human interleukin-2 (IL-2, Roche, 11147528001), 5 ng/ml Human IL-7 (Miltenyi Biotec 130-095-367), and 5 ng/ml Human IL-15 (Miltenyi Biotec, 130-095-760) (hereinafter, non-activation culture medium).

### <Preparation and non-activation culture of T cells>

Frozen T cells (Human PB Pan-T, Cryo, STEMCELL Technologies, ST-70024) derived from peripheral blood of a healthy human donor were thawed by being placed in a water bath at 37°C for several minutes. The thawed T cells were resuspended in TexMACS^{TM} Medium containing 1% BSA and 20 U/ml DNase I, further washed by centrifugation, and resuspended in a non-activation culture medium. The T cells were adjusted to a cell concentration of 1.0 × 10⁶ cells/ml using the present culture medium, seeded in a 24-well plate for cell culture, and cultured in a 37°C, 5% CO₂ incubator for 3 days. The T cells pretreated as described above were used as non-activated T cells.

### <Flow cytometry>

On the 4th day after the start of the culture, the GFP-positive rate of the T cells to which the nucleic acid was delivered by each method was evaluated by flow cytometry.

For the evaluation of the GFP-positive rate, the T cells were stained with dead cells using BD Horizon^{™} Fixable Viability Stain (FVS) Reagents (BD, 565388). After the staining operation, the cells were fixed and washed, and the cell state was analyzed with an Attune device (Thermo Fisher). The analysis data was analyzed using Flowjo software. The T cells were gated by size, single cell, and living cell, and then the ratio of GFP-positive cells and the median fluorescence intensity (MFI) were analyzed.

### <Test Example 2-1> Delivery of nucleic acid to activated T cells

The activated T cells on the 3rd day of culture were collected in the required number of cells, centrifuged, and the supernatant was removed. The activated T cells were adjusted to a concentration of 1.0 × 10⁶ cells/ml in an activation culture medium containing recombinant human apolipoprotein E3 (ApoE3) (FUJIFILM Wako Pure Chemical Corporation, 010-20261) at a final concentration of 1 µg/ml, which was prepared in advance, and seeded in a 96-well plate.

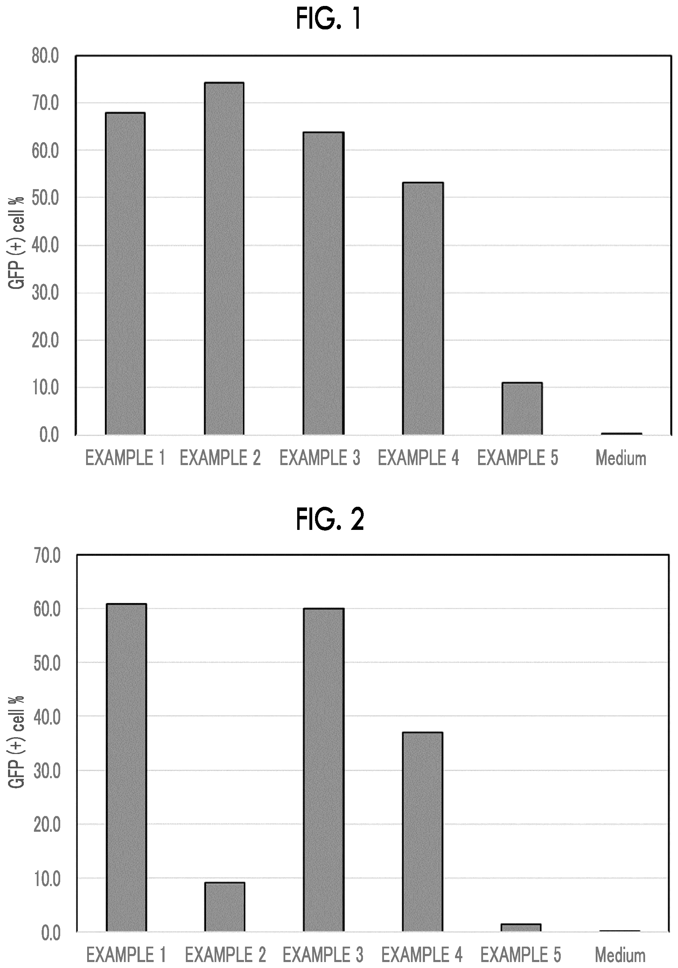

Using the GFP mRNA-encapsulating LNPs prepared in Examples 1 to 5, the LNPs were added such that the amount of RNA was 1.0 µg (total RNA amount) per 1.0 × 10⁶ cells, and the cells were cultured in a 37°C, 5% CO₂ incubator. The cells were collected 24 hours after the addition of the LNP, the GFP-positive cell ratio and MFI of the T cells treated with each LNP was measured by flow cytometry, and the GFP mRNA introduction efficiency was evaluated. The present results are shown in FIG. 1.

All of the LNP of Examples 1 to 5 were capable of efficiently delivering mRNA to activated T cells.

### <Test Example 2-2> Delivery of nucleic acid to non-activated T cells

The non-activated T cells on the 3rd day of the culture were collected in the required number of cells, centrifuged, and the supernatant was removed. The non-activated T cells were adjusted to a concentration of 1.0 × 10⁶ cells/ml in an activation culture medium containing ApoE3 at a final concentration of 1 µg/ml, which had been prepared in advance, and seeded in a 96-well plate. Using the GFP mRNA-encapsulating LNPs prepared in Examples 1 to 5, the LNPs were added such that the amount of RNA was 1.0 µg (total RNA amount) per 1.0 × 10⁶ cells, and the cells were cultured in a 37°C, 5% CO₂ incubator.

The cells were collected 24 hours after the addition of the LNP, the GFP-positive cell ratio and MFI of the T cells treated with each LNP was measured by flow cytometry, and the GFP mRNA introduction efficiency was evaluated. The present results are shown in FIG. 2.

It was shown that all of the LNPs of Examples 1 to 5 were capable of delivering mRNA to non-activated T cells, and particularly, the LNP of Examples 1, 3, and 4 were highly efficient.

### <Test Example 3-1> Delivery of nucleic acid to activated T cells

The activated T cells on the 3rd day of culture were collected in the required number of cells, centrifuged, and the supernatant was removed. The activated T cells were adjusted to a concentration of 1.0 × 10⁶ cells/ml in an activation culture medium containing no ApoE3 and seeded in a 96-well plate.

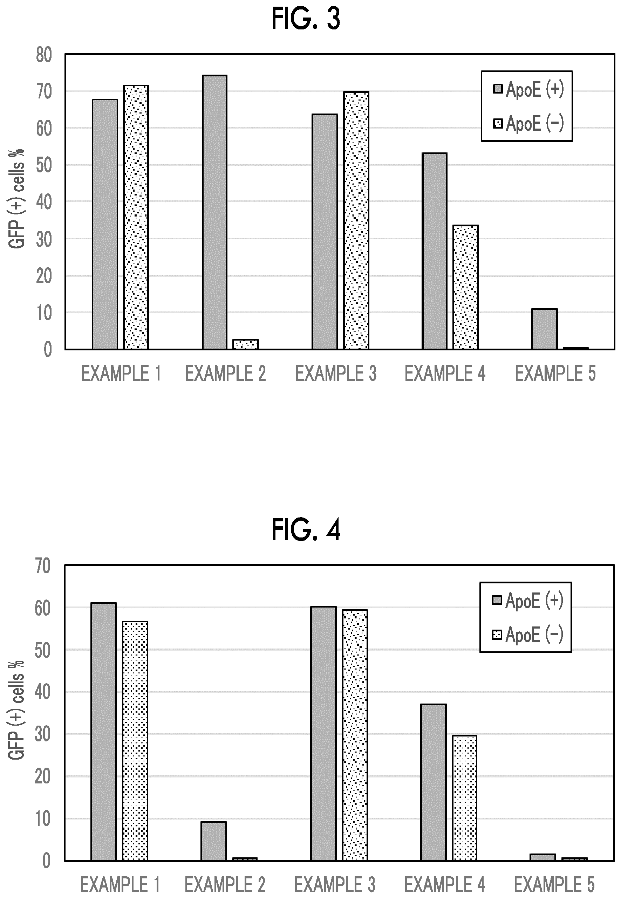

Using the GFP mRNA-encapsulating LNPs prepared in Examples 1 to 5, the LNPs were added such that the amount of RNA was 1.0 µg (total RNA amount) per 1.0 × 10⁶ cells, and the cells were cultured in a 37°C, 5% CO₂ incubator. The cells were collected 24 hours after the addition of the LNP, the GFP-positive cell ratio and MFI of the T cells treated with each LNP was measured by flow cytometry, and the GFP mRNA introduction efficiency was evaluated. The present results are shown in FIG. 3 (the data in the presence of ApoE3 shown in FIG. 1 as a reference example are also shown together).

The LNP of Examples 1, 3, and 4 was capable of efficiently delivering mRNA to activated T cells even in an activation environment in which the ApoE3 was not present.

### <Test Example 3-2> Delivery of nucleic acid to non-activated T cells

The non-activated T cells on the 3rd day of the culture were collected in the required number of cells, centrifuged, and the supernatant was removed. The non-activated T cells were adjusted to a concentration of 1.0 × 10⁶ cells/ml in an activation culture medium containing no ApoE3, and seeded in a 96-well plate. Using the GFP mRNA-encapsulating LNPs prepared in Examples 1 to 5, the LNPs were added such that the amount of RNA was 1.0 µg (total RNA amount) per 1.0 × 10⁶ cells, and the cells were cultured in a 37°C, 5% CO₂ incubator.

The cells were collected 24 hours after the addition of the LNP, the GFP-positive cell ratio and MFI of the T cells treated with each LNP was measured by flow cytometry, and the GFP mRNA introduction efficiency was evaluated. The present results are shown in FIG. 4 (the data in the presence of ApoE3 shown in FIG. 2 as a reference example are also shown together).

The LNP of Examples 1, 3, and 4 was capable of efficiently delivering mRNA to non-activated T cells even in an activation environment in which the ApoE3 was not present.

### <Test Example 4> Delivery of plasmid DNA to activated T cells using conventional method LNP

### <1> LNP treatment of activated T cells

The activated T cells on the 3rd day of culture were collected in the required number of cells, centrifuged, and the supernatant was removed. The activated T cells were adjusted to a concentration of 1.0 × 10⁶ cells/ml in an activation culture medium or an activation CDM culture medium, which contained recombinant human apolipoprotein E3 (ApoE3) (FUJIFILM Wako Pure Chemical Corporation, 010-20261) at a final concentration of 1 µg/ml, and seeded in a 96-well plate.

Using the GFP pDNA-encapsulating LNPs of Examples 130 to 147, the LNPs were added such that the amount of DNA was in an amount corresponding to 0.4 to 10.0 µg of total DNA per 1.0 × 10⁶ cells, and the cells were cultured in a 37°C, 5% CO₂ incubator.

### <2> GFP-positive cell ratio in activated T cells

On the 4th day after the start of the culture, the GFP-positive cell ratio of the T cells treated with each method was measured by flow cytometry, and the plasmid DNA introduction efficiency was evaluated. The results are shown in Table 8.

**[Table 8]**

| <Table 8> pDNA introduction efficiency in activated T cells | | |
|---|---|---|
| | Addition concentration (µg/10⁶ cells) | GFP-positive cell ratio (%) |
| Example 130 | 1.8 | 1.1 |
| Example 131 | 1.8 | 1.3 |
| Example 133 | 1.8 | 0.6 |
| Example 135 | 1.8 | 1.8 |
| Example 136 | 1.8 | 2.7 |
| Example 137 | 1.8 | 4.7 |
| Example 138 | 1.8 | 5.0 |
| Example 139 | 1.8 | 8.5 |
| Example 140 | 1.8 | 1.7 |
| Example 141 | 1.8 | 2.2 |
| Example 142 | 1.8 | 1.0 |
| Example 143 | 1.8 | 0.9 |
| Example 144 | 1.8 | 1.2 |
| Example 145 | 1.8 | 2.1 |
| Example 147 | 1.8 | 0.3 |

### <Test Example 5> Nucleic acid delivery to activated T cells using post-addition LNP (TCR KO)

### <1> LNP treatment of activated T cells

The activated T cells on the 3rd day of culture were collected in the required number of cells, centrifuged, and the supernatant was removed. The activated T cells were adjusted to a concentration of 1.0 × 10⁶ cells/ml in an activation culture medium containing recombinant human apolipoprotein E3 (ApoE3) (FUJIFILM Wako Pure Chemical Corporation, 010-20261) at a final concentration of 1 µg/ml, which was prepared in advance, and seeded in a 96-well plate.

Using the RNA-encapsulating LNP prepared by the post-addition method of Examples 8 to 129 and Example 405, the LNP was added such that the amount of RNA was 1.8 to 4.0 µg (total RNA amount) per 1.0 × 10⁶ cells, and the cells were cultured in a 37°C, 5% CO₂ incubator.

24 hours after the addition of LNP, the activation culture medium was added to the T cell suspension at a volume ratio of 1:3, and the cells were expanded and cultured for another 3 days.

### <2> TCR KO efficiency in activated T cells

On the 7th day of the start of the culture, the TCR-negative cell ratio of the T cells treated with each method was measured by flow cytometry, and the TCR KO efficiency was evaluated. The results are shown in Table 9.

**[Table 9]**

| <Table 9> TCR KO efficiency in activated T cells | | |
|---|---|---|
| | Addition concentration (µg/10⁶ cells) | TCR KO efficiency |
| Example 8 | 1.8 | 2% |
| Example 9 | 1.8 | 1% |
| Example 10 | 1.8 | 2% |
| Example 11 | 1.8 | 17% |
| Example 12 | 1.8 | 2% |
| Example 13 | 1.8 | 1% |
| Example 14 | 1.8 | 1% |
| Example 15 | 1.8 | 2% |
| Example 16 | 1.8 | 3% |
| Example 17 | 1.8 | 1% |
| Example 18 | 1.8 | 2% |
| Example 19 | 1.8 | 7% |
| Example 20 | 1.8 | 6% |
| Example 21 | 1.8 | 1% |
| Example 22 | 1.8 | 1% |
| Example 23 | 1.8 | 8% |
| Example 24 | 1.8 | 6% |
| Example 25 | 1.8 | 2% |
| Example 26 | 1.8 | 1% |
| Example 27 | 1.8 | 1% |
| Example 28 | 1.8 | 1% |
| Example 29 | 1.8 | 1% |
| Example 30 | 1.8 | 2% |
| Example 31 | 1.8 | 2% |
| Example 32 | 1.8 | 2% |
| Example 33 | 1.8 | 2% |
| Example 34 | 1.8 | 2% |
| Example 35 | 1.8 | 80% |
| Example 36 | 1.8 | 5% |
| Example 37 | 1.8 | 2% |
| Example 38 | 1.8 | 2% |
| Example 39 | 1.8 | 9% |
| Example 40 | 1.8 | 87% |
| Example 41 | 1.8 | 58% |
| Example 42 | 1.8 | 96% |
| Example 43 | 1.8 | 92% |
| Example 44 | 1.8 | 39% |
| Example 45 | 1.8 | 6% |
| Example 46 | 1.8 | 2% |
| Example 47 | 1.8 | 2% |
| Example 48 | 1.8 | 2% |
| Example 49 | 1.8 | 2% |
| Example 50 | 1.8 | 2% |
| Example 51 | 1.8 | 2% |
| Example 52 | 1.8 | 2% |
| Example 53 | 1.8 | 3% |
| Example 54 | 1.8 | 4% |
| Example 55 | 1.8 | 2% |
| Example 56 | 1.8 | 3% |
| Example 57 | 1.8 | 2% |
| Example 58 | 1.8 | 1% |
| Example 59 | 1.8 | 2% |
| Example 60 | 1.8 | 34% |
| Example 61 | 1.8 | 2% |
| Example 62 | 1.8 | 91% |
| Example 63 | 1.8 | 96% |
| Example 64 | 1.8 | 30% |
| Example 65 | 1.8 | 78% |
| Example 66 | 1.8 | 92% |
| Example 67 | 1.8 | 66% |
| Example 68 | 1.8 | 96% |
| Example 69 | 1.8 | 91% |
| Example 70 | 1.8 | 56% |
| Example 71 | 1.8 | 70% |
| Example 72 | 1.8 | 71% |
| Example 73 | 1.8 | 26% |
| Example 74 | 1.8 | 43% |
| Example 75 | 1.8 | 25% |
| Example 76 | 1.8 | 64% |
| Example 77 | 1.8 | 83% |
| Example 78 | 1.8 | 68% |
| Example 79 | 1.8 | 21% |
| Example 80 | 1.8 | 2% |
| Example 81 | 1.8 | 2% |
| Example 82 | 1.8 | 2% |
| Example 83 | 1.8 | 2% |
| Example 84 | 1.8 | 2% |
| Example 85 | 1.8 | 11% |
| Example 86 | 1.8 | 95% |
| Example 87 | 1.8 | 98% |
| Example 88 | 4.0 | 97% |
| Example 89 | 4.0 | 93% |
| Example 90 | 4.0 | 96% |
| Example 91 | 4.0 | 92% |
| Example 92 | 1.8 | 96% |
| Example 93 | 1.8 | 56% |
| Example 94 | 1.8 | 98% |
| Example 95 | 1.8 | 80% |
| Example 96 | 1.8 | 92% |
| Example 97 | 1.8 | 92% |
| Example 98 | 1.8 | 92% |
| Example 99 | 1.8 | 77% |
| Example 100 | 1.8 | 42% |
| Example 101 | 1.8 | 94% |
| Example 102 | 1.8 | 94% |
| Example 103 | 1.8 | 93% |
| Example 104 | 1.8 | 51% |
| Example 105 | 1.8 | 2% |
| Example 106 | 1.8 | 59% |
| Example 107 | 1.8 | 2% |
| Example 108 | 1.8 | 5% |
| Example 109 | 1.8 | 36% |
| Example 110 | 1.8 | 96% |
| Example 111 | 1.8 | 85% |
| Example 112 | 1.8 | 82% |
| Example 113 | 1.8 | 97% |
| Example 114 | 1.8 | 91% |
| Example 115 | 1.8 | 65% |
| Example 116 | 1.8 | 62% |
| Example 117 | 1.8 | 2% |
| Example 118 | 1.8 | 28% |
| Example 119 | 1.8 | 83% |
| Example 120 | 1.8 | 96% |
| Example 121 | 1.8 | 85% |
| Example 122 | 1.8 | 82% |
| Example 123 | 1.8 | 97% |
| Example 124 | 1.8 | 91% |
| Example 125 | 1.8 | 65% |
| Example 126 | 1.8 | 62% |
| Example 127 | 1.8 | 2% |
| Example 128 | 1.8 | 28% |
| Example 129 | 1.8 | 83% |
| Example 405 | 1.8 | 2% |

### <Test Example 6> Delivery of plasmid DNA to activated T cells using post-addition LNP

### <1> LNP treatment of activated T cells

The activated T cells on the 3rd day of culture were collected in the required number of cells, centrifuged, and the supernatant was removed. The activated T cells were adjusted to a concentration of 1.0 × 10⁶ cells/ml in an activation culture medium or an activation CDM culture medium, which contained recombinant human apolipoprotein E3 (ApoE3) (FUJIFILM Wako Pure Chemical Corporation, 010-20261) at a final concentration of 1 µg/ml, and seeded in a 96-well plate.

Using the plasmid DNA-encapsulating LNP prepared by the post-addition method of Examples 148 to 404, the LNP was added thereto such that the amount of RNA was 0.4 to 10.0 µg (total RNA amount) per 1.0 × 10⁶ cells, and cultured in a 37°C, 5% CO₂ incubator.

### <2> GFP-positive cell ratio in activated T cells

On the 4th day after the start of the culture, the GFP-positive cell ratio of the T cells treated with each method was measured by flow cytometry, and the plasmid DNA introduction efficiency was evaluated. The results are shown in Table 10.

**[Table 10]**

| <Table 10> pDNA introduction efficiency in activated T cells | | |
|---|---|---|
| | Addition concentration (µg/10⁶ cells) | GFP-positive cell ratio (%) |
| Example 148 | 1.8 | 0.5 |
| Example 149 | 1.8 | 0.5 |
| Example 153 | 1.8 | 12.8 |
| Example 154 | 1.8 | 2.9 |
| Example 155 | 1.8 | 11.7 |
| Example 156 | 1.8 | 6.3 |
| Example 157 | 1.8 | 0.9 |
| Example 159 | 1.8 | 0.8 |
| Example 162 | 1.8 | 0.6 |
| Example 163 | 1.8 | 2.1 |
| Example 166 | 1.8 | 5.2 |
| Example 167 | 1.8 | 2.2 |
| Example 168 | 1.8 | 4.2 |
| Example 169 | 1.8 | 1.2 |
| Example 170 | 1.8 | 0.7 |
| Example 172 | 1.8 | 0.3 |
| Example 176 | 1.8 | 7.9 |
| Example 177 | 1.8 | 1.0 |
| Example 178 | 1.8 | 3.0 |
| Example 179 | 1.8 | 4.5 |
| Example 180 | 1.8 | 0.6 |
| Example 181 | 1.8 | 0.8 |
| Example 182 | 1.8 | 1.8 |
| Example 183 | 1.8 | 3.7 |
| Example 186 | 1.8 | 9.3 |
| Example 188 | 1.8 | 1.2 |
| Example 189 | 1.8 | 1.8 |
| Example 190 | 0.6 | 3.4 |
| Example 191 | 0.6 | 4.3 |
| Example 192 | 0.6 | 0.4 |
| Example 193 | 0.6 | 0.5 |
| Example 194 | 0.2 | 1.5 |
| Example 195 | 0.2 | 1.9 |
| Example 197 | 0.2 | 0.4 |
| Example 203 | 1.8 | 1.5 |
| Example 207 | 1.8 | 1.3 |
| Example 208 | 1.8 | 2.8 |
| Example 209 | 1.8 | 3.8 |
| Example 212 | 1.8 | 0.4 |
| Example 213 | 1.8 | 3.1 |
| Example 222 | 1.8 | 1.1 |
| Example 225 | 1.8 | 0.7 |
| Example 230 | 1.8 | 2.6 |
| Example 231 | 1.8 | 2.7 |
| Example 232 | 1.8 | 0.5 |
| Example 233 | 1.8 | 2.8 |
| Example 234 | 1.8 | 2.0 |
| Example 235 | 1.8 | 1.8 |
| Example 236 | 1.8 | 7.0 |
| Example 237 | 1.8 | 4.1 |
| Example 238 | 1.8 | 5.5 |
| Example 239 | 1.8 | 3.9 |
| Example 240 | 1.8 | 5.2 |
| Example 241 | 1.8 | 4.3 |
| Example 242 | 1.8 | 2.4 |
| Example 243 | 1.8 | 2.4 |
| Example 244 | 1.8 | 1.7 |
| Example 245 | 1.8 | 1.8 |
| Example 246 | 1.8 | 3.6 |
| Example 247 | 1.8 | 1.8 |
| Example 253 | 1.8 | 0.8 |
| Example 254 | 1.8 | 6.4 |
| Example 255 | 1.8 | 13.6 |
| Example 257 | 1.8 | 5.5 |
| Example 258 | 1.8 | 13.6 |
| Example 259 | 1.8 | 1.0 |
| Example 260 | 1.8 | 4.0 |
| Example 261 | 1.8 | 3.3 |
| Example 262 | 1.8 | 5.1 |
| Example 263 | 1.8 | 2.0 |
| Example 264 | 1.8 | 1.2 |
| Example 265 | 1.8 | 1.6 |
| Example 266 | 1.8 | 1.4 |
| Example 267 | 1.8 | 1.9 |
| Example 268 | 1.8 | 2.2 |
| Example 272 | 1.8 | 0.5 |
| Example 273 | 1.8 | 1.3 |
| Example 274 | 1.8 | 29.0 |
| Example 275 | 1.8 | 9.8 |
| Example 276 | 1.8 | 39.8 |
| Example 277 | 1.8 | 31.9 |
| Example 278 | 1.8 | 10.1 |
| Example 279 | 1.8 | 20.9 |
| Example 280 | 1.8 | 16.7 |
| Example 281 | 1.8 | 1.8 |
| Example 282 | 1.8 | 7.7 |
| Example 283 | 1.8 | 22.5 |
| Example 284 | 1.8 | 7.3 |
| Example 285 | 1.8 | 1.7 |
| Example 286 | 1.8 | 1.0 |
| Example 287 | 1.8 | 9.6 |
| Example 288 | 1.8 | 7.2 |
| Example 289 | 1.8 | 10.2 |
| Example 291 | 1.8 | 1.5 |
| Example 293 | 1.8 | 1.1 |
| Example 294 | 1.8 | 0.7 |
| Example 295 | 1.8 | 1.2 |
| Example 298 | 1.8 | 0.3 |
| Example 299 | 1.8 | 35.2 |
| Example 300 | 1.8 | 0.5 |
| Example 301 | 1.8 | 1.1 |
| Example 302 | 1.8 | 38.3 |
| Example 304 | 1.8 | 2.2 |
| Example 305 | 1.8 | 2.0 |
| Example 306 | 1.8 | 2.1 |
| Example 307 | 1.8 | 0.8 |
| Example 308 | 1.8 | 2.9 |
| Example 309 | 1.8 | 0.4 |
| Example 310 | 1.8 | 36.2 |
| Example 311 | 1.8 | 2.4 |
| Example 312 | 1.8 | 31.3 |
| Example 313 | 1.8 | 25.6 |
| Example 314 | 1.8 | 42.2 |
| Example 315 | 1.8 | 36.7 |
| Example 316 | 1.8 | 3.4 |
| Example 317 | 1.8 | 0.8 |
| Example 319 | 1.8 | 1.2 |
| Example 320 | 1.8 | 1.2 |
| Example 321 | 1.8 | 0.5 |
| Example 322 | 1.8 | 0.8 |
| Example 324 | 1.8 | 31.9 |
| Example 325 | 1.8 | 62.7 |
| Example 326 | 1.8 | 39.0 |
| Example 327 | 1.8 | 27.5 |
| Example 328 | 1.8 | 51.9 |
| Example 329 | 1.8 | 18.3 |
| Example 330 | 1.8 | 51.8 |
| Example 332 | 1.8 | 0.4 |
| Example 334 | 1.8 | 0.5 |
| Example 337 | 1.8 | 0.9 |
| Example 338 | 1.8 | 1.5 |
| Example 339 | 1.8 | 6.1 |
| Example 340 | 1.8 | 1.0 |
| Example 341 | 1.8 | 1.6 |
| Example 342 | 1.8 | 3.3 |
| Example 343 | 1.8 | 1.0 |
| Example 344 | 1.8 | 0.3 |
| Example 345 | 1.8 | 0.6 |
| Example 346 | 1.8 | 1.1 |
| Example 347 | 1.8 | 39.4 |
| Example 348 | 1.8 | 35.8 |
| Example 349 | 1.8 | 13.9 |
| Example 350 | 1.8 | 53.6 |
| Example 351 | 1.8 | 31.4 |
| Example 352 | 1.8 | 32.9 |
| Example 353 | 1.8 | 64.2 |
| Example 354 | 1.8 | 18.1 |
| Example 356 | 1.8 | 0.4 |
| Example 361 | 1.8 | 0.5 |
| Example 363 | 1.8 | 2.1 |
| Example 364 | 1.8 | 40.9 |
| Example 365 | 1.8 | 16.2 |
| Example 366 | 1.8 | 0.4 |
| Example 367 | 1.8 | 0.7 |
| Example 368 | 1.8 | 0.3 |
| Example 372 | 1.8 | 0.6 |
| Example 373 | 1.8 | 0.4 |
| Example 375 | 1.8 | 0.6 |
| Example 376 | 1.8 | 0.3 |
| Example 377 | 1.8 | 0.6 |
| Example 378 | 1.8 | 0.3 |
| Example 380 | 1.8 | 1.1 |
| Example 381 | 1.8 | 0.9 |
| Example 382 | 1.8 | 1.7 |
| Example 383 | 1.8 | 0.5 |
| Example 384 | 1.8 | 0.7 |
| Example 385 | 1.8 | 1.4 |
| Example 386 | 1.8 | 0.8 |
| Example 387 | 1.8 | 3.4 |
| Example 388 | 1.8 | 2.1 |
| Example 389 | 1.8 | 0.5 |
| Example 390 | 1.8 | 0.5 |
| Example 391 | 1.8 | 1.2 |
| Example 392 | 1.8 | 2.5 |
| Example 393 | 1.8 | 0.3 |
| Example 396 | 1.8 | 1.7 |
| Example 397 | 1.8 | 0.8 |
| Example 398 | 1.8 | 2.2 |
| Example 399 | 1.8 | 1.2 |
| Example 400 | 1.8 | 0.6 |
| Example 401 | 1.8 | 1.3 |
| Example 402 | 1.8 | 0.5 |
| Example 404 | 1.8 | 1.2 |

### <Test Example 7> Nucleic acid delivery to activated T cells using post-addition LNP (TCR KO)

### <1> LNP treatment of activated T cells

The activated T cells on the 3rd day of culture were collected in the required number of cells, centrifuged, and the supernatant was removed. The activated T cells were adjusted to a concentration of 1.0 × 10⁶ cells/ml in an activation culture medium containing recombinant human apolipoprotein E3 (ApoE3) (FUJIFILM Wako Pure Chemical Corporation, 010-20261) at a final concentration of 1 µg/ml, which was prepared in advance, and seeded in a 96-well plate.

Using the RNA-encapsulating LNP prepared by the post-addition method of Examples 88 to 90, the RNA-encapsulating LNP was added thereto such that the amount of RNA was 0.4 to 4.0 µg (total RNA amount) per 1.0 × 10⁶ cells, and cultured in a 37°C, 5% CO₂ incubator.

24 hours after the addition of LNP, the activation culture medium was added to the T cell suspension at a volume ratio of 1:3, and the cells were expanded and cultured for another 3 days.

### <2> TCR KO efficiency in activated T cells

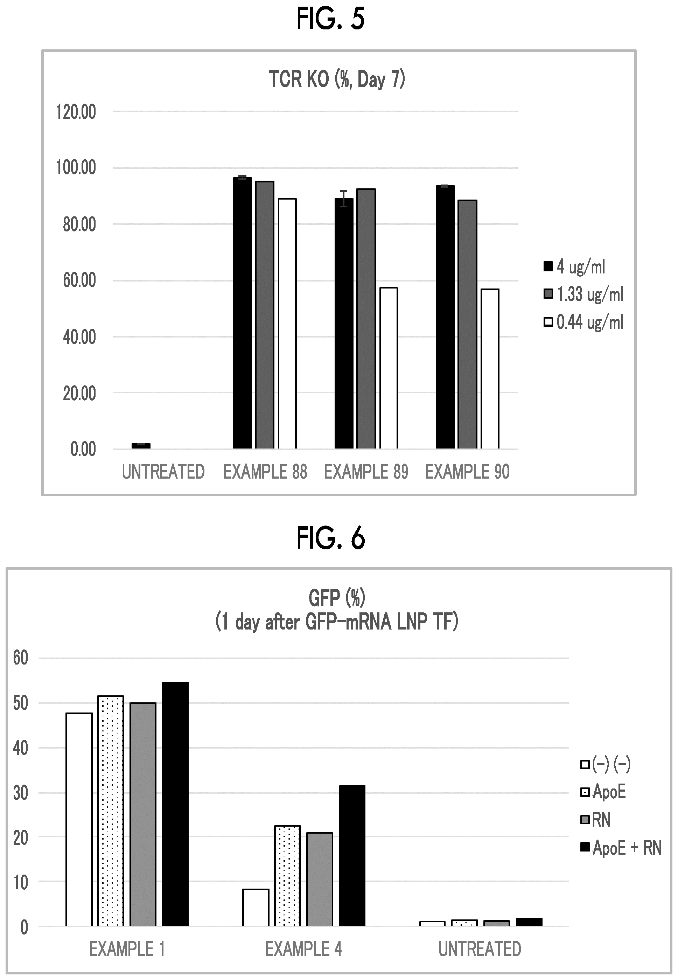

On the 7th day of the start of the culture, the TCR-negative cell ratio of the T cells treated with each method was measured by flow cytometry, and the TCR KO efficiency was evaluated. The results are shown in FIG. 5.

### <Test Example 8> Nucleic acid delivery to activated T cells using addtive

The activated T cells on the 3rd day of culture were collected in the required number of cells, centrifuged, and the supernatant was removed. The activated T cells were adjusted to a concentration of 1.0 × 10⁶ cells/ml in an activation culture medium containing recombinant human apolipoprotein E3 (ApoE3) (FUJIFILM Wako Pure Chemical Corporation, 010-20261) or RetroNectin (Takara Bio Inc.) at a final concentration of 1 µg/ml, which was prepared in advance, and seeded in a 96-well plate.

Using the GFP mRNA-encapsulating LNPs prepared in Examples 1 and 4, the LNPs were added such that the amount of RNA was 4.0 µg (total RNA amount) per 1.0 × 10⁶ cells, and the cells were cultured in a 37°C, 5% CO₂ incubator. The cells were collected 24 hours after the addition of the LNP, the GFP-positive cell ratio of the T cells treated with each LNP was measured by flow cytometry, and the GFP mRNA introduction efficiency was evaluated. The present results are shown in FIG. 6.

In the LNP of Examples 1 and 4, the efficiency of mRNA delivery to the activated T cells was improved by the addition of RetroNectin alone, and the efficiency was further improved by the combined use of ApoE3 and RetroNectin.

### <Test Example 9> Nucleic acid delivery to T cells after long-term culture

The T cells on the 10th day of the culture (7 days after activation) were collected in the required number of cells, centrifuged, and the supernatant was removed. The activated T cells were adjusted to a concentration of 1.0 × 10⁶ cells/ml in an activation culture medium containing recombinant human apolipoprotein E3 (ApoE3) (FUJIFILM Wako Pure Chemical Corporation, 010-20261) at a final concentration of 1 µg/ml, which was prepared in advance, and seeded in a 96-well plate.

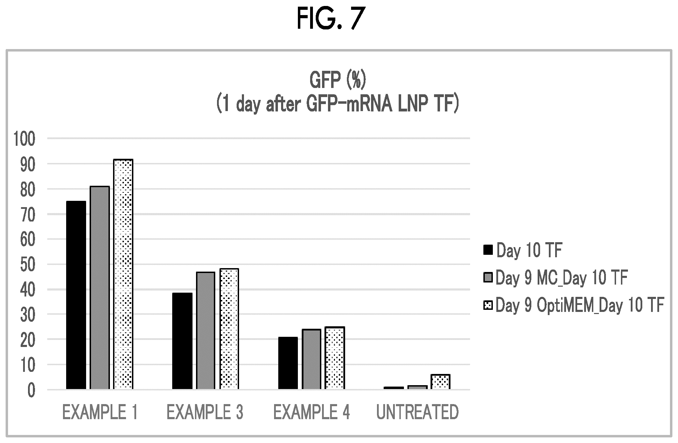

Using the GFP mRNA-encapsulating LNPs prepared in Examples 1, 3, and 4, the LNPs were added such that the amount of RNA was 4.0 µg (total RNA amount) per 1.0 × 10⁶ cells, and the cells were cultured in a 37°C, 5% CO₂ incubator. The cells were collected 24 hours after the addition of the LNP, the GFP-positive cell ratio of the T cells treated with each LNP was measured by flow cytometry, and the GFP mRNA introduction efficiency was evaluated. The present results are shown in FIG. 7 (Day10 TF condition).

### <Test Example 10> Nucleic acid delivery to T cells after long-term culture

The T cells on the 9th day of the culture (6 days after activation) in the activation culture medium were collected, resuspended in a newly adjusted activation culture medium or 5 ng/ml IL-2-containing Opti-MEM (TM) I Reduced Serum Medium (Thermo Fisher, 31985062), and cultured for 1 day. The T cells on the 10th day of the culture were collected in the required number of cells, centrifuged, and the supernatant was removed. The activated T cells were adjusted to a concentration of 1.0 × 10⁶ cells/ml in an activation culture medium containing recombinant human apolipoprotein E3 (ApoE3) (FUJIFILM Wako Pure Chemical Corporation, 010-20261) at a final concentration of 1 µg/ml, which was prepared in advance, and seeded in a 96-well plate.

Using the GFP mRNA-encapsulating LNPs prepared in Examples 1, 3, and 4, the LNPs were added such that the amount of RNA was 4.0 µg (total RNA amount) per 1.0 × 10⁶ cells, and the cells were cultured in a 37°C, 5% CO₂ incubator. The cells were collected 24 hours after the addition of the LNP, the GFP-positive cell ratio of the T cells treated with each LNP was measured by flow cytometry, and the GFP mRNA introduction efficiency was evaluated. The present results are shown in FIG. 7 (Day9 MC_Day10 TF condition, Day9 OptiMEM_Day10 TF condition).

## Claims

1. A nucleic acid delivery agent for an immune cell, comprising:
a lipid composition containing an ionizable lipid that is a compound represented by Formula (1) or a salt thereof, a non-ionizable lipid, a lipid having a nonionic polymer, and a nucleic acid, 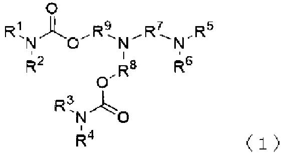
in the formula, R¹, R², R³, and R⁴ each independently represent a hydrogen atom or a hydrocarbon group having 1 to 24 carbon atoms which may be substituted,
substituents on the hydrocarbon groups having 1 to 24 carbon atoms which may be substituted, the hydrocarbon groups being represented by R¹, R², R³, and R⁴, each independently represent -C(O)O-R¹¹, -OC(O)-R¹², -O-R¹³, -CO-R¹⁴, -OC(O)O-R¹⁵, or -S-S-R¹⁶,
R¹¹, R¹², R¹³, R¹⁴, R¹⁵, and R¹⁶ each independently represent a hydrocarbon group having 1 to 24 carbon atoms which may be substituted with -S-R¹⁷, where R¹⁷ represents a hydrocarbon group having 1 to 12 carbon atoms,
R⁵ and R⁶ each independently represent a hydrocarbon group having 1 to 18 carbon atoms which may be substituted,
substituents on the hydrocarbon groups having 1 to 18 carbon atoms which may be substituted, the hydrocarbon groups being represented by R⁵ and R⁶, each independently represent -OH, -COOH, -NR²¹R²², -OC(O)O-R²³, -C(O)O-R²⁴, -OC(O)-R²⁵, -Q-R²⁶, -C(O)NR²⁷R²⁸, -NR²⁹C(O)R³⁰, -N(R³¹)S(O)₂R³², -N(R³³)C(O)N(R³⁴)R³⁵, -N(R³⁶)C(S)N(R³⁷)R³⁸, -OC(O)N(R³⁹)R⁴⁰, or -N(R⁴¹)C(O)OR⁴²,
R²¹ and R²² each independently represent a hydrogen atom or a hydrocarbon group having 1 to 8 carbon atoms,
R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, and R⁴² each independently represent a hydrogen atom or a hydrocarbon group having 1 to 24 carbon atoms which may be substituted, and substituents on the hydrocarbon groups having 1 to 24 carbon atoms which may be substituted, the hydrocarbon group being represented by R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, and R⁴², represent an aryl group having 6 to 20 carbon atoms, a heterocyclic group, -OH, -COOH, or -NR⁵¹R⁵², where R⁵¹ and R⁵² each independently represent a hydrogen atom or a hydrocarbon group having 1 to 8 carbon atoms,
R⁷, R⁸, and R⁹ each independently represent a hydrocarbon group having 2 to 8 carbon atoms, and
R⁵ and R⁶, or R⁵ and R⁷ may be taken together to form a 4- to 7-membered ring.

2. The nucleic acid delivery agent for an immune cell according to claim 1,
wherein a molar ratio of the ionizable lipid to total lipids in the lipid composition is 20 to 60 mol%.

3. The nucleic acid delivery agent for an immune cell according to claim 1,
wherein the non-ionizable lipid contains a sterol or a derivative thereof, and a phospholipid.

4. The nucleic acid delivery agent for an immune cell according to claim 3,
wherein the phospholipid is selected from the group consisting of distearoylphosphatidylcholine, dioleoylphosphatidylcholine, and dioleoylphosphatidylethanolamine.

5. The nucleic acid delivery agent for an immune cell according to claim 3,
wherein a molar ratio of the sterol or the derivative thereof to total lipids in the lipid composition is 30 to 70 mol%.

6. The nucleic acid delivery agent for an immune cell according to claim 3,
wherein a molar ratio of the phospholipid to total lipids in the lipid composition is 1 to 30 mol%.

7. The nucleic acid delivery agent for an immune cell according to any one of claims 1 to 6,
wherein the lipid having the nonionic polymer is a lipid having a polyethylene glycol chain.

8. The nucleic acid delivery agent for an immune cell according to claim 7,
wherein the lipid having the polyethylene glycol chain is selected from dimyristoyl-rac-glycerol polyethylene glycol, distearoyl-rac-glycerol polyethylene glycol, and distearoylphosphatidylethanolamine polyethylene glycol.

9. The nucleic acid delivery agent for an immune cell according to any one of claims 1 to 6,
wherein a molar ratio of the lipid having the nonionic polymer to total lipids in the lipid composition is 0.1 to 3 mol%.

10. The nucleic acid delivery agent for an immune cell according to any one of claims 1 to 6,
wherein a mass ratio of total lipids in the lipid composition to the nucleic acid is 7:1 to 1000:1.

11. The nucleic acid delivery agent for an immune cell according to any one of claims 1 to 6, wherein the immune cell is an activated cell or a non-activated cell.

12. The nucleic acid delivery agent for an immune cell according to any one of claims 1 to 6, wherein the ionizable lipid is one or more of the following compounds,

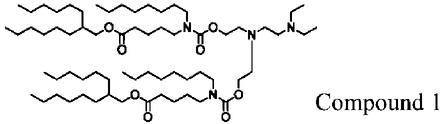

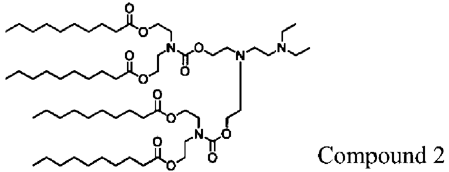

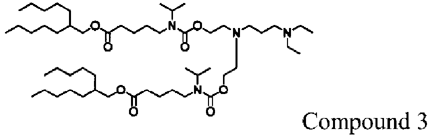

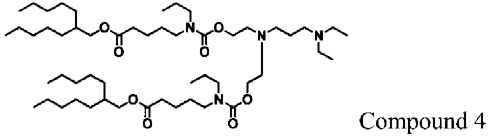

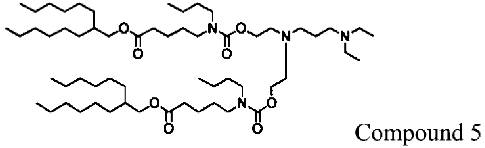

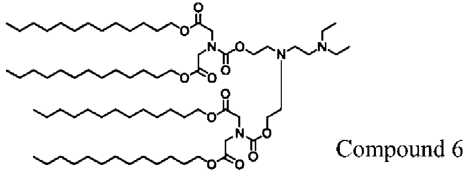

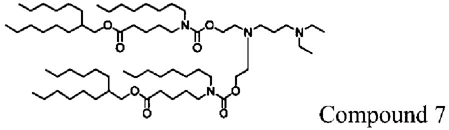

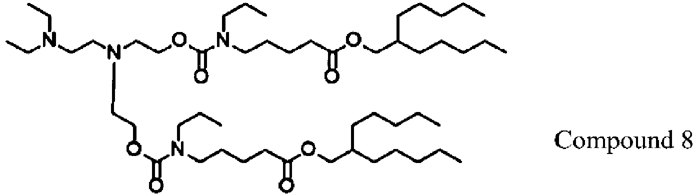

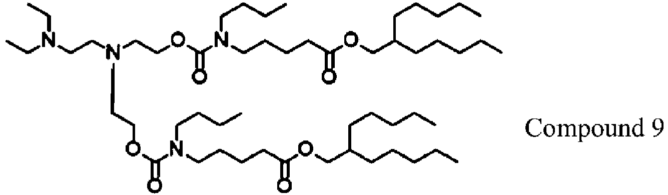

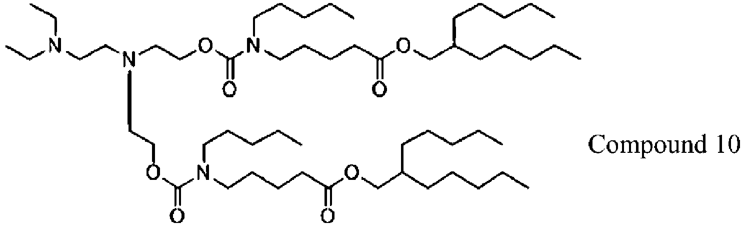

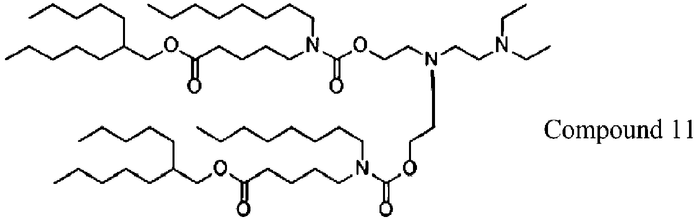

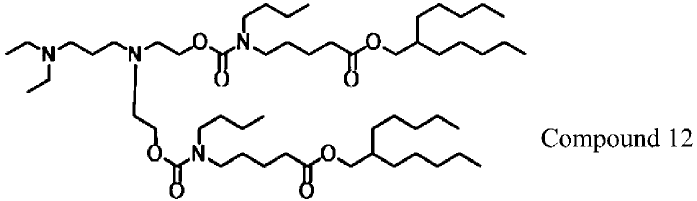

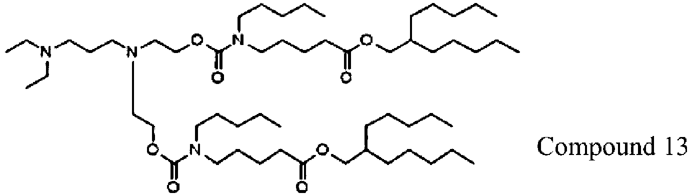

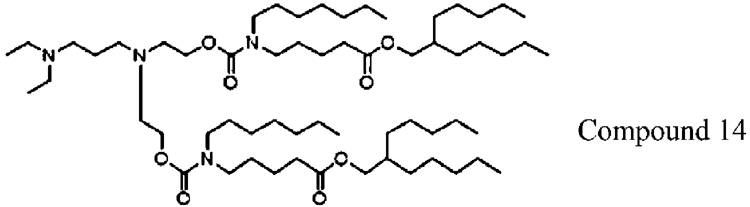

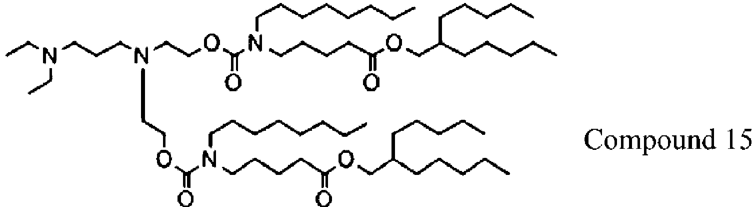

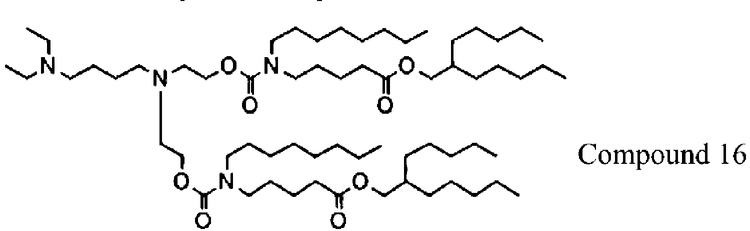

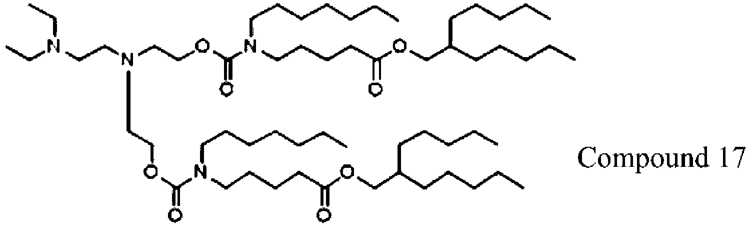

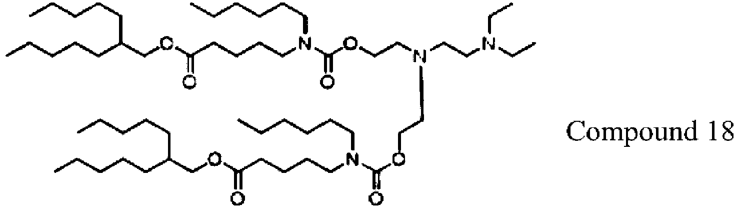

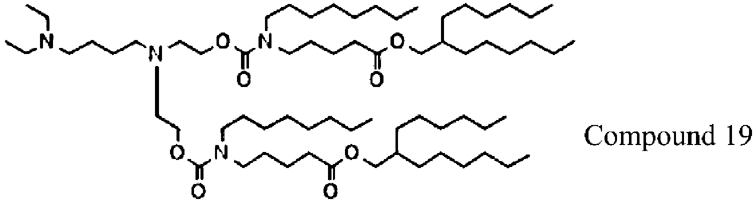

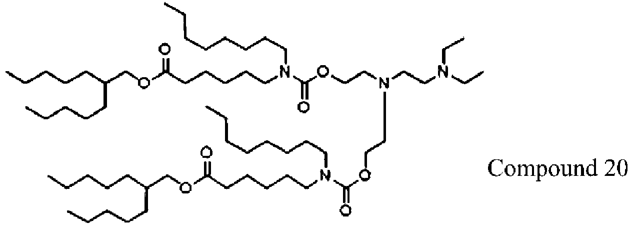

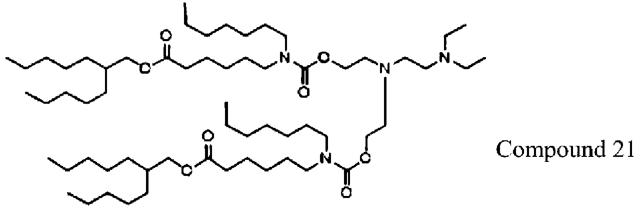

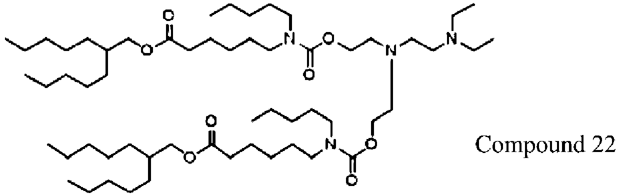

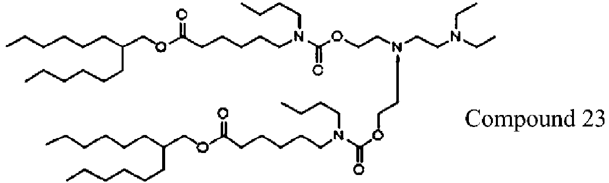

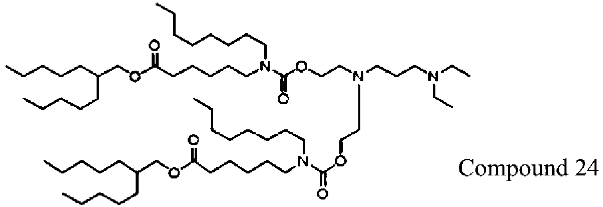

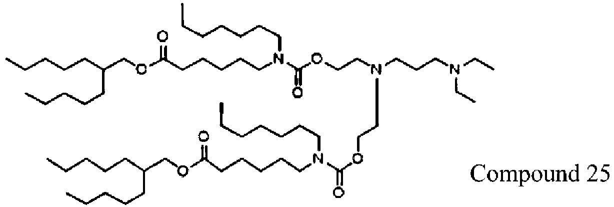

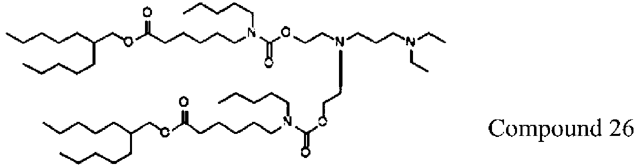

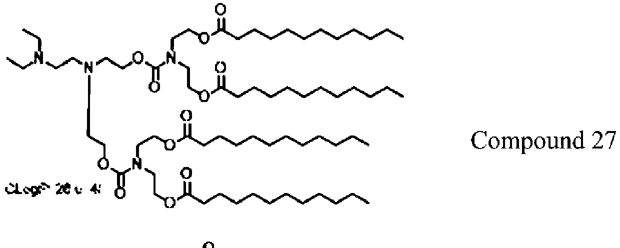

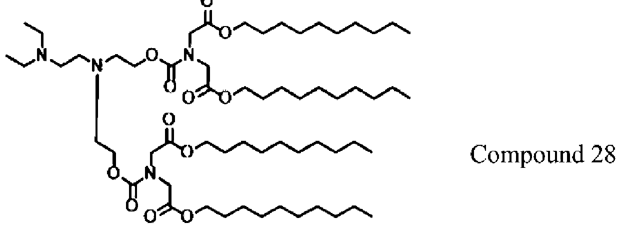

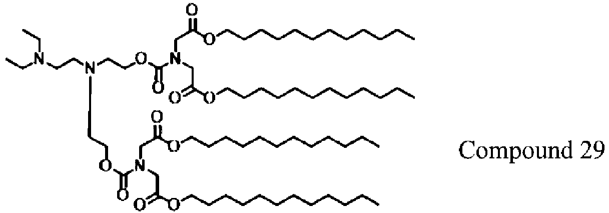

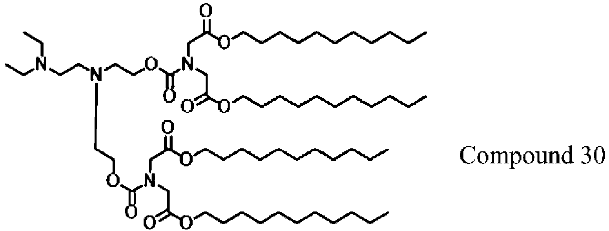

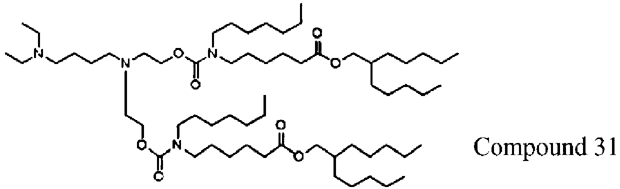

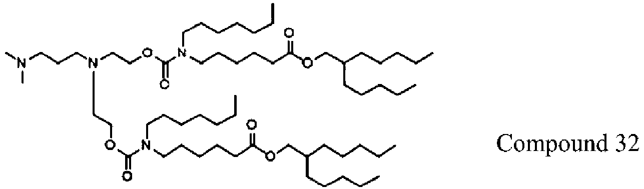

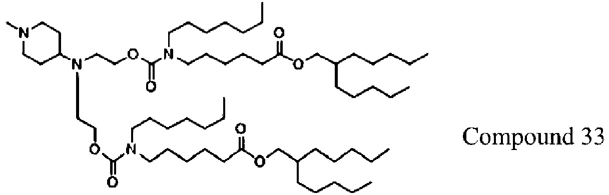

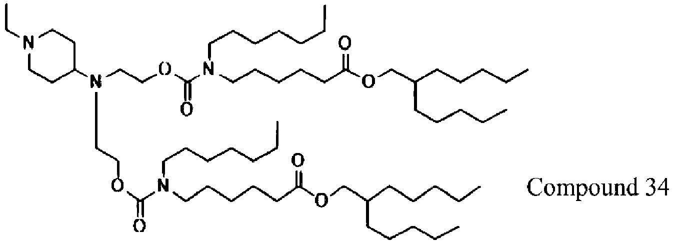

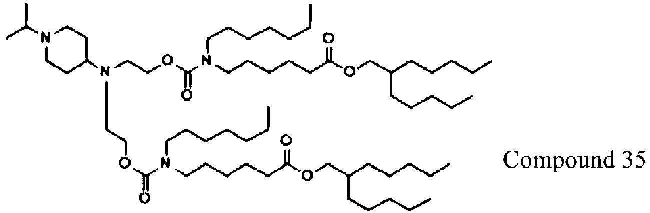

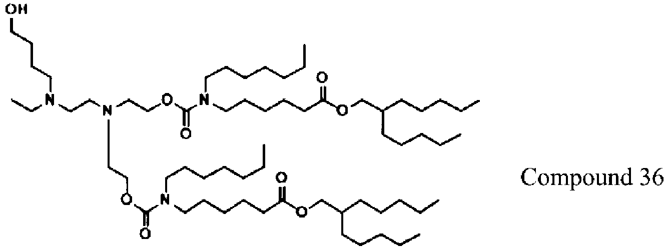

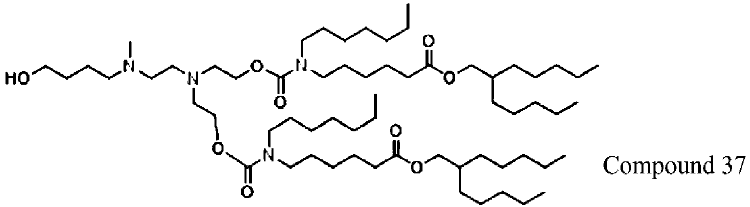

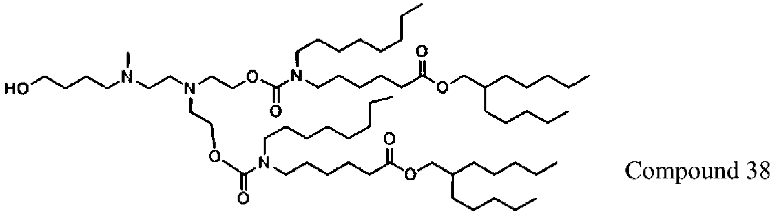

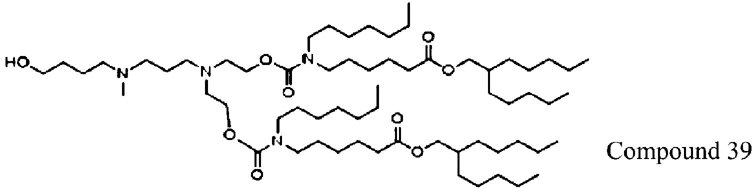

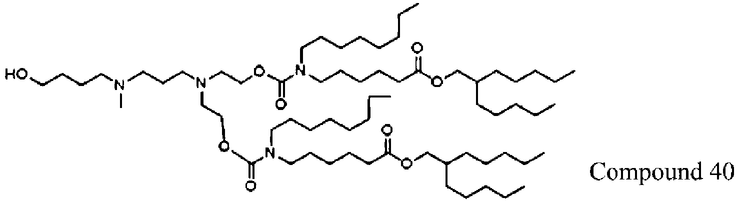

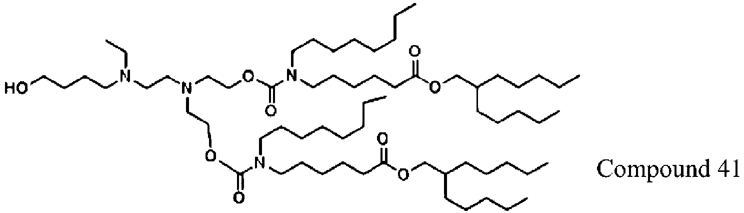

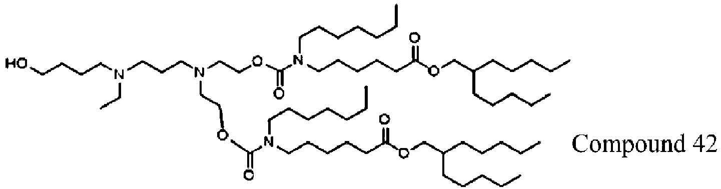

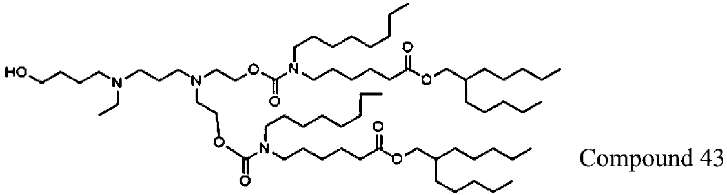

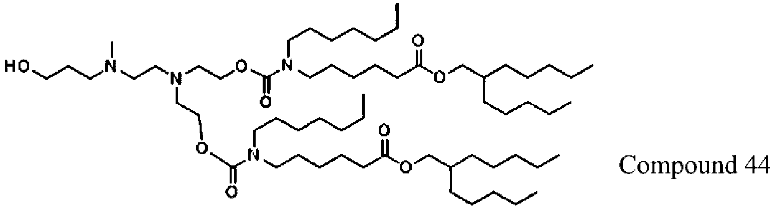

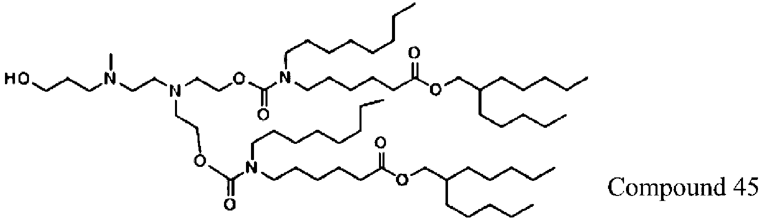

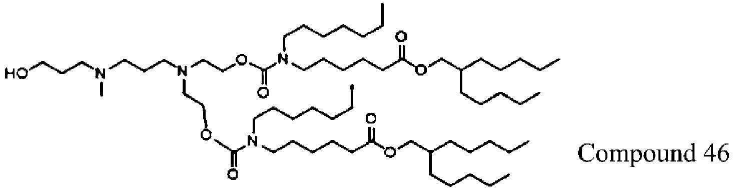

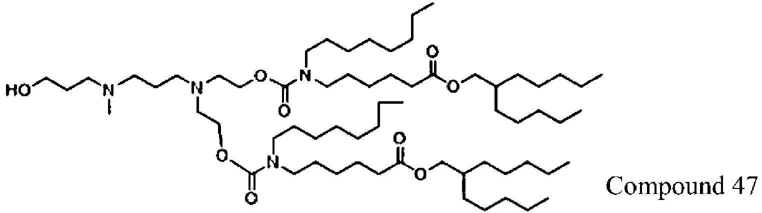

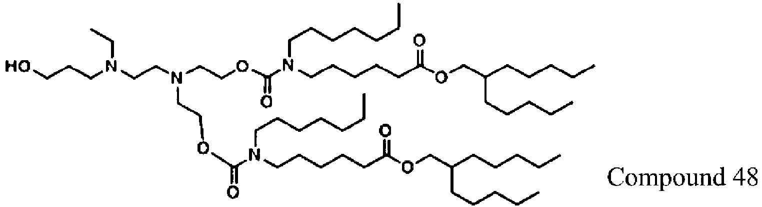

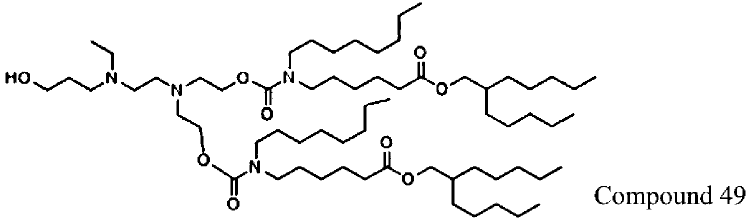

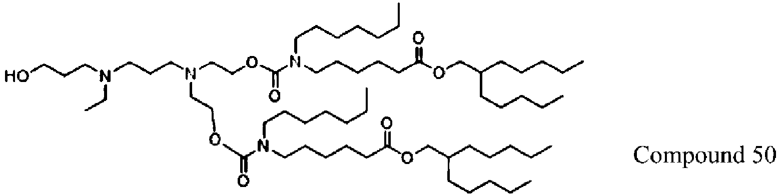

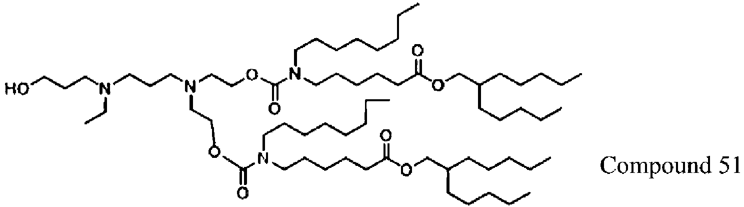

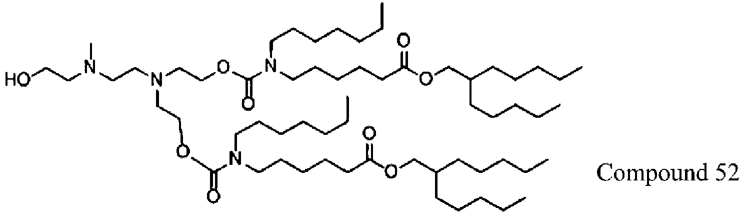

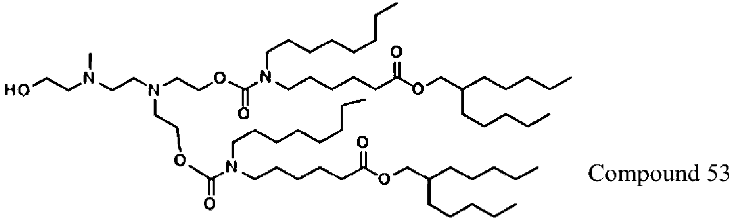

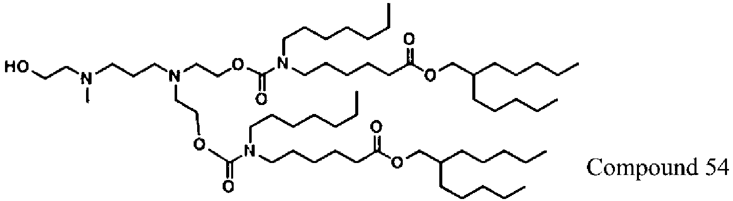

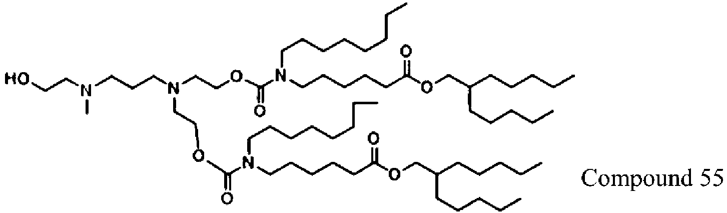

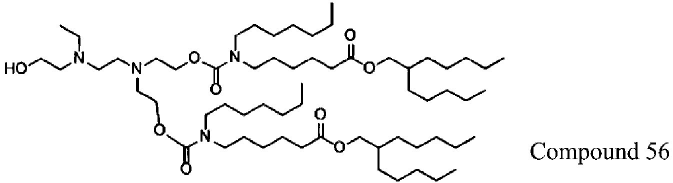

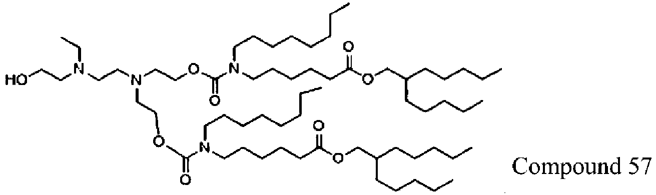

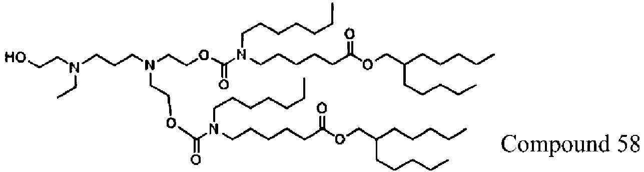

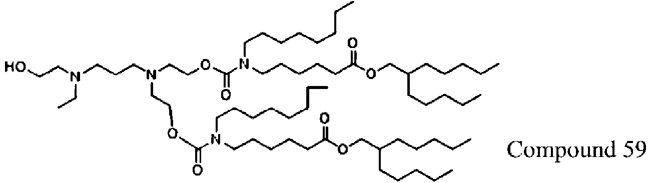

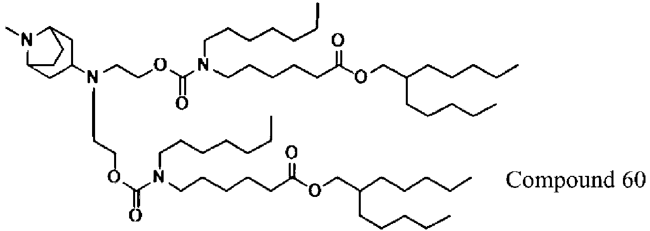

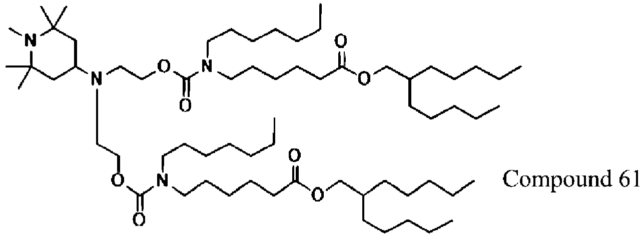

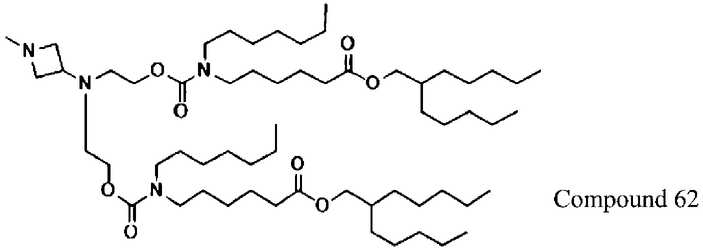

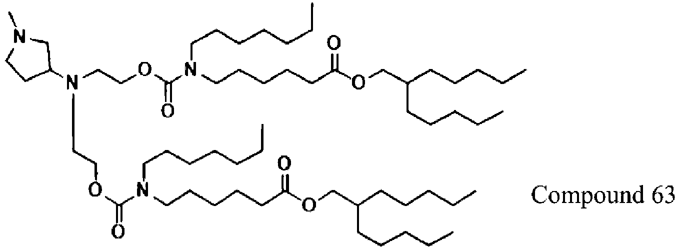

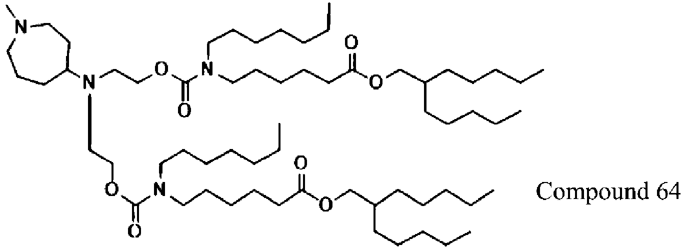

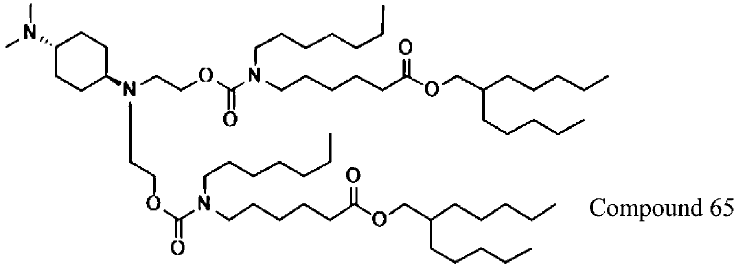

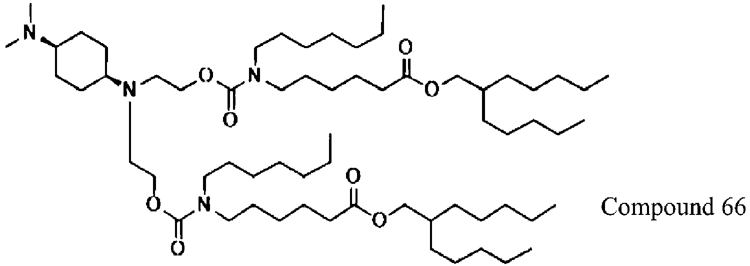

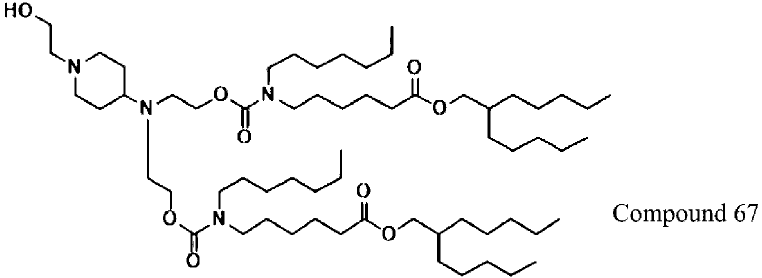

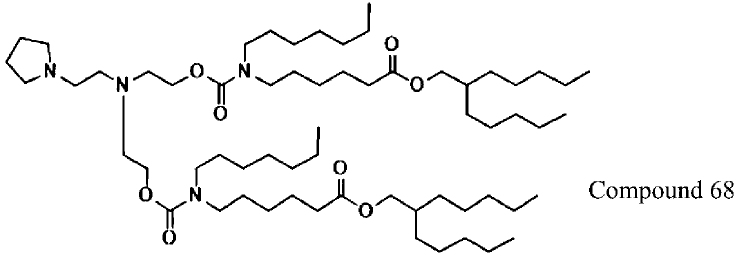

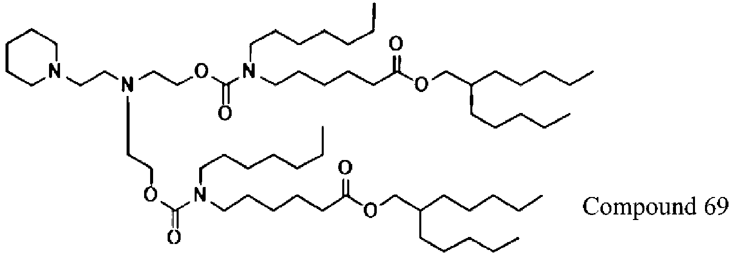

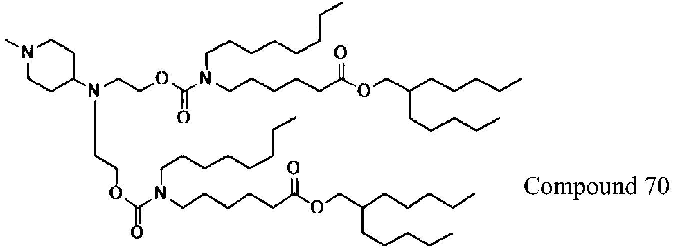

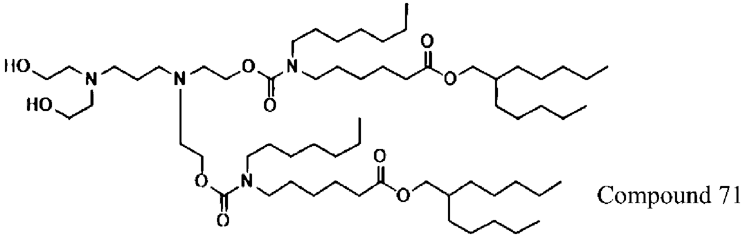

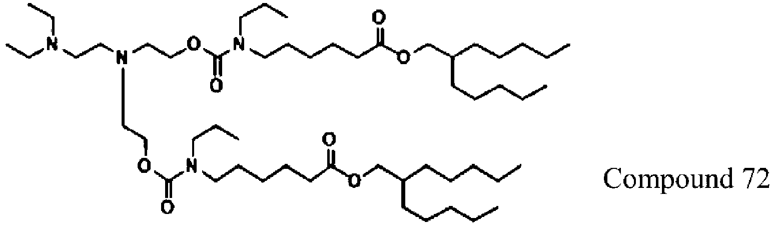

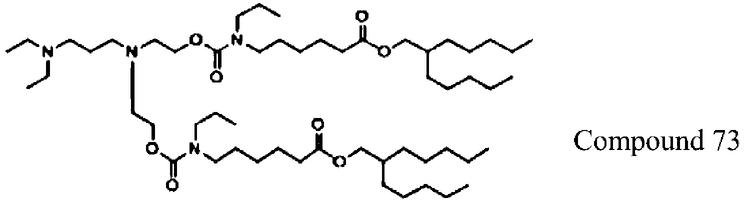

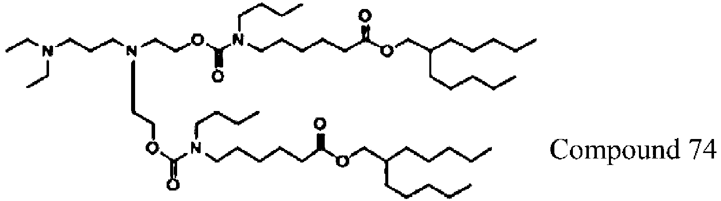

13. A method for delivering nucleic acid to an immune cell (excluding an in vivo delivery method), the method comprising:
bringing the nucleic acid delivery agent for an immune cell according to any one of claims 1 to 6 into contact with the immune cell.

14. The method according to claim 13,
wherein the immune cell is an activated cell or a non-activated cell.

15. The method according to claim 13, further comprising:
a step of adding, before bringing the nucleic acid delivery agent into contact with the immune cell, (i) an apolipoprotein and/or (ii) a protein including a cell-binding domain and a heparin-binding domain to the nucleic acid delivery agent or the immune cell.

16. A method for producing the nucleic acid delivery agent according to claim 1, the method comprising:
a step of preparing lipid particles not containing nucleic acid using an ionizable lipid that is a compound represented by Formula (1), a non-ionizable lipid, and a lipid having a nonionic polymer; and
a step of mixing the lipid particles not containing nucleic acid with the nucleic acid, 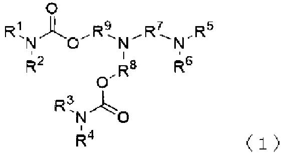
in the formula, R¹, R², R³, and R⁴ each independently represent a hydrogen atom or a hydrocarbon group having 1 to 24 carbon atoms which may be substituted,
substituents on the hydrocarbon groups having 1 to 24 carbon atoms which may be substituted, the hydrocarbon groups being represented by R¹, R², R³, and R⁴, each independently represent -C(O)O-R¹¹, -OC(O)-R¹², -O-R¹³, -CO-R¹⁴, -OC(O)O-R¹⁵, or -S-S-R¹⁶,
R¹¹, R¹², R¹³, R¹⁴, R¹⁵, and R¹⁶ each independently represent a hydrocarbon group having 1 to 24 carbon atoms which may be substituted with -S-R¹⁷, where R¹⁷ represents a hydrocarbon group having 1 to 12 carbon atoms,
R⁵ and R⁶ each independently represent a hydrocarbon group having 1 to 18 carbon atoms which may be substituted,
substituents on the hydrocarbon groups having 1 to 18 carbon atoms which may be substituted, the hydrocarbon groups being represented by R⁵ and R⁶, each independently represent -OH, -COOH, -NR²¹R²², -OC(O)O-R²³, -C(O)O-R²⁴, -OC(O)-R²⁵, -Q-R²⁶, -_{C}(O)_{NR}27_{R}28, -NR²⁹C(O)R³⁰, -N(R³¹)S(O)₂R³², -N(R³³)C(O)N(R³⁴)R³⁵, -N(R³⁶)C(S)N(R³⁷)R³⁸, -OC(O)N(R³⁹)R⁴⁰, or -N(R⁴¹)C(O)OR⁴²,
R²¹ and R²² each independently represent a hydrogen atom or a hydrocarbon group having 1 to 8 carbon atoms,
R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, and R⁴² each independently represent a hydrogen atom or a hydrocarbon group having 1 to 24 carbon atoms which may be substituted, and substituents on the hydrocarbon groups having 1 to 24 carbon atoms which may be substituted, the hydrocarbon group being represented by R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, and R⁴², represent an aryl group having 6 to 20 carbon atoms, a heterocyclic group, -OH, -COOH, or -NR⁵¹R⁵², where R⁵¹ and R⁵² each independently represent a hydrogen atom or a hydrocarbon group having 1 to 8 carbon atoms,
R⁷, R⁸, and R⁹ each independently represent a hydrocarbon group having 2 to 8 carbon atoms, and
R⁵ and R⁶, or R⁵ and R⁷ may be taken together to form a 4- to 7-membered ring.

17. At least one compound selected from the group consisting of the following compounds, or a salt thereof, 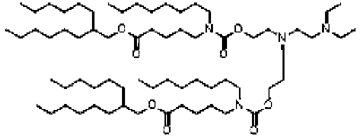
bis(2-hexyloctyl) 11-(2-(diethylamino)ethyl)-6,16-dioctyl-7,15-dioxo-8,14-dioxa-6,11,16-triazahenicosanedioat e, 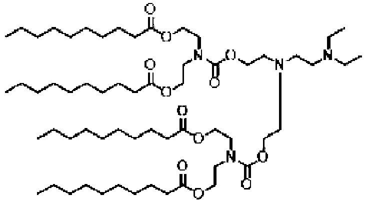
2-(2-(2-(bis(2-decanoyloxyethyl)carbamoyloxy)ethyl-(2-(diethylamino)ethyl)amino)e thoxycarbonyl-(2-decanoyloxyethyl)amino)ethyl decanoate, 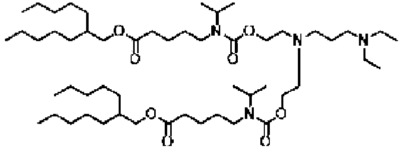
bis(2-pentylheptyl) 11-(3-(diethylamino)propyl)-6,16-diisopropyl-7,15-dioxo-8,14-dioxa-6,11,16-triazahenicosane dioate, 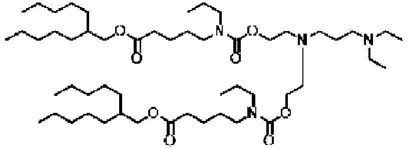
bis(2-pentylheptyl) 11-(3-(diethylamino)propyl)-7,15-dioxo-6,16-dipropyl-8,14-dioxa-6,11,16-triazahenicosanedi oate, 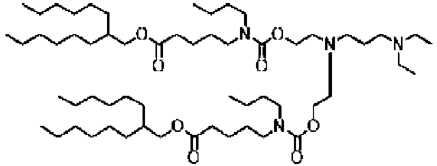
bis(2-hexyloctyl) 6,16-dibutyl-11-(3-(diethylamino)propyl)-7,15-dioxo-8,14-dioxa-6,11,16-triazahenicosanedioa te, 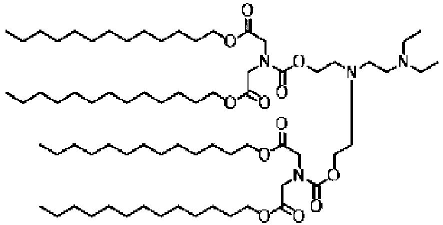
ditridecyl 8-(2-(diethylamino)ethyl)-4,12-dioxo-3,13-bis(2-oxo-2-(tridecyloxy)ethyl)-5,11-dioxa-3,8,13-t riazapentadecanedioate, 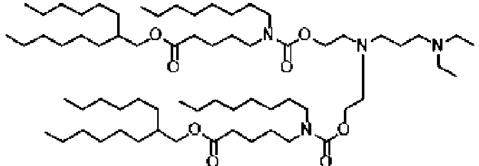
bis(2-hexyloctyl) 11-(3-(diethylamino)propyl)-6,16-dioctyl-7,15-dioxo-8,14-dioxa-6,11,16-triazahenicosanedioa te, 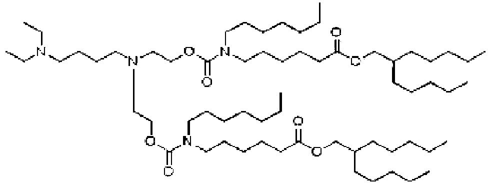
bis(2-pentylheptyl) 12-(4-(diethylamino)butyl)-7,17-diheptyl-8,16-dioxo-9,15-dioxa-7,12,17-triazatricosanedioate , 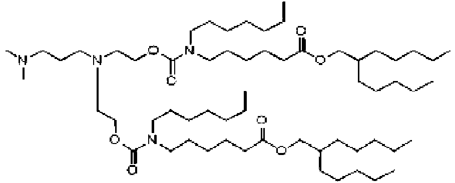
bis(2-pentylheptyl) 12-(3-(dimethylamino)propyl)-7,17-diheptyl-8,16-dioxo-9,15-dioxa-7,12,17-triazatricosanedio ate, 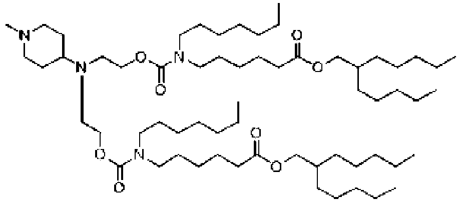
bis(2-pentylheptyl) 7,17-diheptyl-12-(1-methylpiperidin-4-yl)-8,16-dioxo-9,15-dioxa-7,12,17-triazatricosanedioat e, 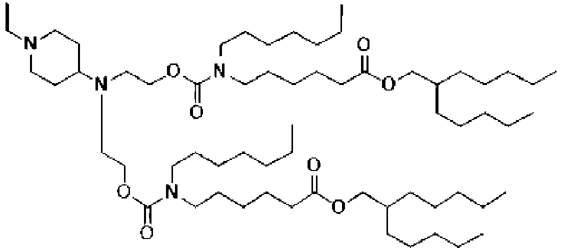
bis(2-pentylheptyl) 12-(1-ethylpiperidin-4-yl)-7,17-diheptyl-8,16-dioxo-9,15-dioxa-7,12,17-triazatricosanedioate, 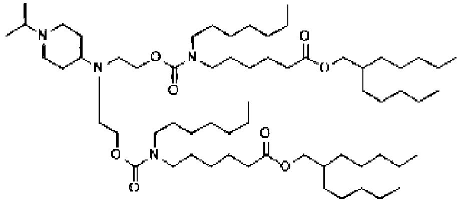
bis(2-pentylheptyl) 7,17-diheptyl-12-(1-isopropylpiperidin-4-yl)-8,16-dioxo-9,15-dioxa-7,12,17-triazatricosanedio ate, 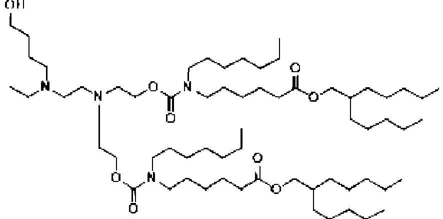
bis(2-pentylheptyl) 12-(2-(ethyl(4-hydroxybutyl)amino)ethyl)-7,17-diheptyl-8,16-dioxo-9,15-dioxa-7,12,17-triaza tricosanedioate, 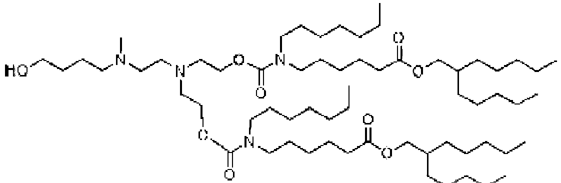
bis(2-pentylheptyl) 7,17-diheptyl-12-(2-((4-hydroxybutyl)(methyl)amino)ethyl)-8,16-dioxo-9,15-dioxa-7,12,17-tri azatricosanedioate, 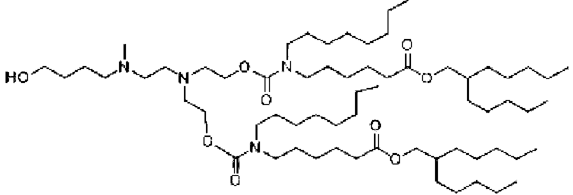
bis(2-pentylheptyl) 12-(2-((4-hydroxybutyl)(methyl)amino)ethyl)-7,17-dioctyl-8,16-dioxo-9,15-dioxa-7,12,17-tria zatricosanedioate, 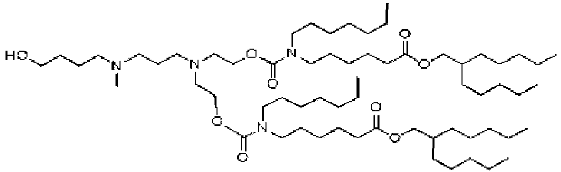
bis(2-pentylheptyl) 7,17-diheptyl-12-(3-((4-hydroxybutyl)(methyl)amino)propyl)-8,16-dioxo-9,15-dioxa-7,12,17-t riazatricosanedioate, 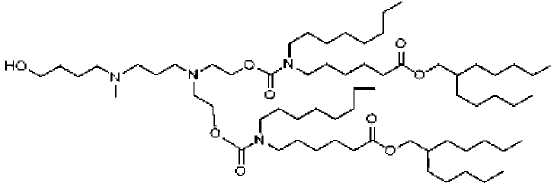
bis(2-pentylheptyl) 12-(3-((4-hydroxybutyl)(methyl)amino)propyl)-7,17-dioctyl-8,16-dioxo-9,15-dioxa-7,12,17-tri azatricosanedioate, 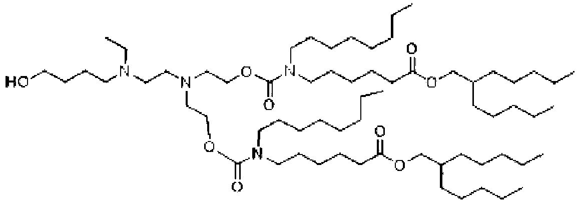
bis(2-pentylheptyl) 12-(2-(ethyl(4-hydroxybutyl)amino)ethyl)-7,17-dioctyl-8,16-dioxo-9,15-dioxa-7,12,17-triazatr icosanedioate, 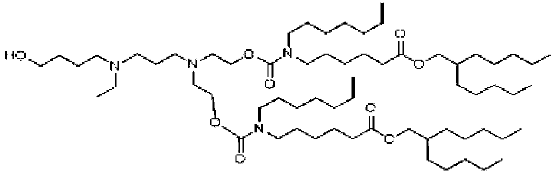
bis(2-pentylheptyl) 12-(3-(ethyl(4-hydroxybutyl)amino)propyl)-7,17-diheptyl-8,16-dioxo-9,15-dioxa-7,12,17-triaz atricosanedioate, 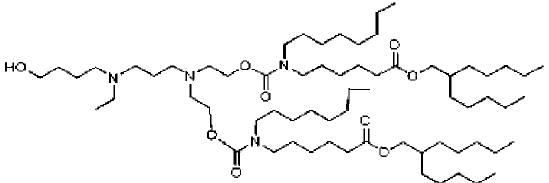
bis(2-pentylheptyl) 12-(3-(ethyl(4-hydroxybutyl)amino)propyl)-7,17-dioctyl-8,16-dioxo-9,15-dioxa-7,12,17-triaza tricosanedioate, 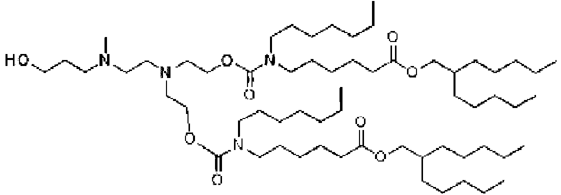
bis(2-pentylheptyl) 7,17-diheptyl-12-(2-((3-hydroxypropyl)(methyl)amino)ethyl)-8,16-dioxo-9,15-dioxa-7,12,17-t riazatricosanedioate, 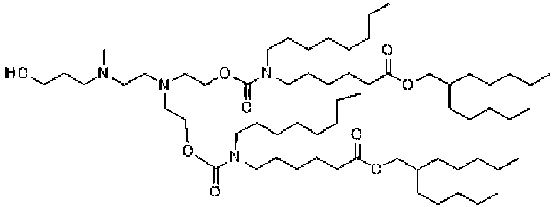
bis(2-pentylheptyl) 12-(2-((3-hydroxypropyl)(methyl)amino)ethyl)-7,17-dioctyl-8,16-dioxo-9,15-dioxa-7,12,17-tri azatricosanedioate, 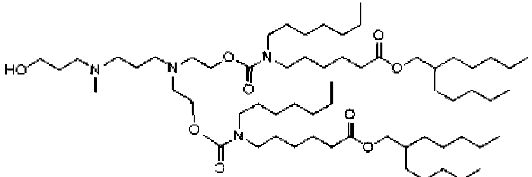
bis(2-pentylheptyl) 7,17-diheptyl-12-(3-((3-hydroxypropyl)(methyl)amino)propyl)-8,16-dioxo-9,15-dioxa-7,12,17 -triazatricosanedioate, 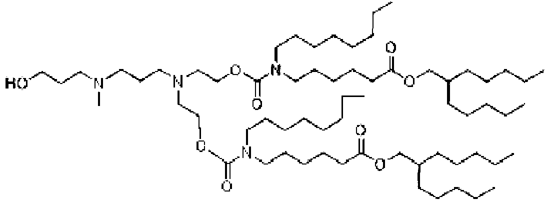
bis(2-pentylheptyl) 12-(3-((3-hydroxypropyl)(methyl)amino)propyl)-7,17-dioctyl-8,16-dioxo-9,15-dioxa-7,12,17-t riazatricosanedioate, 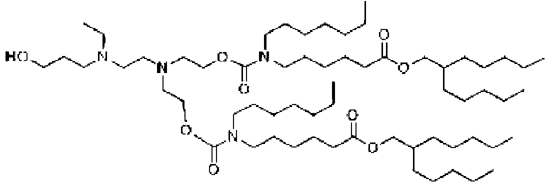
bis(2-pentylheptyl) 12-(2-(ethyl(3-hydroxypropyl)amino)ethyl)-7,17-diheptyl-8,16-dioxo-9,15-dioxa-7,12,17-triaz atricosanedioate, 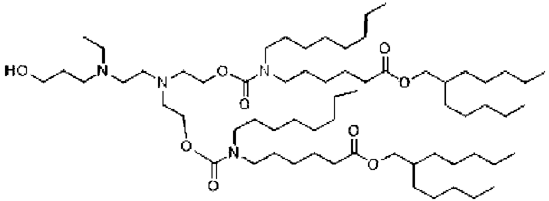
bis(2-pentylheptyl) 12-(2-(ethyl(3-hydroxypropyl)amino)ethyl)-7,17-dioctyl-8,16-dioxo-9,15-dioxa-7,12,17-triaza tricosanedioate, 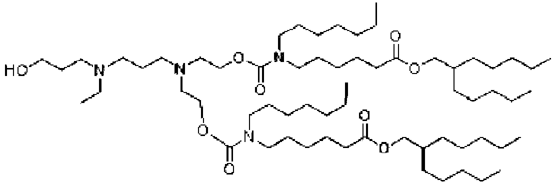
bis(2-pentylheptyl) 12-(3-(ethyl(3-hydroxypropyl)amino)propyl)-7,17-diheptyl-8,16-dioxo-9,15-dioxa-7,12,17-tri azatricosanedioate, 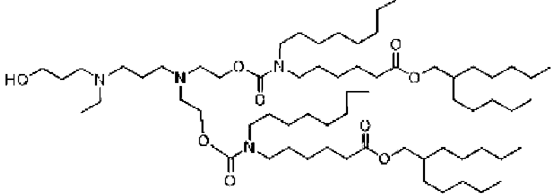
bis(2-pentylheptyl) 12-(3-(ethyl(3-hydroxypropyl)amino)propyl)-7,17-dioctyl-8,16-dioxo-9,15-dioxa-7,12,17-triaz atricosanedioate, 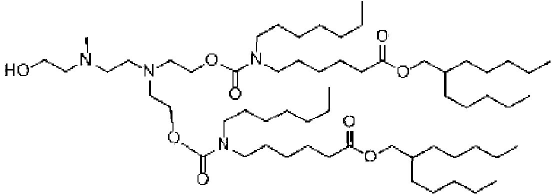
bis(2-pentylheptyl) 7,17-diheptyl-12-(2-((2-hydroxyethyl)(methyl)amino)ethyl)-8,16-dioxo-9,15-dioxa-7,12,17-tri azatricosanedioate, 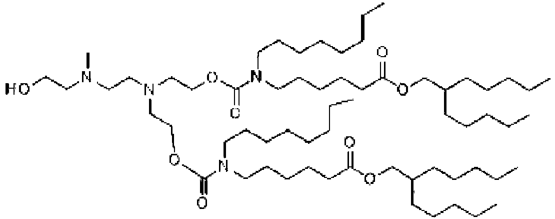
bis(2-pentylheptyl) 12-(2-((2-hydroxyethyl)(methyl)amino)ethyl)-7,17-dioctyl-8,16-dioxo-9,15-dioxa-7,12,17-tria zatricosanedioate, 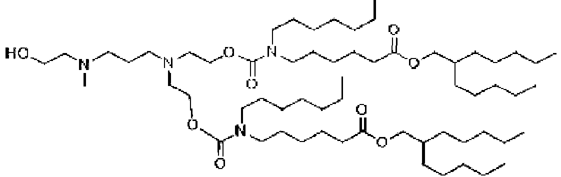
bis(2-pentylheptyl) 7,17-diheptyl-12-(3-((2-hydroxyethyl)(methyl)amino)propyl)-8,16-dioxo-9,15-dioxa-7,12,17-t riazatricosanedioate, 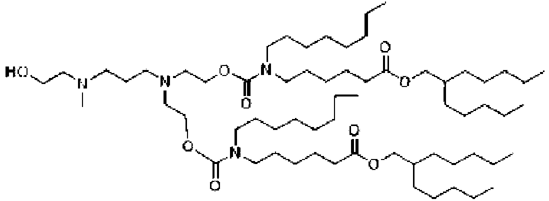
bis(2-pentylheptyl) 12-(3-((2-hydroxyethyl)(methyl)amino)propyl)-7,17-dioctyl-8,16-dioxo-9,15-dioxa-7,12,17-tri azatricosanedioate, 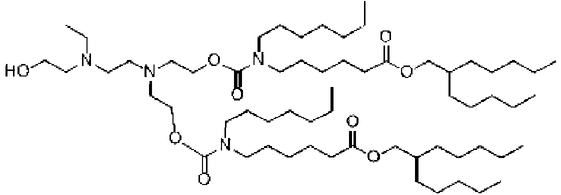
bis(2-pentylheptyl) 12-(2-(ethyl(2-hydroxyethyl)amino)ethyl)-7,17-diheptyl-8,16-dioxo-9,15-dioxa-7,12,17-triazat ricosanedioate, 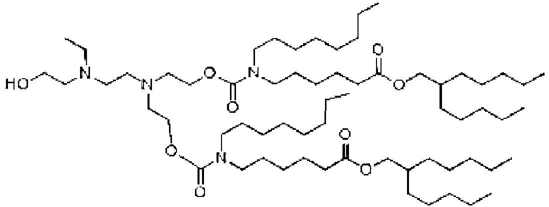
bis(2-pentylheptyl) 12-(2-(ethyl(2-hydroxyethyl)amino)ethyl)-7,17-dioctyl-8,16-dioxo-9,15-dioxa-7,12,17-triazatr icosanedioate, 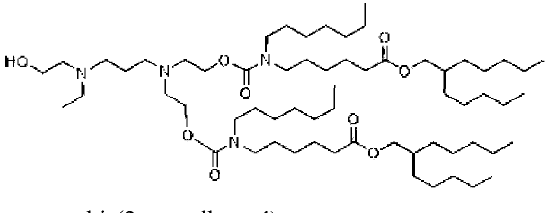
bis(2-pentylheptyl) 12-(3-(ethyl(2-hydroxyethyl)amino)propyl)-7,17-diheptyl-8,16-dioxo-9,15-dioxa-7,12,17-triaz atricosanedioate, 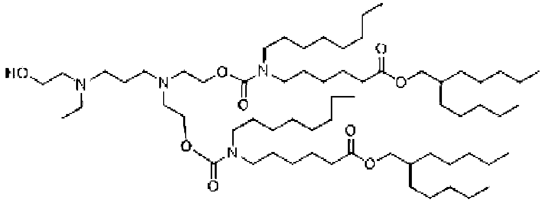
bis(2-pentylheptyl) 12-(3-(ethyl(2-hydroxyethyl)amino)propyl)-7,17-dioctyl-8,16-dioxo-9,15-dioxa-7,12,17-triaza tricosanedioate, 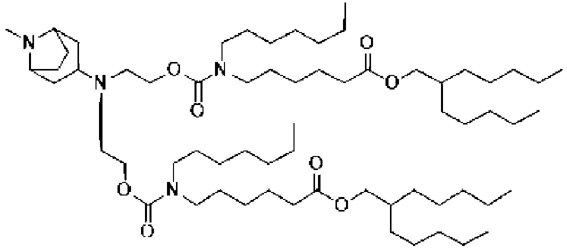
bis(2-pentylheptyl) 7,17-diheptyl-12-(8-methyl-8-azabicyclo[3.2.1]octan-3-yl)-8,16-dioxo-9,15-dioxa-7,12,17-tria zatricosanedioate, 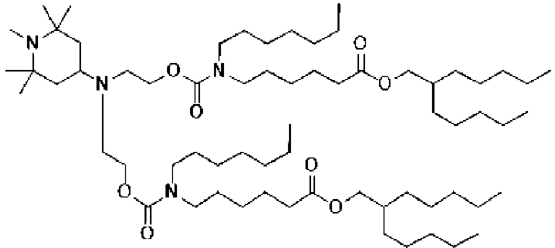
bis(2-pentylheptyl) 7,17-diheptyl-8,16-dioxo-12-(1,2,2,6,6-pentamethylpiperidin-4-yl)-9,15-dioxa-7,12,17-triazatr icosanedioate, 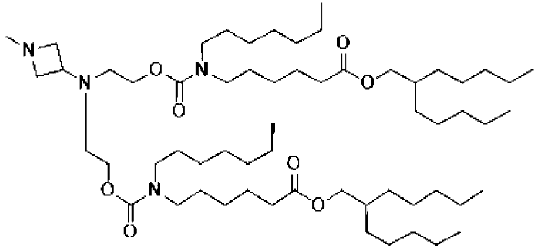
bis(2-pentylheptyl) 7,17-diheptyl-12-(1-methylazetidin-3-yl)-8,16-dioxo-9,15-dioxa-7,12,17-triazatricosanedioate, 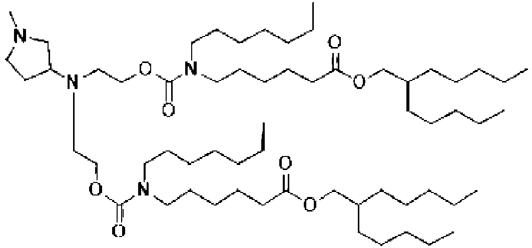
bis(2-pentylheptyl) 7,17-diheptyl-12-(1-methylpyrrolidin-3-yl)-8,16-dioxo-9,15-dioxa-7,12,17-triazatricosanedioa te, 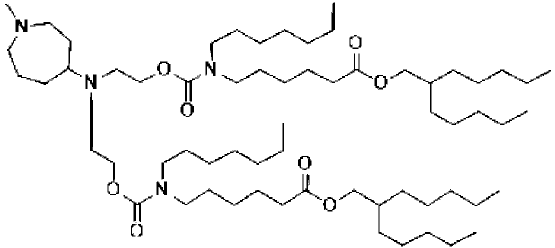
bis(2-pentylheptyl) 7,17-diheptyl-12-(1-methylazepan-4-yl)-8,16-dioxo-9,15-dioxa-7,12,17-triazatricosanedioate, 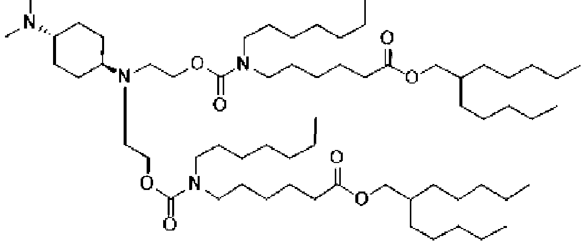
bis(2-pentylheptyl) 12-((1r,4r)-4-(dimethylamino)cyclohexyl)-7,17-diheptyl-8,16-dioxo-9,15-dioxa-7,12,17-triaza tricosanedioate, 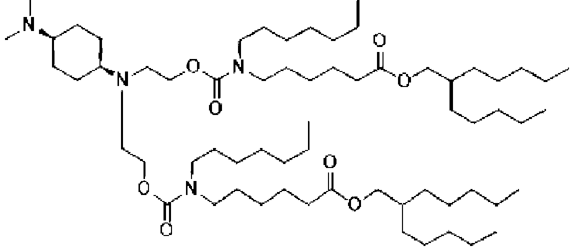
bis(2-pentylheptyl) 12-((1s,4s)-4-(dimethylamino)cyclohexyl)-7,17-diheptyl-8,16-dioxo-9,15-dioxa-7,12,17-triaza tricosanedioate, 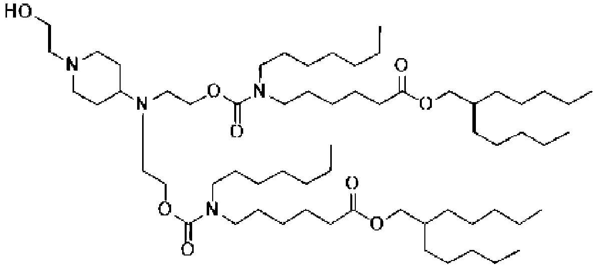
bis(2-pentylheptyl) 7,17-diheptyl-12-(1-(2-hydroxyethyl)piperidin-4-yl)-8,16-dioxo-9,15-dioxa-7,12,17-triazatrico sanedioate, 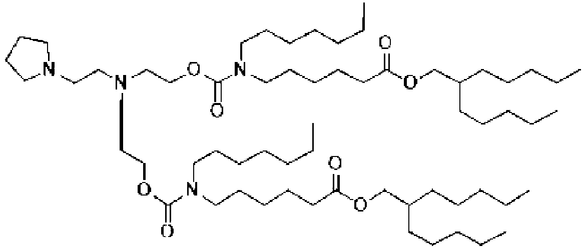
bis(2-pentylheptyl) 7,17-diheptyl-8,16-dioxo-12-(2-(pyrrolidin-1-yl)ethyl)-9,15-dioxa-7,12,17-triazatricosanedioat e, 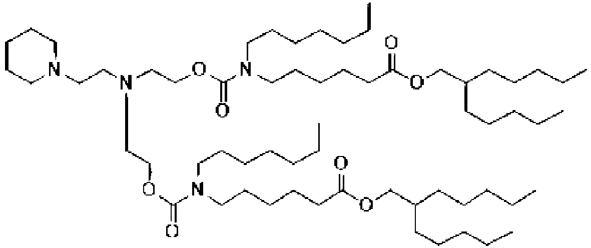
bis(2-pentylheptyl) 7,17-diheptyl-8,16-dioxo-12-(2-(piperidin-1-yl)ethyl)-9,15-dioxa-7,12,17-triazatricosanedioate , 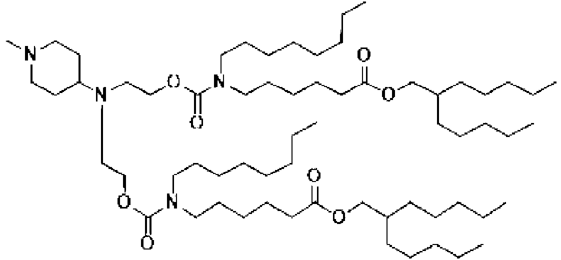
bis(2-pentylheptyl) 12-(1-methylpiperidin-4-yl)-7,17-dioctyl-8,16-dioxo-9,15-dioxa-7,12,17-triazatricosanedioate, 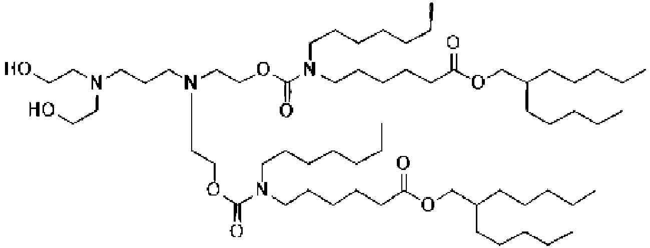
bis(2-pentylheptyl) 12-(3-(bis(2-hydroxyethyl)amino)propyl)-7,17-diheptyl-8,16-dioxo-9,15-dioxa-7,12,17-triazat ricosanedioate, 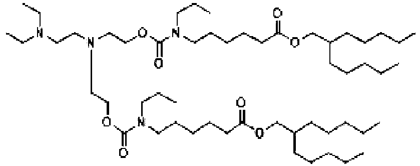
bis(2-pentylheptyl) 12-(2-(diethylamino)ethyl)-8,16-dioxo-7,17-dipropyl-9,15-dioxa-7,12,17-triazatricosanedioate , 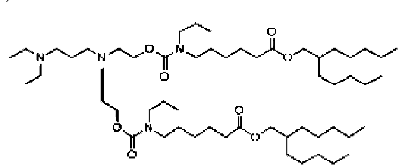
bis(2-pentylheptyl) 12-(3-(diethylamino)propyl)-8,16-dioxo-7,17-dipropyl-9,15-dioxa-7,12,17-triazatricosanedioat e, and 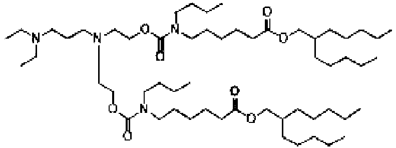
bis(2-pentylheptyl) 7,17-dibutyl-12-(3-(diethylamino)propyl)-8,16-dioxo-9,15-dioxa-7,12,17-triazatricosanedioate
